# EUROPEAN PATENT SPECIFICATION

(11) **EP 3 273 976 B1**
(45) Date of publication and mention of the grant of the patent: **26.07.2023**
(21) Application number: 16773942.4
(22) Date of filing: 28.03.2016
(51) Int. Cl.: C12N 5/0783, C12N 5/10, A61K 35/12, A61K 35/17, C12N 15/85

(54) **MODIFIED T CELLS AND METHODS OF MAKING AND USING THE SAME**
MODIFIZIERTE T-ZELLEN UND VERFAHREN ZUR HERSTELLUNG UND VERWENDUNG DAVON
CELLULES T MODIFIÉES, LEURS PROCÉDÉS DE PRÉPARATION ET UTILISATION

(30) Priority: 27.03.2015 US 201562139479 P
(43) Date of publication of application: 31.01.2018
(73) Proprietor: President and Fellows of Harvard College, Cambridge, MA 02138 (US)
(72) Inventor: MEISSNER, Torsten, B., Somerville, MA 02143 (US); MULUMBA, Kabungo, Y., Cambridge, MD 02138 (US); FERREIRA, Leonardo, M., R., Cambridge, MA 02138 (US); COWAN, Chad A., Boston, MA 02114 (US)
(74) Representative: Lock, Graham James
(86) International application number: PCT/US2016/024554
(87) International publication number: WO 2016/160721

(56) References cited:
- WO-A2-2014/153470
- US-A1- 2007 283 452
- US-A1- 2014 030 240
- US-A1- 2014 065 667
- MANDAL P K ET AL: "Efficient Ablation of Genes in Human Hematopoietic Stem and Effector Cells using CRISPR/Cas9", CELL STEM CELL, vol. 15, no. 5, November 2014 (2014-11), pages 643-652, XP55202004, ISSN: 1934-5909, DOI: 10.1016/j.stem.2014.10.004
- MEISSNER T B ET AL: "Genome editing for human gene therapy", METHODS IN ENZYMOLOGY, vol. 546, 2014, pages 273-295, XP008179752, ISSN: 1557-7988 ISBN: 978-0-12-801185-0 [retrieved on 2014-11-12]
- UREÑA-BAILÉN G ET AL: "CRISPR/Cas9 technology: towards a new generation of improved CAR-T cells for anticancer therapies.", BRIEFINGS IN FUNCTIONAL GENOMICS, vol. 19, no. 3, 20 May 2020 (2020-05-20), pages 191-200, ISSN: 2041-2657
- REN J ET AL: "A versatile system for rapid multiplex genome-edited CAR T cell generation.", ONCOTARGET, vol. 8, no. 10, 7 March 2017 (2017-03-07), pages 17002-17011, ISSN: 1949-2553

## Description

### BACKGROUND OF THE INVENTION

T cell therapy employing chimeric antigen receptors (CAR) represents a major breakthrough in cancer immunotherapy (e.g., adoptive immunotherapy) and as a new form of treatment for viral and fungal infections, among others. Several obstacles currently prevent the safe translation of CAR T therapies, such as the risk of autoreactivity of the endogenous T cell receptor (TCR), T cell inhibition by cancerous or infected cells, and the reliance on autologous T cell transplants.

### SUMMARY OF THE INVENTION

There is a need for modified T cells and methods of making and using such T cells that overcome autoreactivity of the endogenous TCR, T cell inhibition, as well empower the use of allogeneic T cells for CAR T therapies. The present invention is directed toward further solutions to address this need, in addition to having other desirable characteristics. The present invention utilizes genomic editing, such as a CRISPR and/or TALEN system, to remove the above obstacles. In a CRISPR/Cas system the Cas protein may be, for example, Cas9 or Cpfl. For example, to overcome autoreactivity work detailed herein designed and tested CRISPR/Cas guide RNAs (gRNAs) targeting the TCRa and TCRb chains and demonstrated high on-target activity and loss of TCR surface expression in Jurkat T cells and in primary human T cells. Due to the multiplexing capabilities of genomic editing, e.g., using the CRISPR/Cas9 or CRISPR/Cpf1 system, the TCRa and/or TCRb can also be targeted in combination with other molecules (e.g., B2M, CTLA4, PD-1) to overcome T cell inhibition and empower CAR T therapies. The compositions and methods of the present invention are suited for clinical translation and improve on existing and emerging T cell-based therapies (e.g., adoptive immunotherapy).

In some embodiments, the present inventions are directed to modified primary human T cells comprising a modified genome, the cells comprising a first genomic modification in which the cytotoxic T-lymphocyte-associated protein 4 (CTLA4) gene on chromosome 2 has been cleaved edited, e.g., to delete a first contiguous stretch of genomic DNA, thereby reducing or eliminating CTLA4 receptor surface expression and/or activity in the cell; a second genomic modification in which the programmed cell death 1 (PD1) gene on chromosome 2 has been cleaved or edited, e.g., to delete a second contiguous stretch of genomic DNA, thereby reducing or eliminating PD1 receptor surface expression and/or activity in the cell; (i) a third genomic modification in which the gene encoding the T cell receptor (TCR) alpha chain locus on chromosome 14 has been cleaved or edited, e.g., to delete a third contiguous stretch of genomic DNA, and/or (ii) a fourth genomic modification in which the gene encoding the TCR beta chain locus on chromosome 7 has been cleaved or edited, e.g., to delete a fourth contiguous stretch of genomic DNA, thereby reducing or eliminating TCR surface expression and/or activity in the cell; and a fifth has been cleaved or edited, e.g., to delete a fifth contiguous stretch of genomic DNA, thereby reducing or eliminating B2M expression or activity and/or MHC Class I molecule surface expression and/or activity in the cell; and each cell optionally comprising: (i) at least one chimeric antigen receptor that specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof, or an exogenous nucleic acid encoding the at least one chimeric antigen receptor, and/or (ii) at least one exogenous protein that modulates a biological effect of interest in an adjacent cell, tissue, or organ, or an exogenous nucleic acid encoding the protein.

In some embodiments, the present inventions are directed to modified primary human T cells comprising a modified genome, the cells comprising a first genomic modification in which the cytotoxic T-lymphocyte-associated protein 4 (CTLA4) gene on chromosome 2 has been edited to delete a first contiguous stretch of genomic DNA comprising an intron flanked by at least a portion of an adjacent upstream exon and at least a portion of an adjacent downstream exon, and the 3' end of the genomic DNA upstream with respect to the 5' end of the deleted first contiguous stretch of genomic DNA is covalently joined to the 5' end of the genomic DNA downstream with respect to the 3' end of the deleted first contiguous stretch of genomic DNA to result in a modified CTLA4 gene on chromosome 2 that lacks the first contiguous stretch of genomic DNA, thereby reducing or eliminating CTLA4 receptor surface expression and/or activity in the cell; and/or a second genomic modification in which the programmed cell death 1 (PD1) gene on chromosome 2 has been edited to delete a second contiguous stretch of genomic DNA comprising an intron flanked by at least a portion of an adjacent upstream exon and at least a portion of an adjacent downstream exon, and the 3' end of the genomic DNA upstream with respect to the deleted second contiguous stretch of genomic DNA is covalently joined to the 5' end of the genomic DNA downstream with respect to the 3' end of the deleted second contiguous stretch of genomic DNA to result in a modified PD1 gene on chromosome 2 that lacks the second contiguous stretch of genomic DNA, thereby reducing or eliminating PD1 receptor surface expression and/or activity in the cell.

In some embodiments, the present inventions are directed to modified primary human T cells comprising a modified genome, the cells comprising a first genomic modification in which the cytotoxic T-lymphocyte-associated protein 4 (CTLA4) gene on chromosome 2 has been edited to delete a first contiguous stretch of genomic DNA, thereby reducing or eliminating CTLA4 receptor surface expression and/or activity in the cell, wherein the first contiguous stretch of genomic DNA has been deleted by contacting the cell with a Cas protein or a nucleic acid encoding the Cas protein and a first pair of ribonucleic acids having sequences selected from the group consisting of SEQ ID NOs: 1-195 and 797-3637; and/or a second genomic modification in which the programmed cell death 1 (PD1) gene on chromosome 2 has been edited to delete a second contiguous stretch of genomic DNA, thereby reducing or eliminating PD1 receptor surface expression and/or activity in the cell, wherein the second contiguous stretch of genomic DNA has been deleted by contacting the cell with the Cas protein or the nucleic acid encoding the Cas protein and a second pair of ribonucleic acids having sequences selected from the group consisting of SEQ ID NOs: 196-531 and 4047-8945.

In some embodiments, the first pair of ribonucleic acids comprises SEQ ID NO: 128 and SEQ ID NO: 72, and wherein the second pair of ribonucleic acids comprises SEQ ID NO: 462 and SEQ ID NO: 421.

In certain embodiments, the modified primary human T cells further comprise (i) a third genomic modification in which the gene encoding the T cell receptor (TCR) alpha chain locus on chromosome 14 has been edited to delete a third contiguous stretch of genomic DNA comprising at least a portion of a coding exon, and/or (ii) a fourth genomic modification in which the gene encoding the TCR beta chain locus on chromosome 7 has been edited to delete a fourth contiguous stretch of genomic DNA comprising at least a portion of a coding exon, thereby reducing or eliminating TCR surface expression and/or activity in the cell.

In some embodiments, the third contiguous stretch of genomic DNA has been deleted by contacting the cell with the Cas protein or the nucleic acid encoding the Cas protein and a third pair of ribonucleic acids having sequences selected from the group consisting of SEQ ID NOs: 532-609 and 9102-9545, and/or wherein the fourth contiguous stretch of genomic DNA has been deleted by contacting the cell with the Cas protein or the nucleic acid encoding the Cas protein and a fourth pair of ribonucleic acids having sequences selected from the group consisting of SEQ ID NOs: 610-765 and 9798-10532. In certain aspects, the third pair of ribonucleic acids comprises SEQ ID NO: 550 and SEQ ID NO: 573, and/or wherein the fourth pair of ribonucleic acids comprises SEQ ID NO: 657 and SEQ ID NO: 662.

In certain embodiments, the modified primary human T cells further comprise a fifth genomic modification in which the β2-microglobulin (B2M) gene on chromosome 15 has been edited to delete a fifth contiguous stretch of genomic DNA, thereby reducing or eliminating MHC Class I molecule surface expression and/or activity in the cell. In certain aspects, the fifth contiguous stretch of genomic DNA has been deleted by contacting the cell with the Cas protein or the nucleic acid encoding the Cas protein and a fifth pair of ribonucleic acids having sequences selected from the group comprises SEQ ID NOs: 766-780 and 10574-13257. In some embodiments, the fifth pair of ribonucleic acids comprises SEQ ID NO: 773 and SEQ ID NO: 778.

In some embodiments, the modified primary human T cells further comprise a chimeric antigen receptor or an exogenous nucleic acid encoding the chimeric antigen receptor. For example, the chimeric antigen receptor may specifically bind to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof.

In certain embodiments, the modified primary human T cells further comprise at least one exogenous protein that modulates a biological effect of interest in an adjacent cell, tissue, or organ, or an exogenous nucleic acid encoding the protein. In some aspects, the T cell is selected from the group consisting of cytotoxic T-cells, helper T-cells, memory T-cells, regulatory T-cells, tissue infiltrating lymphocytes, and combinations thereof. In certain aspects, the cell is obtained from a subject suffering from, being treated for, diagnosed with, at risk of developing, or suspected of having, a disorder selected from the group consisting of an autoimmune disorder, cancer, a chronic infectious disease, and graft versus host disease (GVHD).

Also disclosed are methods for producing a modified primary human T cell, the method comprising (a) editing the cytotoxic T-lymphocyte-associated protein 4 (CTLA4) gene on chromosome 2 in a primary human T cell to delete a first contiguous stretch of genomic DNA, thereby reducing or eliminating CTLA4 receptor surface expression and/or activity in the cell; (b) editing the programmed cell death 1 (PD1) gene on chromosome 2 in the cell to delete a second contiguous stretch of genomic DNA, thereby reducing or eliminating PD1 receptor surface expression and/or activity in the cell; (c)(i) editing the gene encoding the T cell receptor (TCR) alpha chain locus on chromosome 14 in the cell to delete a third contiguous stretch of genomic DNA, and/or (c)(ii) editing the gene encoding the TCR beta chain locus on chromosome 7 in the cell to delete a fourth contiguous stretch of genomic DNA, thereby reducing or eliminating TCR surface expression and/or activity in the cell; and (d) editing the β2-microglobulin (B2M) gene on chromosome 15 in the cell to delete a fifth contiguous stretch of genomic DNA, thereby reducing or eliminating MHC Class I molecule surface expression and/or activity in the cell; and optionally (e) (i) causing the cell to express at least one chimeric antigen receptor that specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof, and/or (e)(ii) causing the cell to express at least one protein that modulates a biological effect of interest in an adjacent cell, tissue, or organ, wherein the editing in (a)-(d) comprises contacting the cell with a Cas protein or a nucleic acid encoding the Cas protein, and at least one first pair of guide RNA sequences to delete the first contiguous stretch of genomic DNA from the gene in (a), at least one second pair of guide RNA sequences to delete the second contiguous stretch of genomic DNA from the gene in (b), at least one third pair of guide RNA sequences to delete the third contiguous stretch of genomic DNA from the gene in (c)(i), and/or at least one fourth pair of guide RNA sequences to delete the fourth contiguous stretch of genomic DNA from the gene in (c)(ii), and at least one fifth pair of guide RNA sequences to delete the fifth contiguous stretch of genomic DNA from the gene in (d).

Also disclosed herein are methods for producing a modified primary human T cell, the method comprising (a) editing the cytotoxic T-lymphocyte-associated protein 4 (CTLA4) gene on chromosome 2 in a primary human T cell to delete a first contiguous stretch of genomic DNA comprising an intron flanked by at least a portion of an adjacent upstream exon and at least a portion of an adjacent downstream exon, and the 3' end of the genomic DNA upstream with respect to the 5' end of the deleted first contiguous stretch of genomic DNA is covalently joined to the 5' end of the genomic DNA downstream with respect to the 3' end of the deleted first contiguous stretch of genomic DNA to result in a modified CTLA4 gene on chromosome 2 that lacks the first contiguous stretch of genomic DNA, thereby reducing or eliminating CTLA4 receptor surface expression and/or activity in the cell; and/or (b) editing the programmed cell death 1 (PD1) gene on chromosome 2 in a primary human T cell to delete a second contiguous stretch of genomic DNA comprising an intron flanked by at least a portion of an adjacent upstream exon and at least a portion of an adjacent downstream exon, and the 3' end of the genomic DNA upstream with respect to the deleted second contiguous stretch of genomic DNA is covalently joined to the 5' end of the genomic DNA downstream with respect to the 3' end of the deleted second contiguous stretch of genomic DNA to result in a modified PD1 gene on chromosome 2 that lacks the second contiguous stretch of genomic DNA, thereby reducing or eliminating PD1 receptor surface expression and/or activity in the cell.

Also disclosed herein are methods for producing a modified primary human T cell, the method comprising (a) contacting a primary human T cell with a Cas protein or a nucleic acid encoding the Cas protein and a first pair of ribonucleic acids having sequences selected from the group consisting of SEQ ID NOs: 1-195 and 797-3637, thereby editing the cytotoxic T-lymphocyte-associated protein 4 (CTLA4) gene on chromosome 2 to delete a first contiguous stretch of genomic DNA, and reduce or eliminate CTLA4 receptor surface expression and/or activity in the cell; and/or (b) contacting a primary human T cell with the Cas protein or the nucleic acid encoding the Cas protein and a second pair of ribonucleic acids having sequences selected from the group consisting of SEQ ID NOs: 196-531 and 4047-8945, thereby editing the programmed cell death 1 (PD1) gene on chromosome 2 to delete a second contiguous stretch of genomic DNA, and reduce or eliminate PD1 receptor surface expression and/or activity in the cell.

In some aspects, the methods for producing a modified primary human T cell further comprise (c)(i) editing the gene encoding the T cell receptor (TCR) alpha chain locus on chromosome 14 in the cell to delete a third contiguous stretch of genomic DNA comprising at least a portion of a coding exon, and/or (c)(ii) editing the gene encoding the TCR beta chain locus on chromosome 7 in the cell to delete a fourth contiguous stretch of genomic DNA comprising at least a portion of a coding exon, thereby reducing or eliminating TCR surface expression and/or activity in the cell. In certain aspects, the editing in (c)(i) comprises contacting the cell with the Cas protein or the nucleic acid encoding the Cas protein and a third pair of ribonucleic acids having sequences selected from the group consisting of SEQ ID NOs: 532-609 and 9102-9750, and/or wherein the editing in (c)(ii) comprises contacting the cell with the Cas protein or the nucleic acid encoding the Cas protein and a fourth pair of ribonucleic acids having sequences selected from the group consisting of SEQ ID NOs: 610-765 and 9798-10532.

In some aspects, the methods for producing a modified primary human T cell further comprise (d) editing the β2-microglobulin (B2M) gene on chromosome 15 in the cell to delete a fifth contiguous stretch of genomic DNA, thereby reducing or eliminating MHC Class I molecule surface expression and/or activity in the cell. In certain aspects, the editing in (d) comprises contacting the cell with the Cas protein or the nucleic acid encoding the Cas protein and a fifth pair of ribonucleic acids having sequences selected from the group consisting of SEQ ID NOs: 766-780 and 10574-13257.

In certain aspects of the inventions disclosed herein, the first pair of ribonucleic acids comprises SEQ ID NO: 128 and SEQ ID NO: 72, and wherein the second pair of ribonucleic acids comprises SEQ ID NO: 462 and SEQ ID NO: 421. In certain aspects of the inventions disclosed herein, the third pair of ribonucleic acids comprises SEQ ID NO: 550 and SEQ ID NO: 573, and/or wherein the fourth pair of ribonucleic acids comprises SEQ ID NO: 657 and SEQ ID NO: 662. In certain aspects of the inventions disclosed herein, the fifth pair of ribonucleic acids comprises SEQ ID NO: 773 and SEQ ID NO: 778.

In some embodiments, the methods for producing a modified primary human T cell further comprise causing the cell to express at least one chimeric antigen receptor that specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof.

In some embodiments, the methods for producing a modified primary human T cell further comprise causing the cell to express at least one protein that modulates a biological effect of interest in an adjacent cell, tissue, or organ when the cell is in proximity to the adjacent cell, tissue, or organ.

In certain aspects of the inventions disclosed herein, T cell is selected from the group consisting of cytotoxic T-cells, helper T-cells, memory T-cells, regulatory T-cells, tissue infiltrating lymphocytes, and combinations thereof. In certain aspects of the inventions disclosed herein, the cell is obtained from a subject suffering from, being treated for, diagnosed with, at risk of developing, or suspected of having, a disorder selected from the group consisting of an autoimmune disorder, cancer, a chronic infectious disease, and graft versus host disease (GVHD).

Also disclosed herein are methods of treating a patient in need thereof, the methods comprising (a)(i) administering a modified T cell according to any one of claims 1 to 15 to a patient in need of such cells; (a)(ii) administering a modified T cell produced according to the method of any one of claims 16 to 29 to a patient in need of such cells; or (a)(iii) administering a composition according to claim 30 to a patient in need of such cells. For example, the treatment may comprise adoptive immunotherapy.

In some embodiments, the method for treating a patient further comprises expanding the modified T cell prior to the step of administering. In some aspects, the patient is suffering from, being treated for, diagnosed with, at risk of developing, or suspected of having, a disorder selected from the group consisting of an autoimmune disorder, cancer, a chronic infectious disease, and graft versus host disease (GVHD).

Also disclosed herein are compositions comprising a chimeric nucleic acid, the chimeric nucleic acid comprising (a) a nucleic acid sequence encoding a Cas protein; and (b) at least one ribonucleic acid sequence selected from the group consisting of: (i) SEQ ID NOs: 1-195 and 797-3637; (ii) SEQ ID NOs: 196-531 and 4047-8945; (iii) SEQ ID NOs: 532-609 and 9102-9750; (iv) SEQ ID NOs: 610-765 and 9798-10532; (v) SEQ ID NOs: 766-780 and 10574-13257; and (vi) combinations of (i)-(v). For example, the pair of ribonucleic acid sequences in (b) is selected from the group consisting of: (i) SEQ ID NO: 128 and SEQ ID NO: 72; (ii) SEQ ID NO: 462 and SEQ ID NO: 421; (iii) SEQ ID NO: 550 and SEQ ID NO: 573; (iv) SEQ ID NO: 657 and SEQ ID NO: 662; (v) SEQ ID NO: 773 and SEQ ID NO: 778; and (vi) combinations of (i)-(v).

In some embodiments, the compositions comprising a chimeric nucleic acid further comprise a nucleic acid sequence encoding a detectable marker. In some embodiments, the compositions comprising a chimeric nucleic acid further comprise a promoter optimized for increased expression in human cells operably linked to the chimeric nucleic acid, wherein the promoter is selected from the group consisting of a Cytomegalovirus (CMV) early enhancer element and a chicken beta-actin promoter, a chicken beta-actin promoter, an elongation factor-1 alpha promoter, and a ubiquitin promoter.

In some aspects, the Cas protein comprises a Cas9 protein or a functional portion thereof. For example, the nucleic acid encoding Cas protein comprises a messenger RNA (mRNA) encoding Cas9 protein. In certain aspects, the mRNA comprises at least one modified nucleotide selected from the group consisting of pseudouridine, 5-methylcytodine, 2-thio-uridine, 5-methyluridine-5'-triphosphate, 4-thiouridine-5'-triphosphate, 5,6-dihydrouridine-5'-triphosphate, and 5-azauridine-5'-triphosphate. In certain aspects of the invention, the chimeric nucleic acid comprises at least one modified nucleotide selected from the group consisting of pseudouridine, 5-methylcytodine, 2-thio-uridine, 5-methyluridine-5'-triphosphate, 4-thiouridine-5'-triphosphate, 5,6-dihydrouridine-5'-triphosphate, and 5-azauridine-5'-triphosphate.

Also disclosed herein are methods for altering a target CTLA4 polynucleotide sequence in a cell comprising contacting the CTLA4 polynucleotide sequence with a clustered regularly interspaced short palindromic repeats-associated (Cas) protein and from one to two ribonucleic acids, wherein the ribonucleic acids direct Cas protein to and hybridize to a target motif of the target CTLA4 polynucleotide sequence, wherein the target CTLA4 polynucleotide sequence is cleaved, and wherein at least one of the one to two ribonucleic acids are selected from the group consisting of SEQ ID NOs: 1-195 and 797-3637. In certain aspects, each of the one to two ribonucleic acids is selected from the group consisting of SEQ ID NOs: 1-195 and 797-3637. For example, the two ribonucleic acids may comprise SEQ ID NO: 128 and SEQ ID NO: 72.

Also disclosed herein are methods for altering a target PD1 polynucleotide sequence in a cell comprising contacting the PD1 polynucleotide sequence with a clustered regularly interspaced short palindromic repeats-associated (Cas) protein and from one to two ribonucleic acids, wherein the ribonucleic acids direct Cas protein to and hybridize to a target motif of the target PD1 polynucleotide sequence, wherein the target PD1 polynucleotide sequence is cleaved, and wherein at least one of the one to two ribonucleic acids are selected from the group consisting of SEQ ID NOs: 196-531 and 4047-8945. In certain aspects, each of the one to two ribonucleic acids is selected from the group consisting of SEQ ID NOs: 196-531 and 4047-8945. For example, the two ribonucleic acids may comrpise SEQ ID NO: 462 and SEQ ID NO: 421.

Also disclosed herein are methods for altering a target TCRA polynucleotide sequence in a cell comprising contacting the TCRA polynucleotide sequence with a clustered regularly interspaced short palindromic repeats-associated (Cas) protein and from one to two ribonucleic acids, wherein the ribonucleic acids direct Cas protein to and hybridize to a target motif of the target TCRA polynucleotide sequence, wherein the target TCRA polynucleotide sequence is cleaved, and wherein at least one of the one to two ribonucleic acids are selected from the group consisting of SEQ ID NOs: 532-609 and 9102-9750. In certain aspects, each of the one to two ribonucleic acids is selected from the group consisting of SEQ ID NOs: 532-609 and 9102-9750. For example, the two ribonucleic acids may comprise SEQ ID NO: 550 and SEQ ID NO: 573.

Also disclosed herein are methods for altering a target TCRB polynucleotide sequence in a cell comprising contacting the TCRB polynucleotide sequence with a clustered regularly interspaced short palindromic repeats-associated (Cas) protein and from one to two ribonucleic acids, wherein the ribonucleic acids direct Cas protein to and hybridize to a target motif of the target TCRB polynucleotide sequence, wherein the target TCRB polynucleotide sequence is cleaved, and wherein at least one of the one to two ribonucleic acids are selected from the group consisting of SEQ ID NOs: 610-765 and 9798-10532. In certain aspects, each of the one to two ribonucleic acids is selected from the group consisting of SEQ ID NOs: 610-765 and 9798-10532. For example, the two ribonucleic acids may comprise SEQ ID NO: 657 and SEQ ID NO: 662.

In some embodiments, the present inventions are directed to modified primary human T cells, each cell comprising a modified genome, the cells comprising (a) a first genomic modification in which the cytotoxic T-lymphocyte-associated protein 4 (CTLA4) gene on chromosome 2 has been edited to reduce or eliminate CTLA4 receptor surface expression and/or activity in the cell by contacting the cell with a Cas protein or a nucleic acid sequence encoding the Cas protein and a ribonucleic acid having a sequence selected from the group consisting of SEQ ID NOs: 3638-4046; and/or (b) a second genomic modification in which the programmed cell death 1 (PD1) gene on chromosome 2 has been edited to reduce or eliminate PD1 receptor surface expression and/or activity in the cell by contacting the cell with the Cas protein or the nucleic acid encoding the Cas protein and a second ribonucleic acid having a sequence selected from the group consisting of SEQ ID NOs: 8946-9101.

In some embodiments, the modified primary human T cells further comprise (c)(i) a third genomic modification in which the gene encoding the T cell receptor (TCR) alpha chain locus on chromosome 14 has been edited, and/or (c)(ii) a fourth genomic modification in which the gene encoding the TCR beta chain locus on chromosome 7 has been, thereby reducing or eliminating TCR surface expression and/or activity in the cell. In some aspects, the third contiguous stretch of genomic DNA has been edited by contacting the cell with the Cas protein or the nucleic acid sequence encoding the Cas protein and a third ribonucleic acid having a sequence selected from the group consisting of SEQ ID NOs: 9751-9797, and/or wherein the fourth contiguous stretch of genomic DNA has been edited by contacting the cell with the Cas protein or the nucleic acid sequence encoding the Cas protein and a fourth ribonucleic acid having a sequence selected from the group consisting of SEQ ID NOs: 10533-10573.

In some embodiments, the modified primary human T cells further comprise (d) a fifth genomic modification in which the β2-microglobulin (B2M) gene on chromosome 15 has been edited, thereby reducing or eliminating MHC Class I molecule surface expression and/or activity in the cell. In certain aspects, the fifth contiguous stretch of genomic DNA has been edited by contacting the cell with the Cas protein or the nucleic acid sequence encoding the Cas protein and a fifth ribonucleic acid having a sequence selected from the group consisting of SEQ ID NOs: 13258-13719.

In some aspects, the modified primary human T cells further comprise a chimeric antigen receptor or an exogenous nucleic acid encoding the chimeric antigen receptor. In certain apects, the chimeric antigen receptor specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof.

In some aspects, the modified primary human T cells further comprise at least one exogenous protein that modulates a biological effect of interest in an adjacent cell, tissue, or organ, or an exogenous nucleic acid encoding the protein.

Also disclosed herein are methods for producing a modified primary human T cell, the methods comprising (a) contacting a primary human T cell with a Cas protein or a nucleic acid sequence encoding the Cas protein and a first ribonucleic acid having a sequence selected from the group consisting of SEQ ID NOs: 3638-4046, thereby editing the cytotoxic T-lymphocyte-associated protein 4 (CTLA4) gene on chromosome 2 to reduce or eliminate CTLA4 receptor surface expression and/or activity in the cell; and/or (b) contacting a primary human T cell with the Cas protein or the nucleic acid sequence encoding the Cas protein and a second ribonucleic acid having a sequence selected from the group consisting of SEQ ID NOs: 8946-9101, thereby editing the programmed cell death 1 (PD1) gene on chromosome 2 to reduce or eliminate PD1 receptor surface expression and/or activity in the cell.

In some embodiments, the methods further comprise (c)(i) editing the gene encoding the T cell receptor (TCR) alpha chain locus on chromosome 14 in the cell, and/or (c)(ii) editing the gene encoding the TCR beta chain locus on chromosome 7 in the cell, thereby reducing or eliminating TCR surface expression and/or activity in the cell. In certain aspects, the editing in (c)(i) comprises contacting the cell with the Cas protein or the nucleic acid encoding the Cas protein and a third ribonucleic acid having a sequence selected from the group consisting of SEQ ID NOs: 9751-9797, and/or wherein the editing in (c)(ii) comprises contacting the cell with the Cas protein or the nucleic acid encoding the Cas protein and a fourth ribonucleic acid having a sequence selected from the group consisting of SEQ ID NOs: 10533-10573.

In some embodiments, the methods further comprise (d) editing the β2-microglobulin (B2M) gene on chromosome 15 in the cell, thereby reducing or eliminating MHC Class I molecule surface expression and/or activity in the cell. In certain aspects, the editing in (d) comprises contacting the cell with the Cas protein or the nucleic acid encoding the Cas protein and a fifth ribonucleic acid having a sequence selected from the group consisting of SEQ ID NOs: 13258-13719.

In some embodiments, the methods further comprise causing the cell to express at least one chimeric antigen receptor that specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof.

In some embodiments, the methods disclosed herein further comprise causing the cell to express at least one protein that modulates a biological effect of interest in an adjacent cell, tissue, or organ when the cell is in proximity to the adjacent cell, tissue, or organ.

In certain embodiments of the inventions disclosed herein, the T cell is selected from the group consisting of cytotoxic T-cells, helper T-cells, memory T-cells, regulatory T-cells, tissue infiltrating lymphocytes, and combinations thereof. In certain embodiments of the inventions disclosed herein, the cell is obtained from a subject suffering from, being treated for, diagnosed with, at risk of developing, or suspected of having, a disorder selected from the group consisting of an autoimmune disorder, cancer, a chronic infectious disease, and graft versus host disease (GVHD).

Also disclosed herein are compositions comprising the cells of the inventions disclosed herein, or the cells produced in accordance with the methods of the inventions disclosed herein.

Also disclosed herein are compositions comprising a chimeric nucleic acid, the chimeric nucleic acid comprising: (a) a nucleic acid sequence encoding a Cas protein; (b) a ribonucleic acid sequence selected from the group consisting of: (i) SEQ ID NOs: 3638-4046; (ii) SEQ ID NOs: 8946-9101; (iii) SEQ ID NOs: 9751-9797; (iv) SEQ ID NOs: 10533-10573; (v) SEQ ID NOs: 13258-13719; and (vi) combinations of (i)-(v).

In some embodiments, the chimeric nucleic acid further comprises a nucleic acid sequence encoding a detectable marker. In some aspects of the inventions disclosed herein, the Cas protein comprises a Cpfl protein or a functional portion thereof. For example, the nucleic acid encoding Cas protein may comprise a messenger RNA (mRNA) encoding Cpfl protein. In certain asepcts, the mRNA comprises at least one modified nucleotide selected from the group consisting of pseudouridine, 5-methylcytodine, 2-thio-uridine, 5-methyluridine-5'-triphosphate, 4-thiouridine-5'-triphosphate, 5,6-dihydrouridine-5'-triphosphate, and 5-azauridine-5'-triphosphate.

In some embodiments, the chimeric nucleic acid comprises at least one modified nucleotide selected from the group consisting of pseudouridine, 5-methylcytodine, 2-thio-uridine, 5-methyluridine-5'-triphosphate, 4-thiouridine-5'-triphosphate, 5,6-dihydrouridine-5'-triphosphate, and 5-azauridine-5'-triphosphate.

Also disclosed herein are methods for altering a target CTLA4 polynucleotide sequence in a cell, the methods comprising contacting the CTLA4 polynucleotide sequence with a clustered regularly interspaced short palindromic repeats-associated (Cas) protein and a ribonucleic acid, wherein the ribonucleic acid directs Cas protein to and hybridizes to a target motif of the target CTLA4 polynucleotide sequence, wherein the target CTLA4 polynucleotide sequence is cleaved, and wherein the ribonucleic acid is selected from the group consisting of SEQ ID NOs: 3638-4046.

Also disclosed herein are methods for altering a target PD1 polynucleotide sequence in a cell, the methods comprising contacting the PD1 polynucleotide sequence with a clustered regularly interspaced short palindromic repeats-associated (Cas) protein and a ribonucleic acid, wherein the ribonucleic acid directs Cas protein to and hybridizes to a target motif of the target PD1 polynucleotide sequence, wherein the target PD 1 polynucleotide sequence is cleaved, and wherein the ribonucleic acid is selected from the group consisting of SEQ ID NOs: 8946-9101.

Also disclosed are methods for altering a target TCRA polynucleotide sequence in a cell, the methods comprising contacting the TCRA polynucleotide sequence with a clustered regularly interspaced short palindromic repeats-associated (Cas) protein and a ribonucleic acid, wherein the ribonucleic acid directs Cas protein to and hybridizes to a target motif of the target TCRA polynucleotide sequence, wherein the target TCRA polynucleotide sequence is cleaved, and wherein the ribonucleic acid is selected from the group consisting of SEQ ID NOs: 9751-9797.

Also disclosed herein are methods for altering a target TCRB polynucleotide sequence in a cell, the methods comprising contacting the TCRB polynucleotide sequence with a clustered regularly interspaced short palindromic repeats-associated (Cas) protein and a ribonucleic acid, wherein the ribonucleic acid directs Cas protein to and hybridizes to a target motif of the target TCRB polynucleotide sequence, wherein the target TCRB polynucleotide sequence is cleaved, and wherein the ribonucleic acid is selected from the group consisting of SEQ ID NOs: 10533-10573.

The above discussed, and many other features and attendant advantages of the present inventions will become better understood by reference to the following detailed description of the invention.

### BRIEF DESCRIPTION OF THE DRAWINGS

These and other characteristics of the present invention will be more fully understood by reference to the following detailed description in conjunction with the attached drawings.

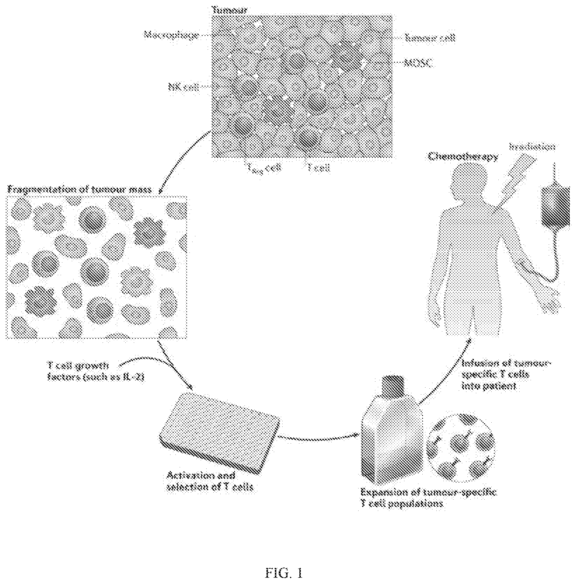

FIG. 1 is a schematic representation of adoptive immunotherapy using Tumor Infiltrating Lymphocytes (TILs). T cells are isolated from tumors, expanded *ex-vivo* and subsequently re-infused into the patient to target the tumor cells. Since the tumor environment does not support sufficient T cell proliferation, this method enables T cells to be activated and to proliferate *ex-vivo* before being reintroduced to mount an immune attack on the tumor (Restifo et al., 2012).
FIG. 2 is a schematic representation of the three generations of CAR T cells. Shown in green are the tumor-associated antigen (TAA)-binding domains that determine antigen specificity. The intracellular domain incorporates different aspects of the TCR transduction machinery: (CD3 ζ chain/ZAP70), blue (CD28/PI3K) and yellow (4-1BB or OX40/TRAF) (Adapted from Casucci and Bondanza, 2011).

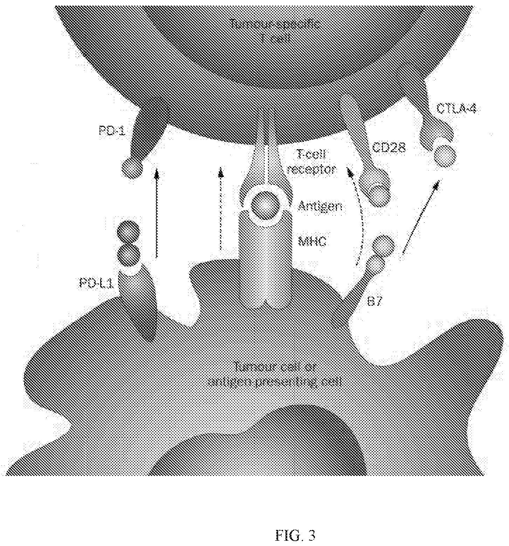

FIG. 3 is a schematic representation of T cell activation and inhibitory mechanisms. Dashed arrows indicate T cell activation through MHC-TCR and CD28-B7 interactions. Solid arrows represent inhibitory actions mediated through CTLA4 and PD1 on T cells. B7-1 and B7-2 are CD80 and CD86, respectively (adapted from Drake et al, C.G, 2014).
FIG. 4 shows an exemplary amino acid sequence of a Cas protein. Yellow highlights indicate Ruv-C-like domain. Underlining indicates HNH nuclease domain.

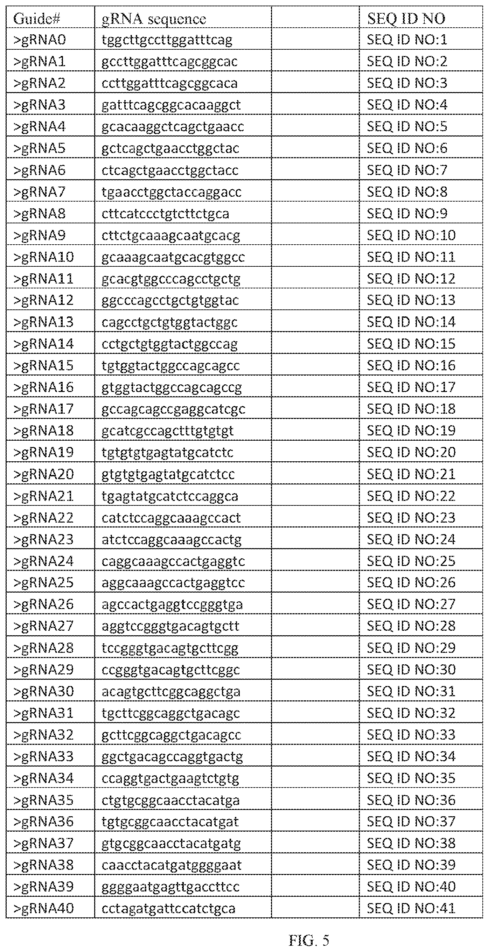

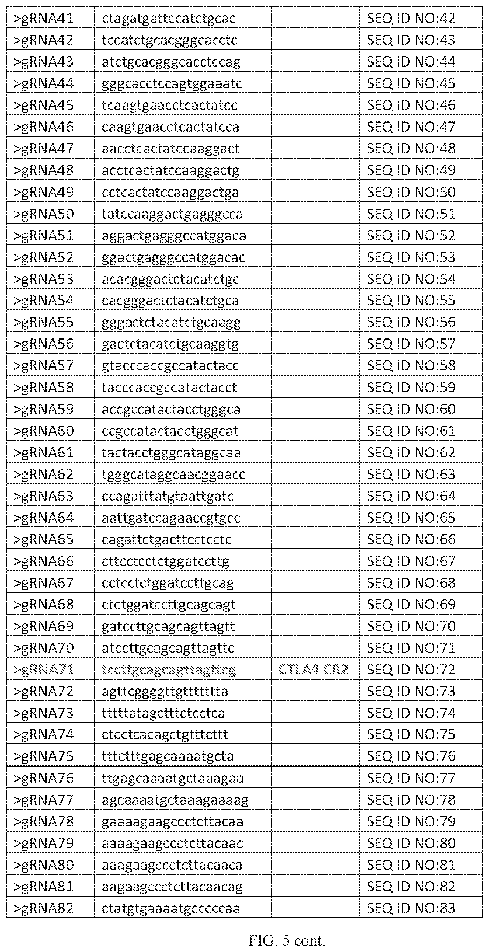

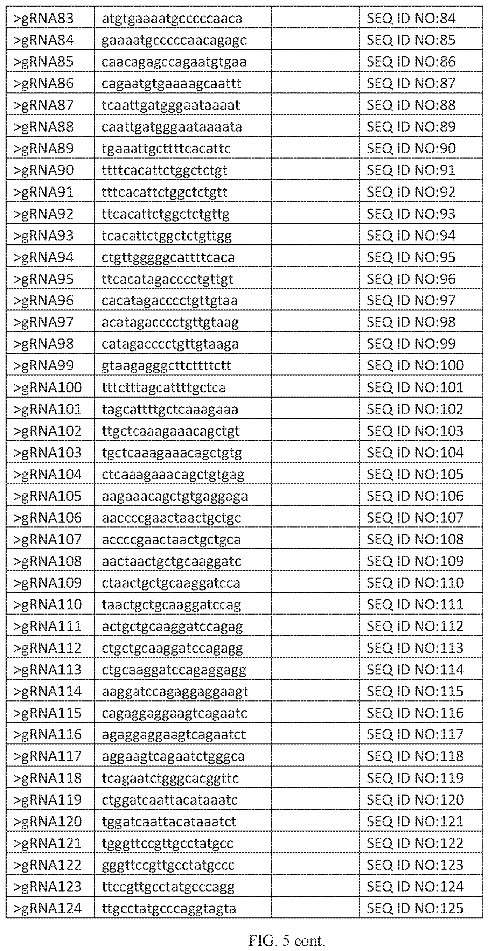

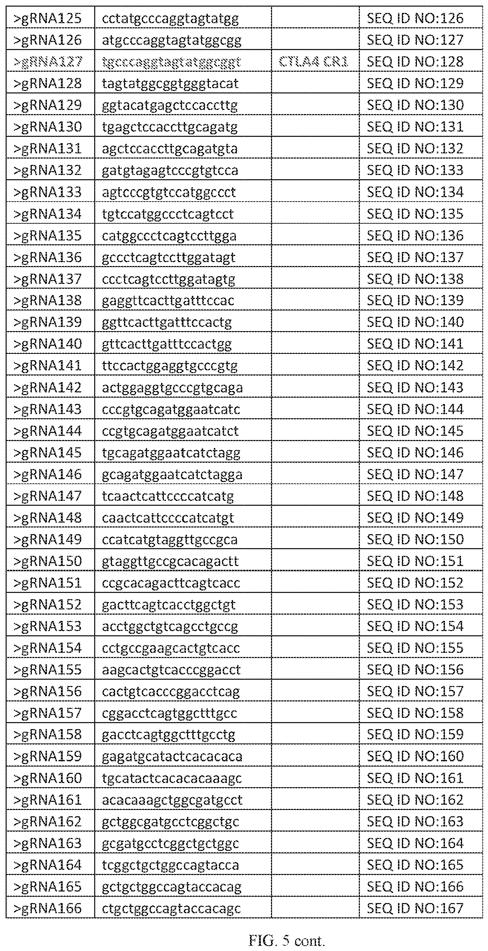

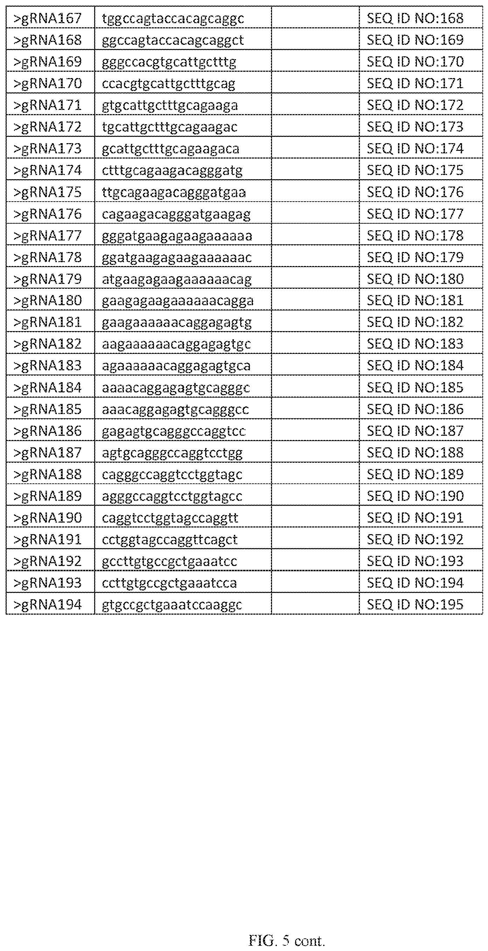

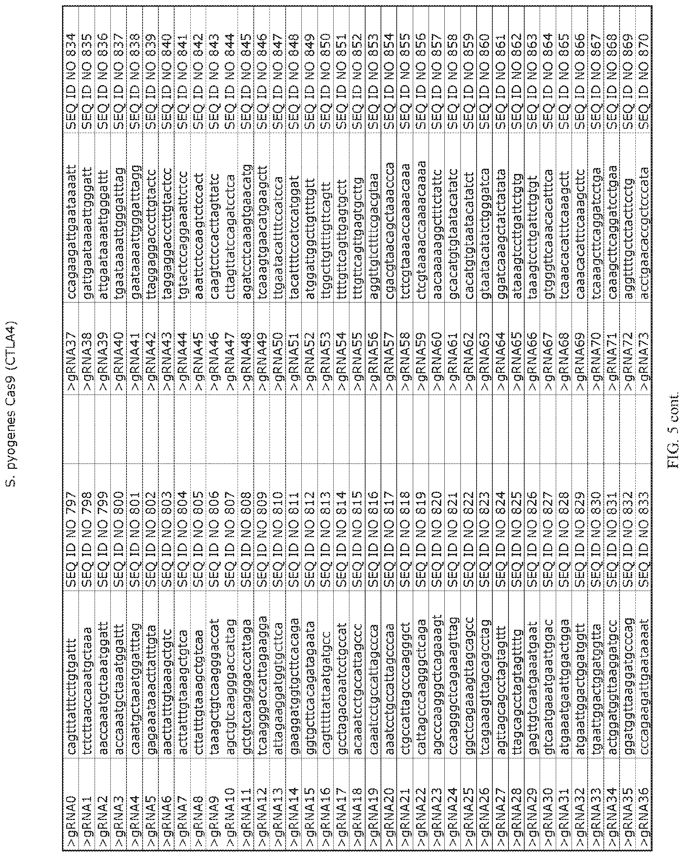

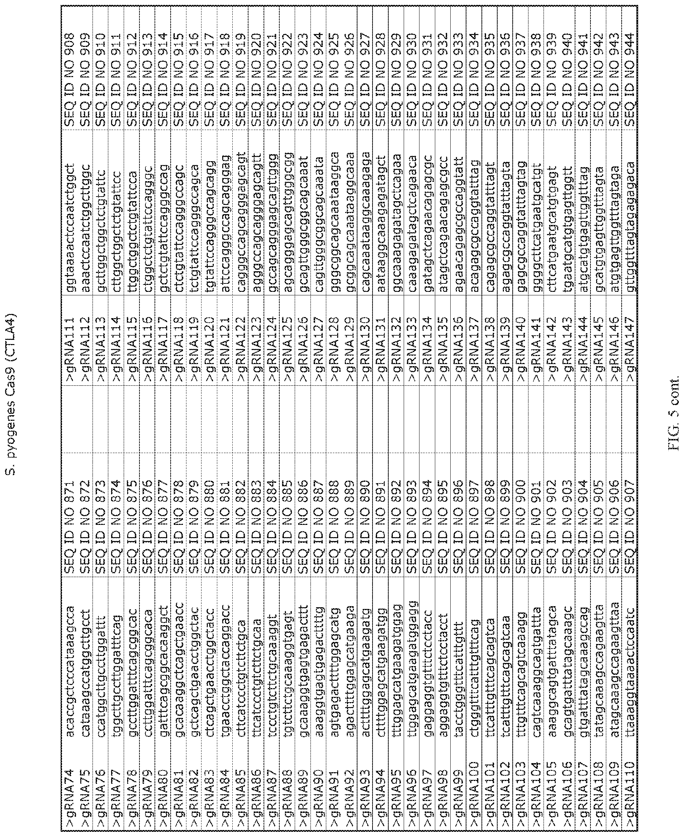

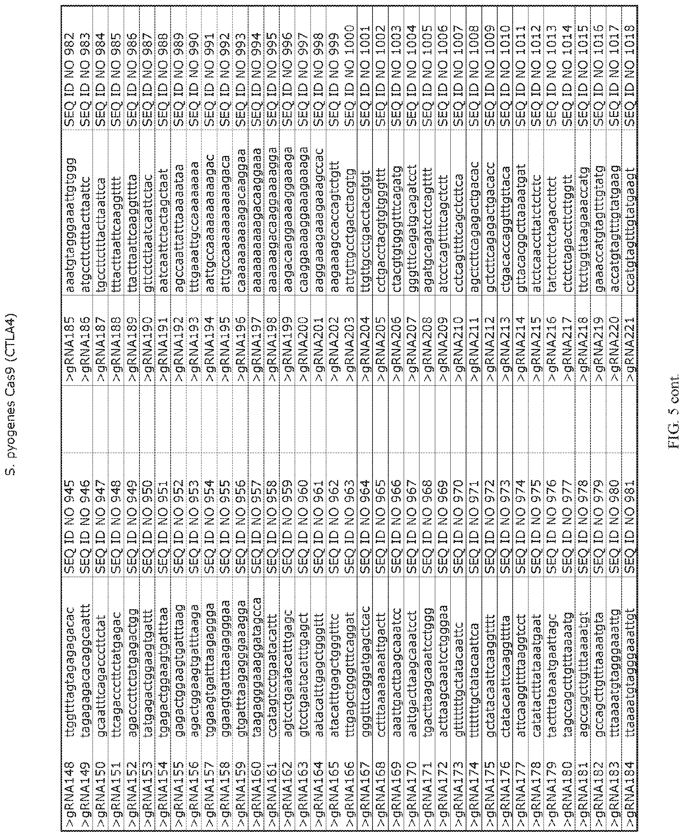

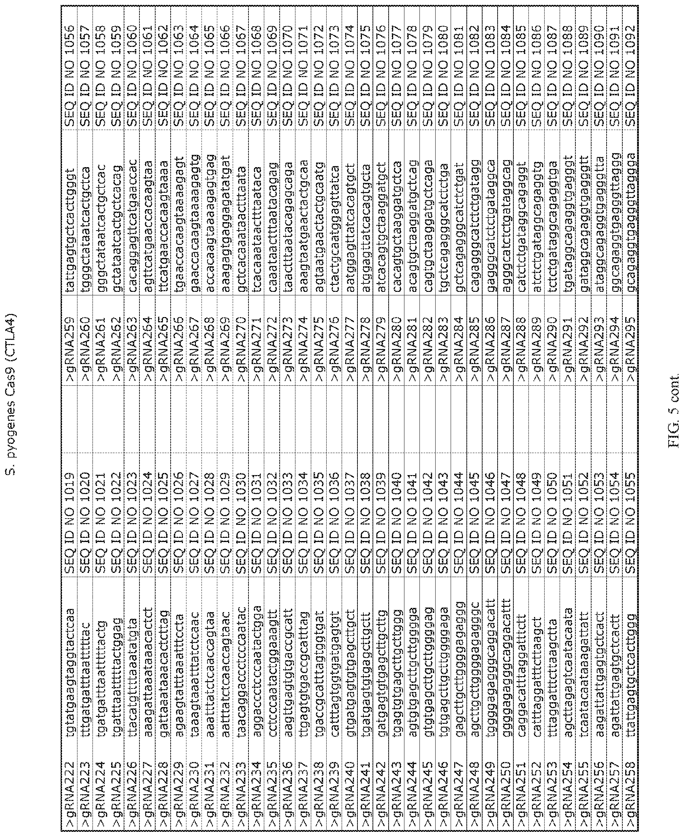

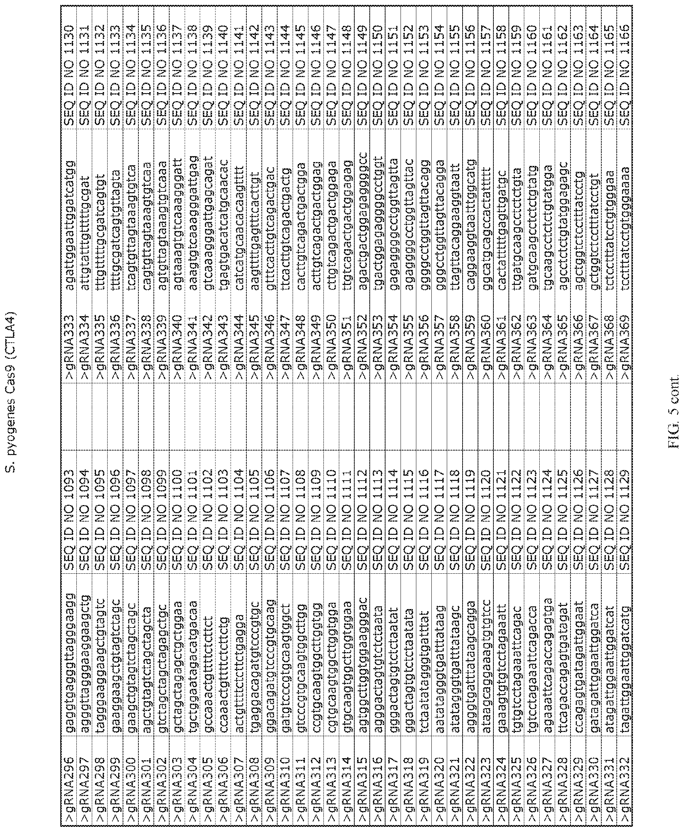

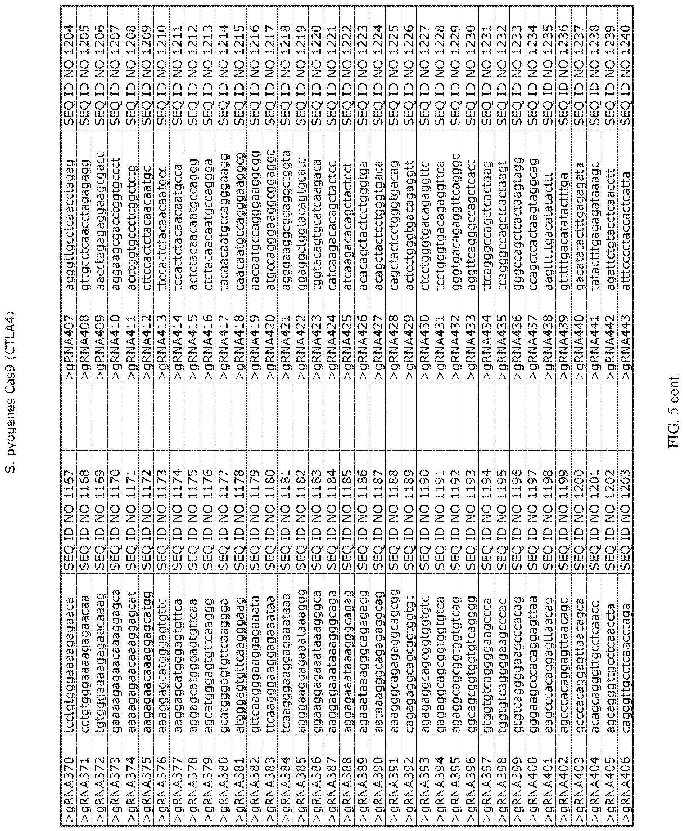

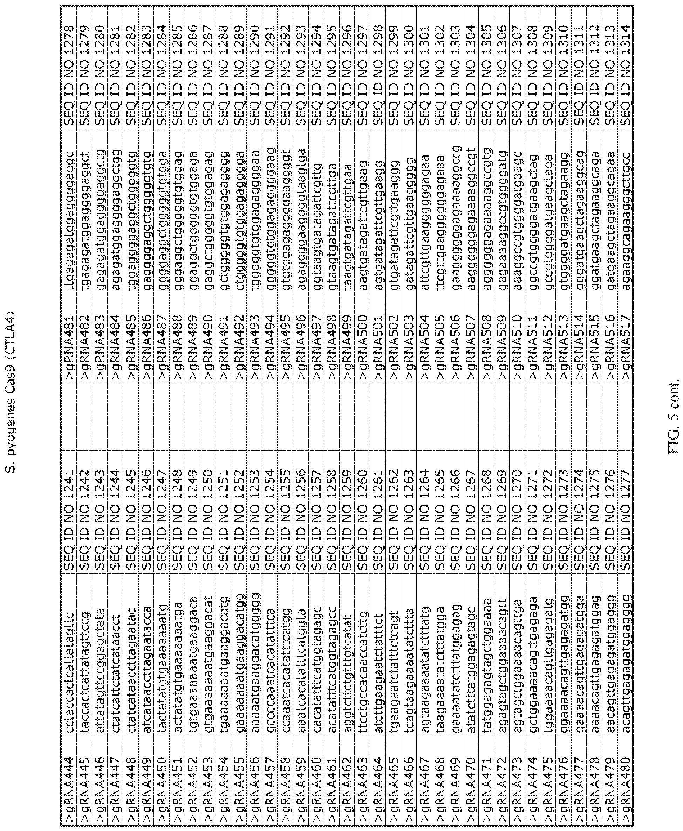

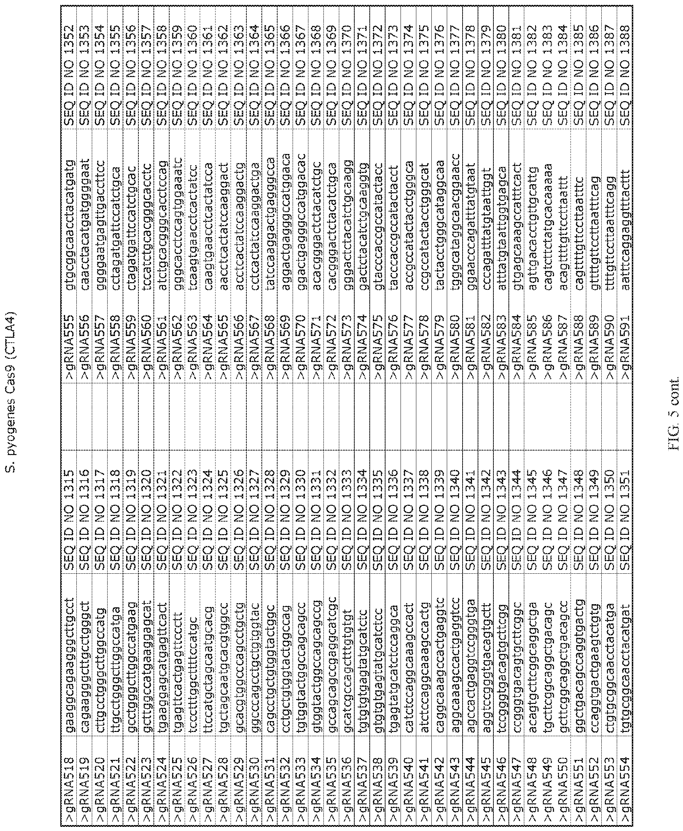

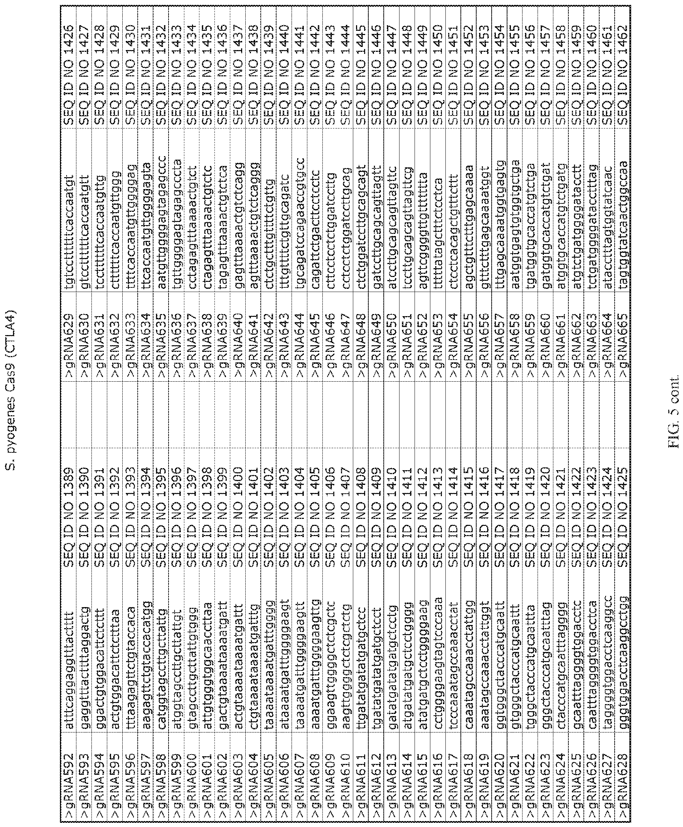

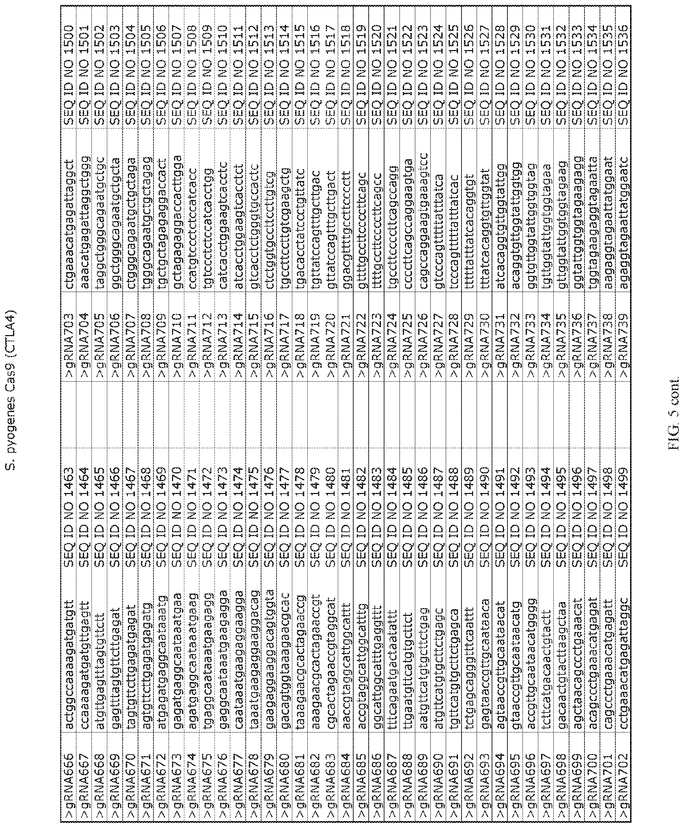

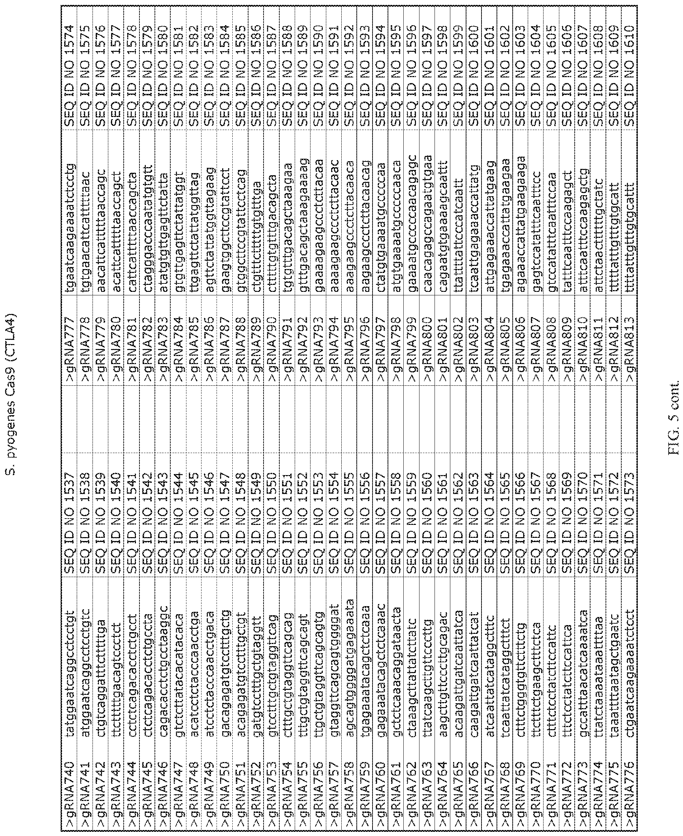

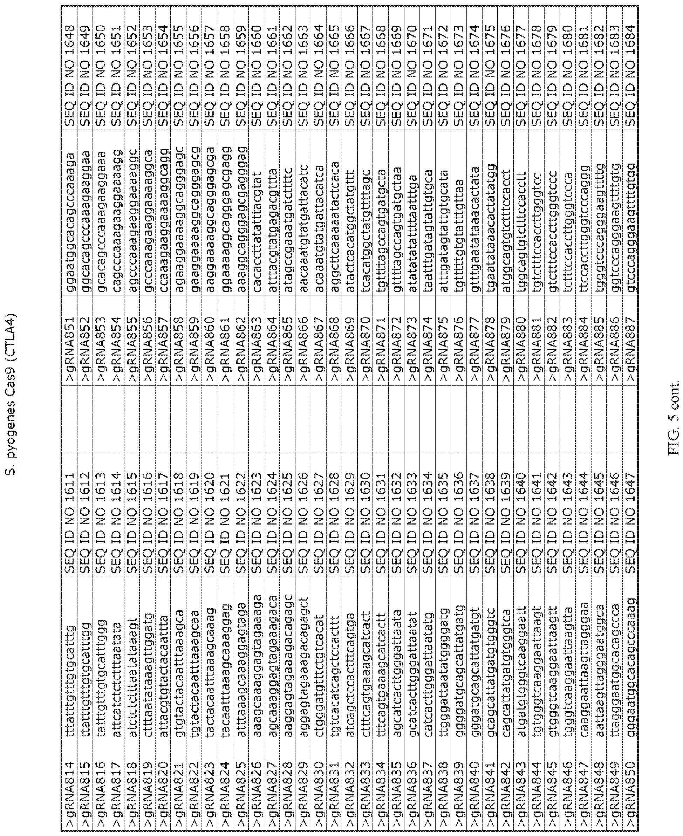

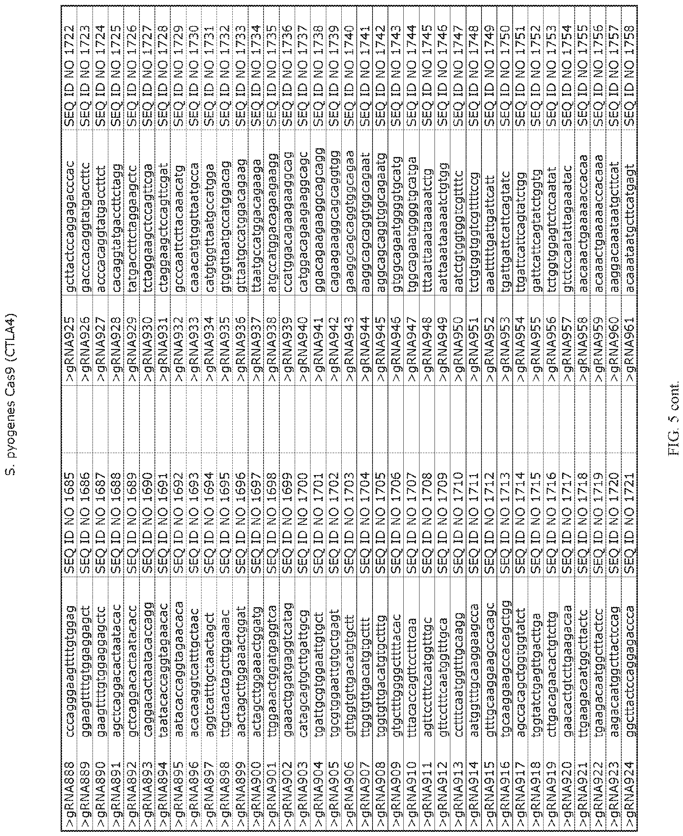

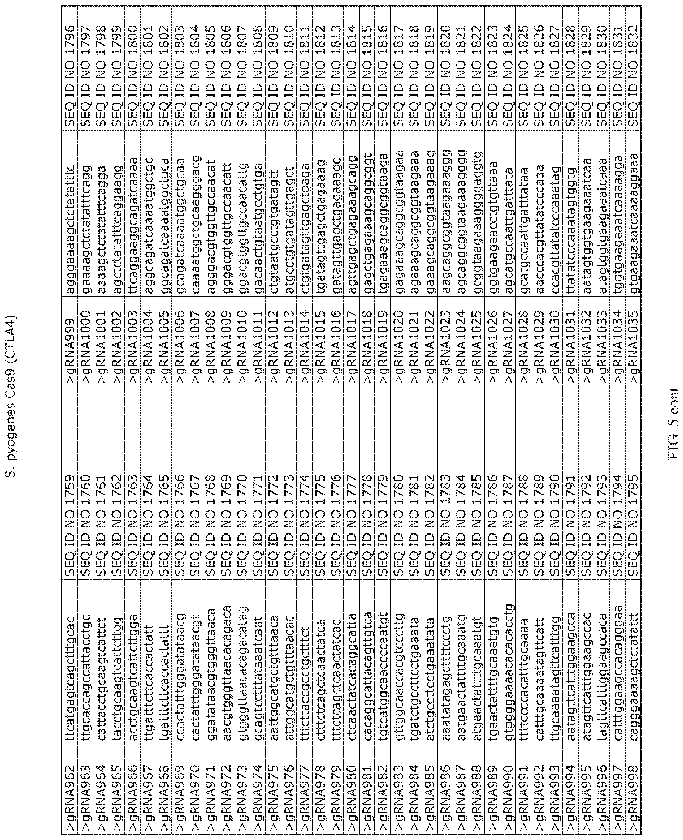

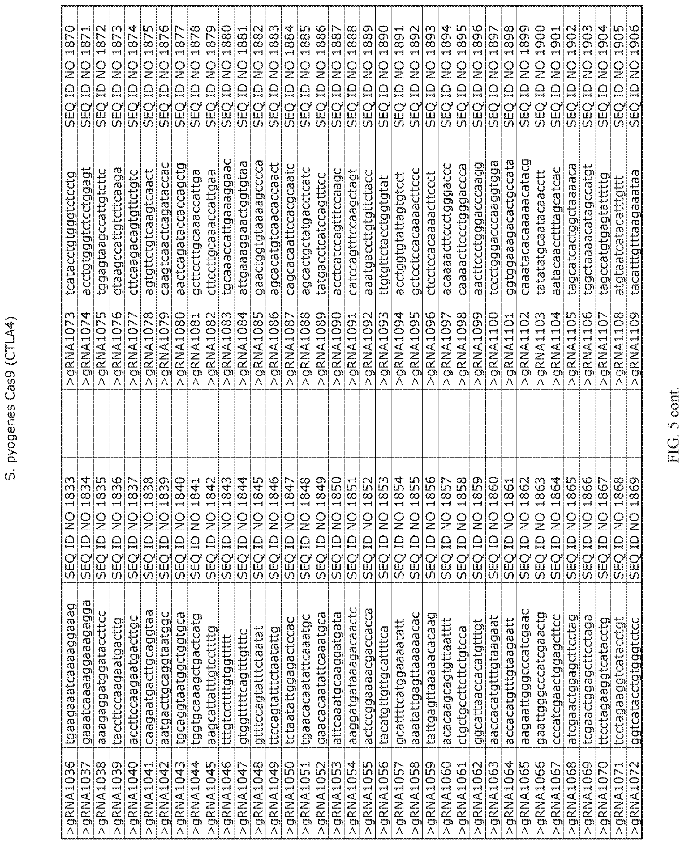

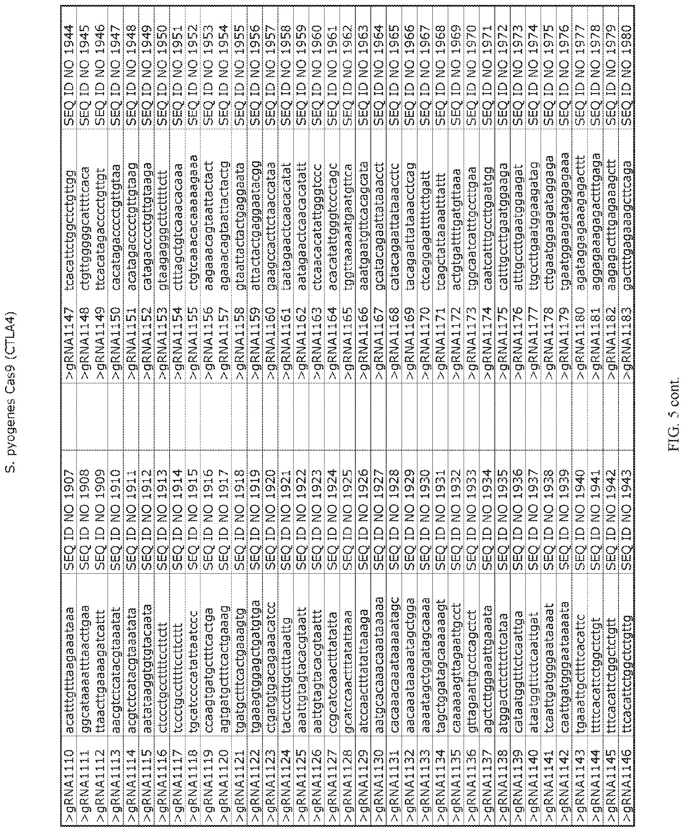

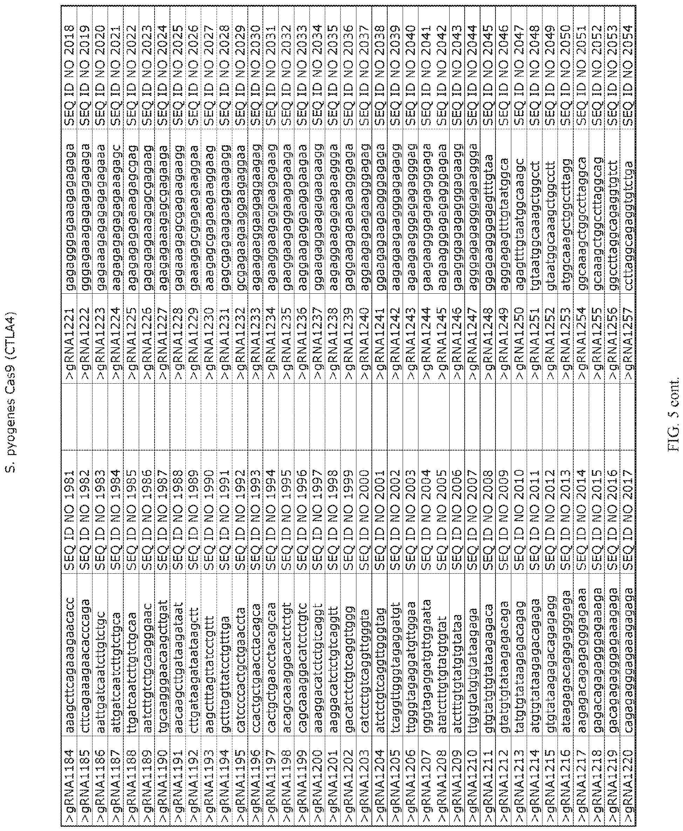

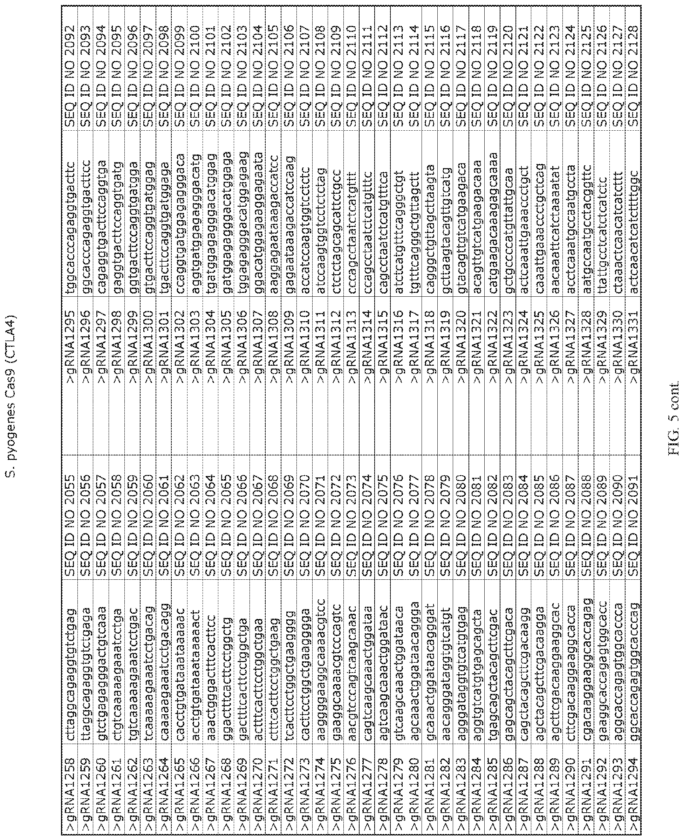

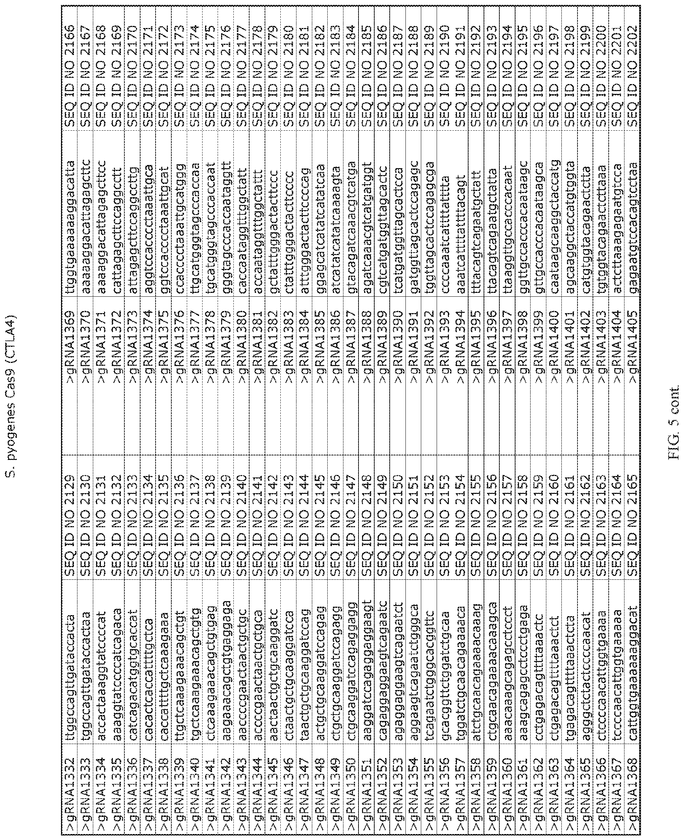

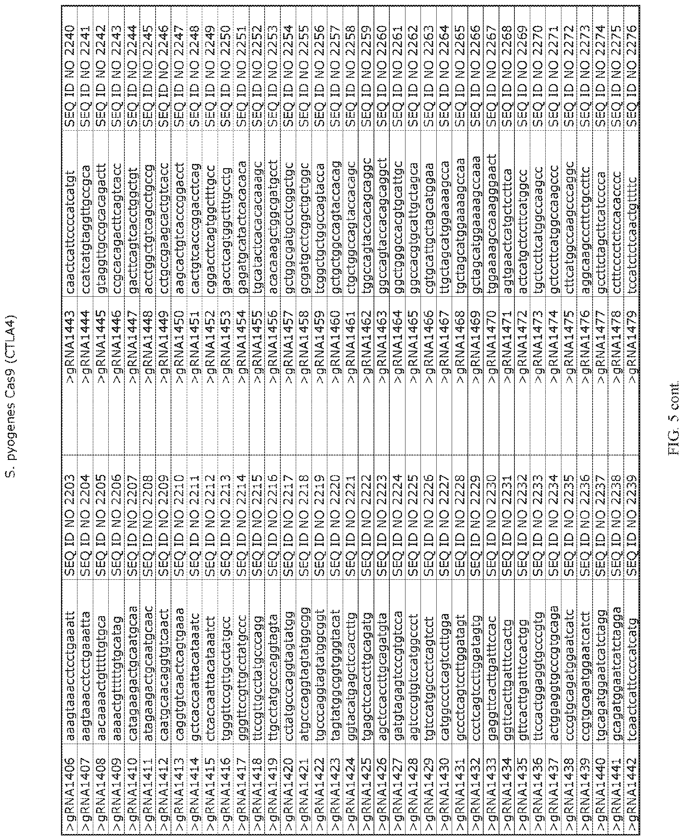

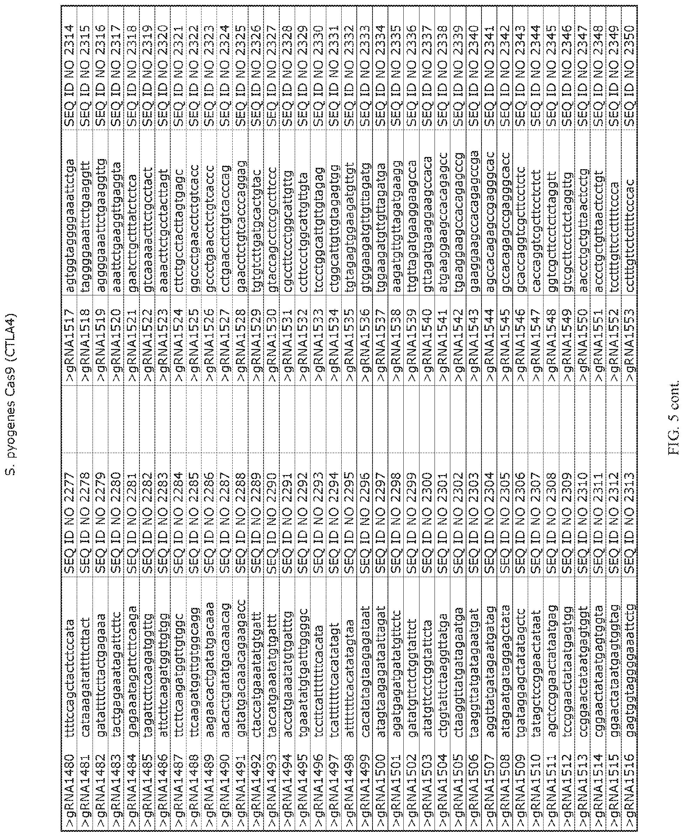

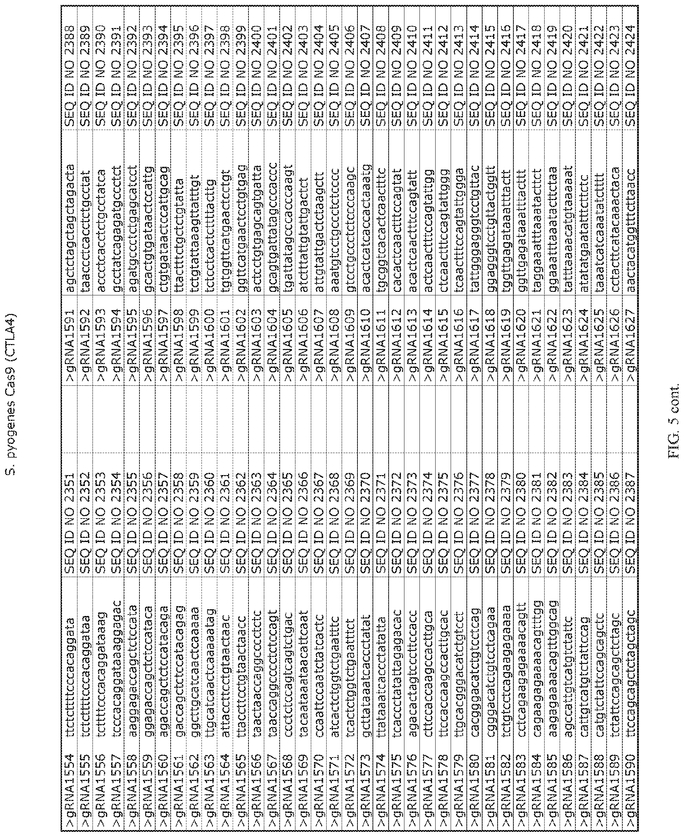

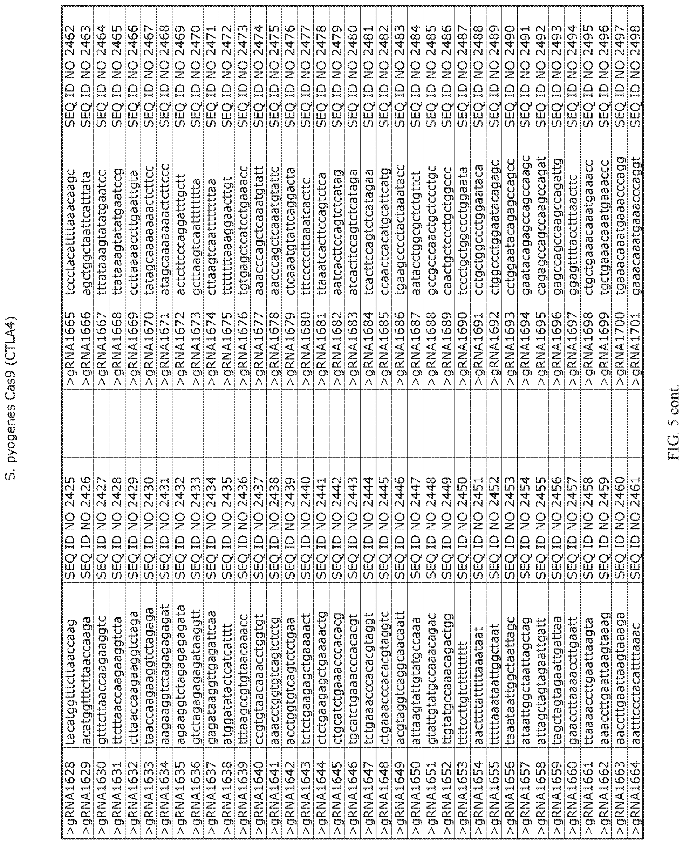

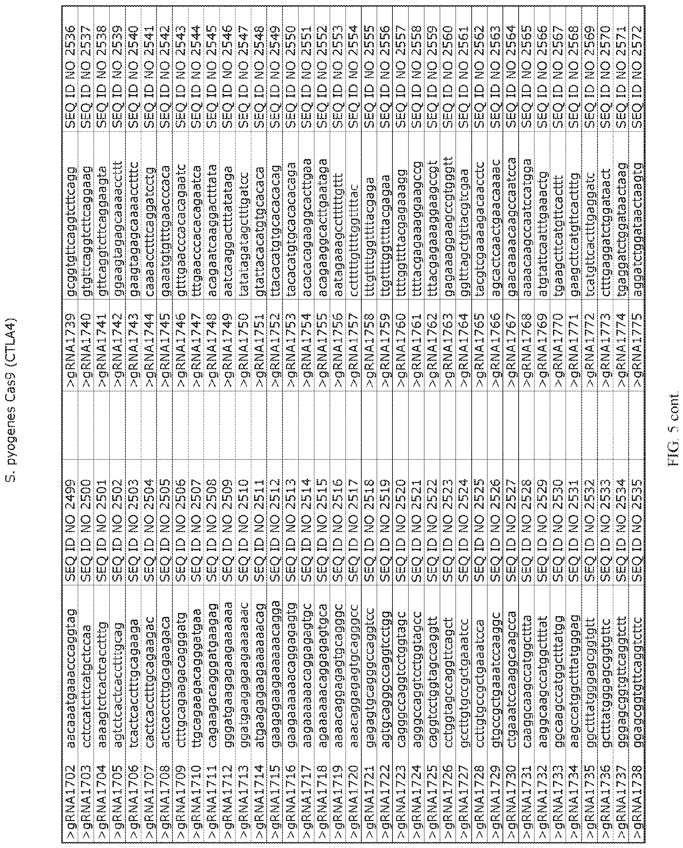

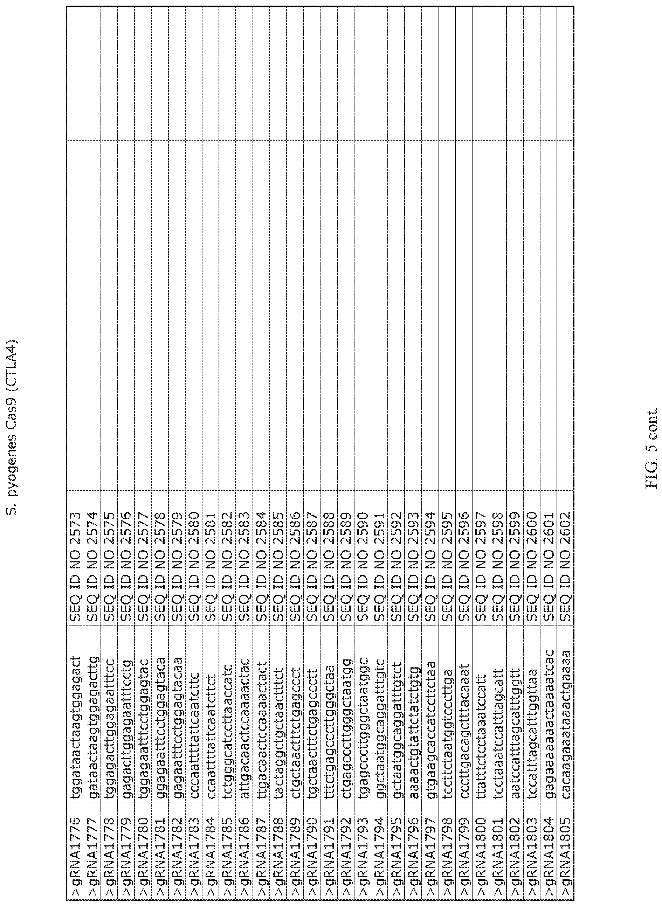

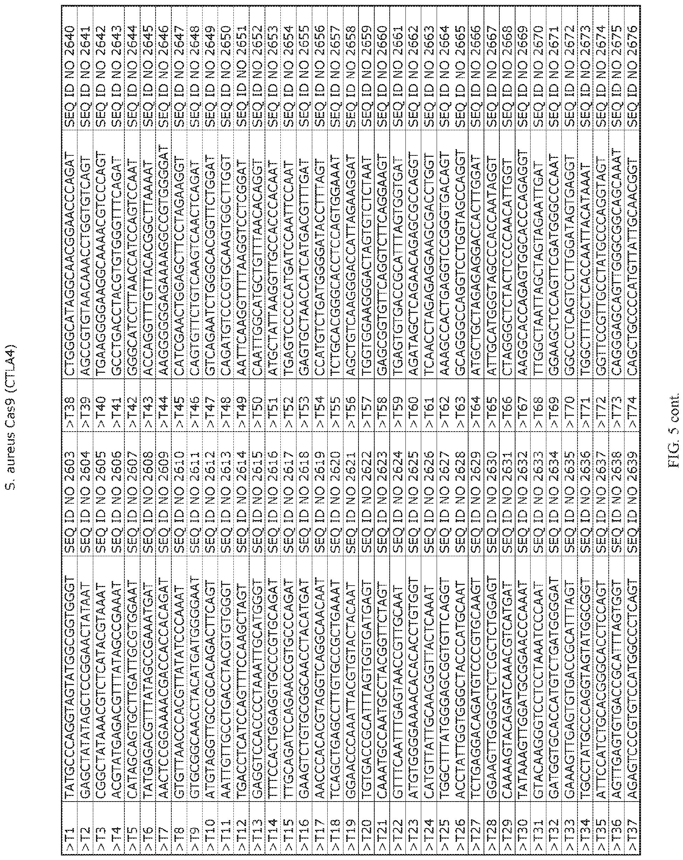

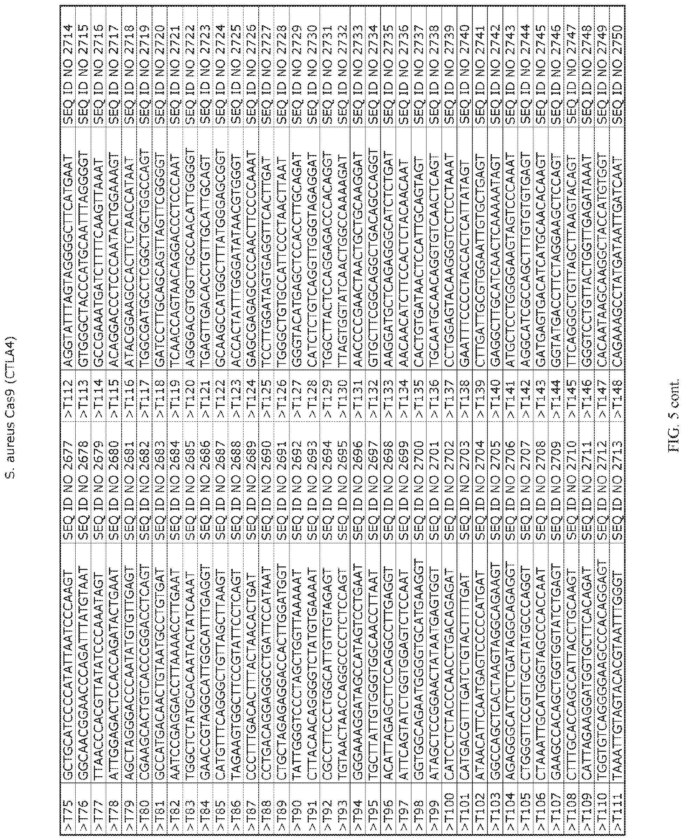

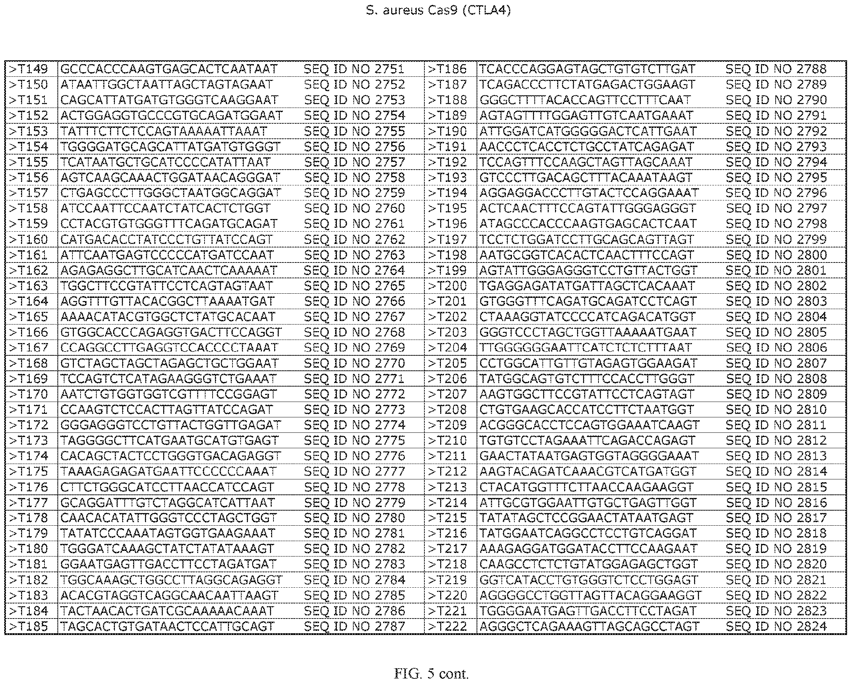

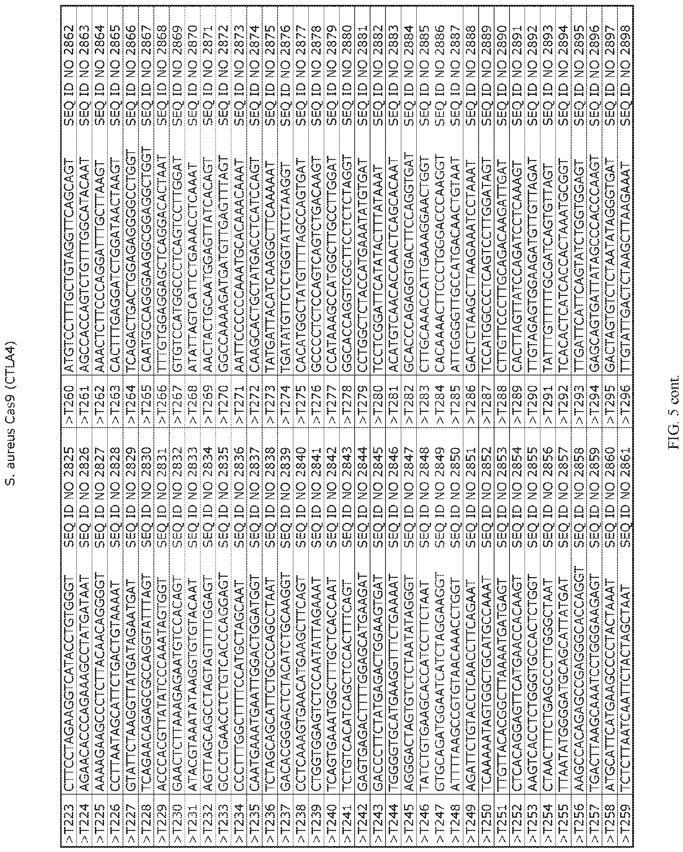

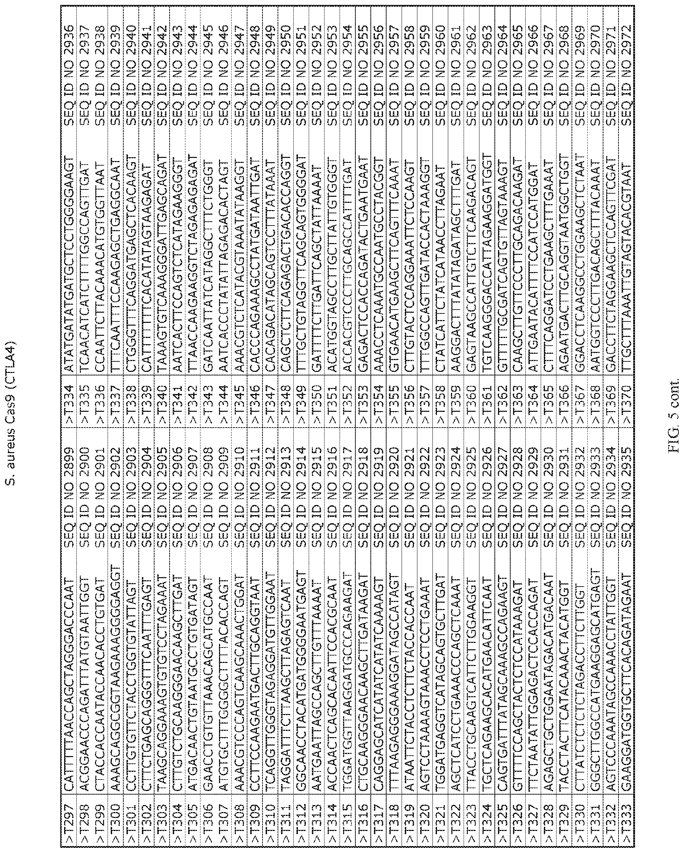

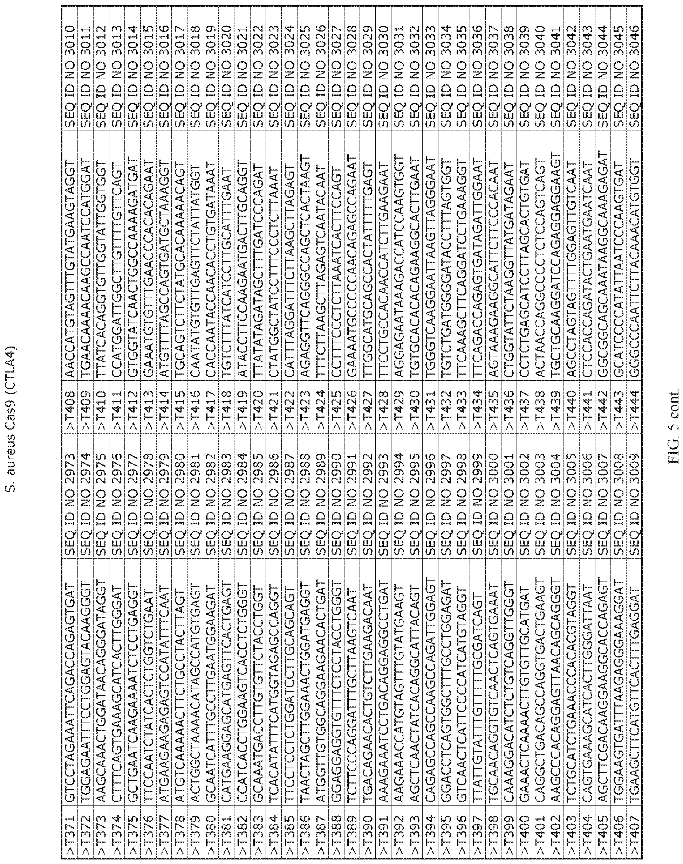

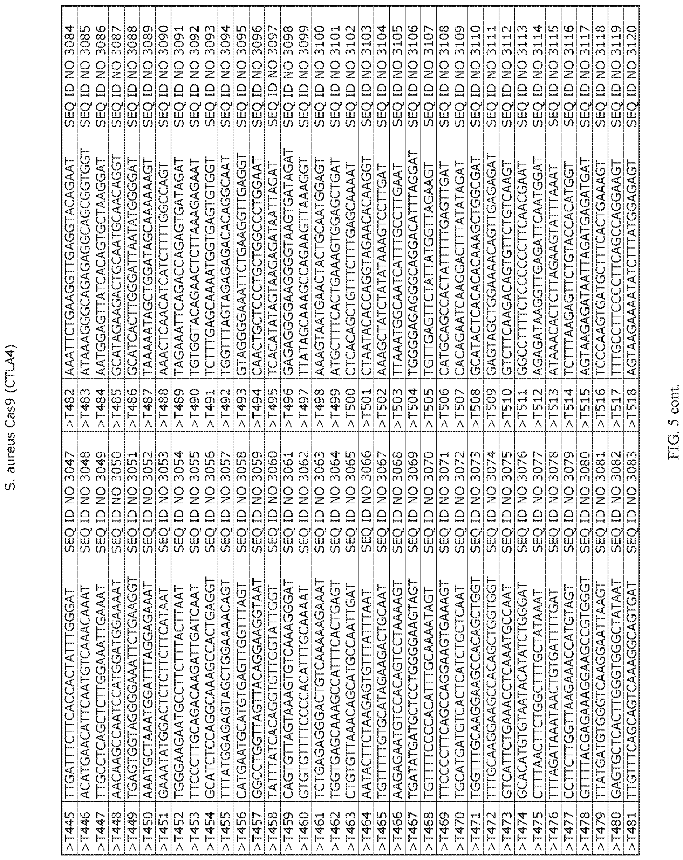

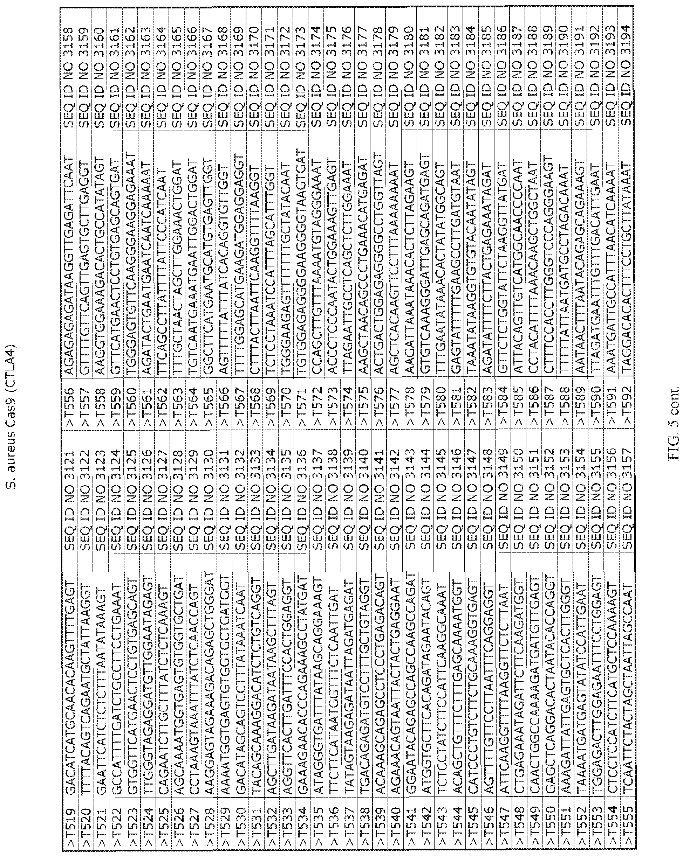

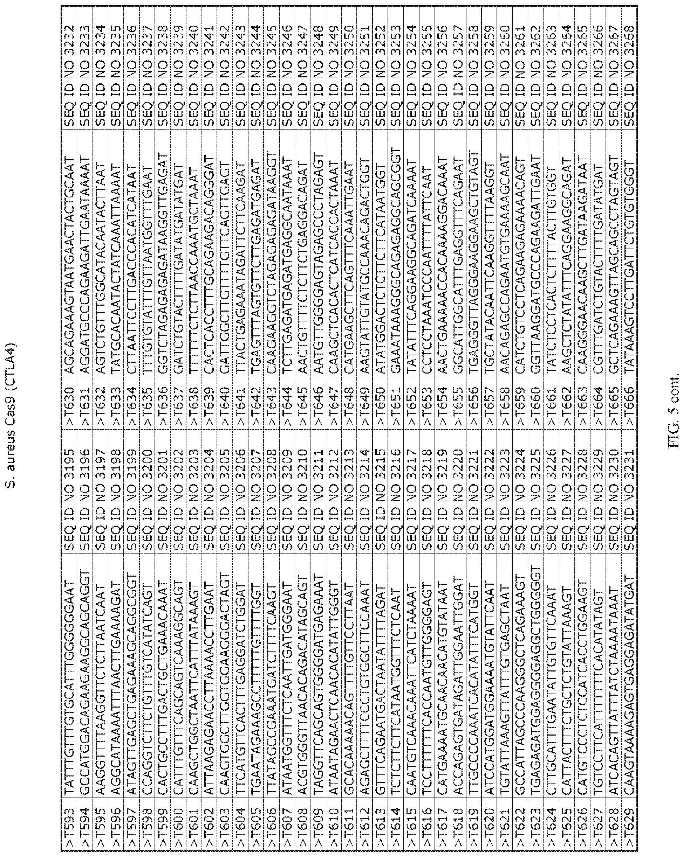

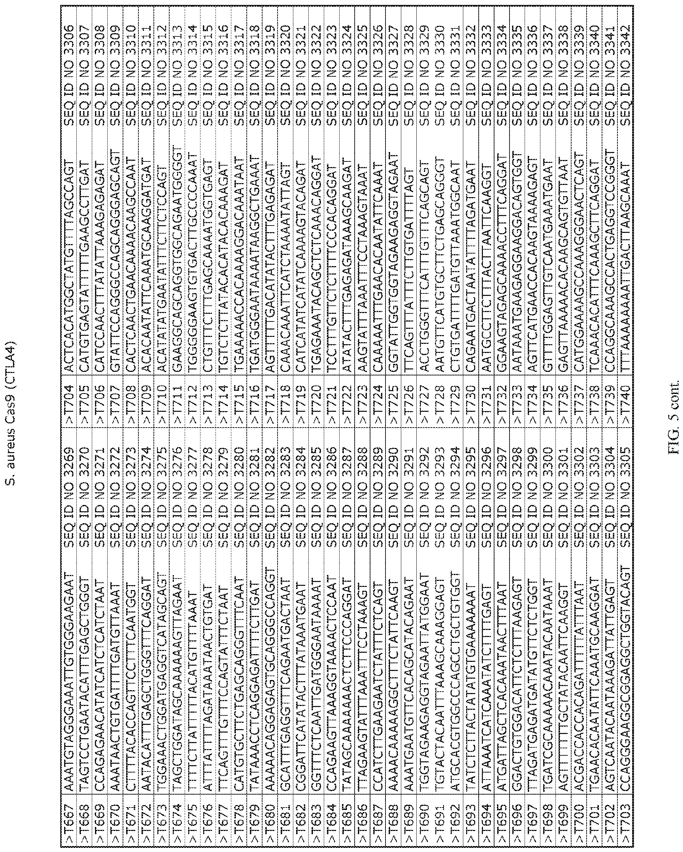

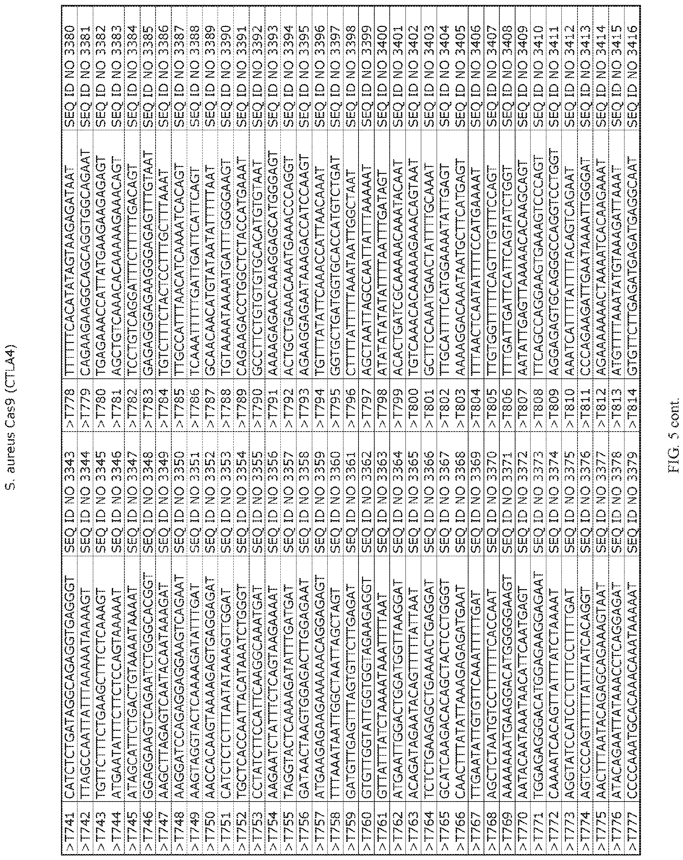

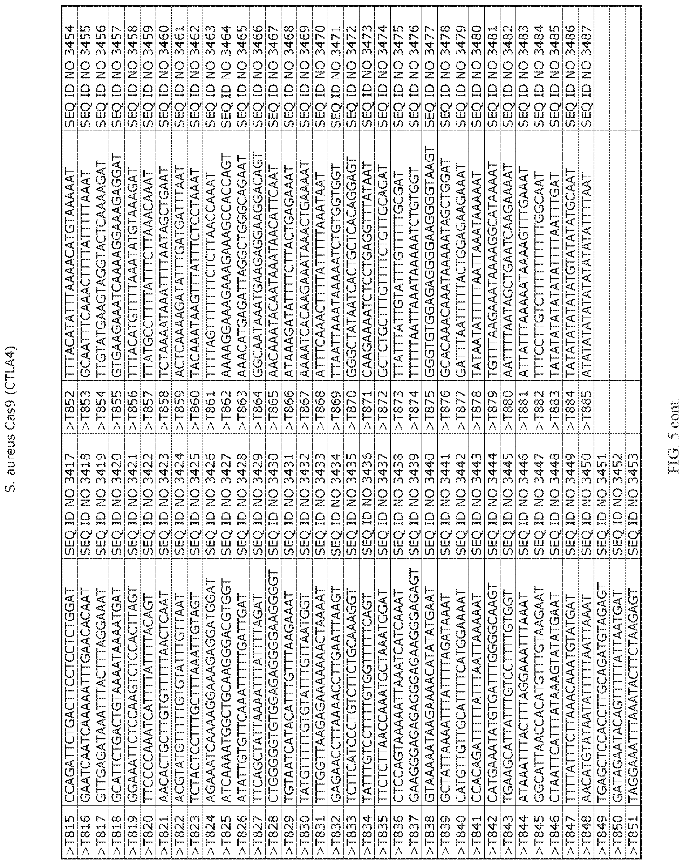

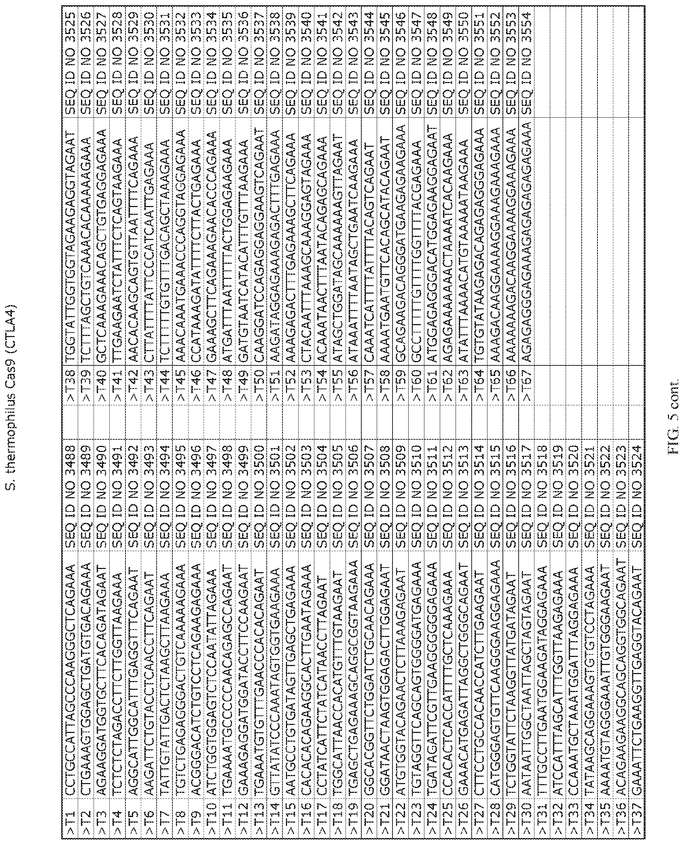

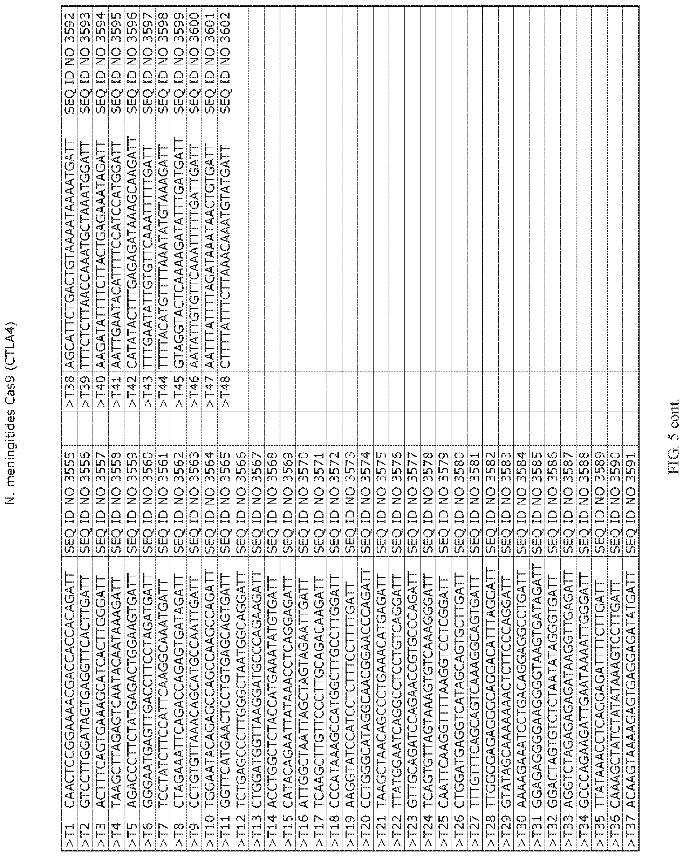

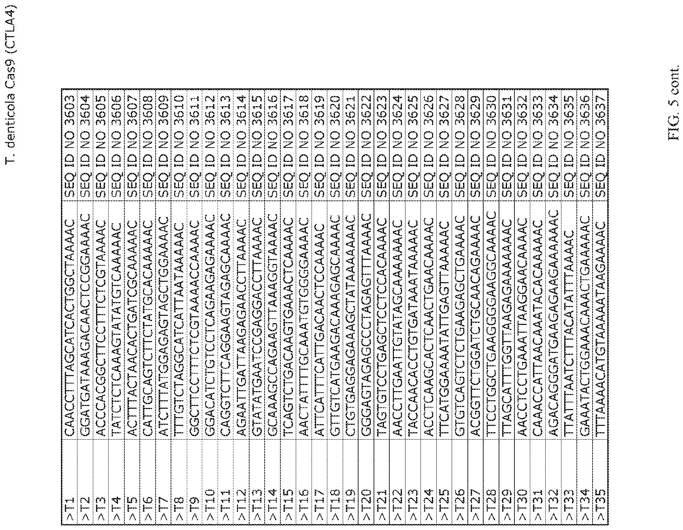

FIG. 5 shows exemplary gRNA sequences useful for targeting the CTLA4 gene using Cas9. The gRNAs described in the experimental examples are identified in red text.

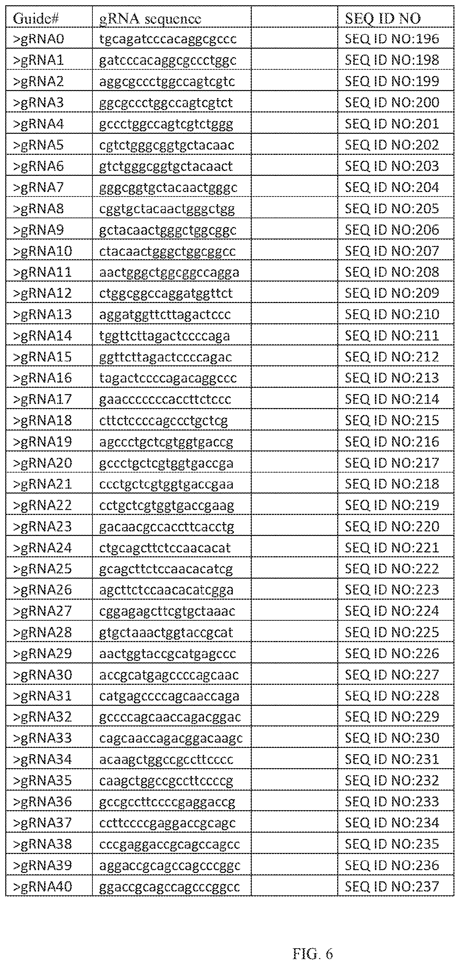

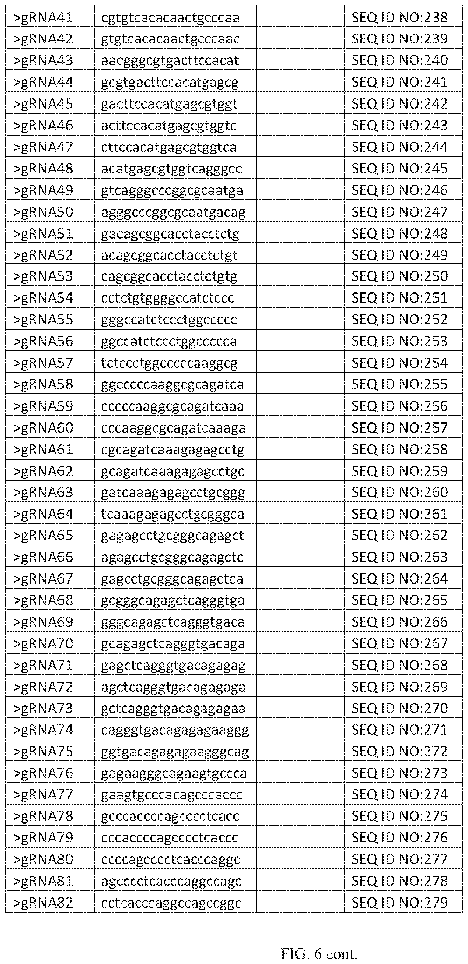

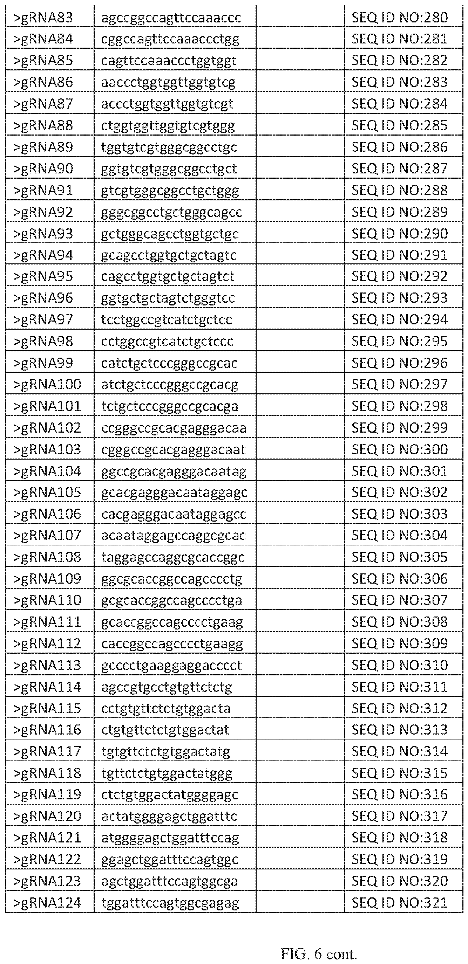

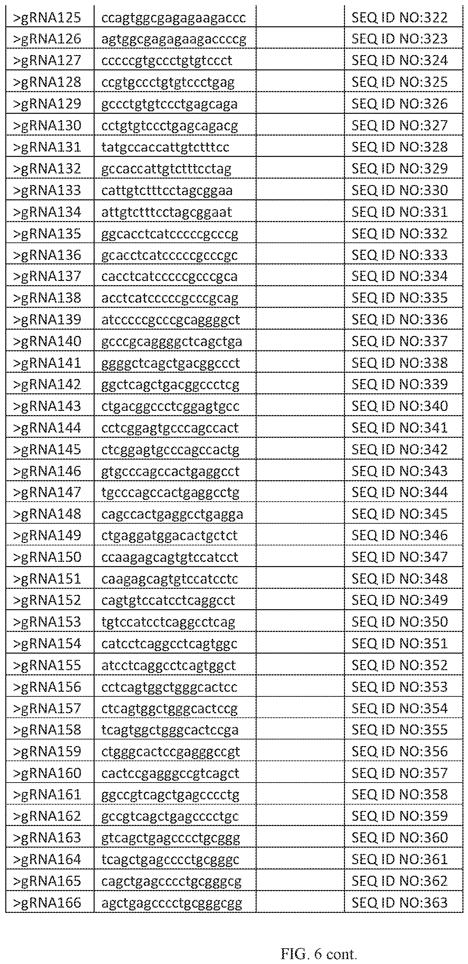

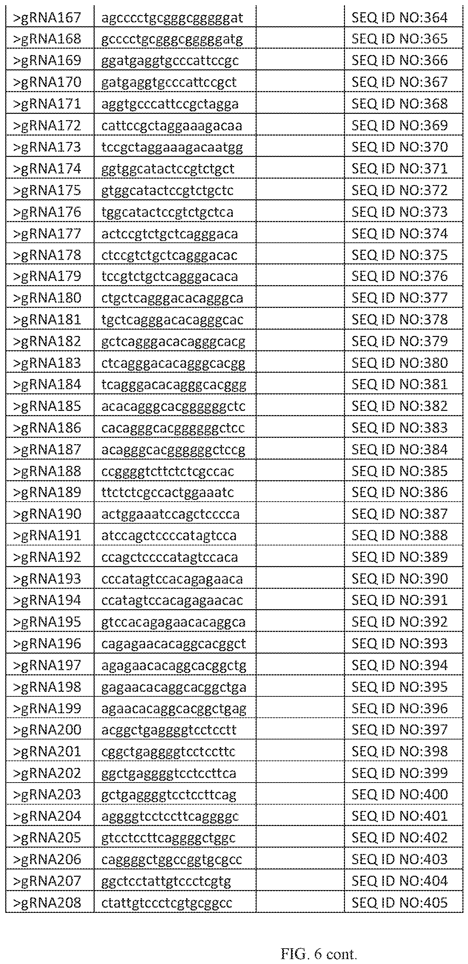

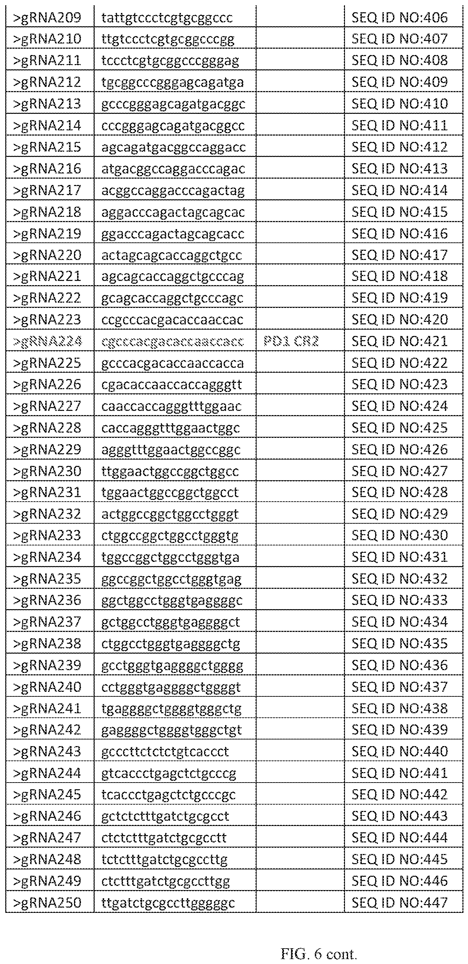

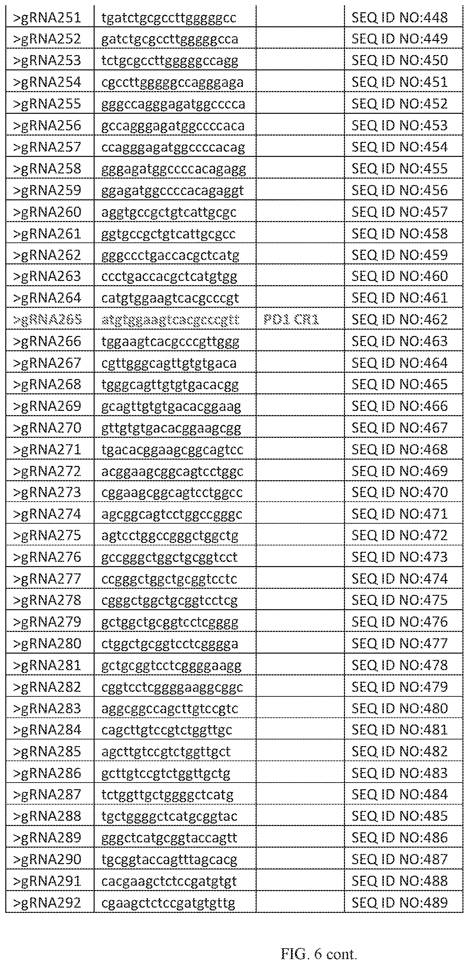

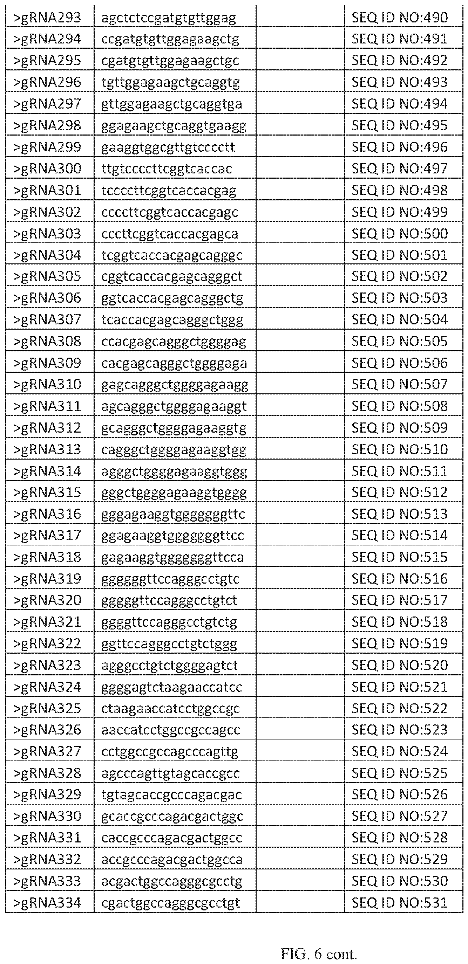

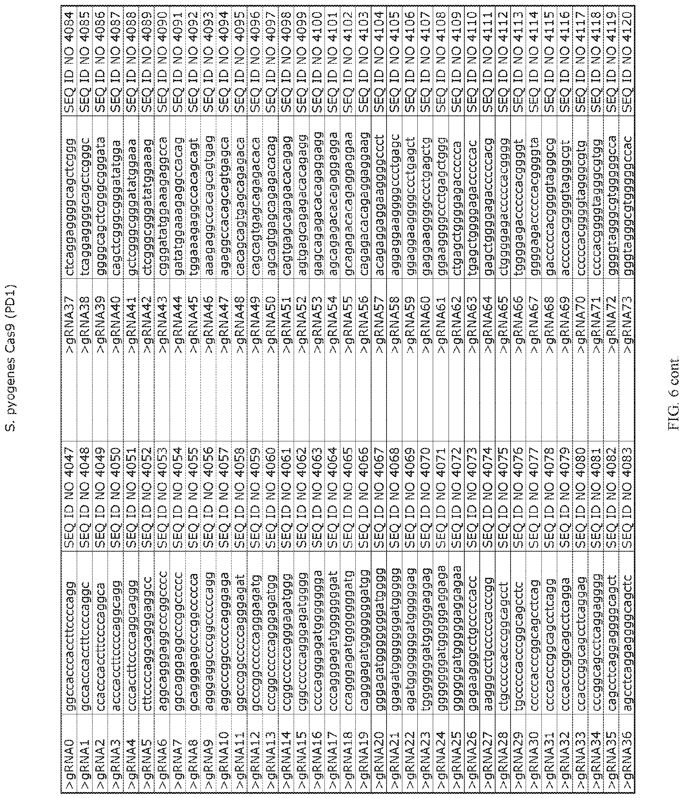

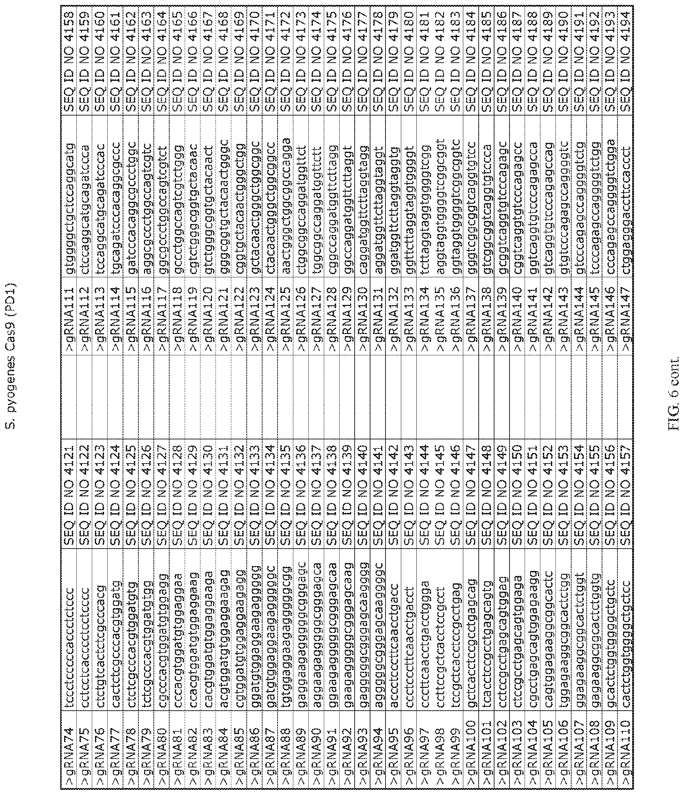

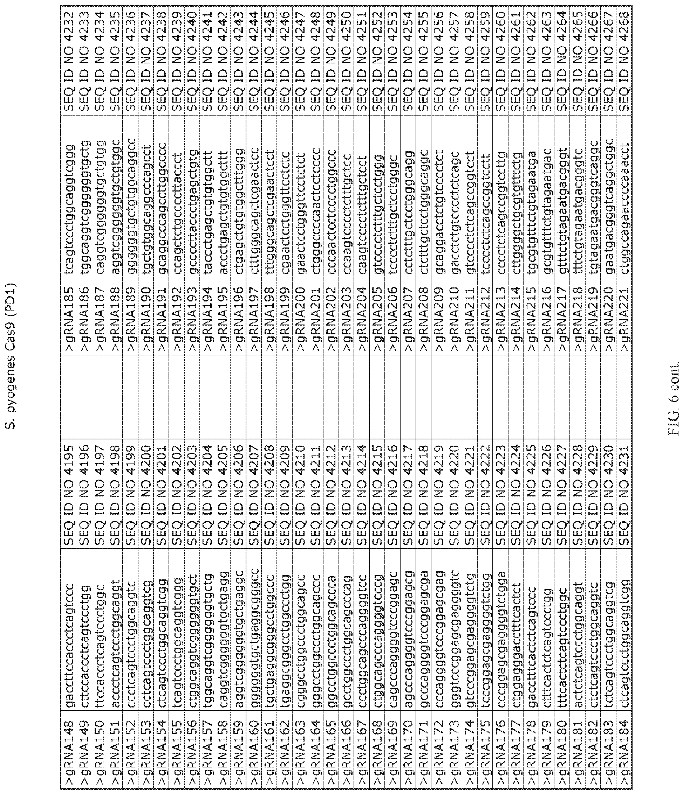

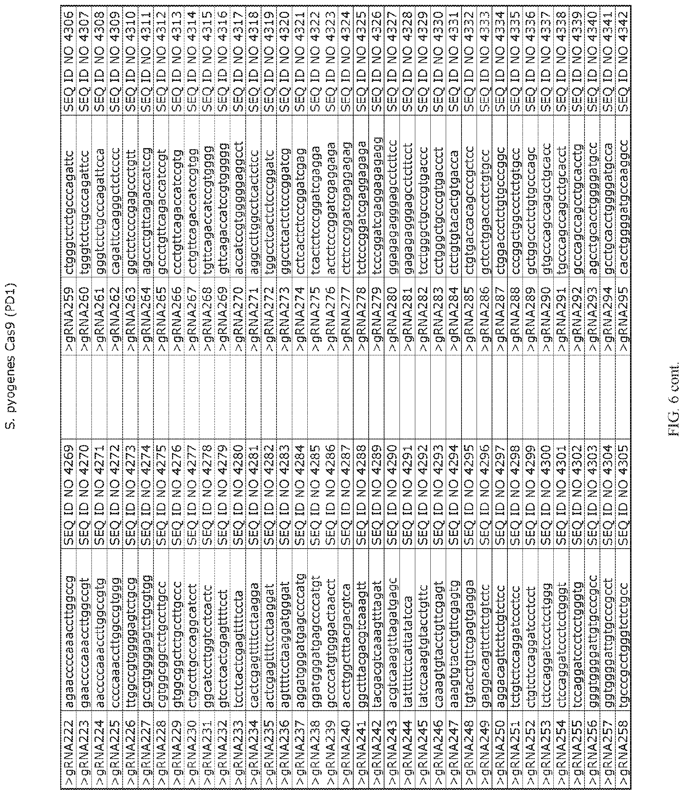

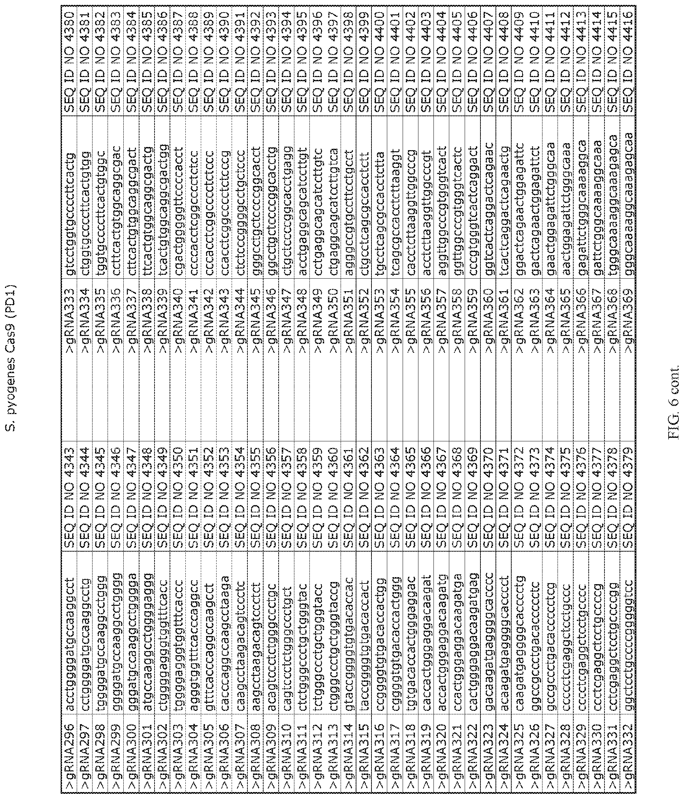

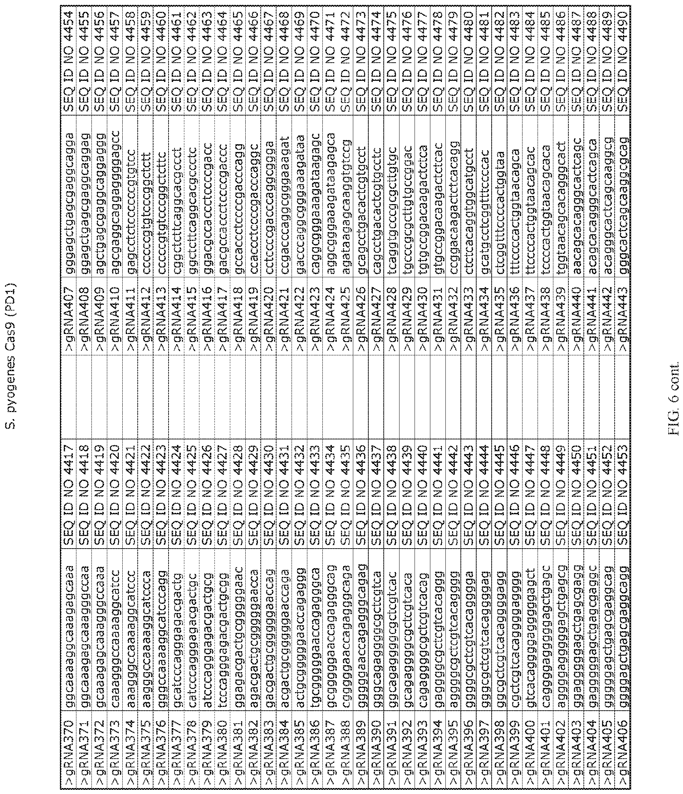

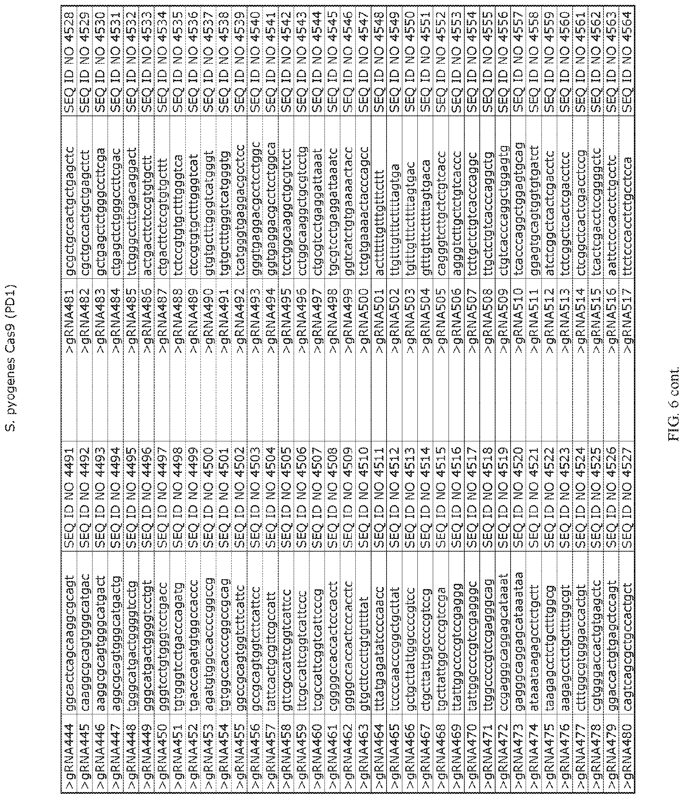

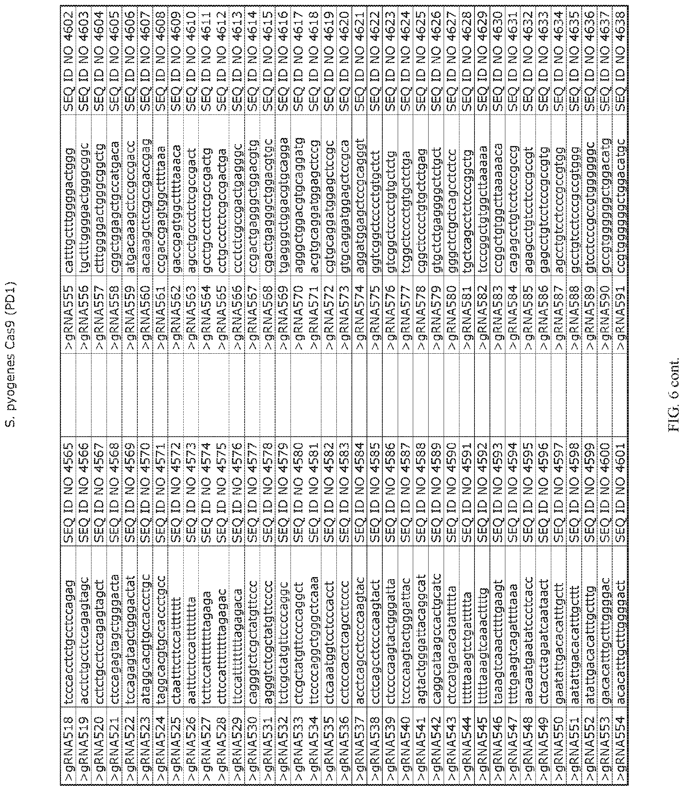

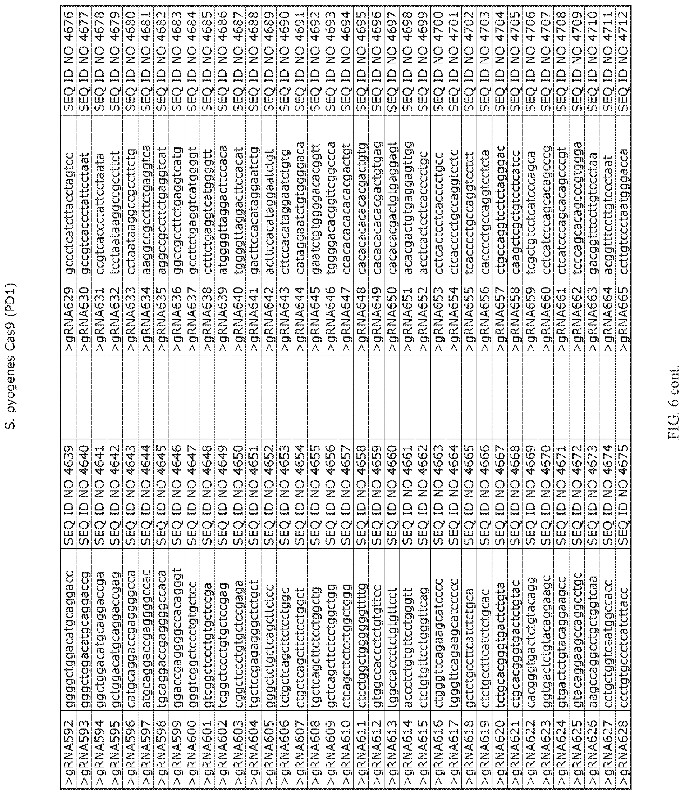

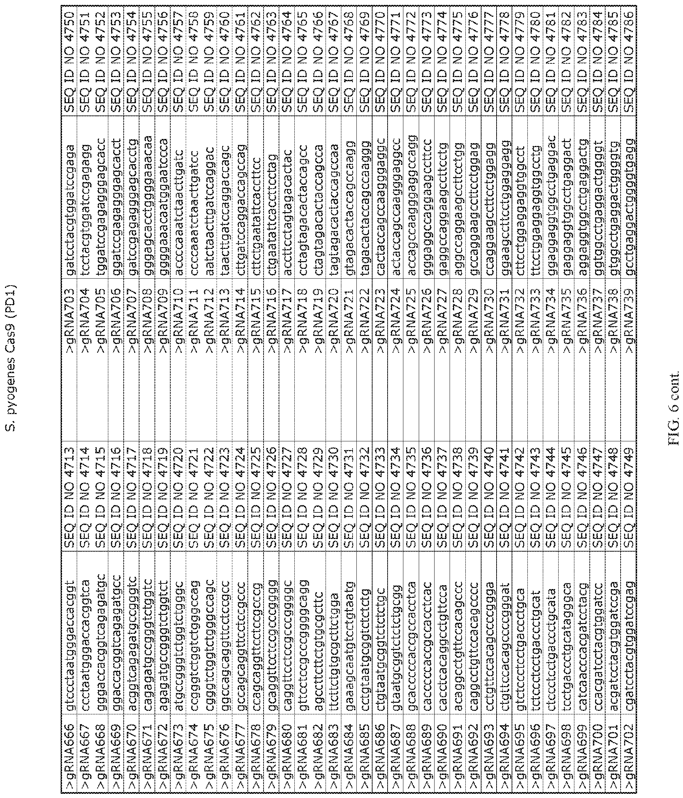

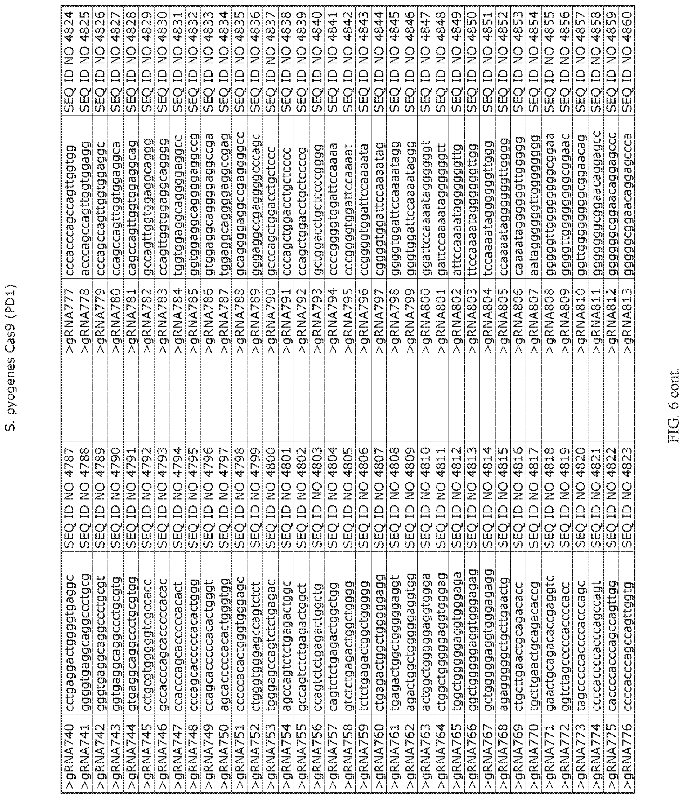

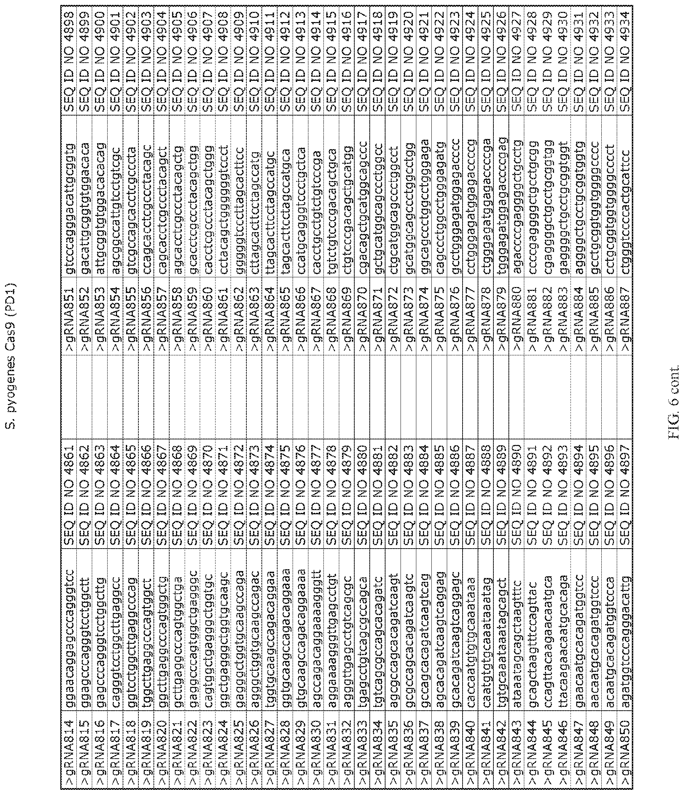

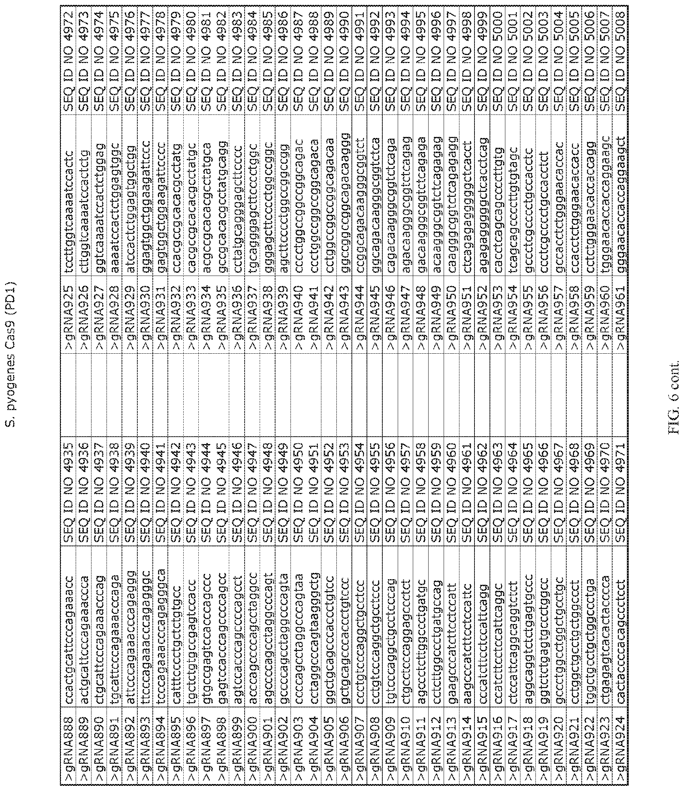

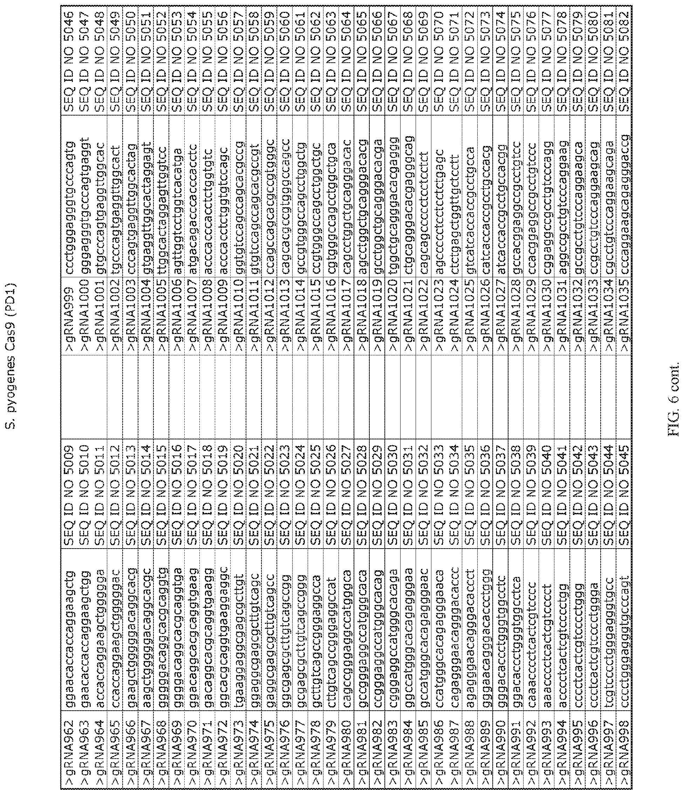

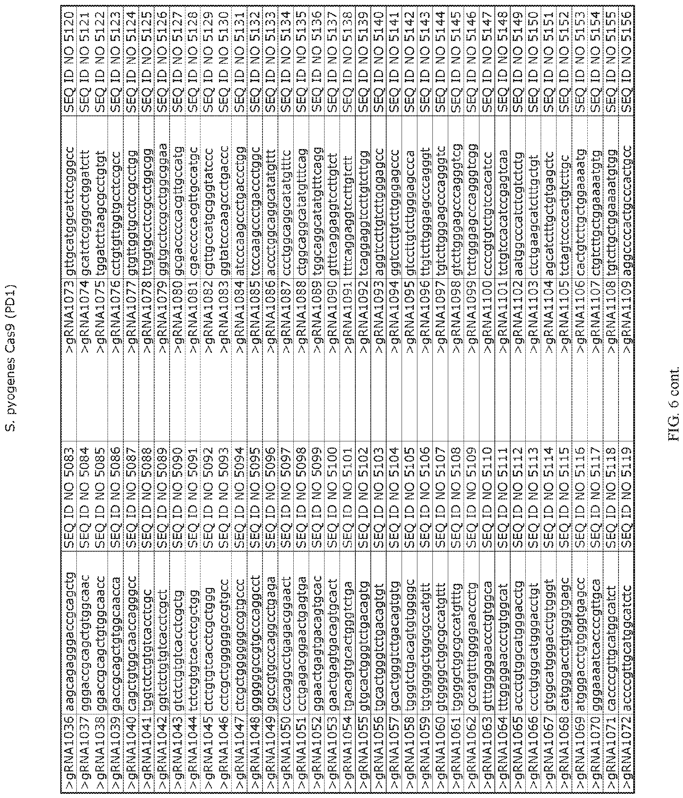

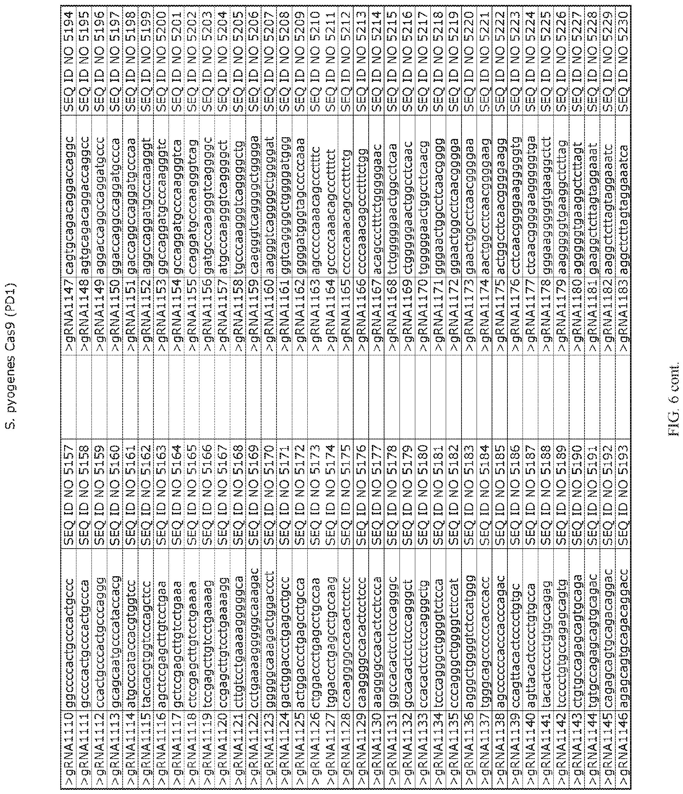

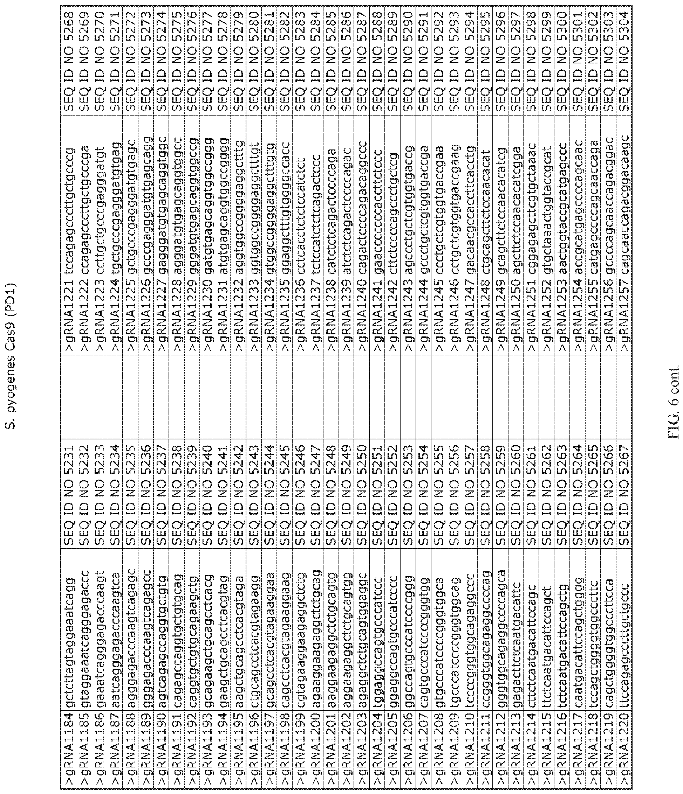

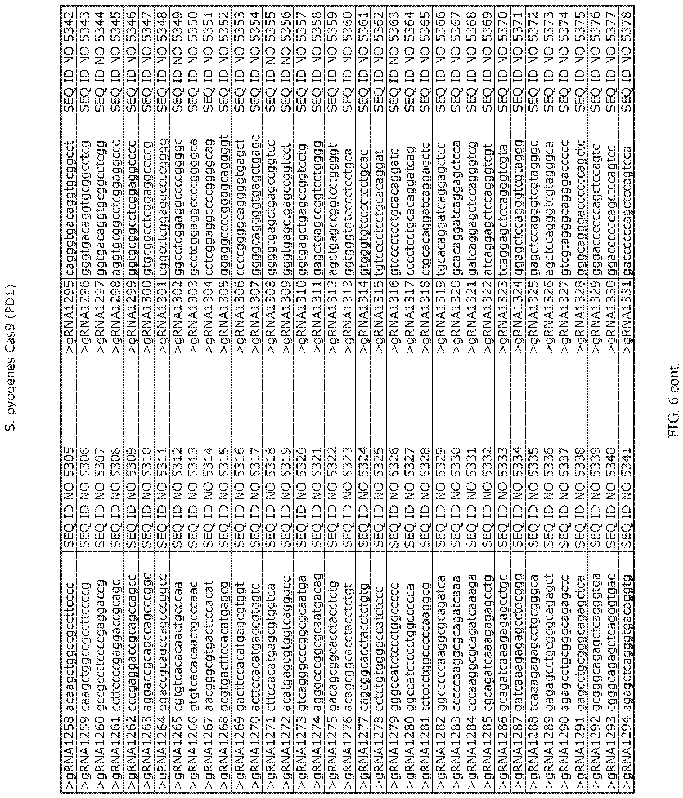

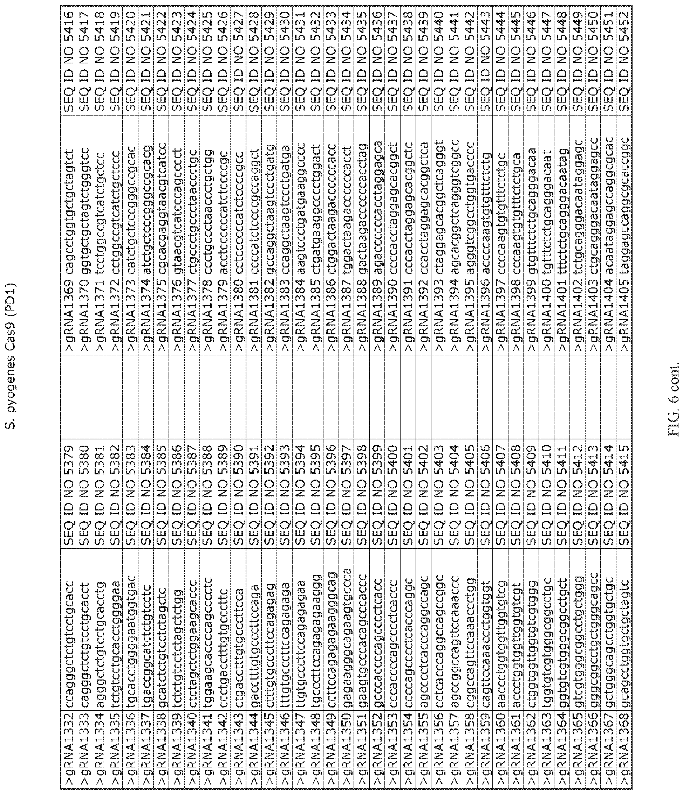

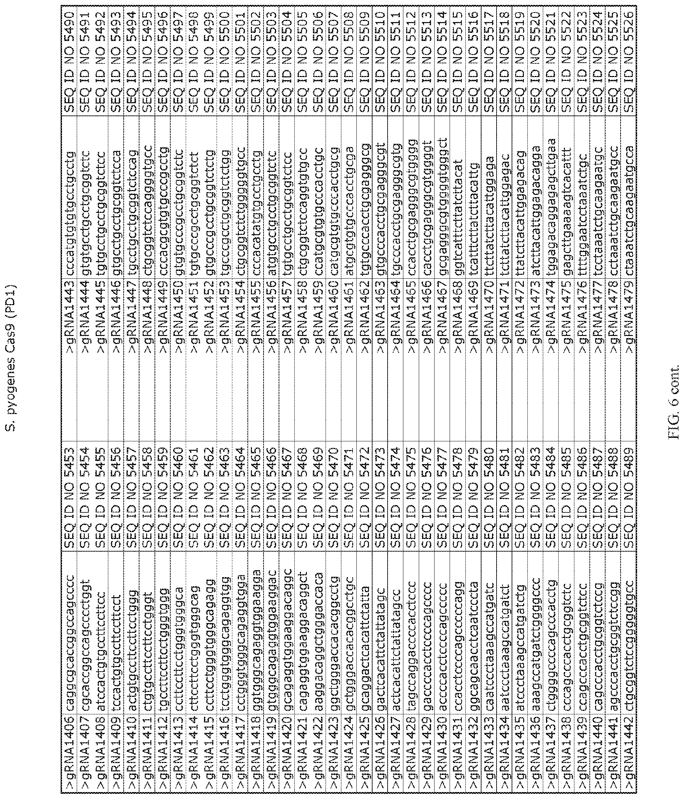

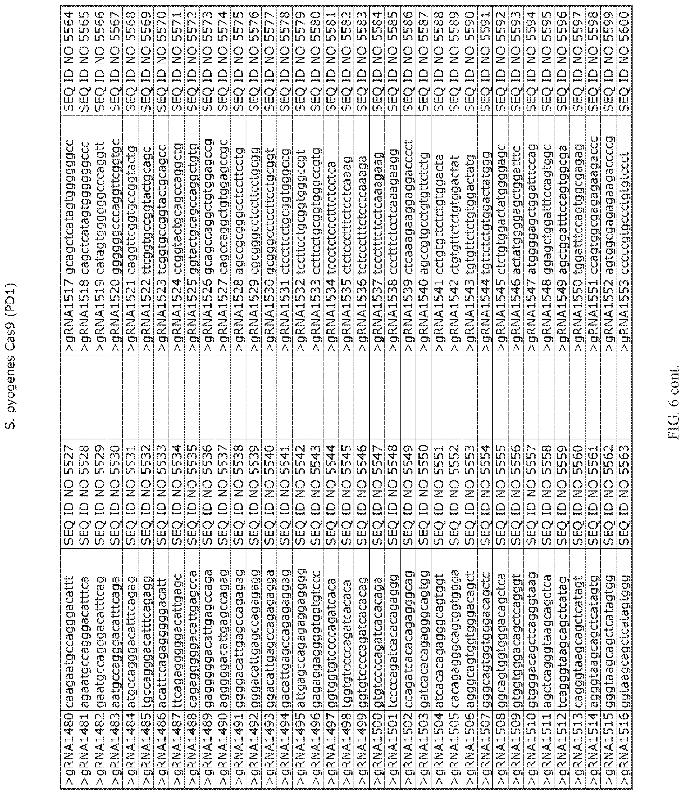

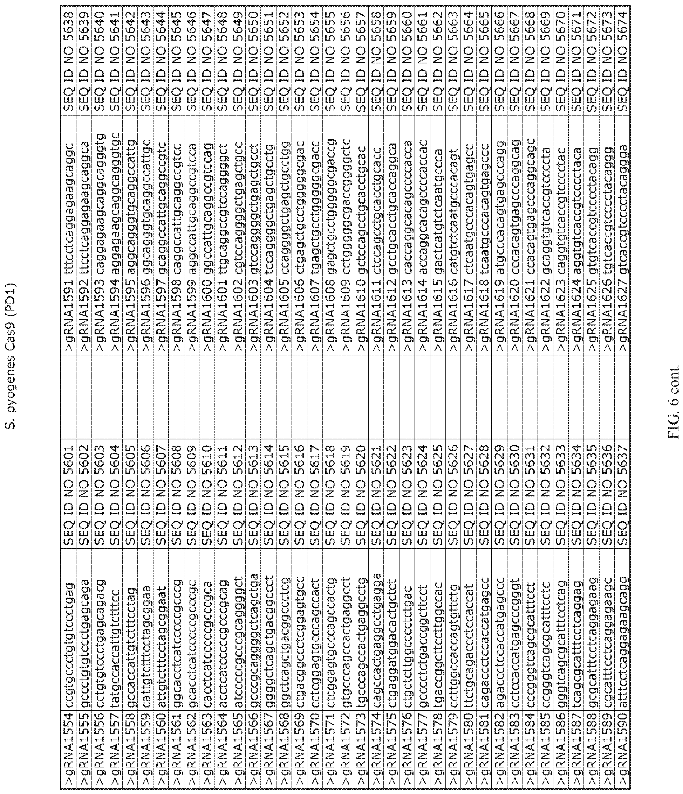

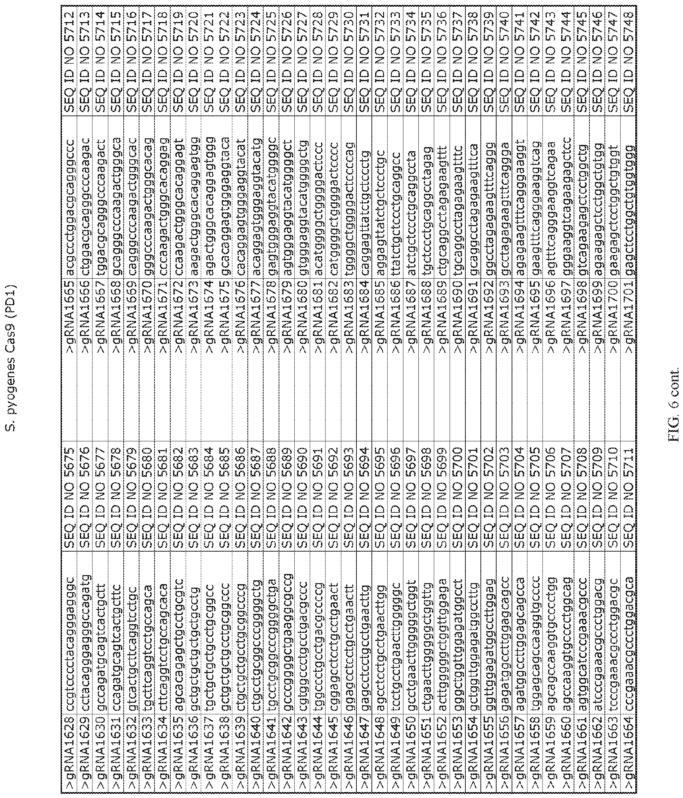

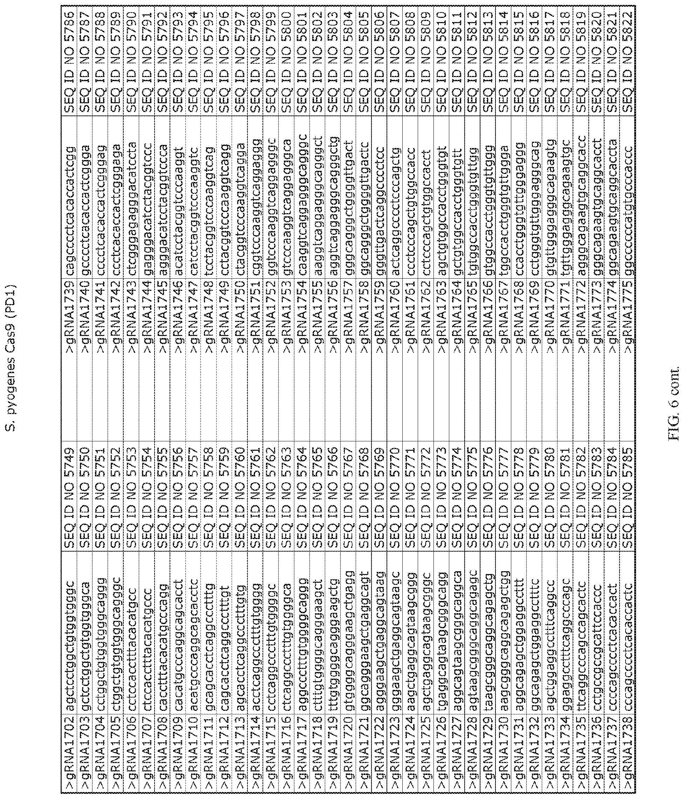

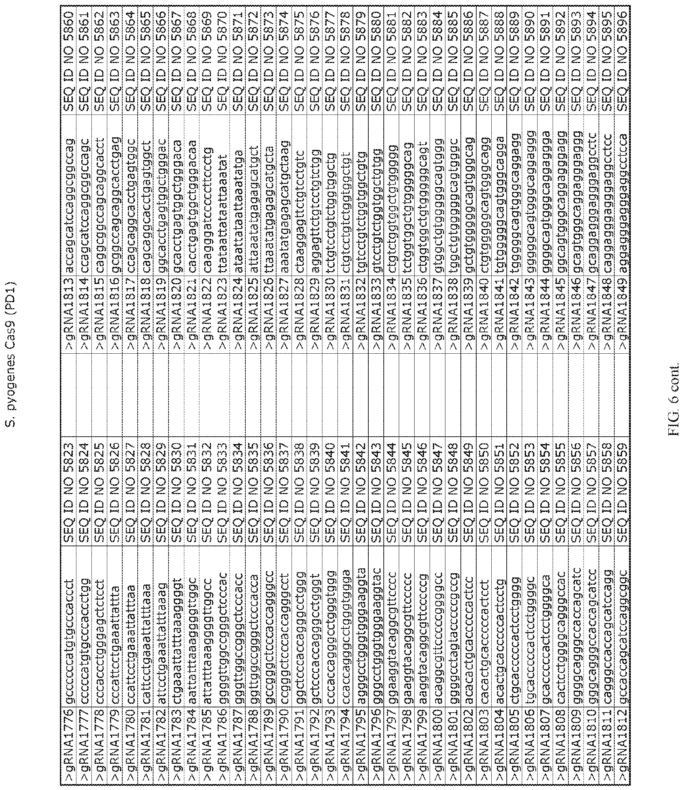

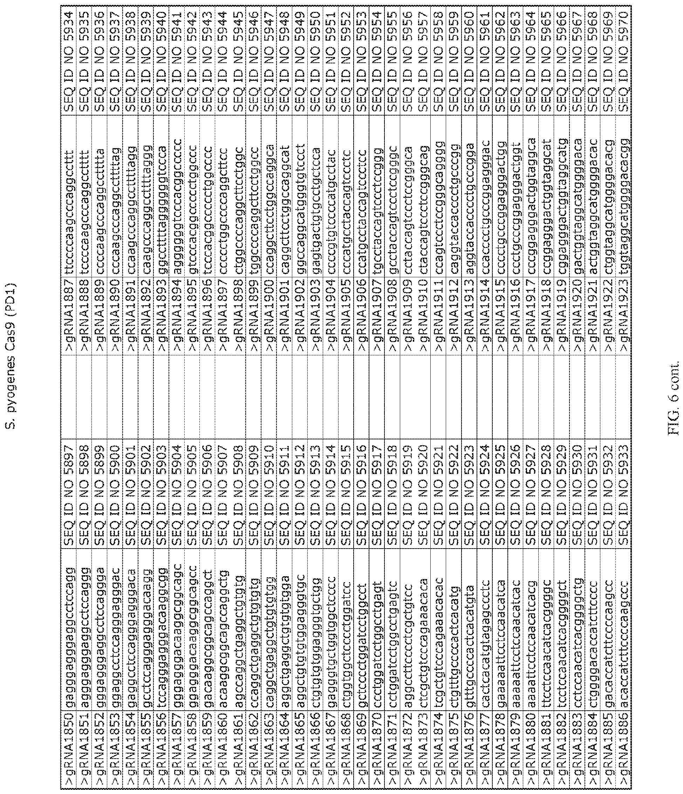

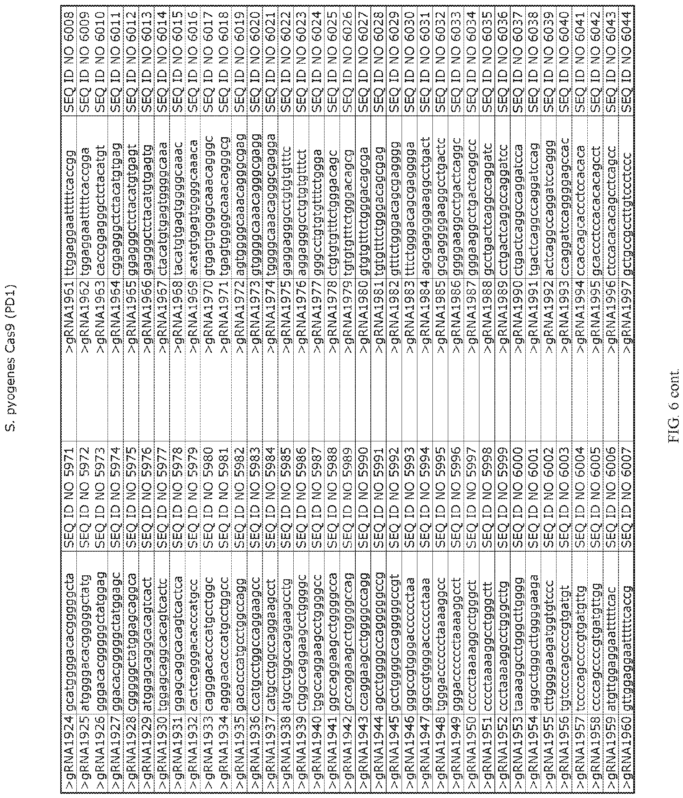

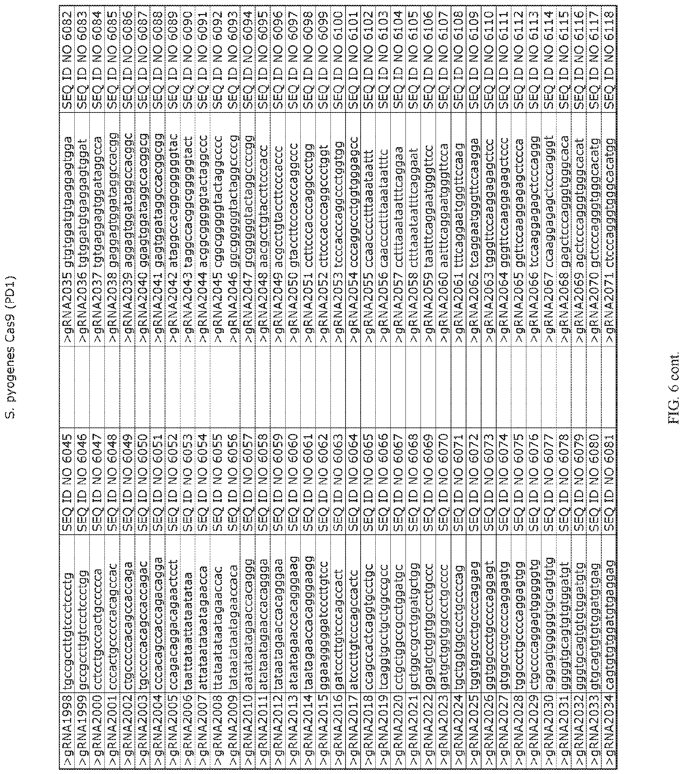

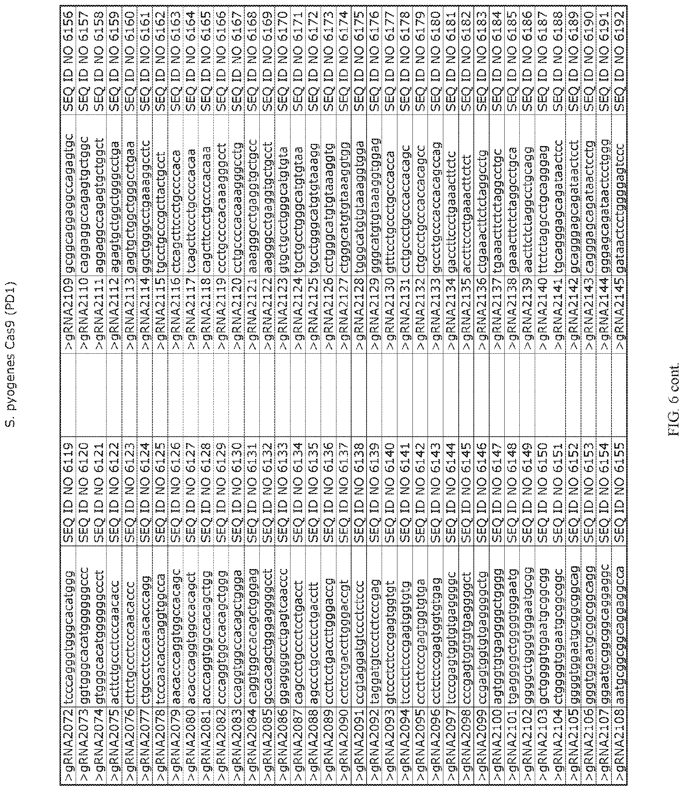

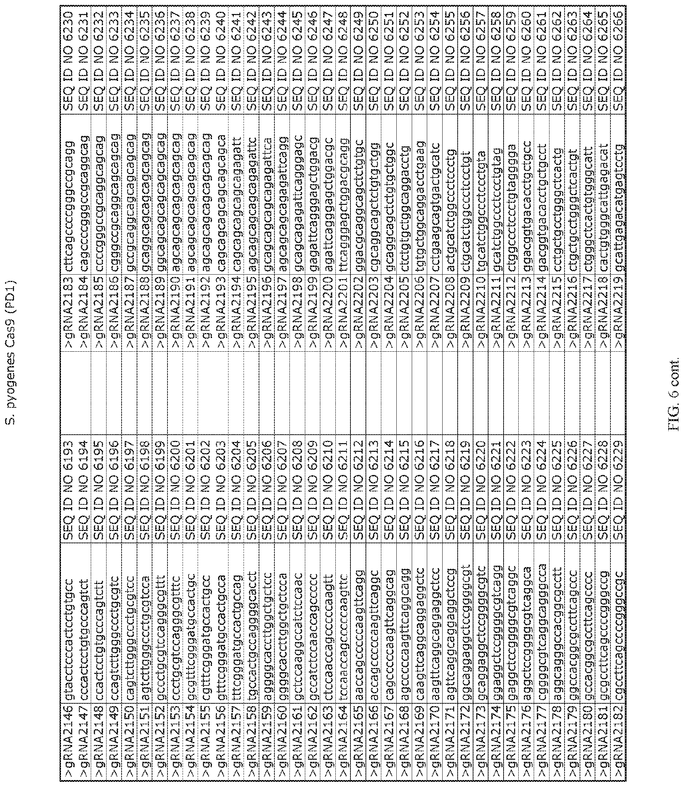

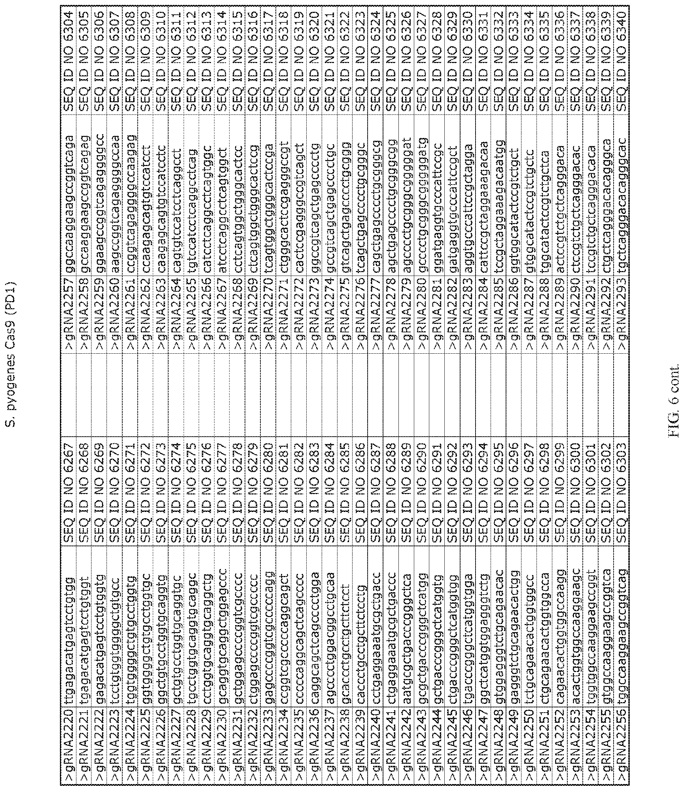

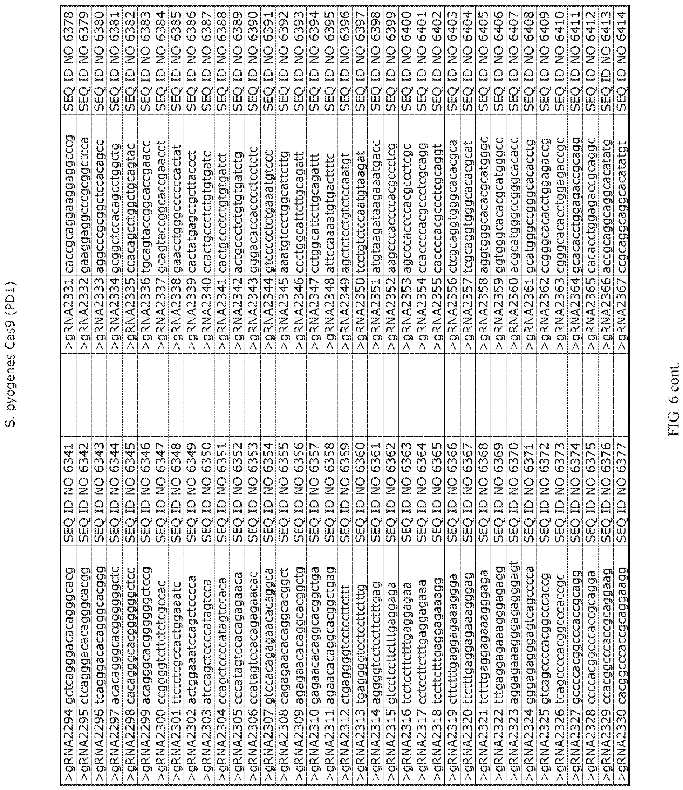

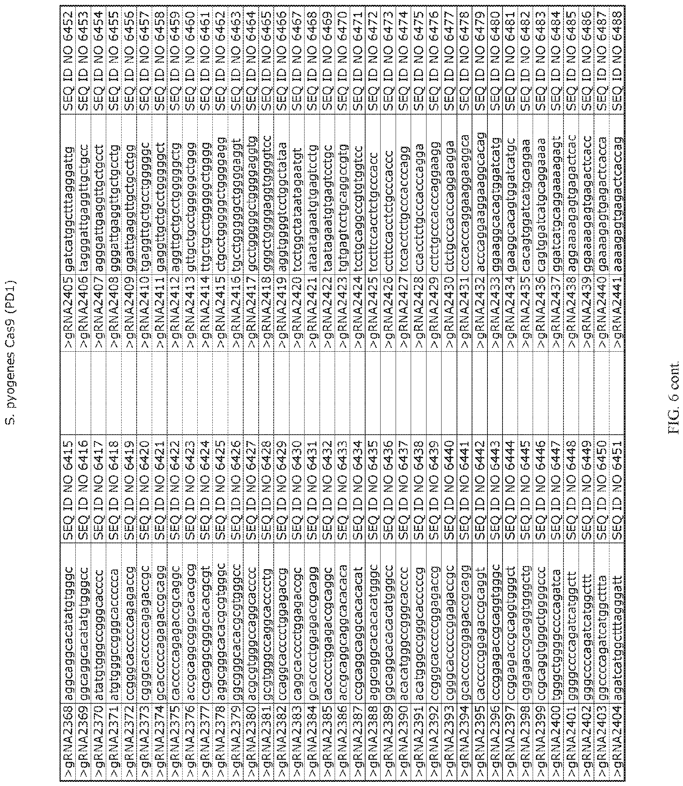

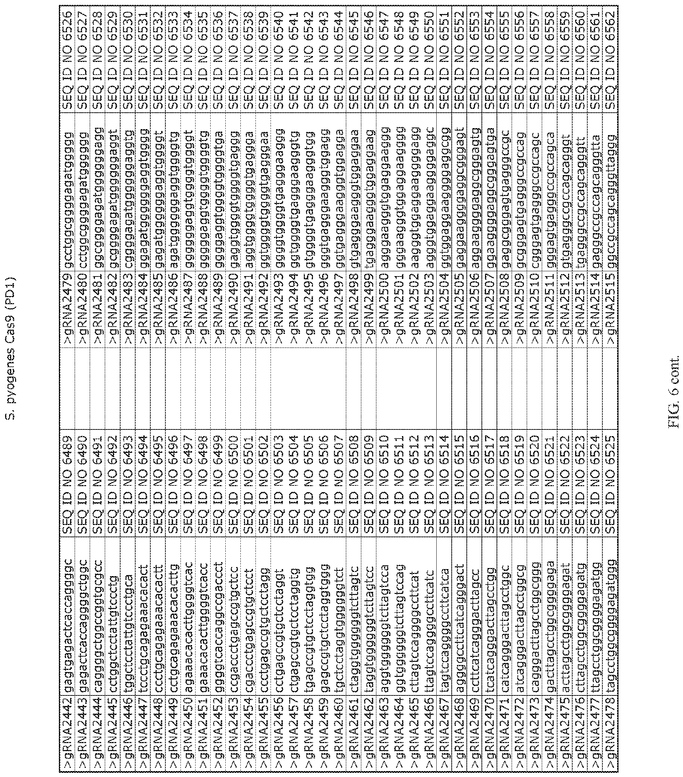

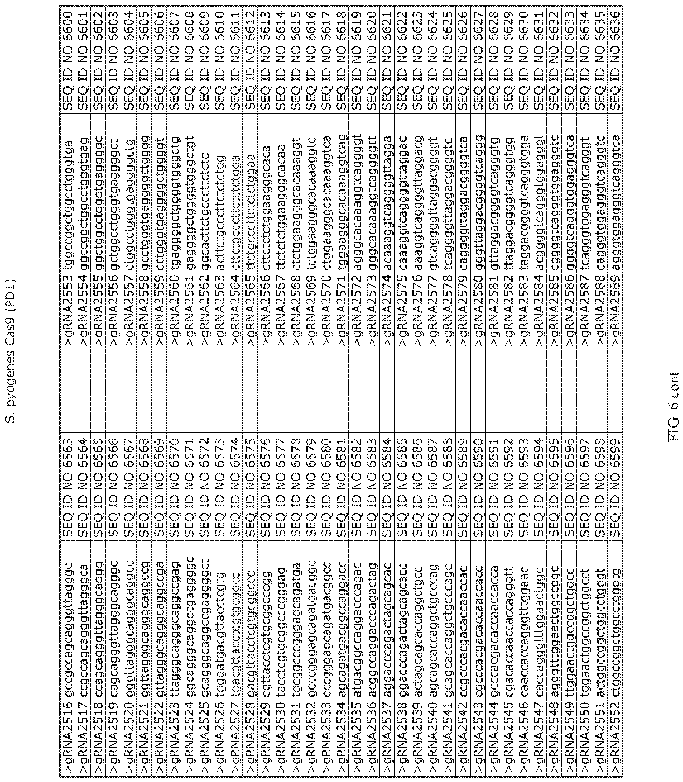

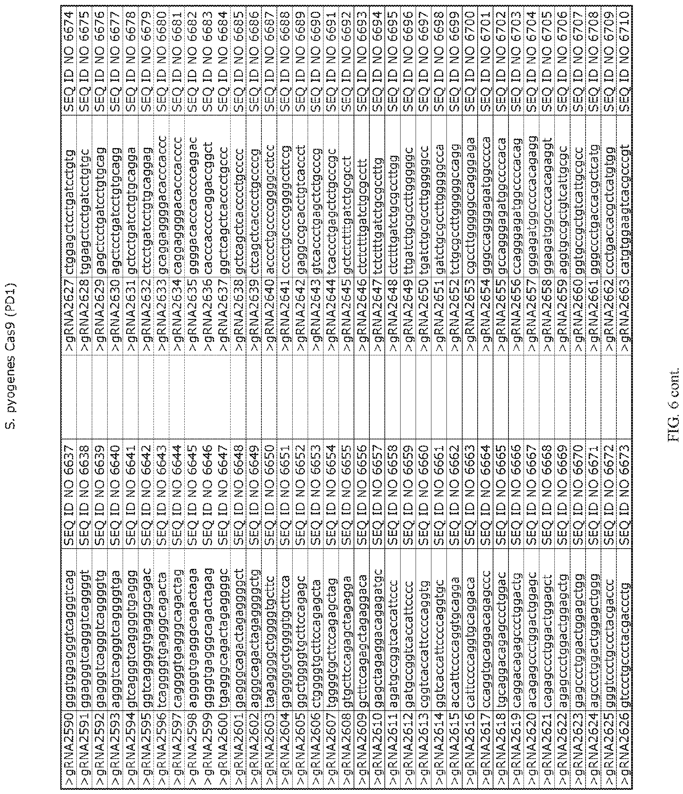

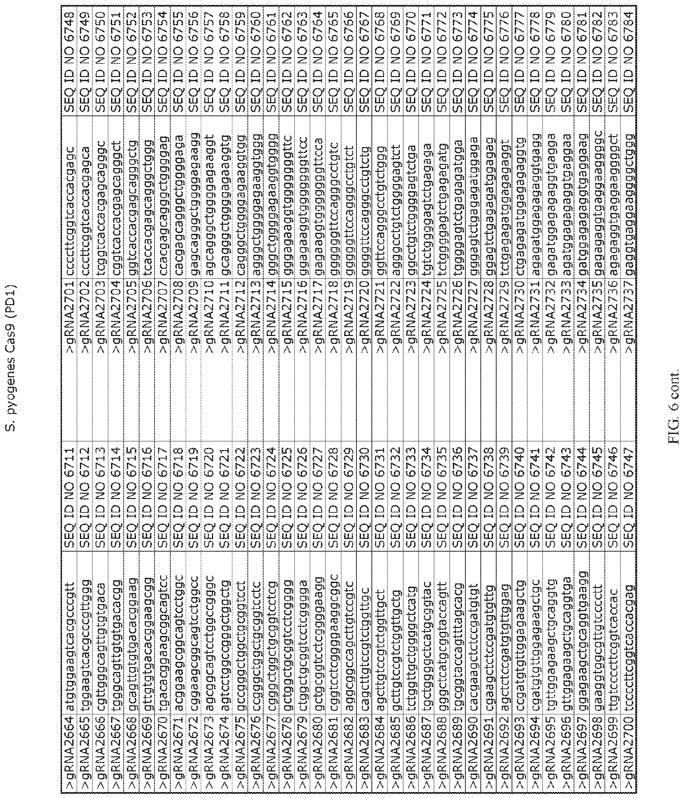

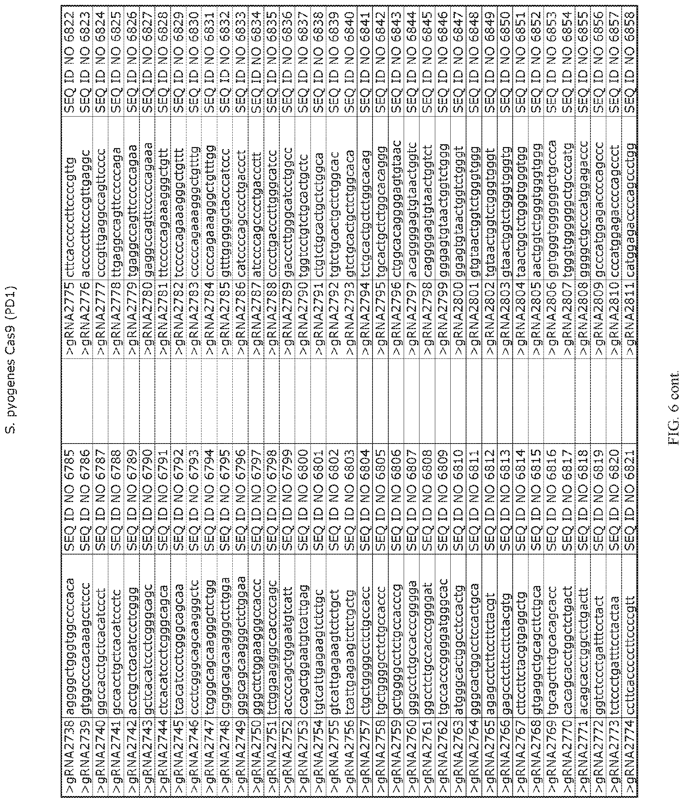

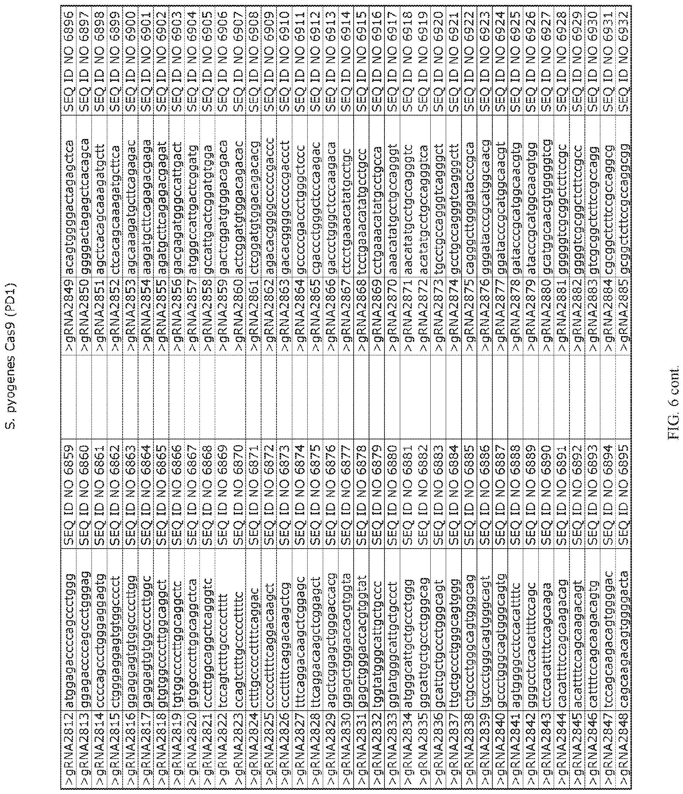

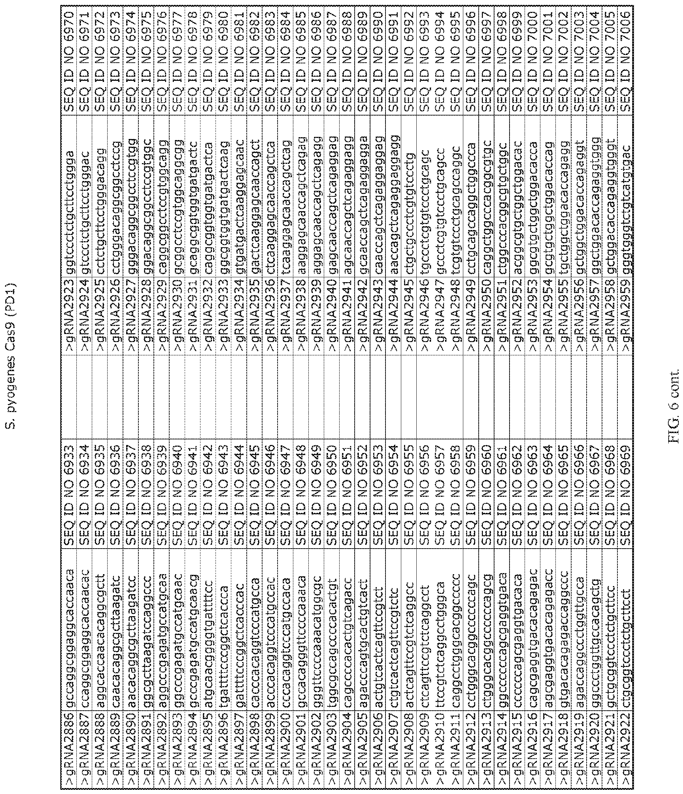

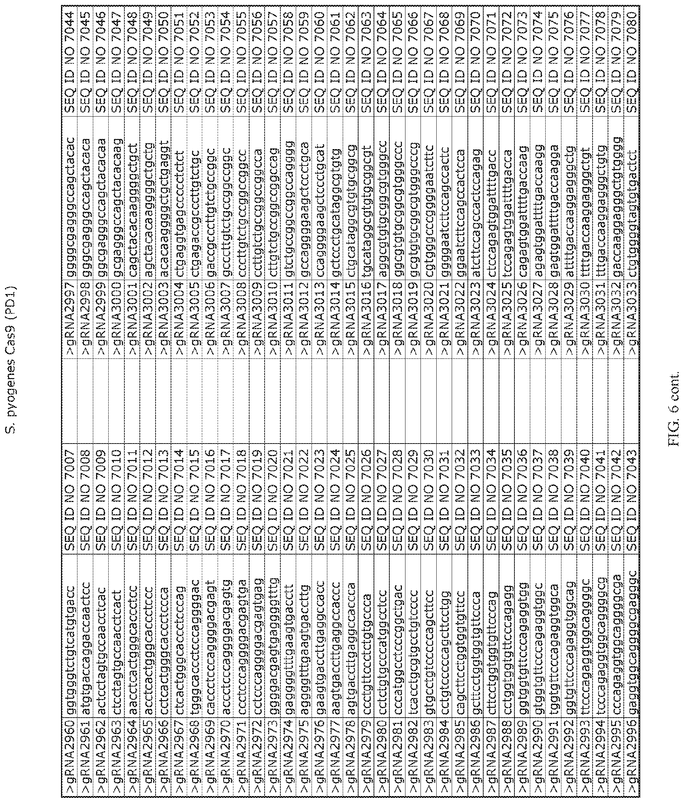

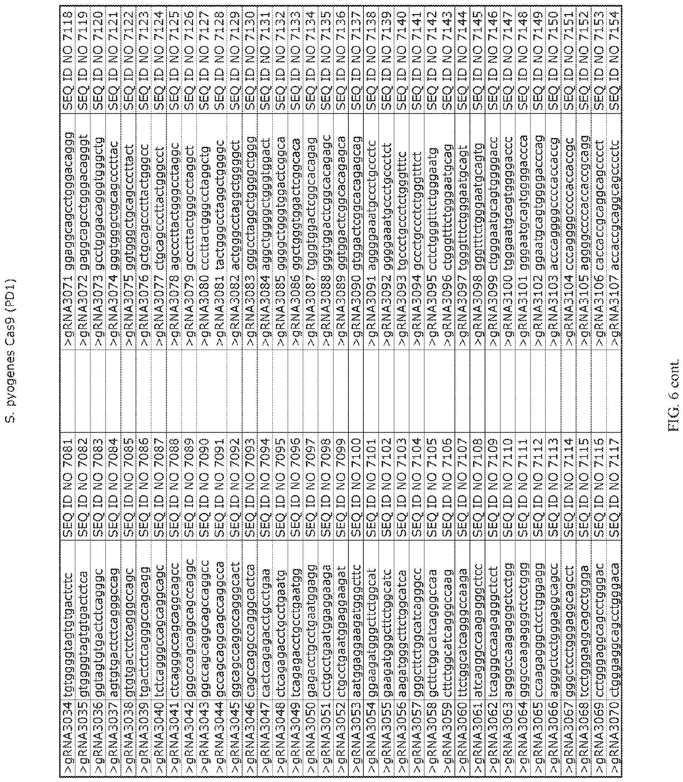

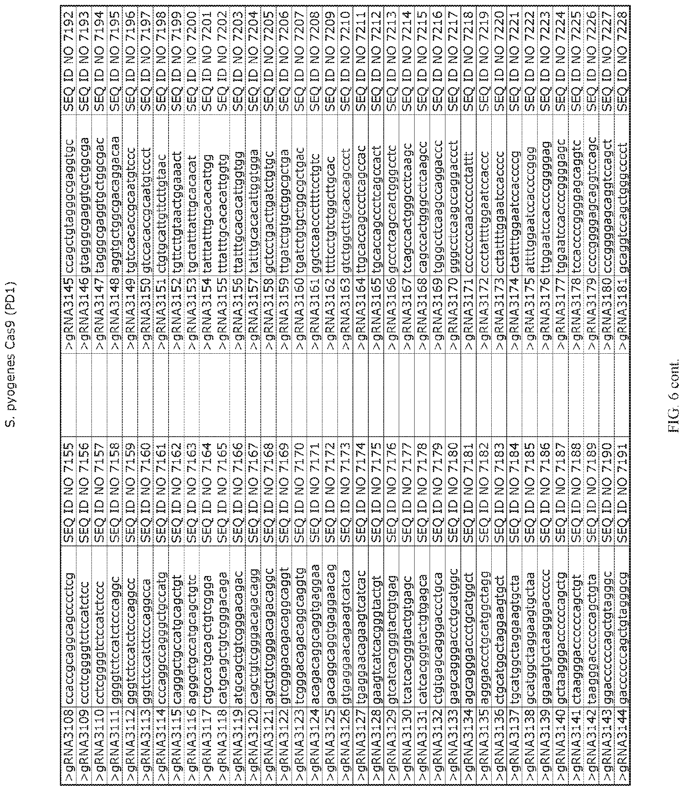

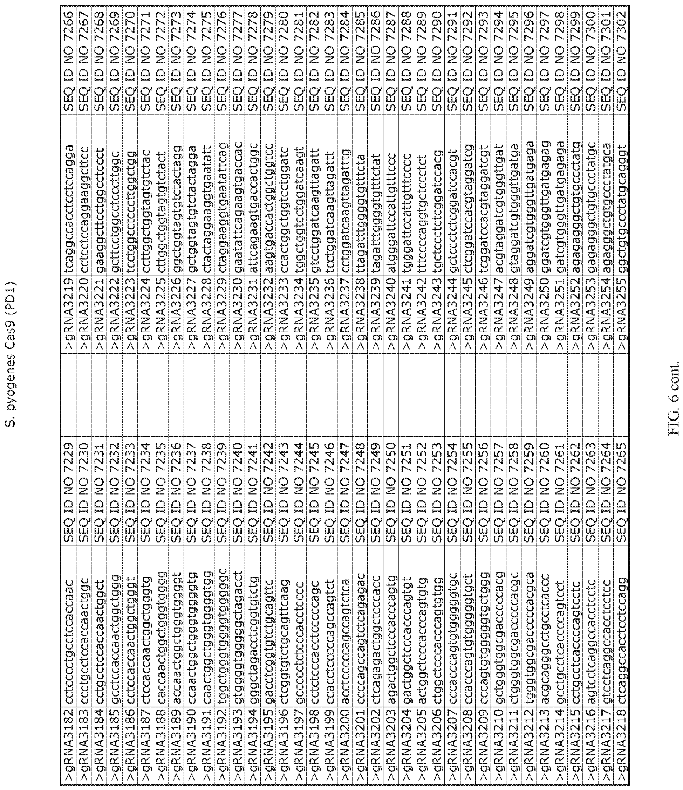

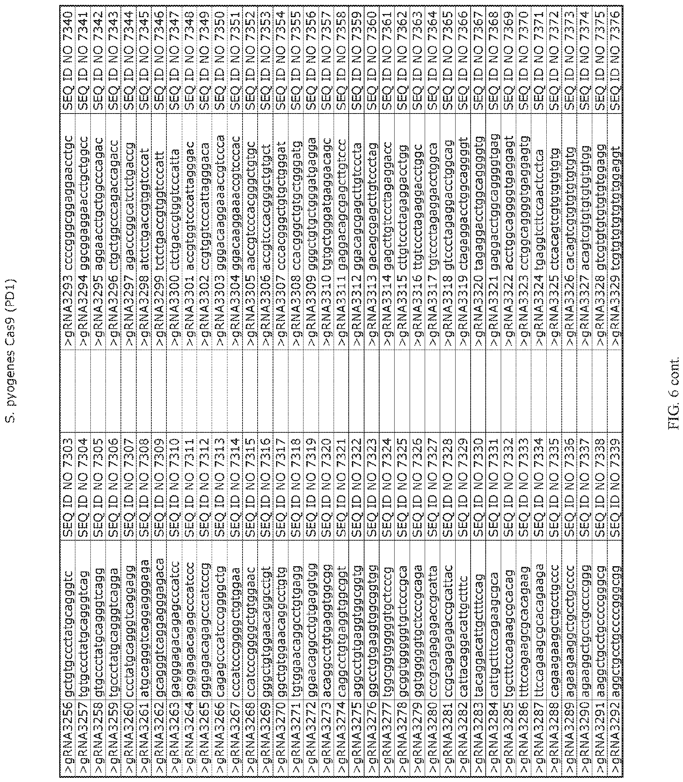

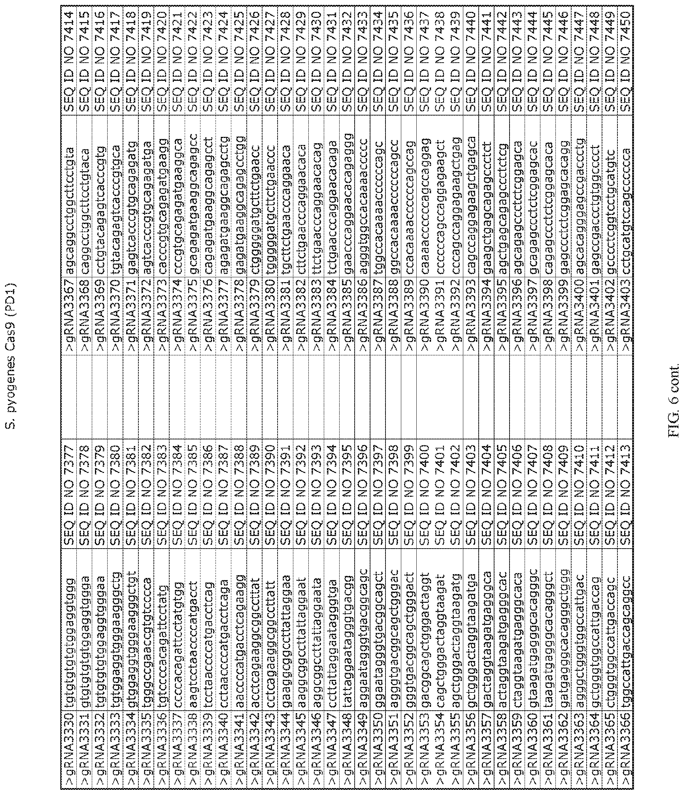

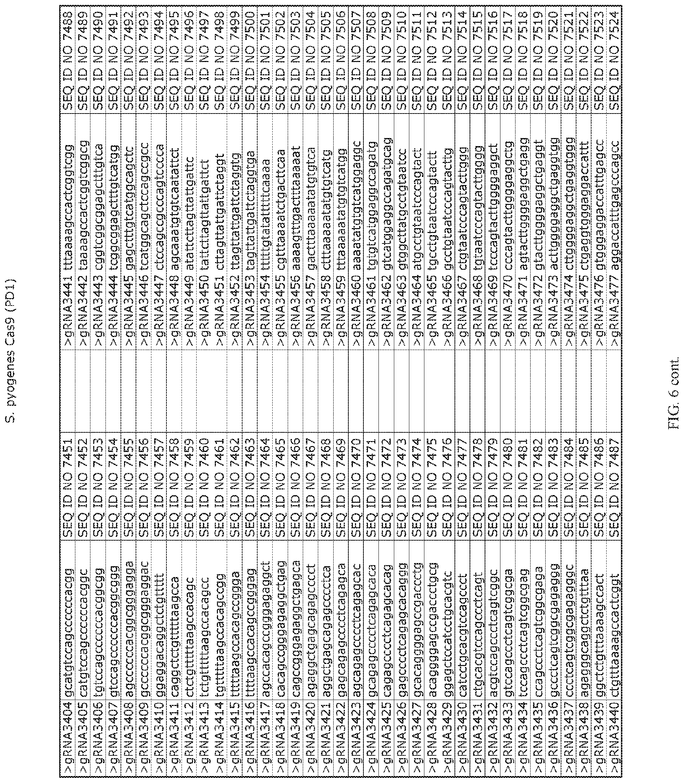

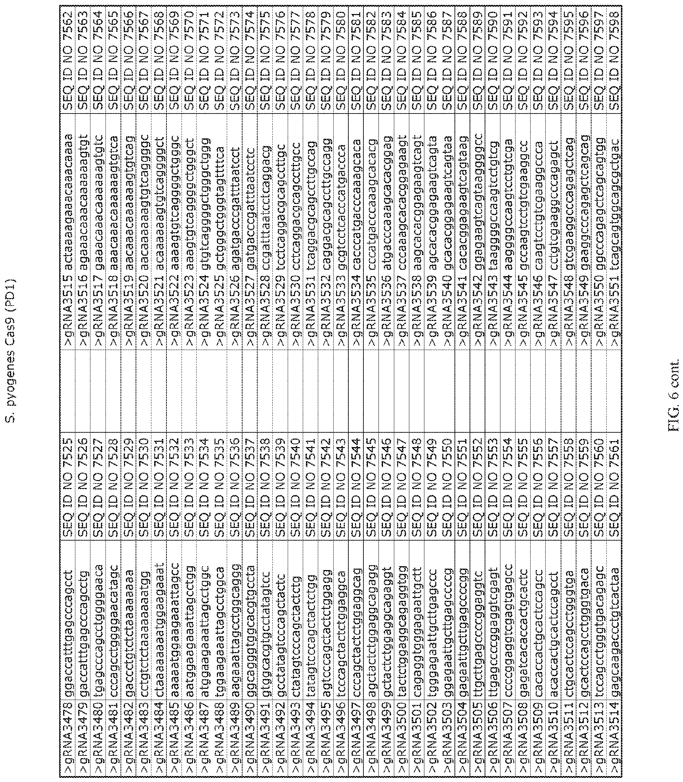

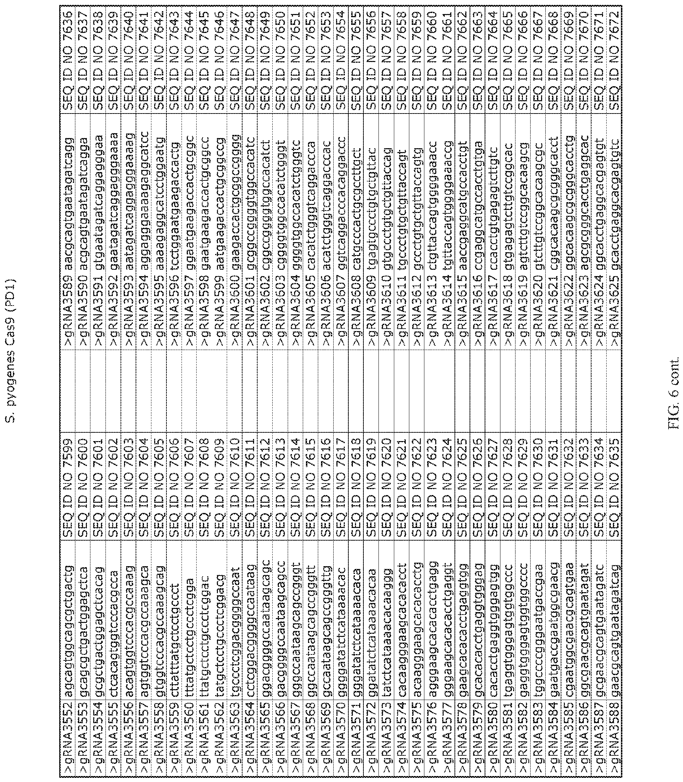

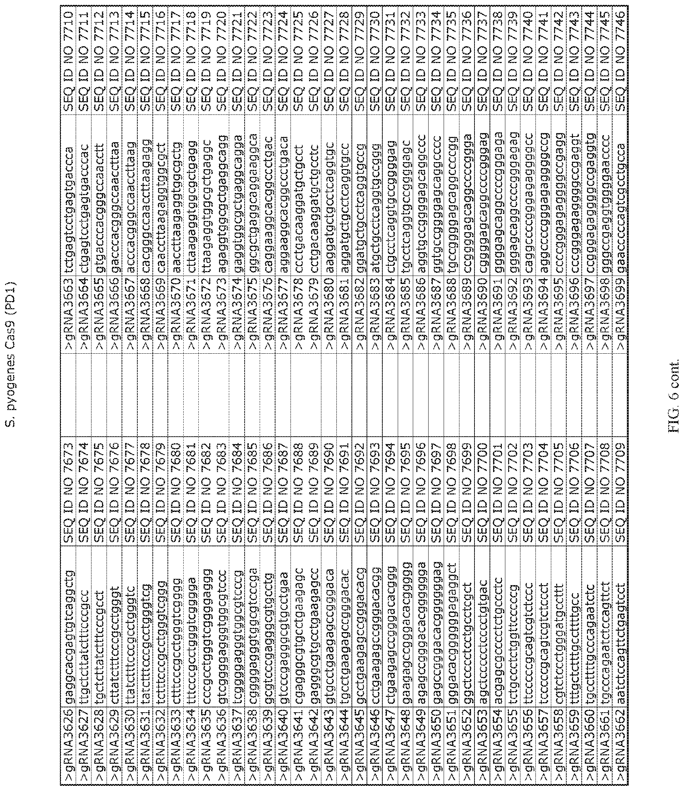

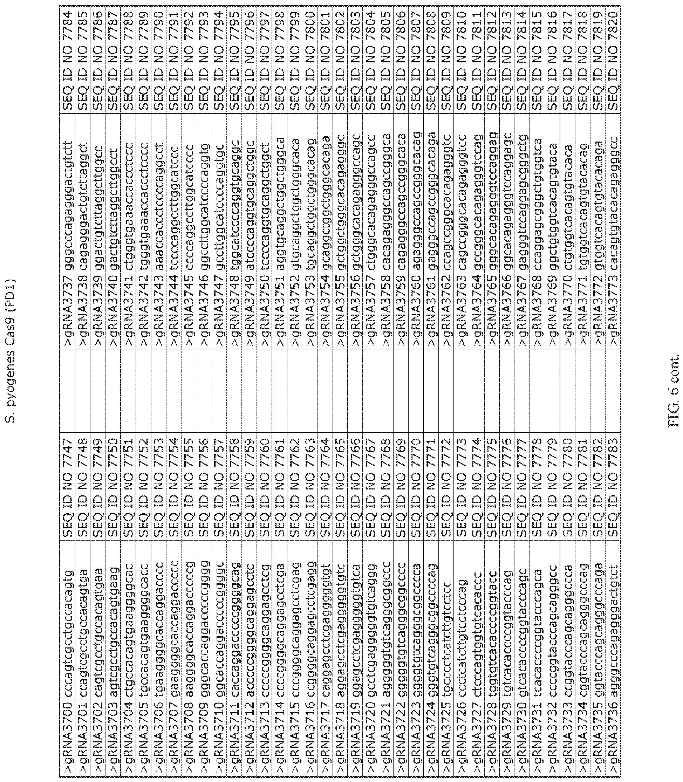

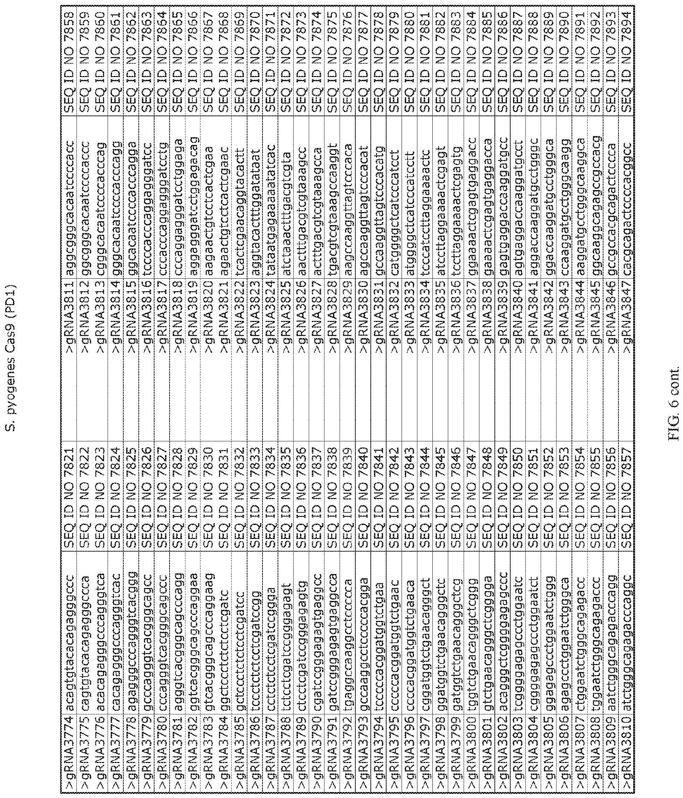

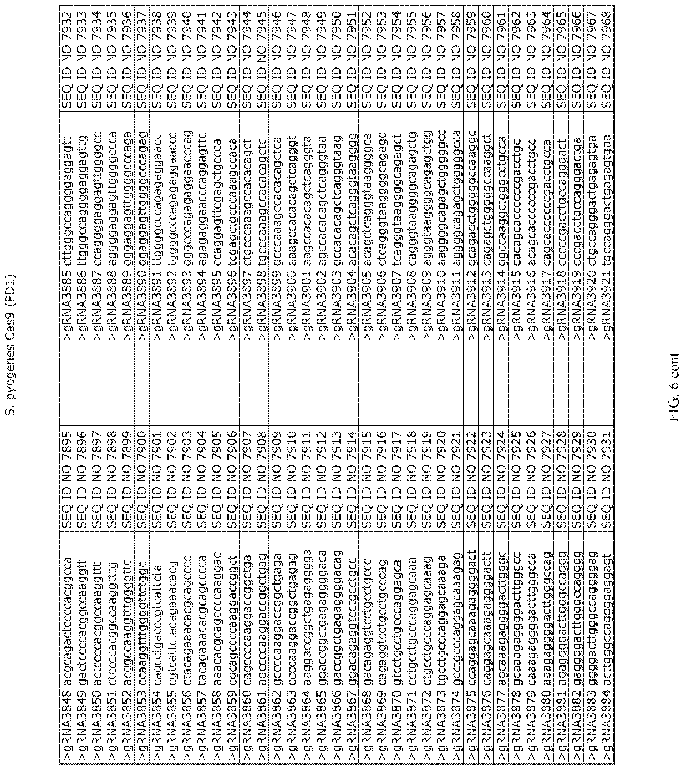

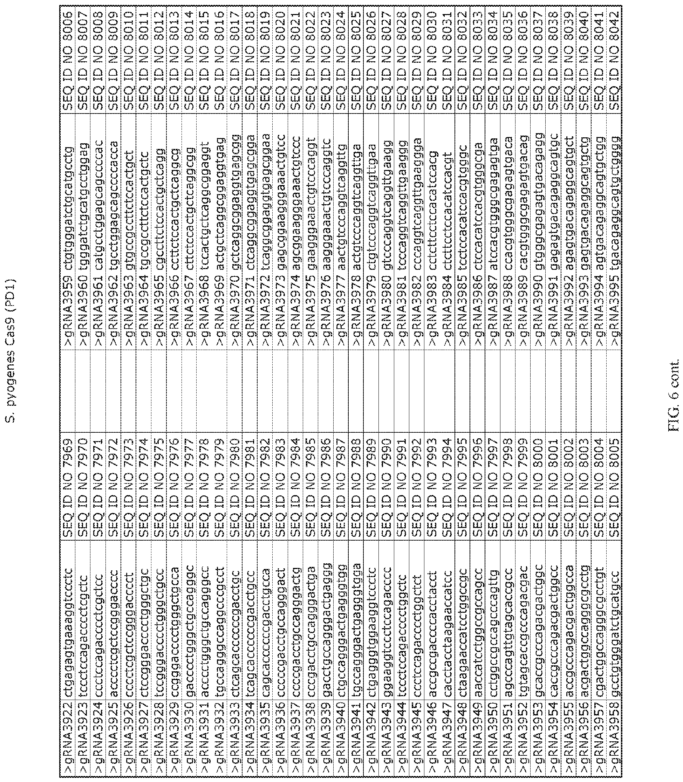

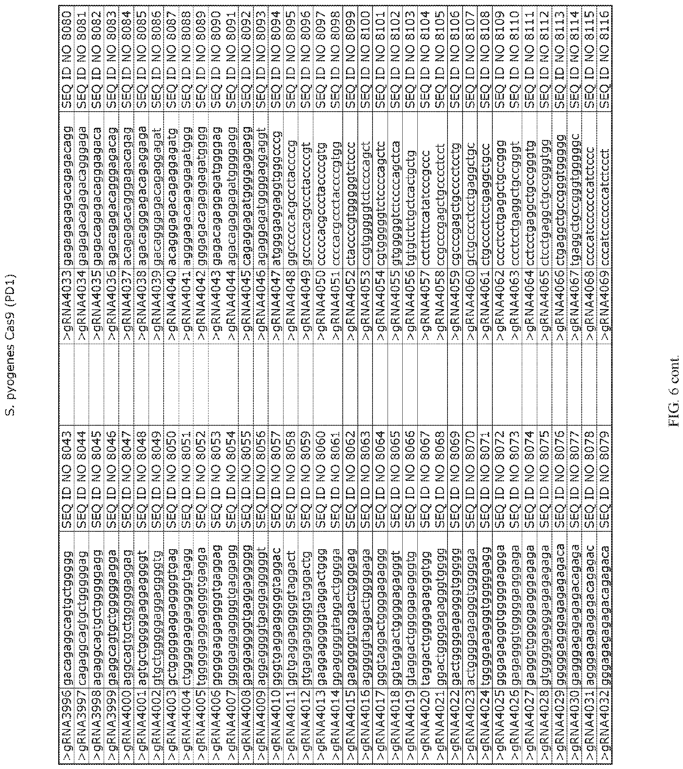

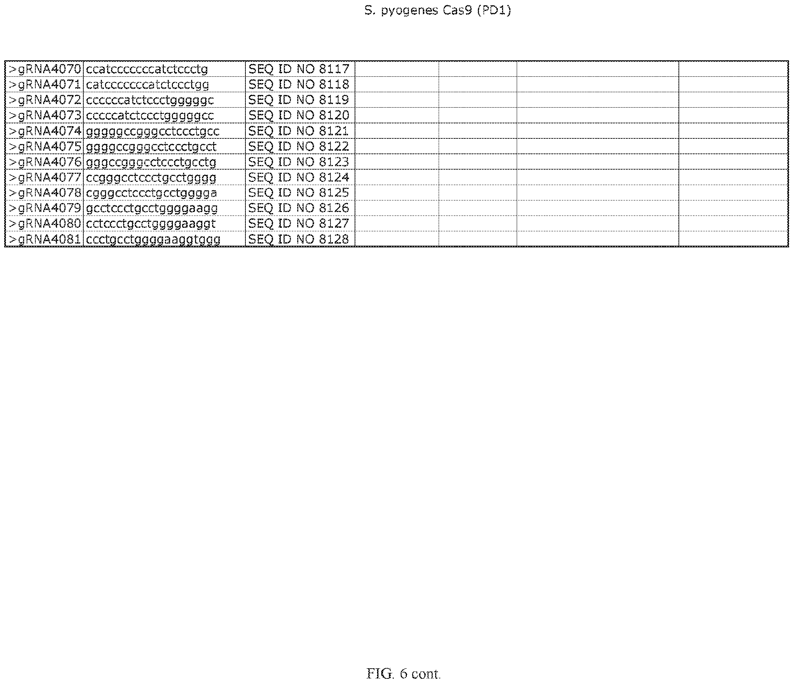

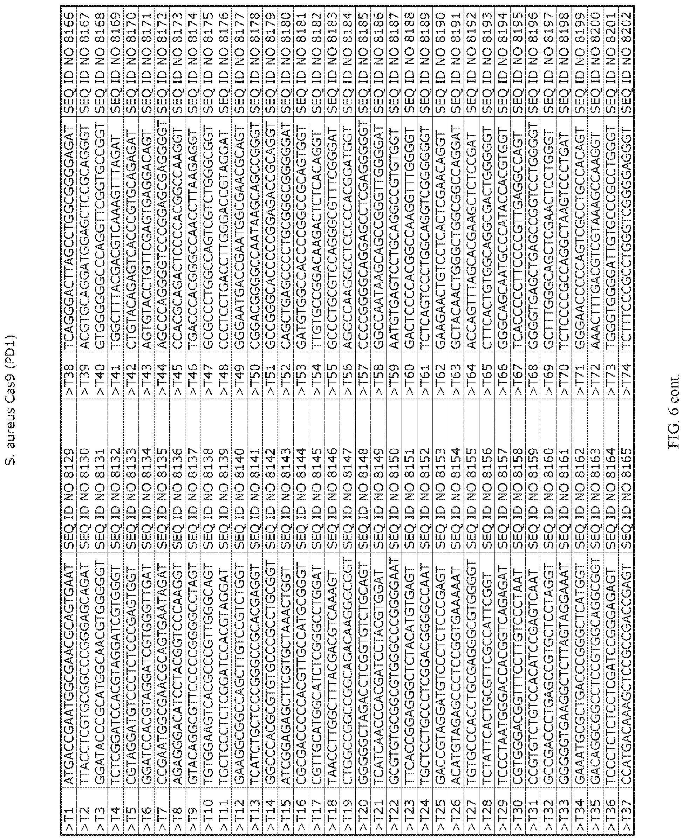

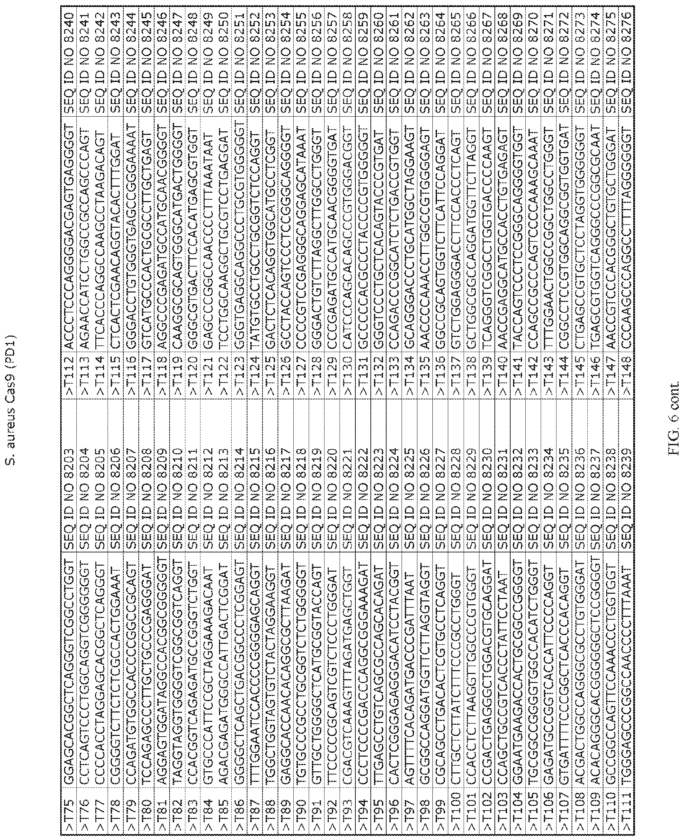

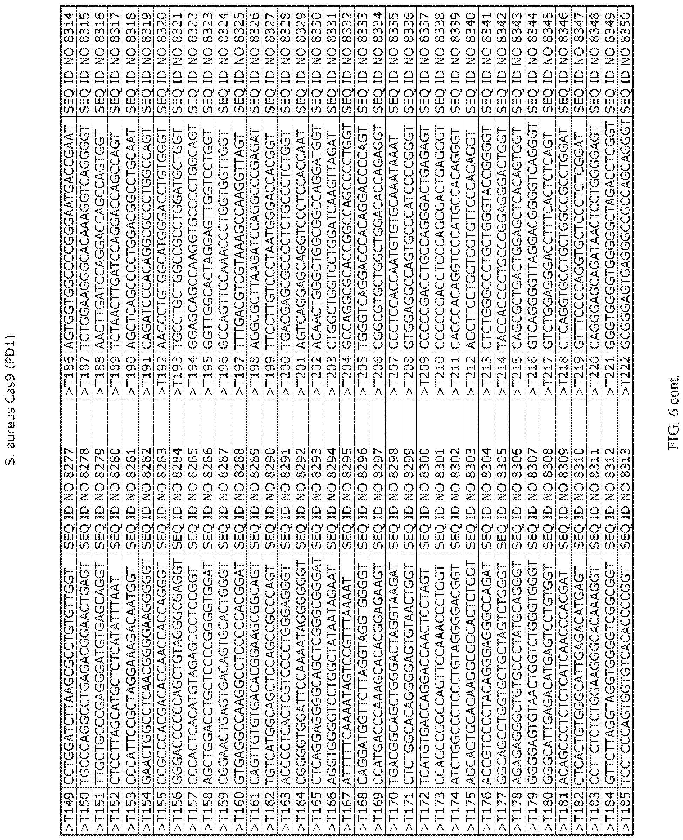

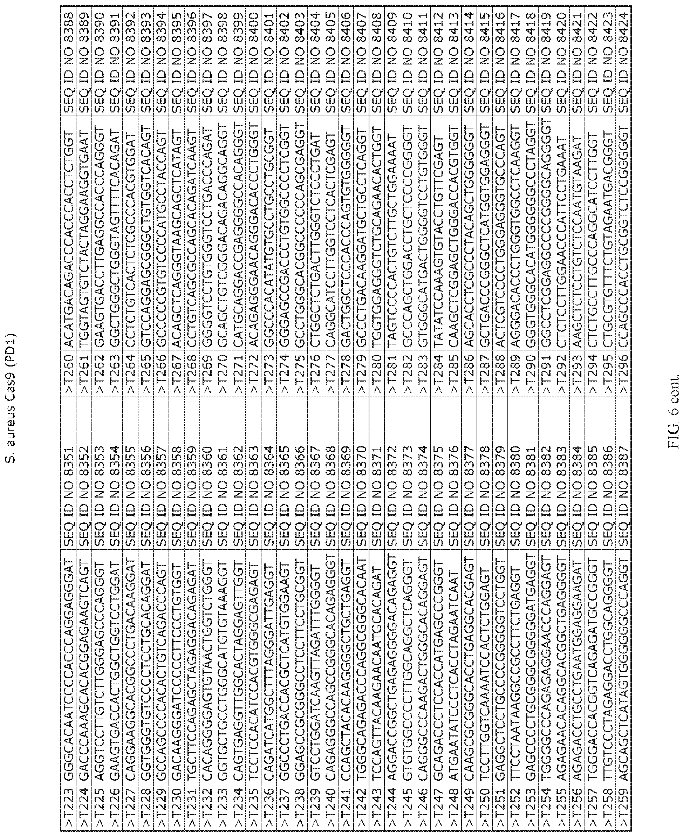

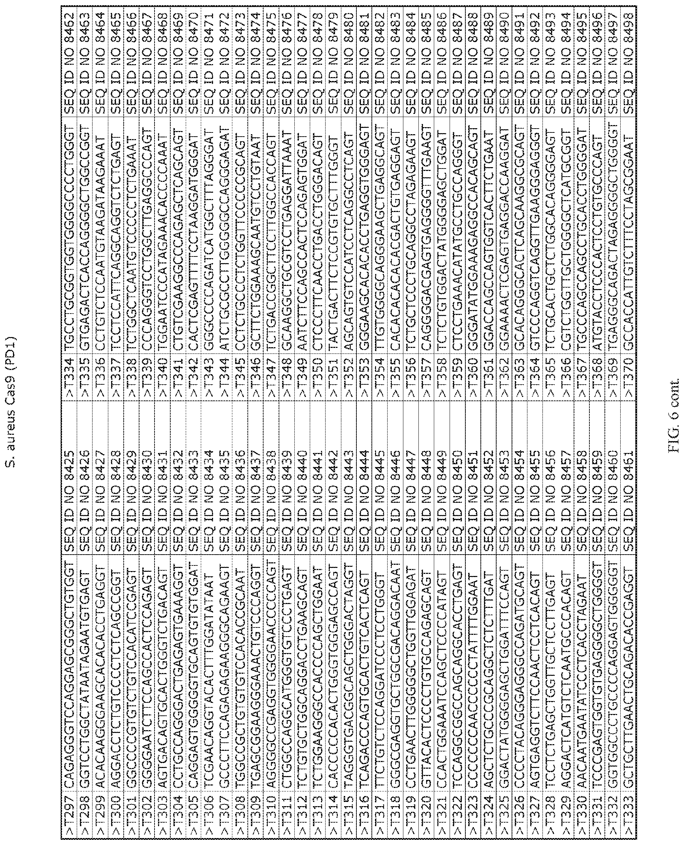

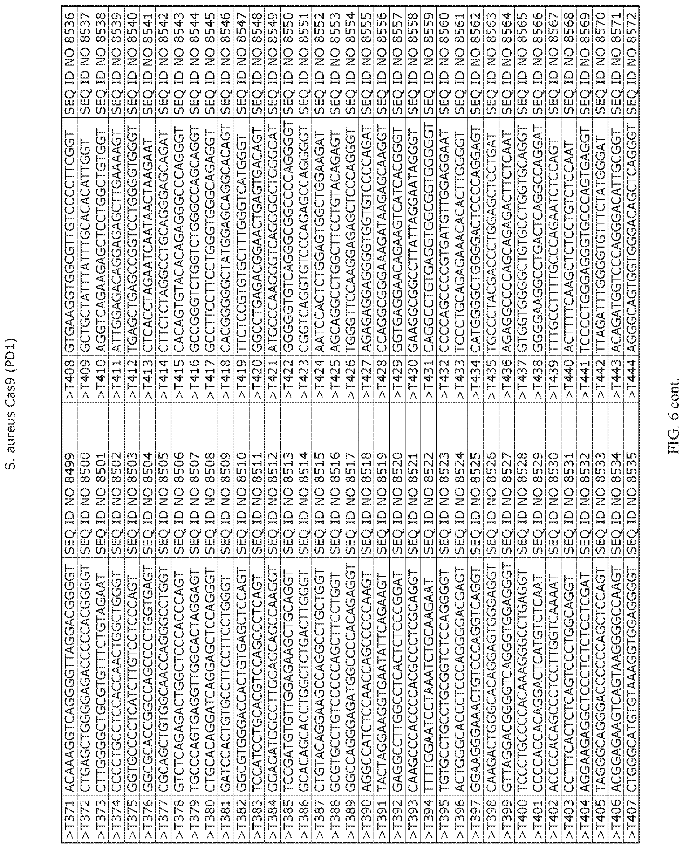

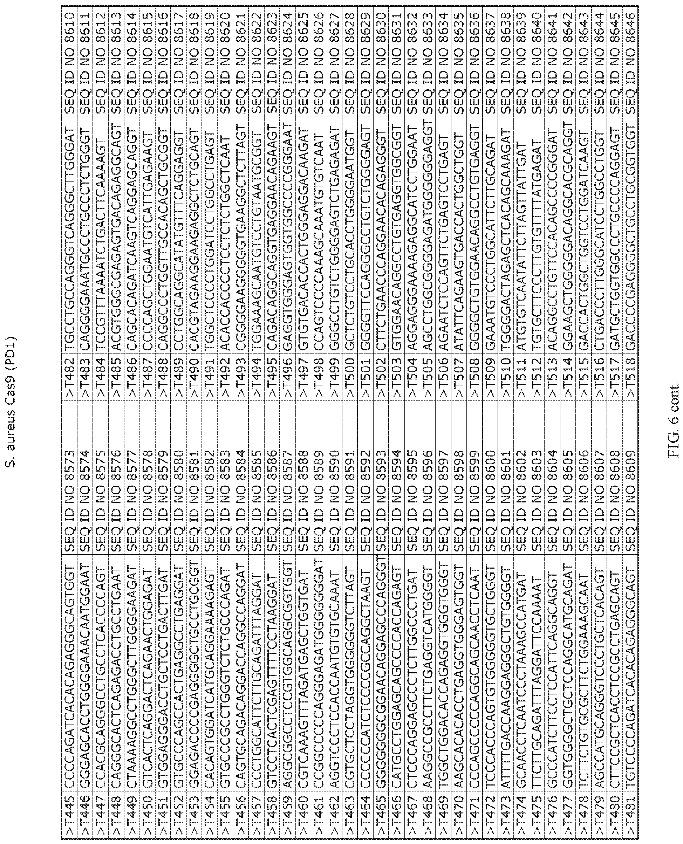

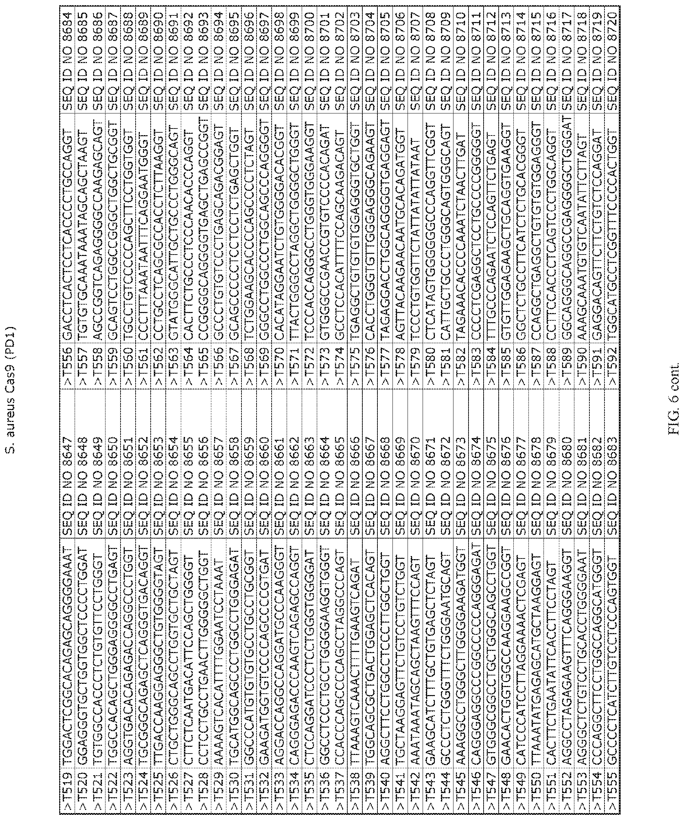

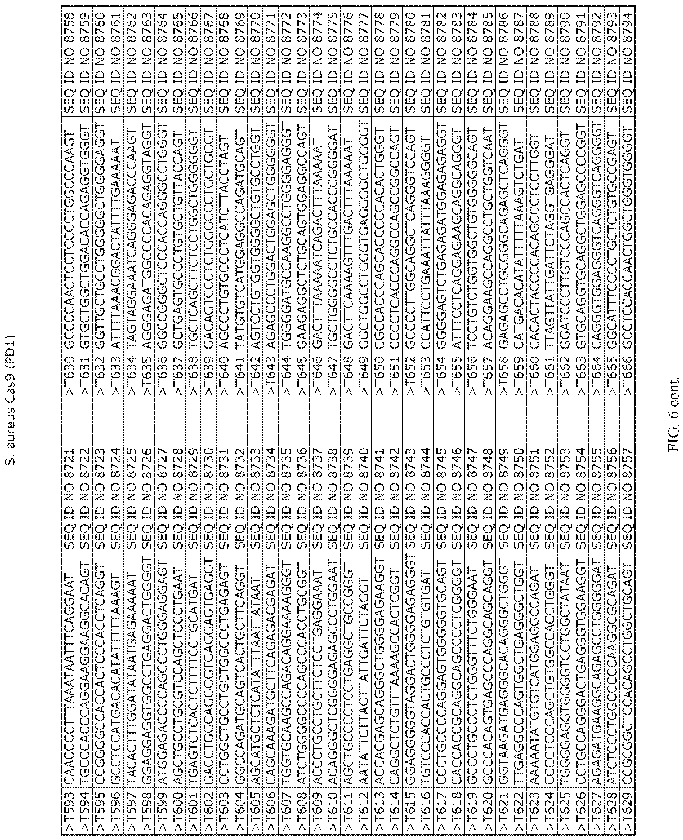

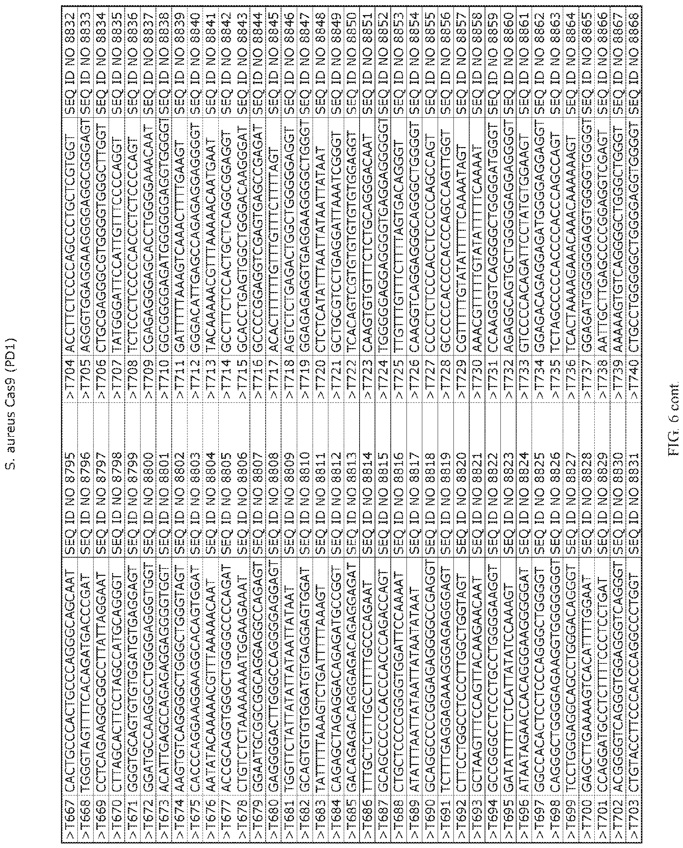

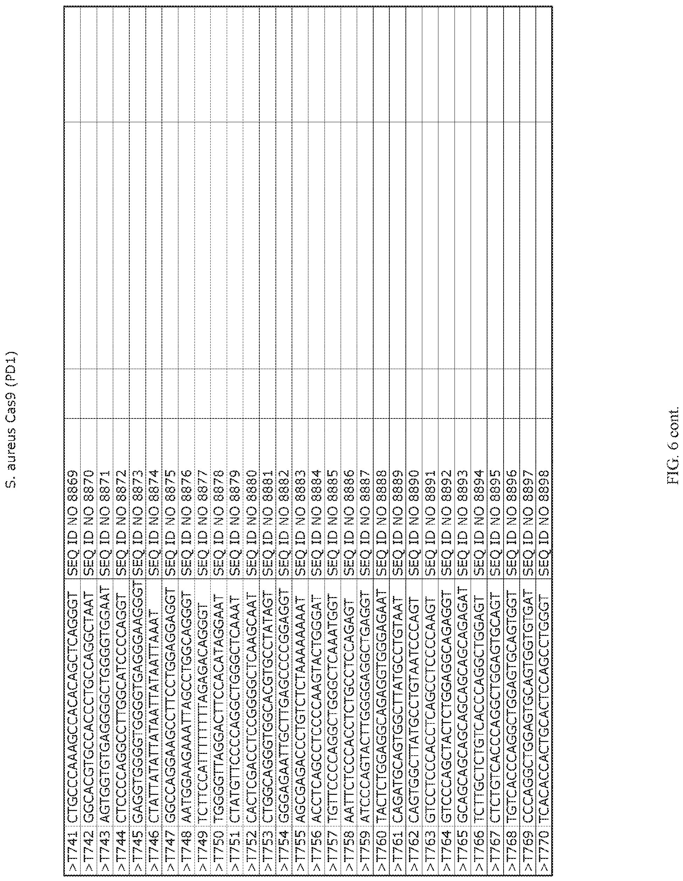

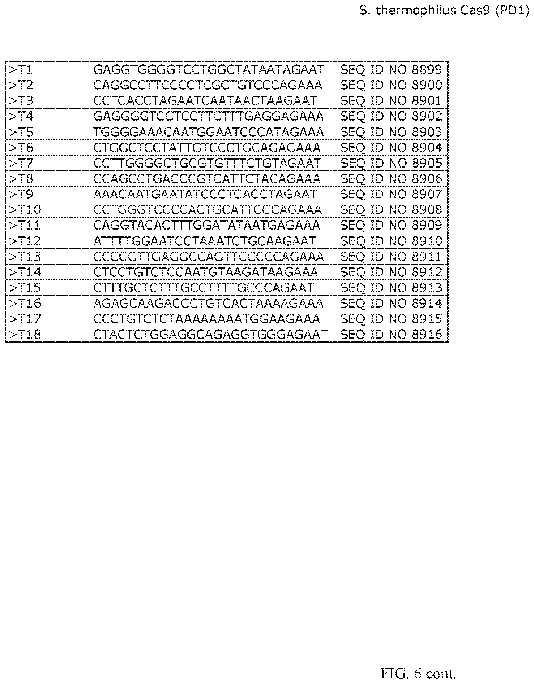

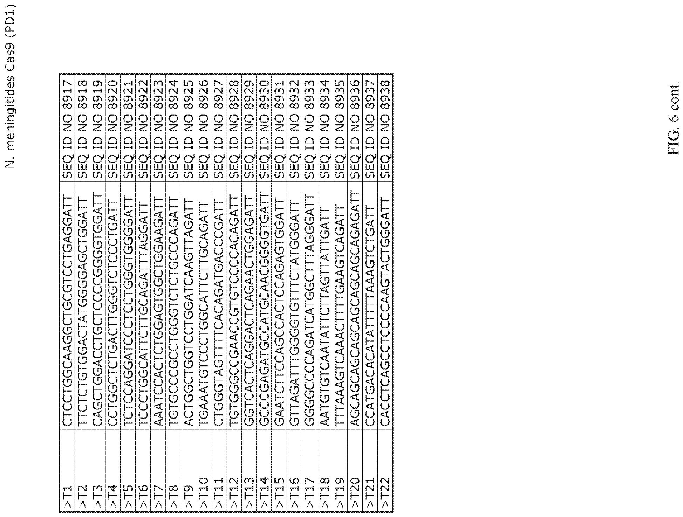

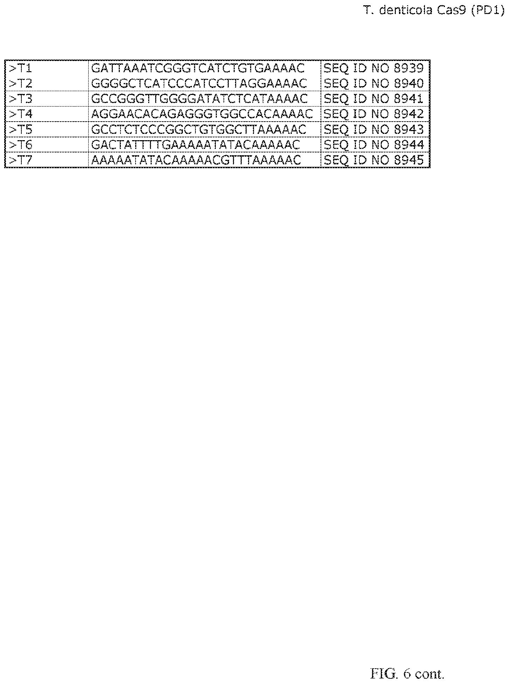

FIG. 6 shows exemplary gRNA sequences useful for targeting the PD1 gene using Cas9. The gRNAs described in the experimental examples are identified in red text.

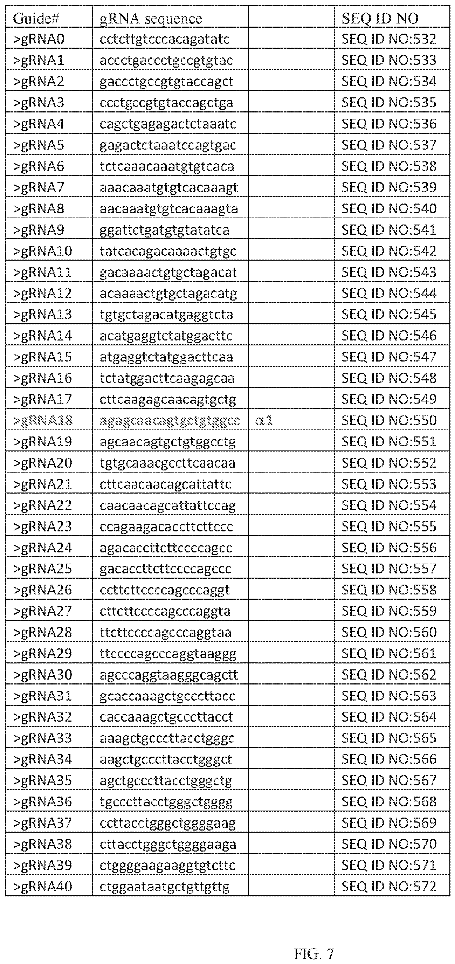

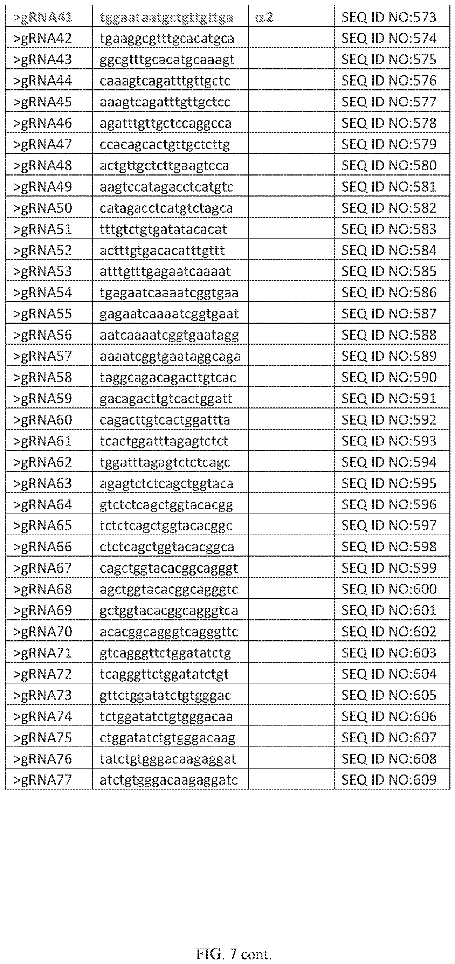

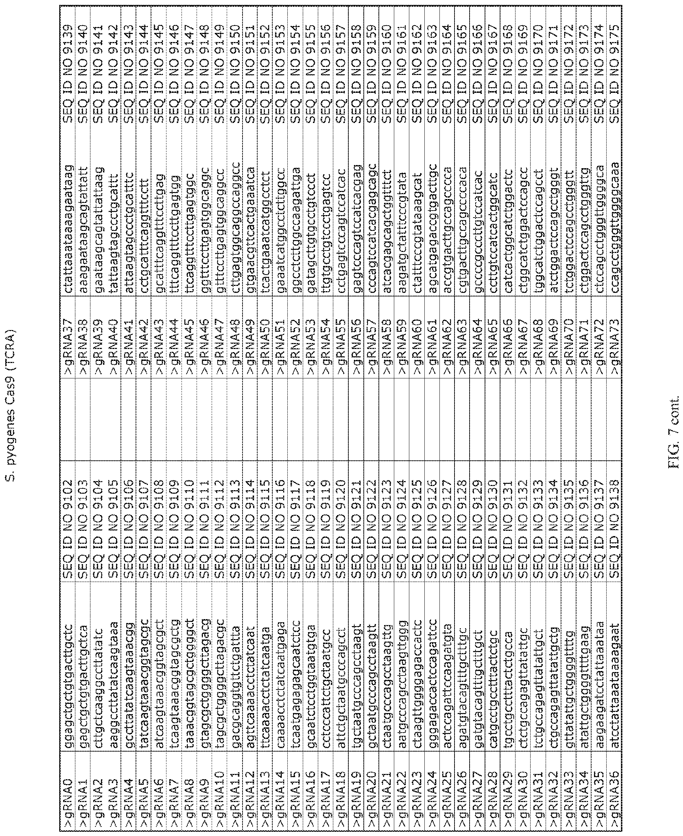

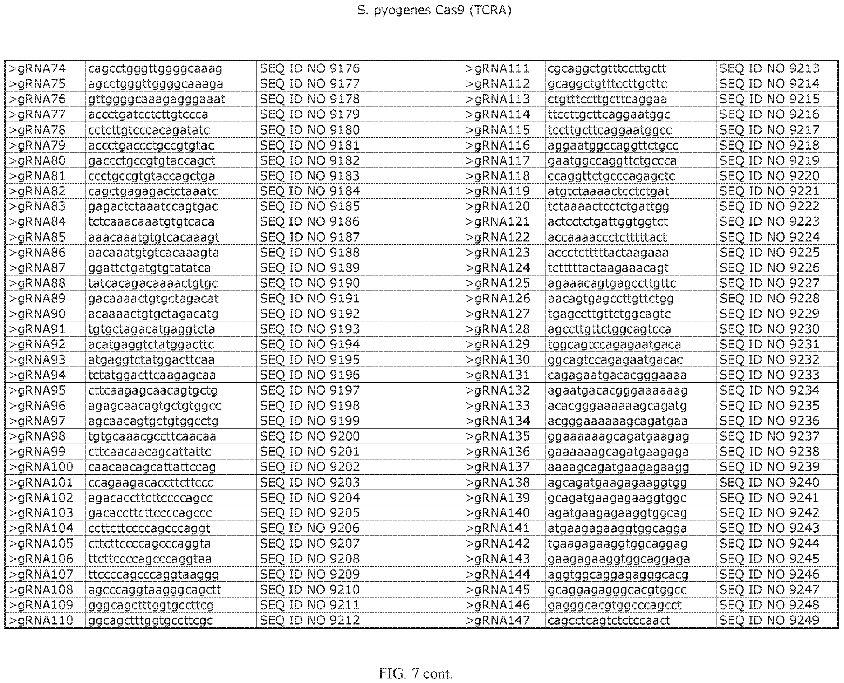

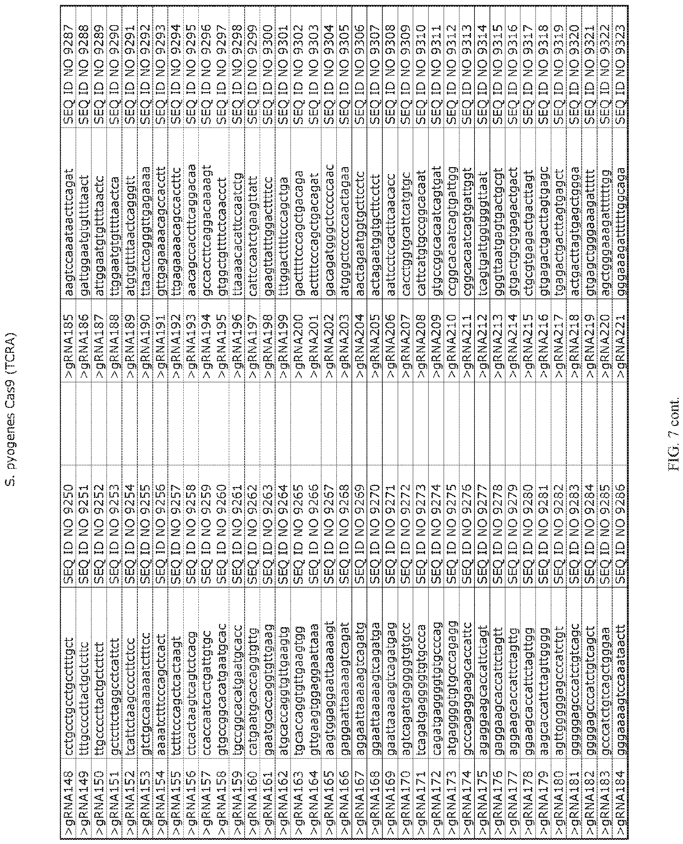

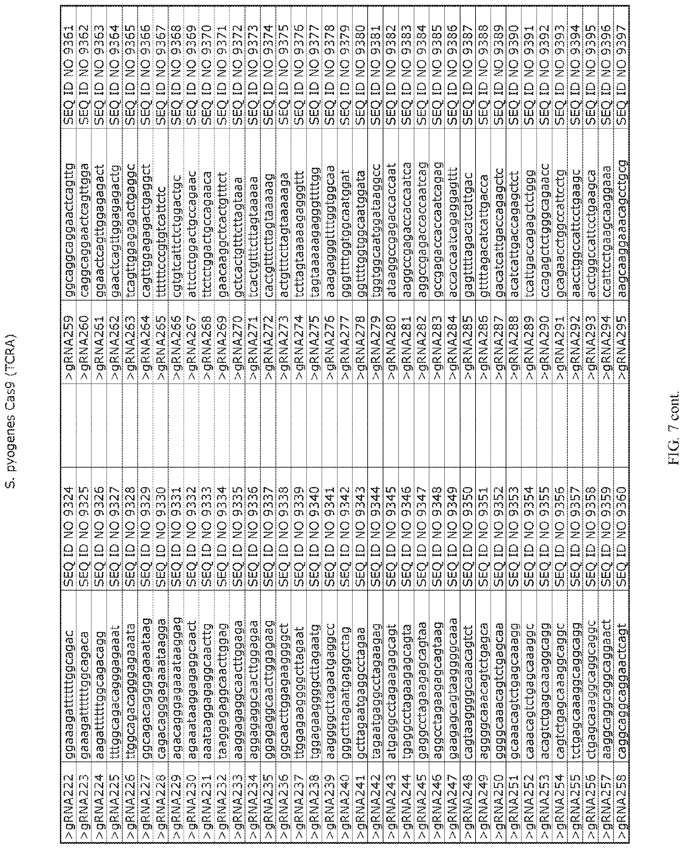

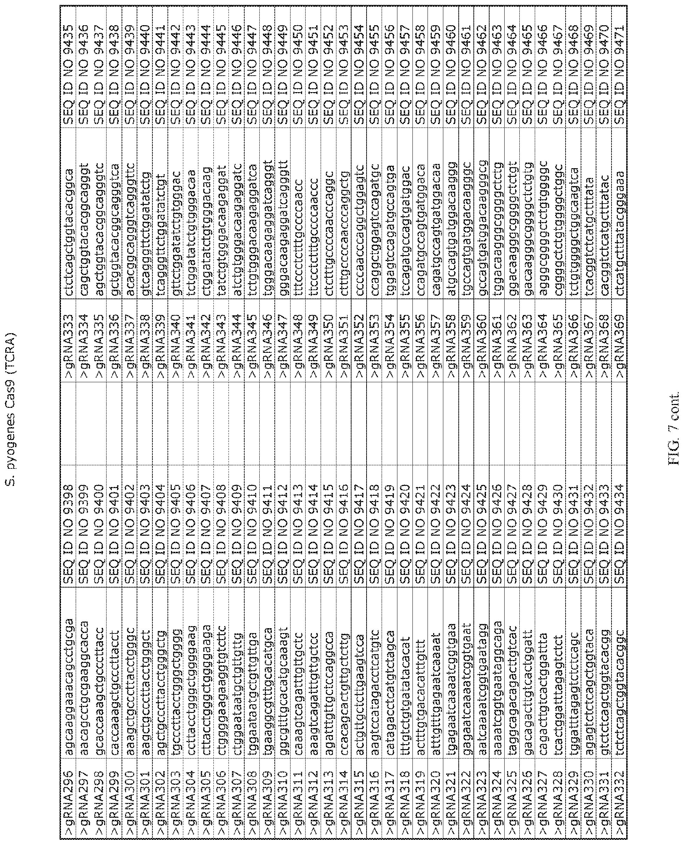

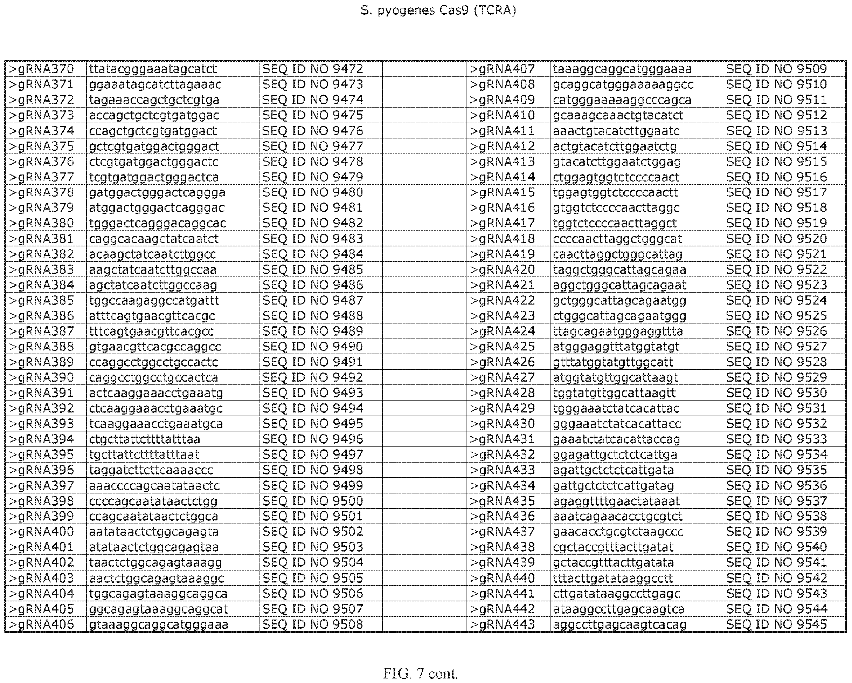

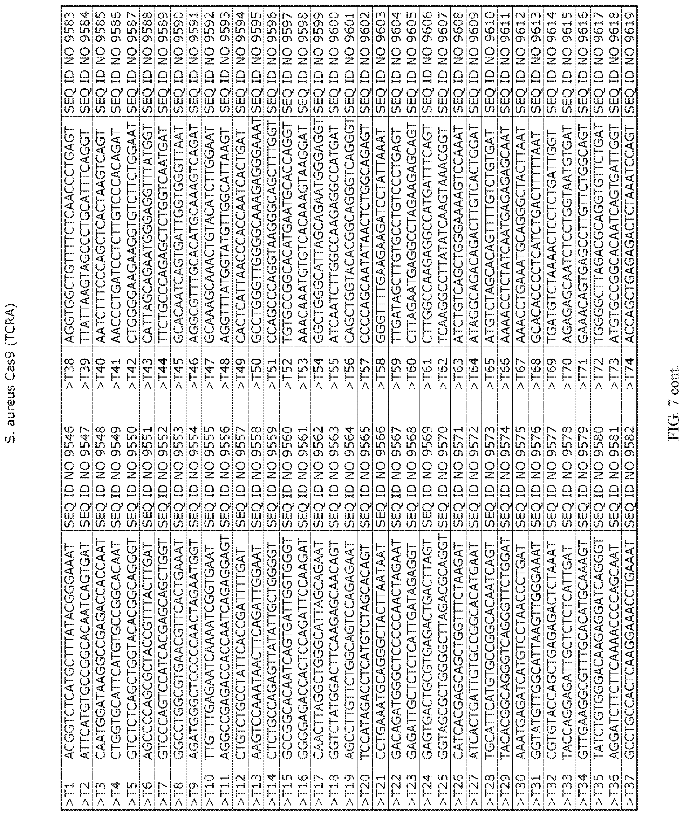

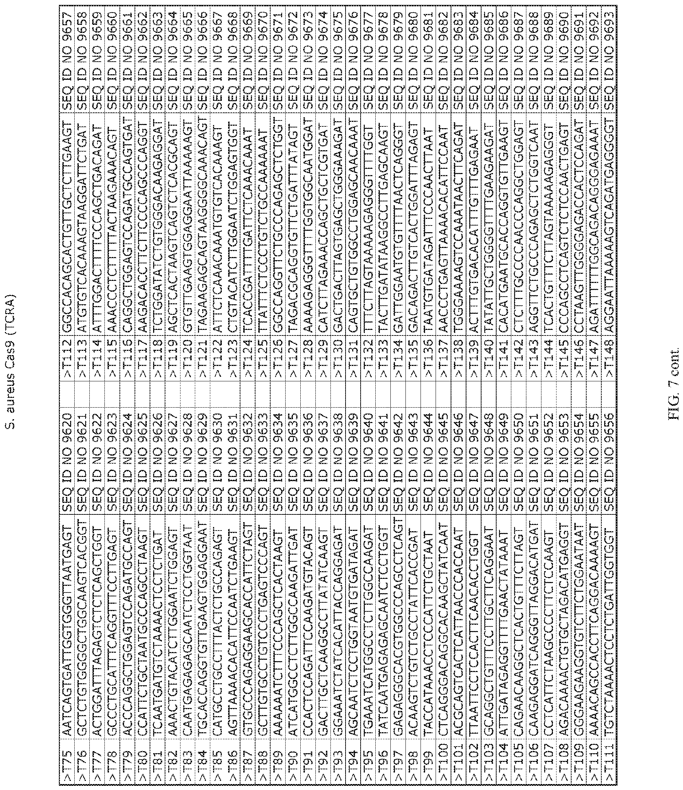

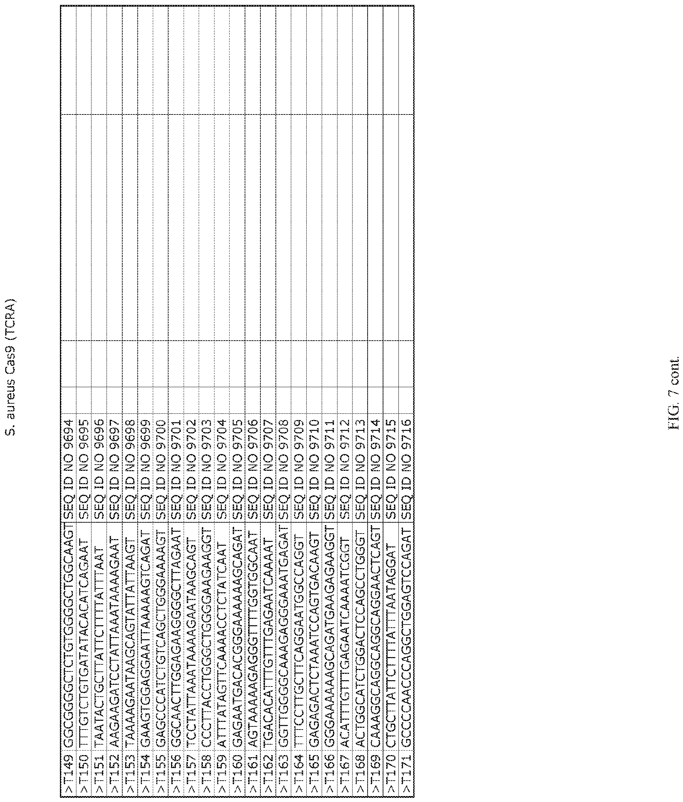

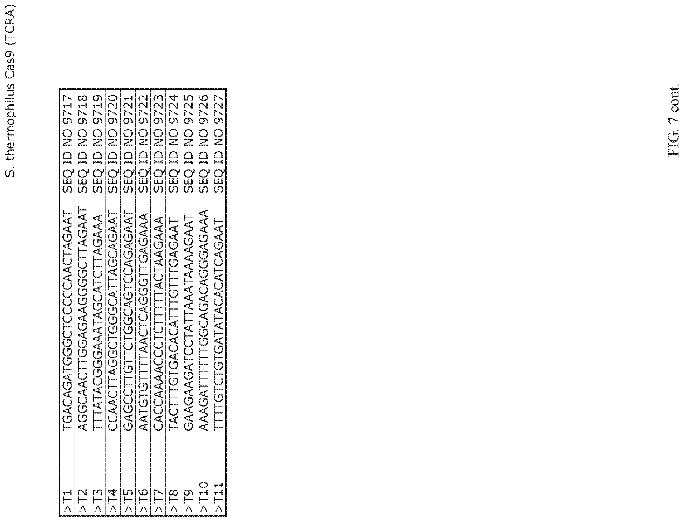

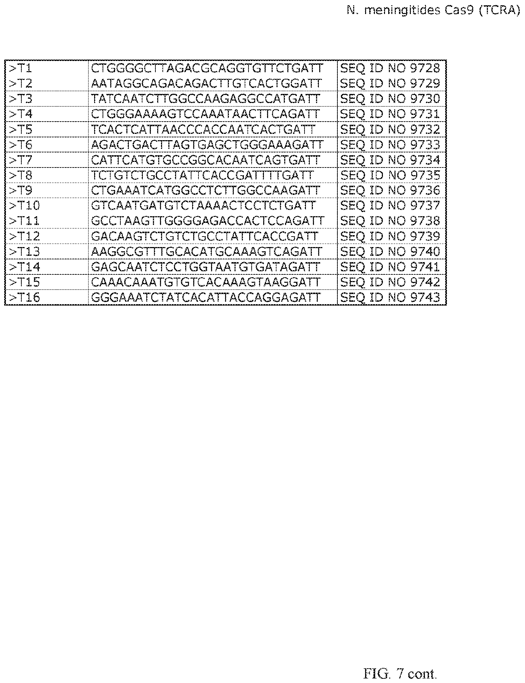

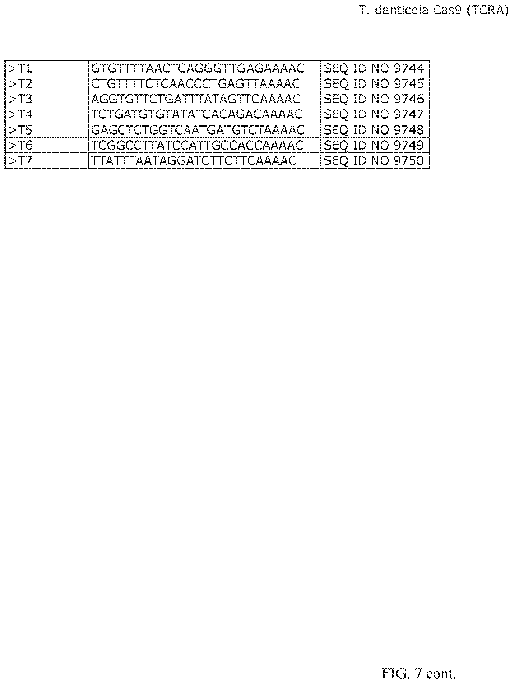

FIG. 7 shows exemplary gRNA sequences useful for targeting the TCRalpha locus using Cas9. The gRNAs described in the experimental examples are identified in red text.

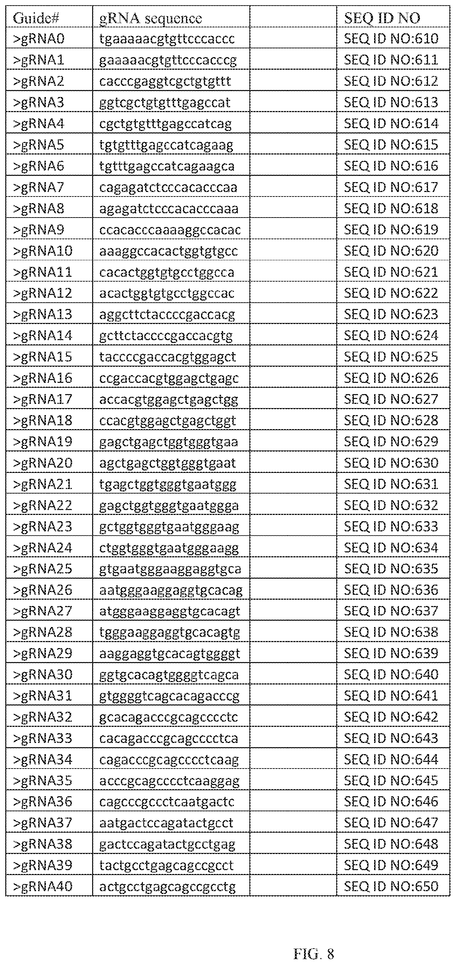

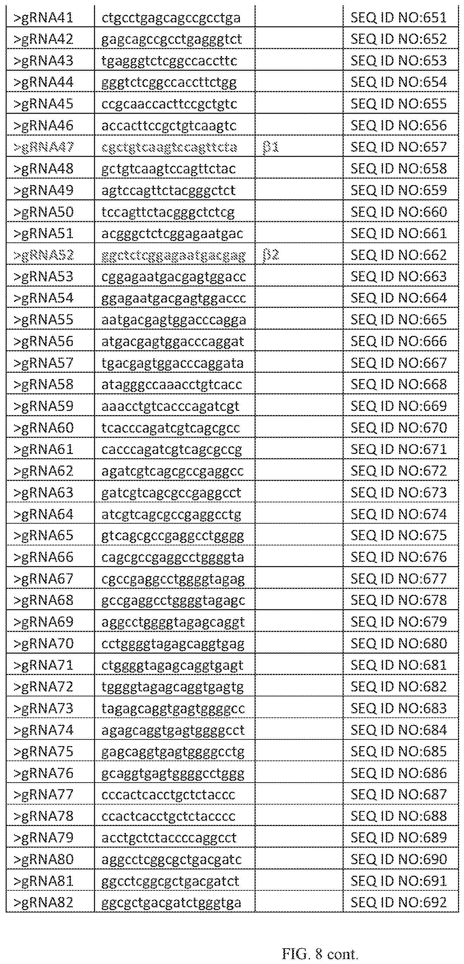

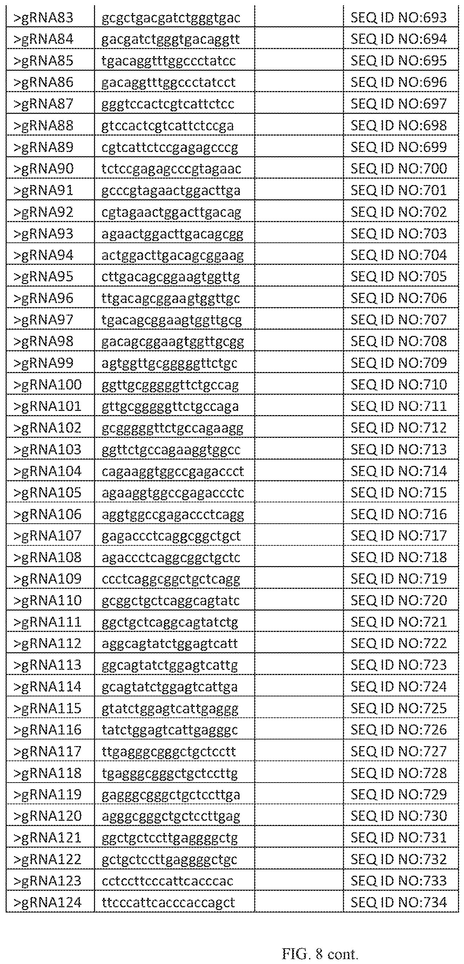

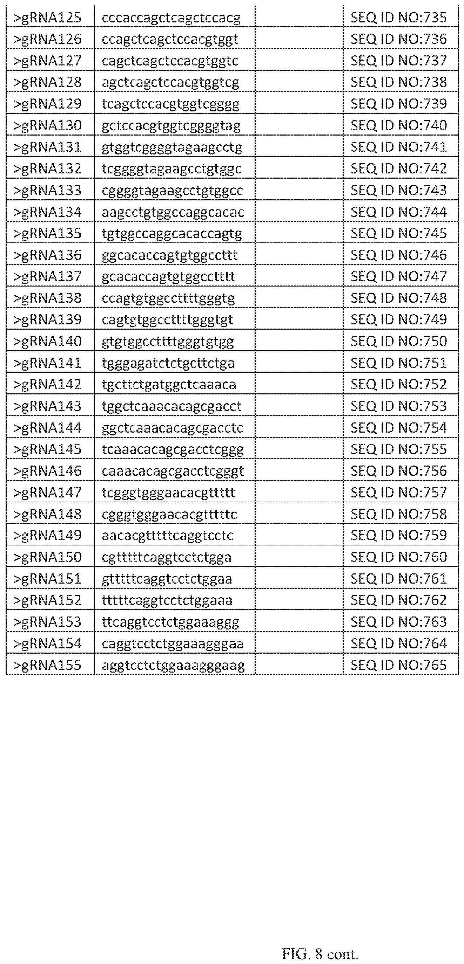

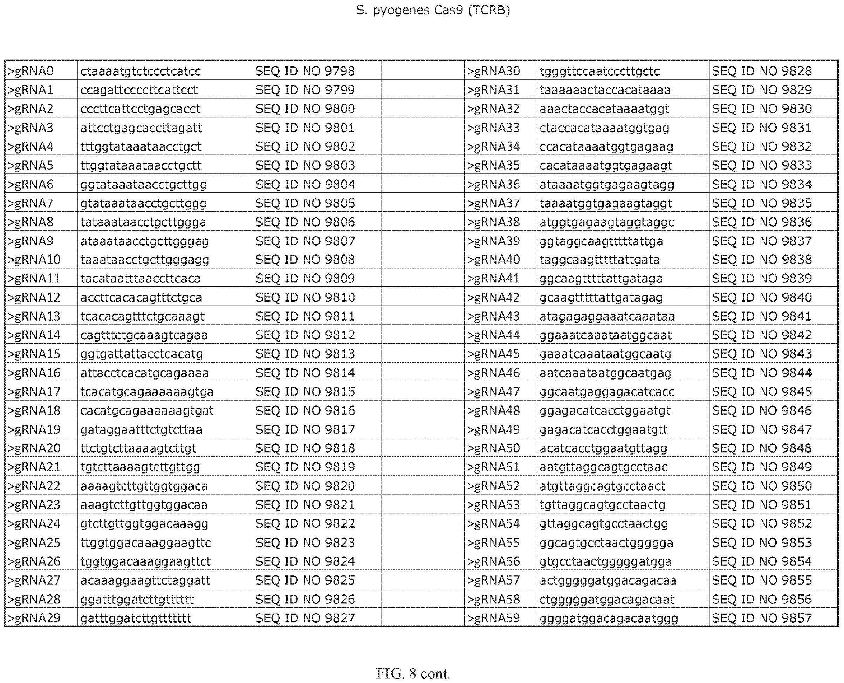

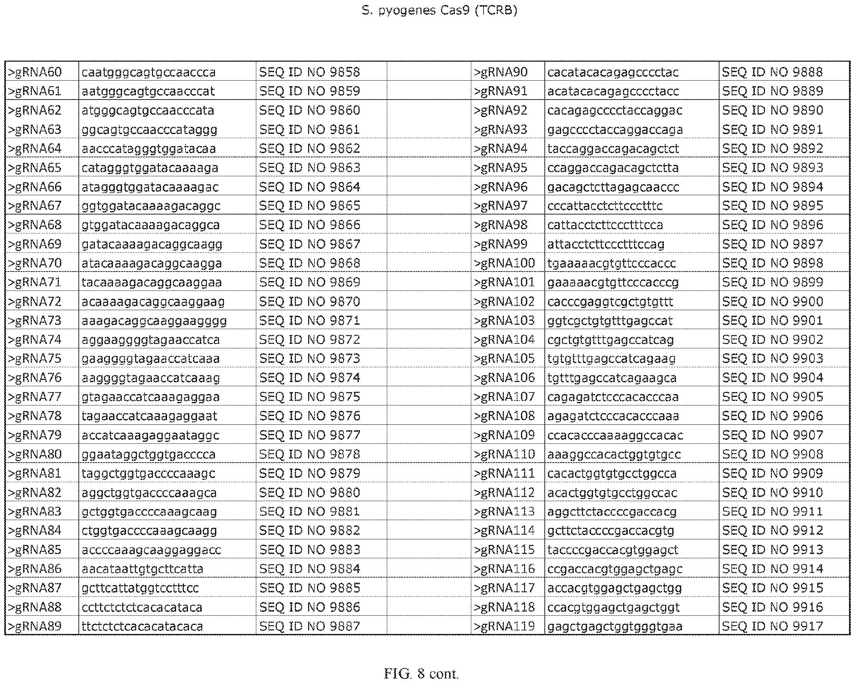

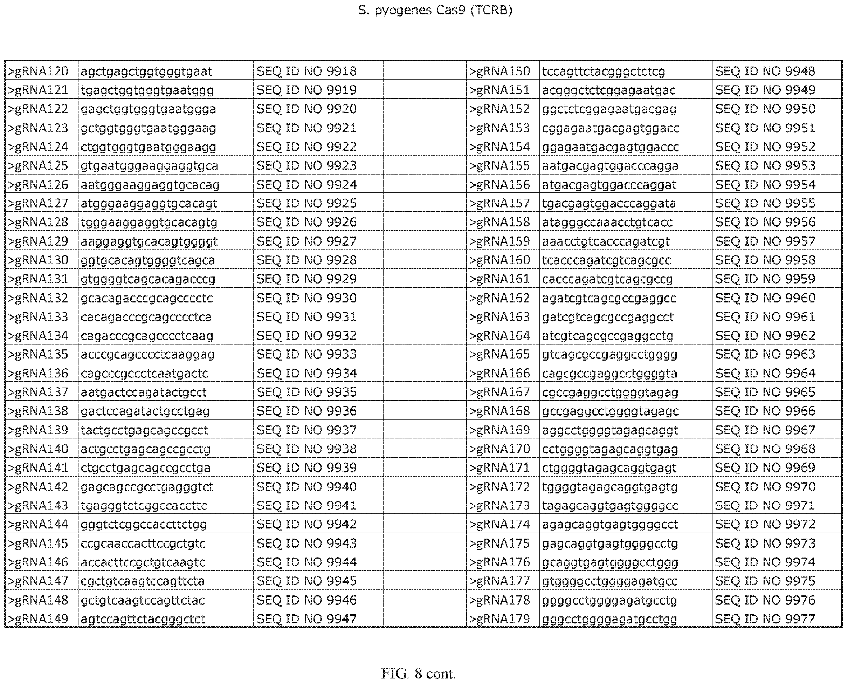

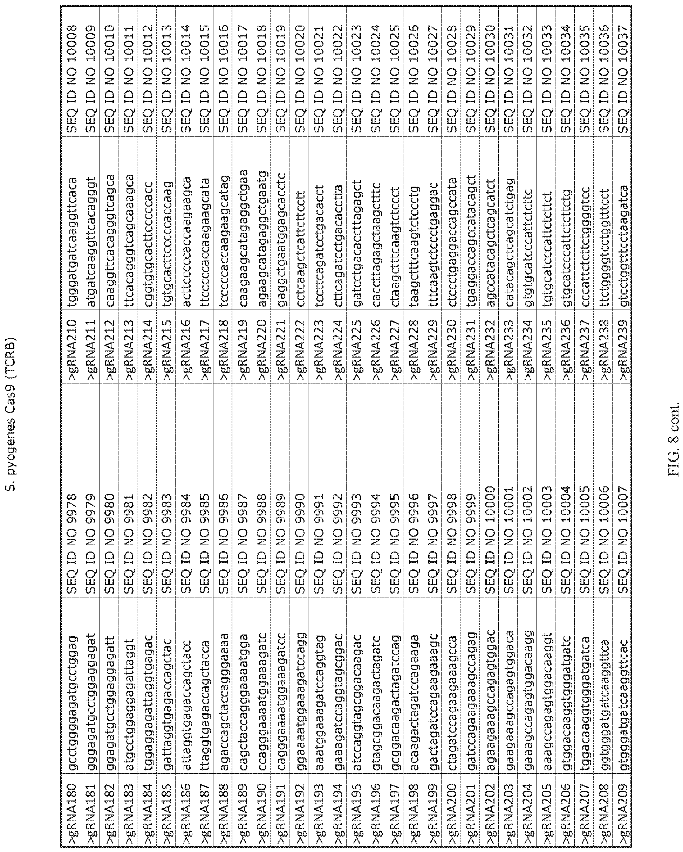

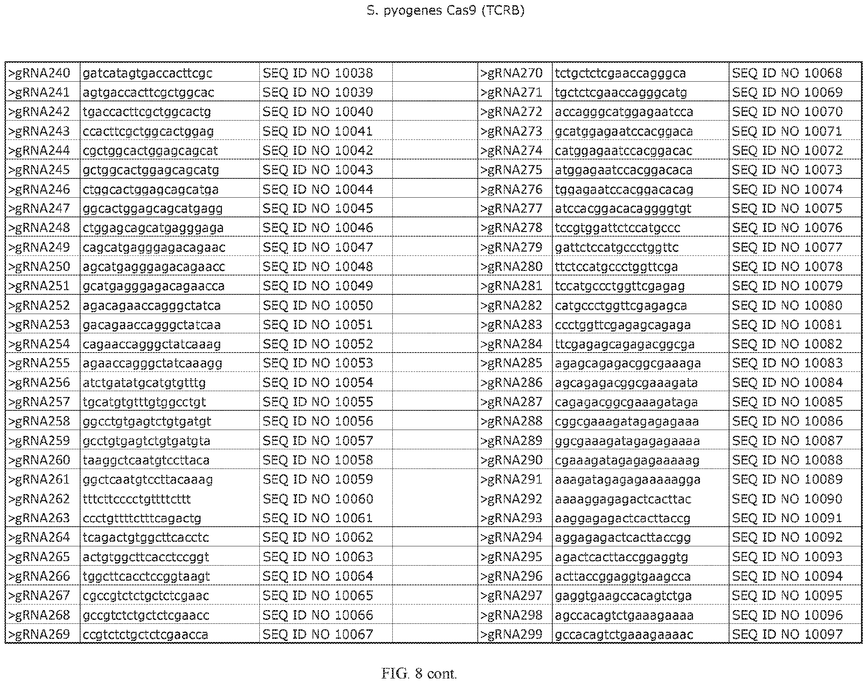

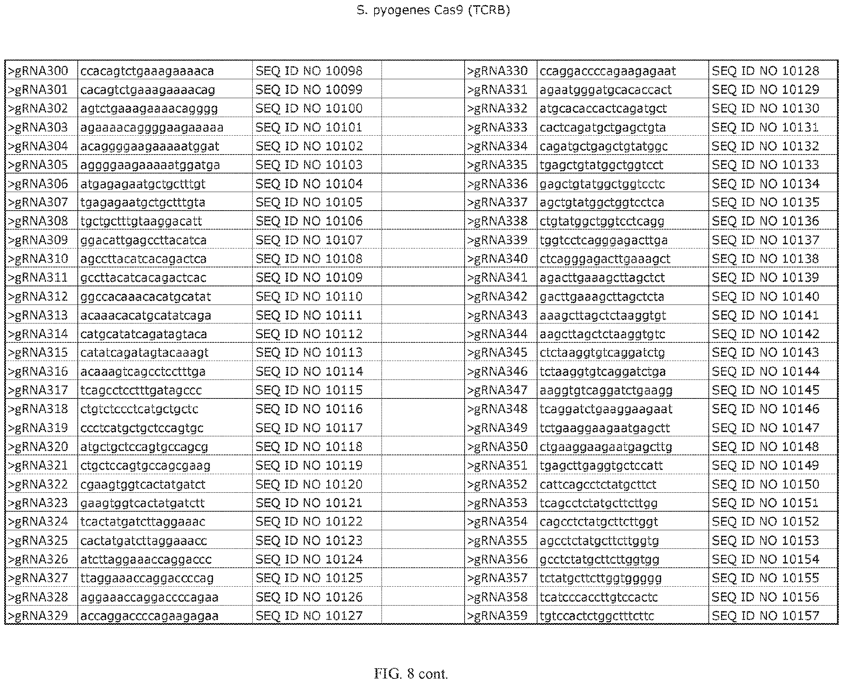

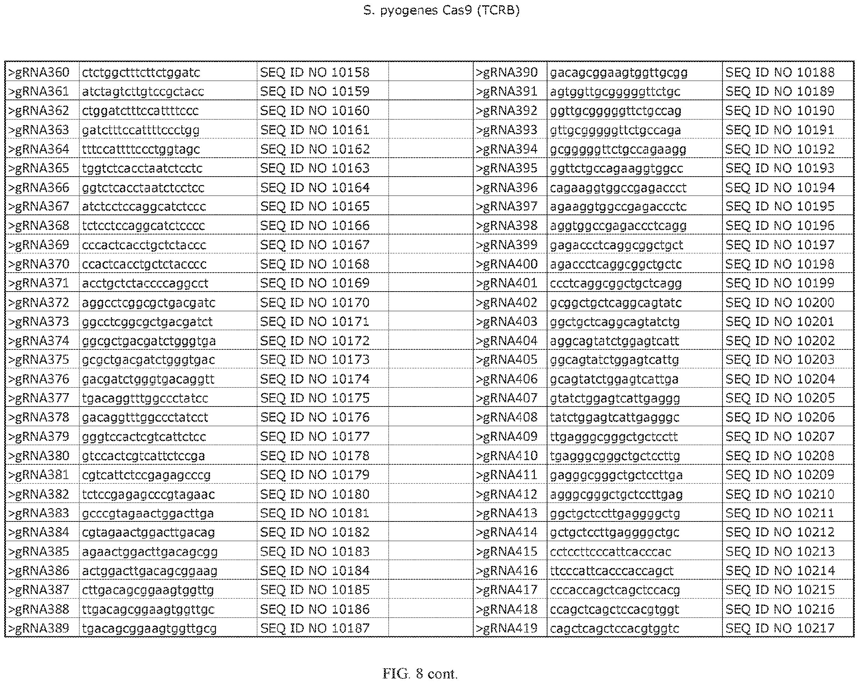

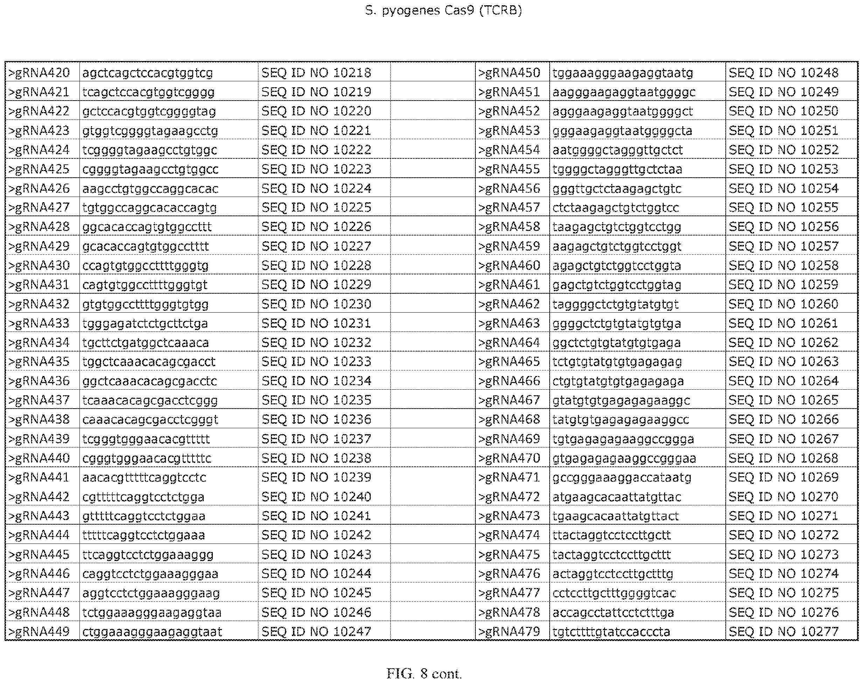

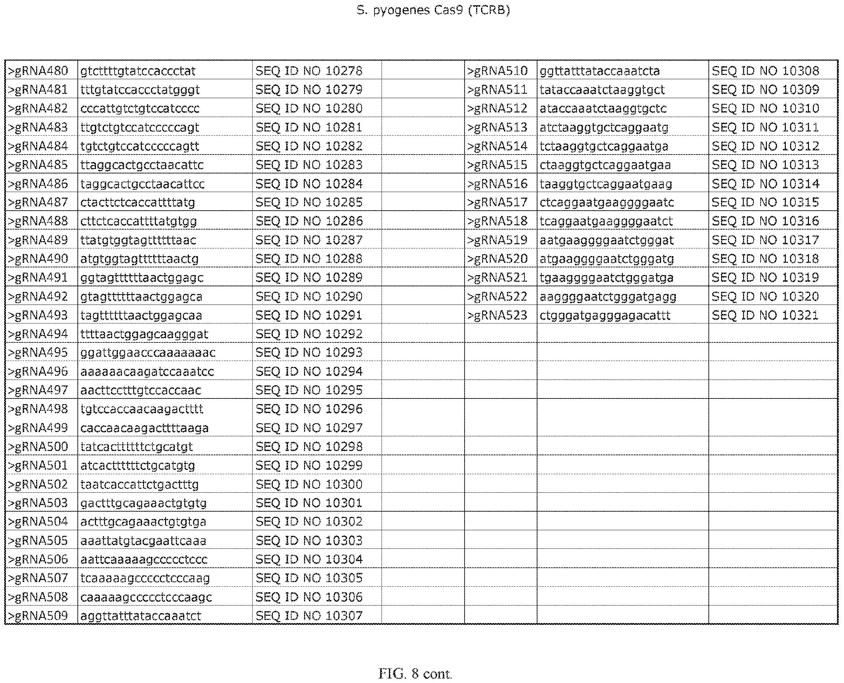

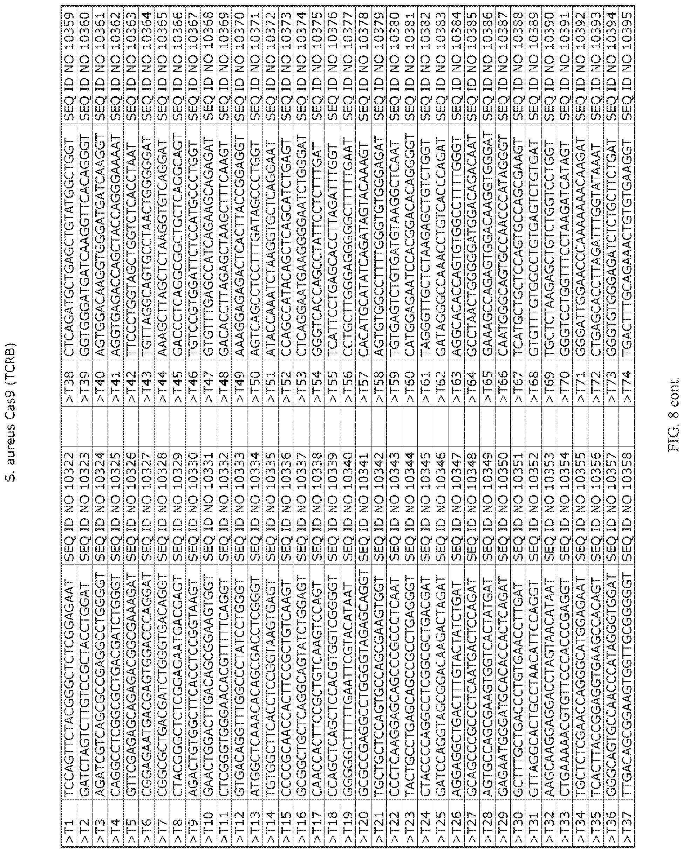

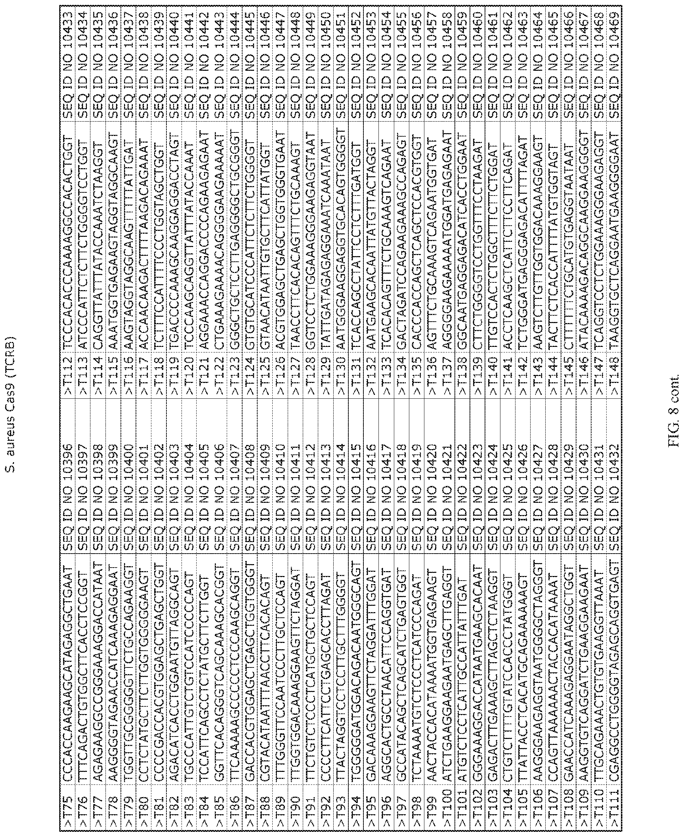

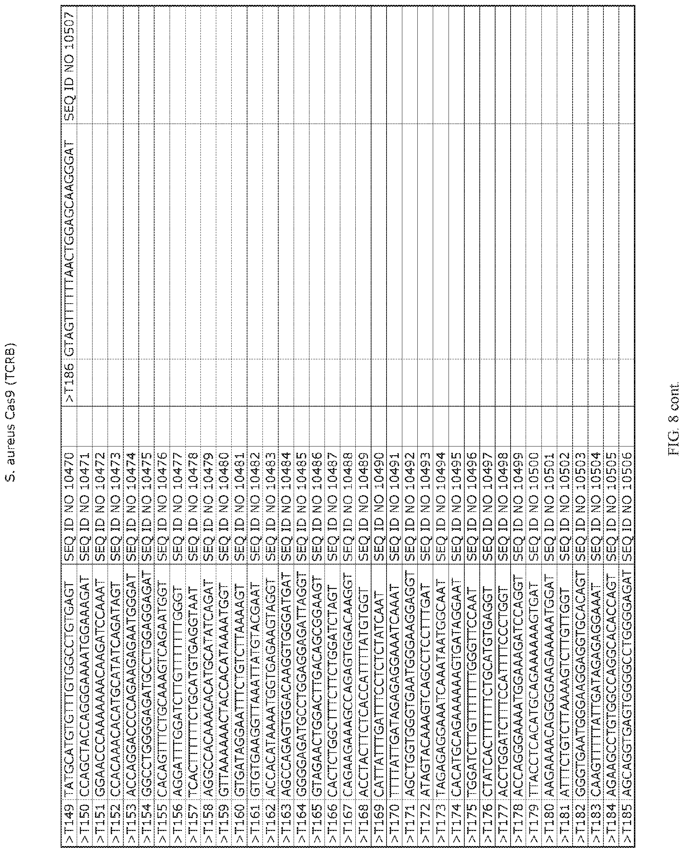

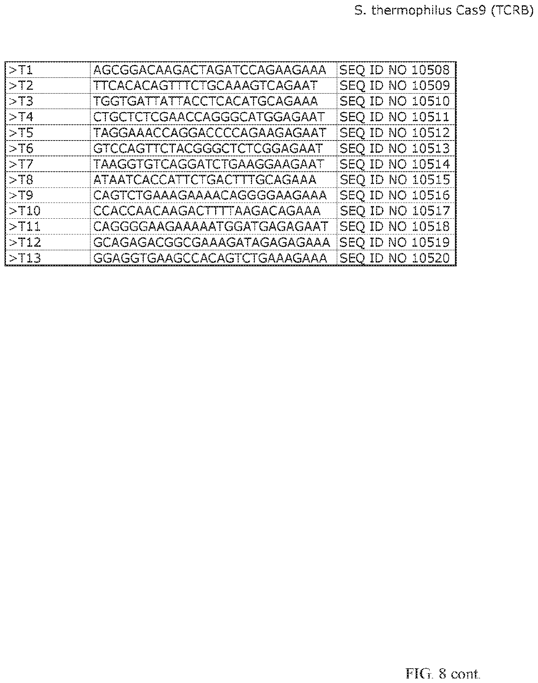

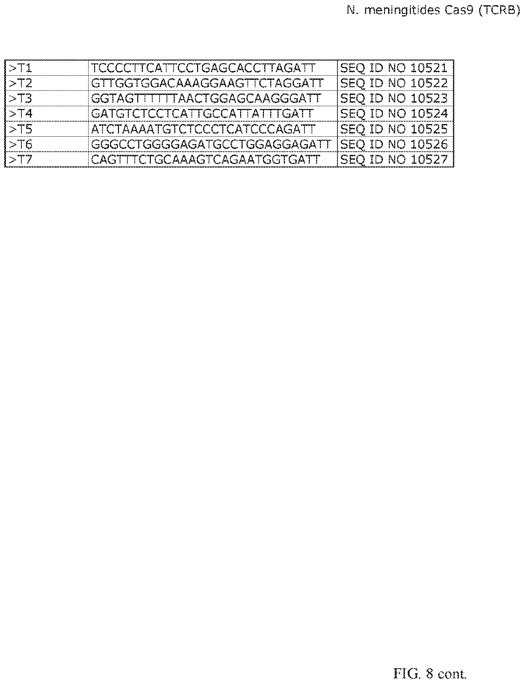

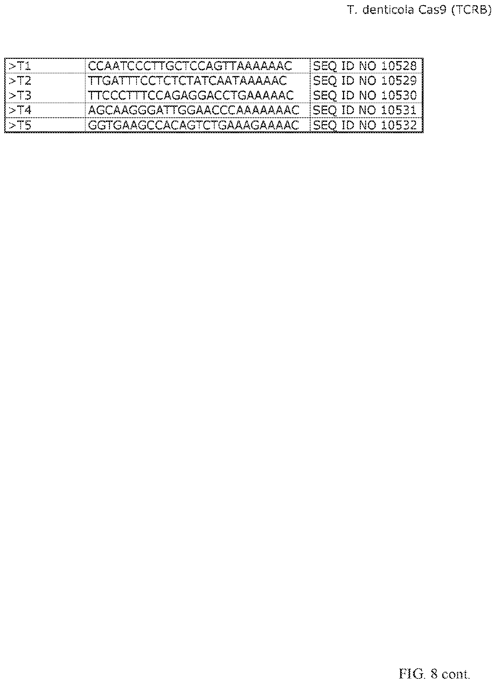

FIG. 8 shows exemplary gRNA sequences useful for targeting the human TCRbeta locus using Cas9. The gRNAs described in the experimental examples are identified in red text.

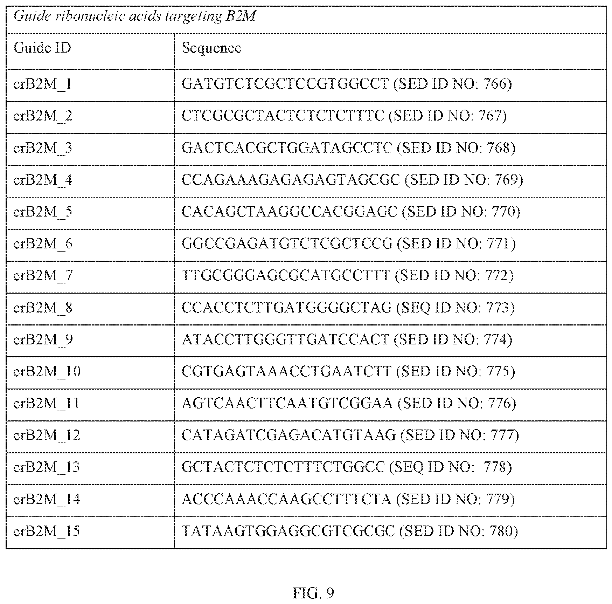

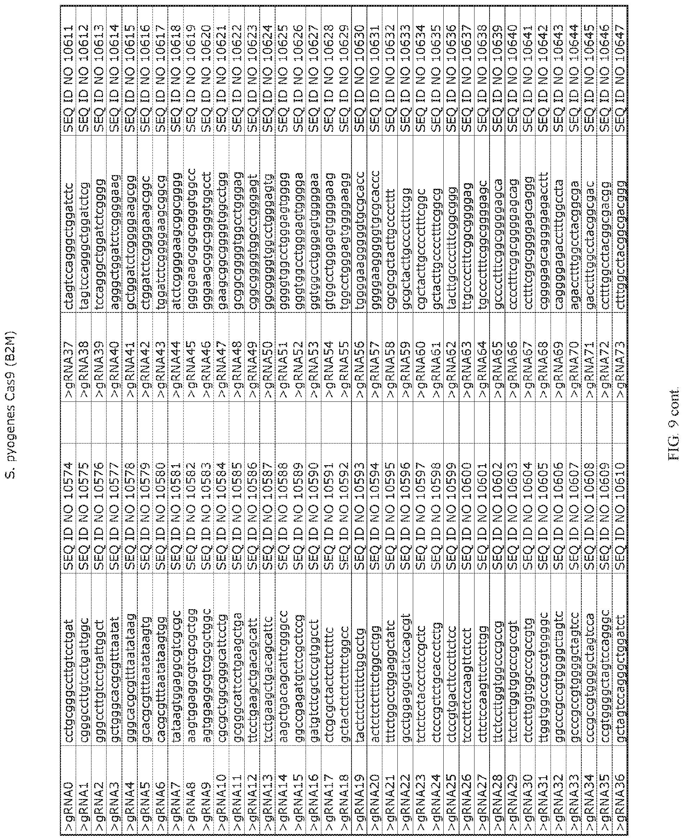

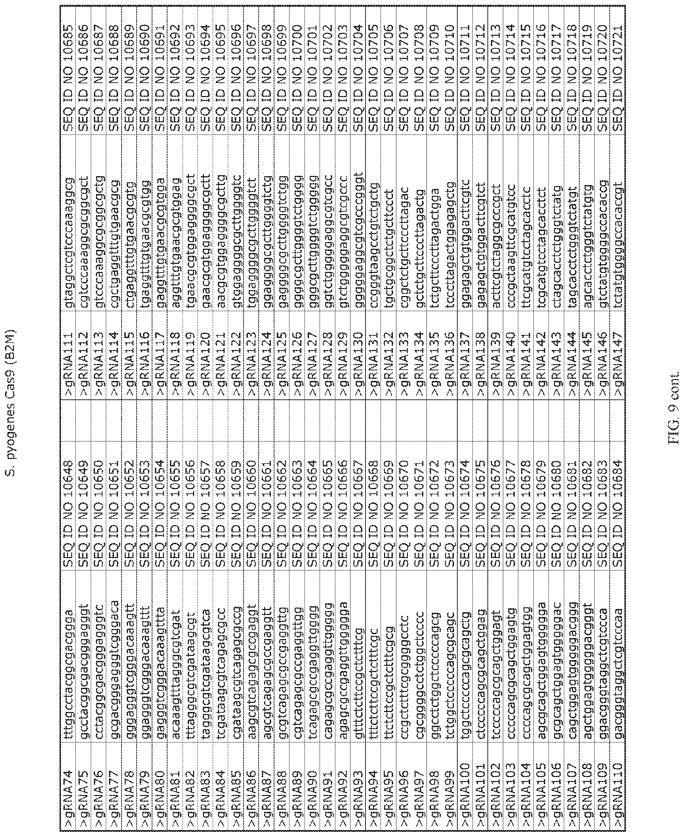

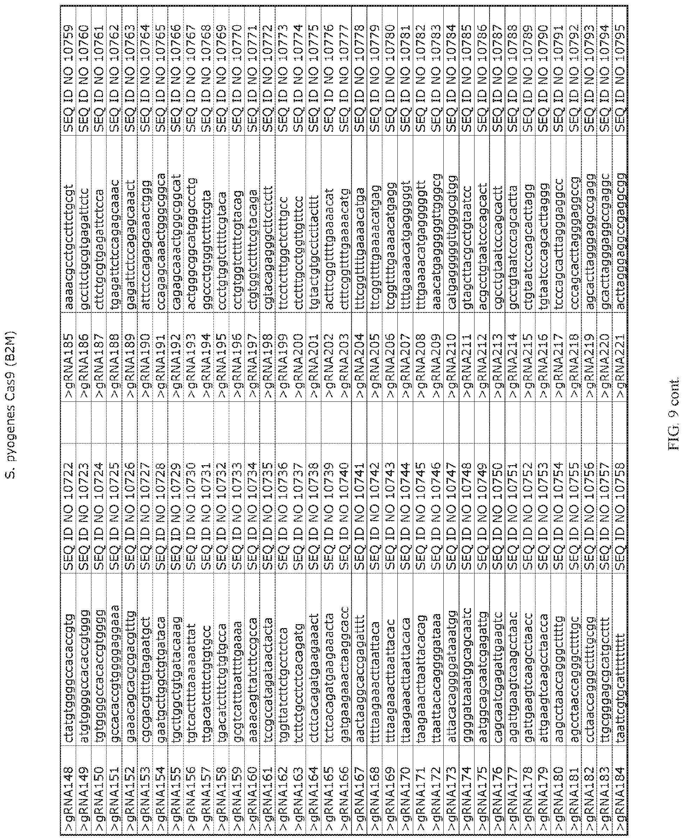

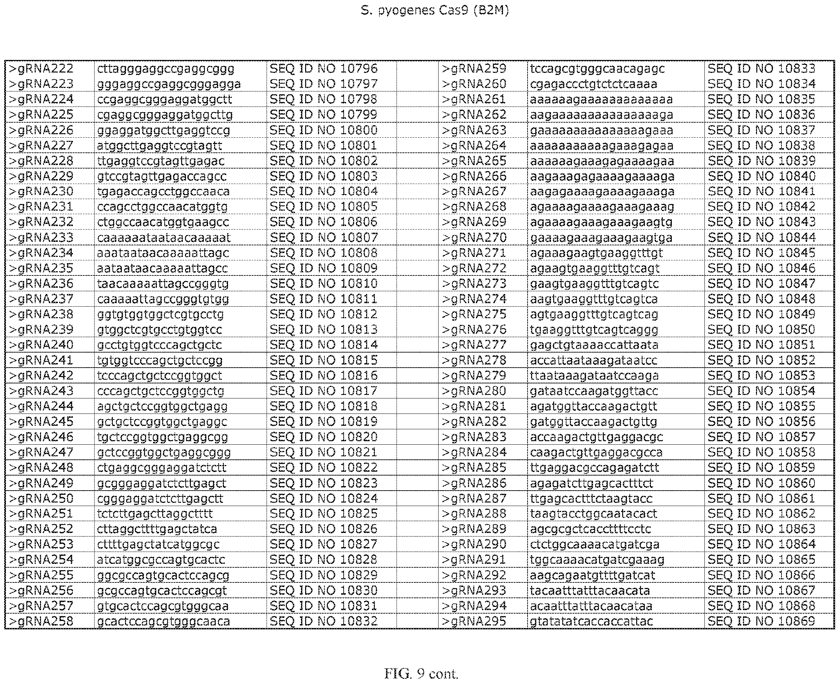

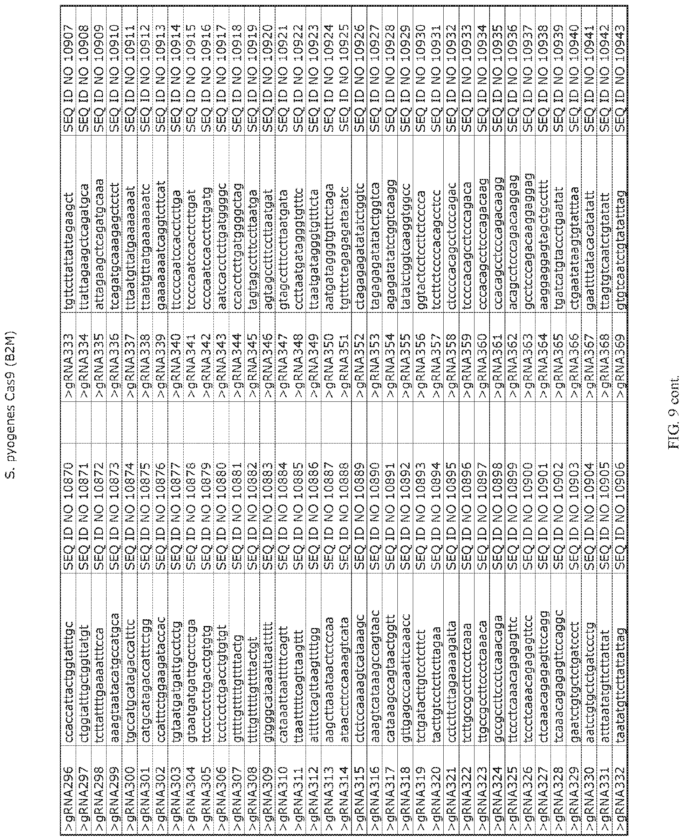

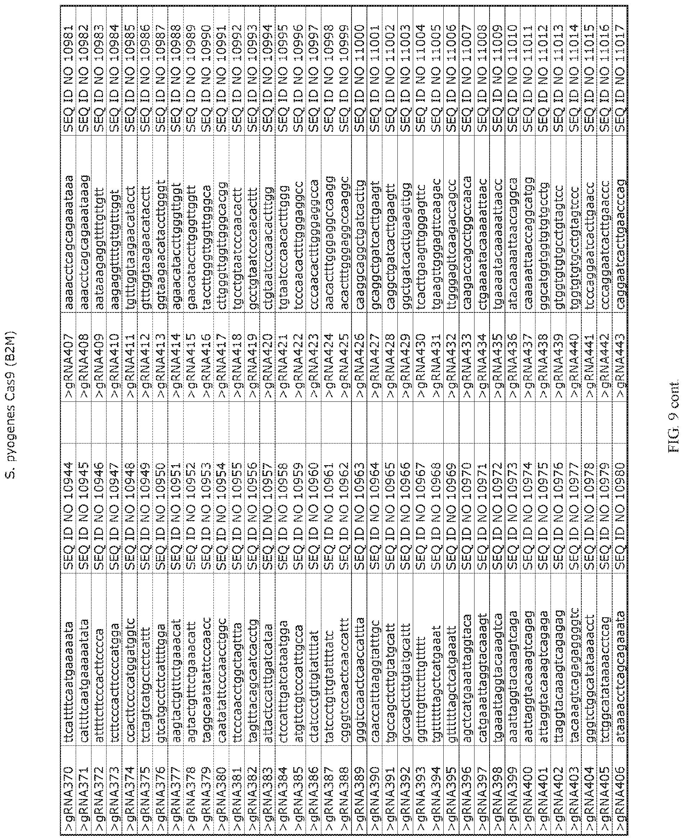

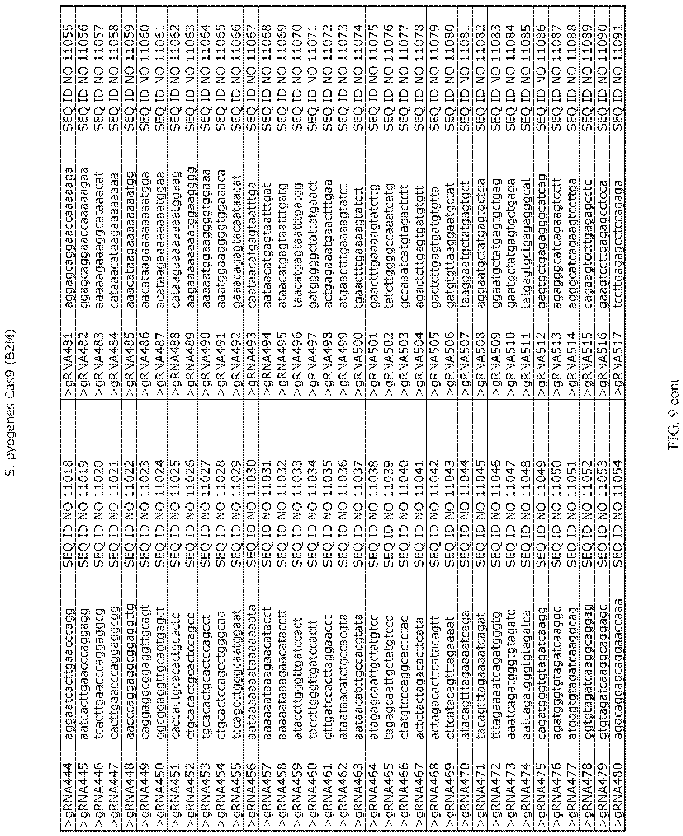

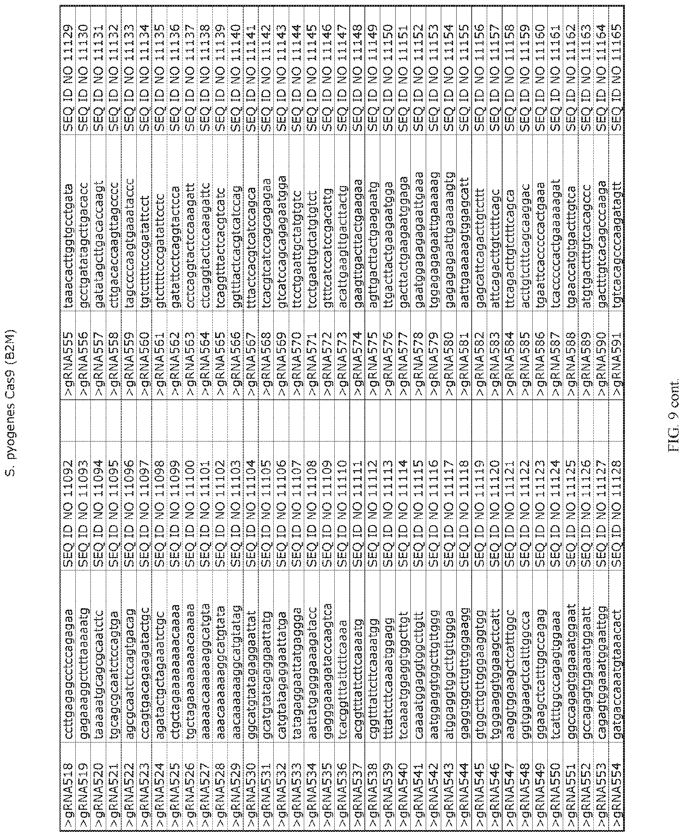

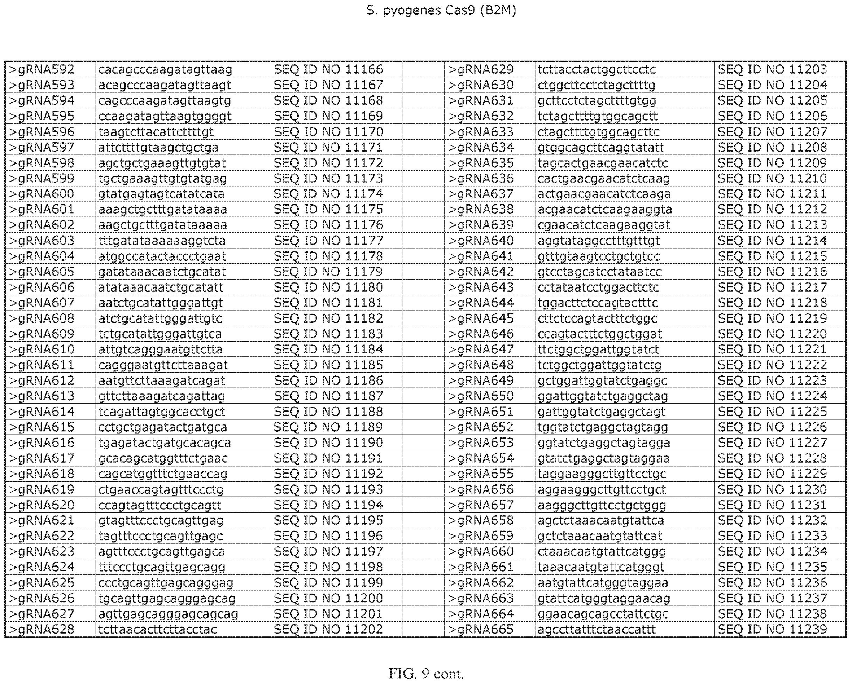

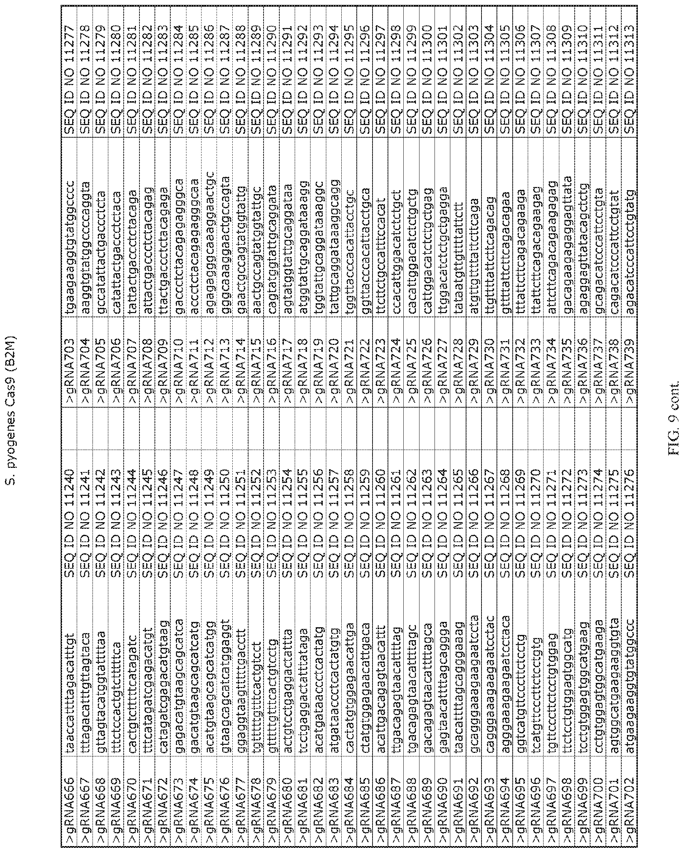

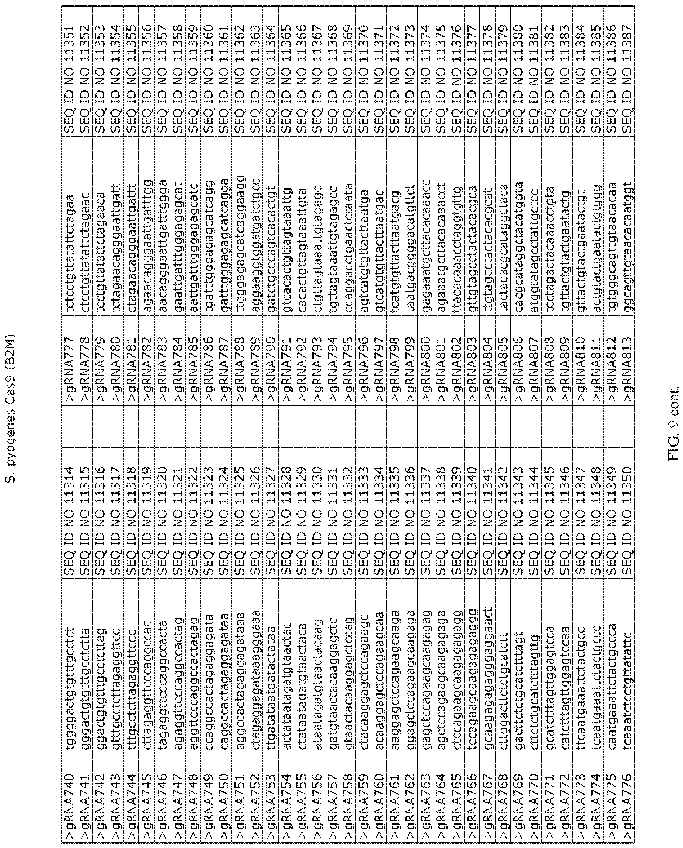

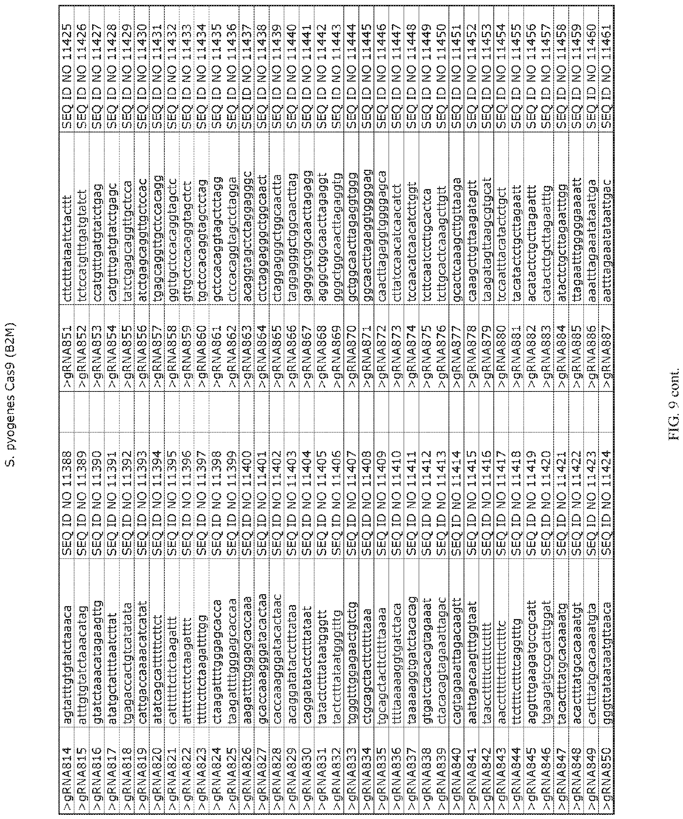

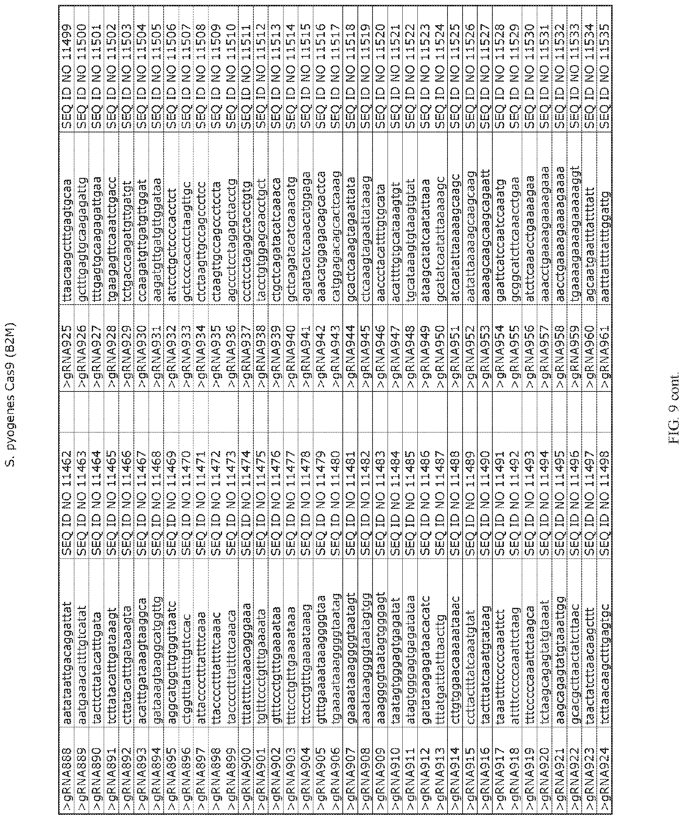

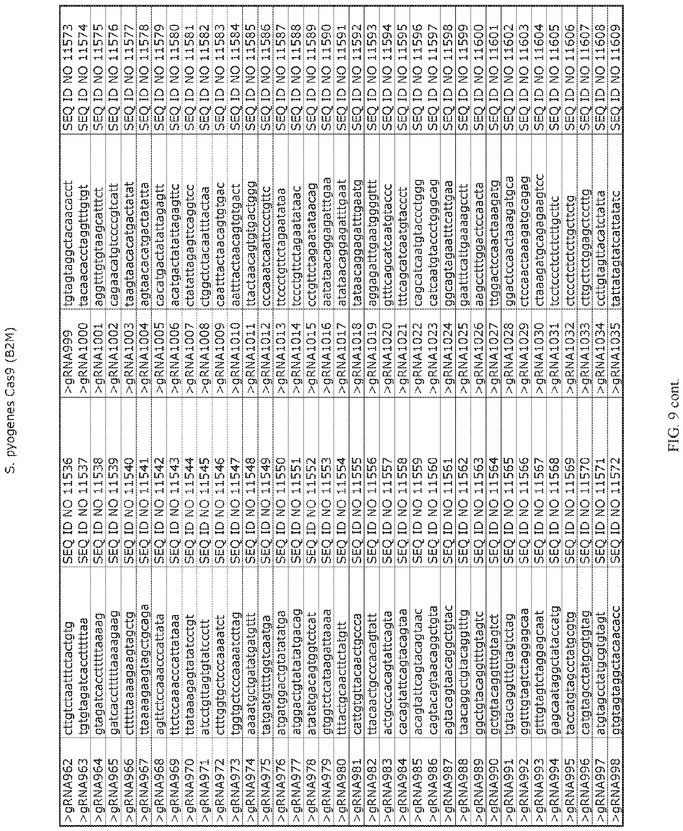

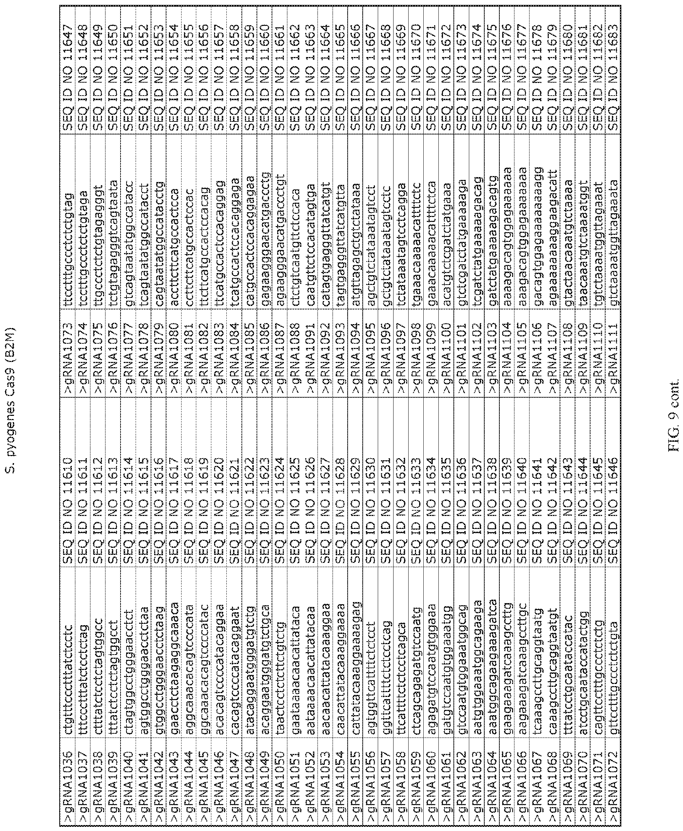

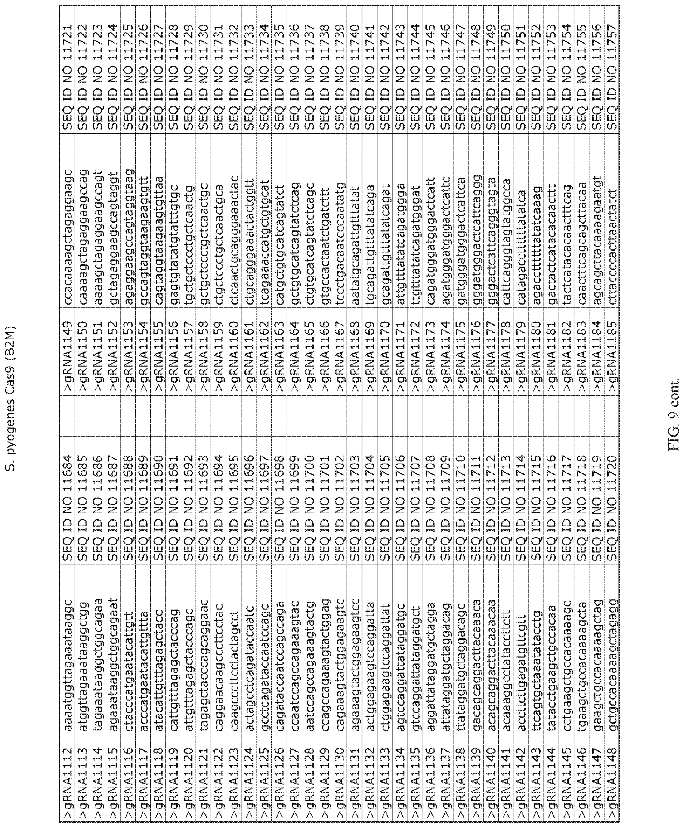

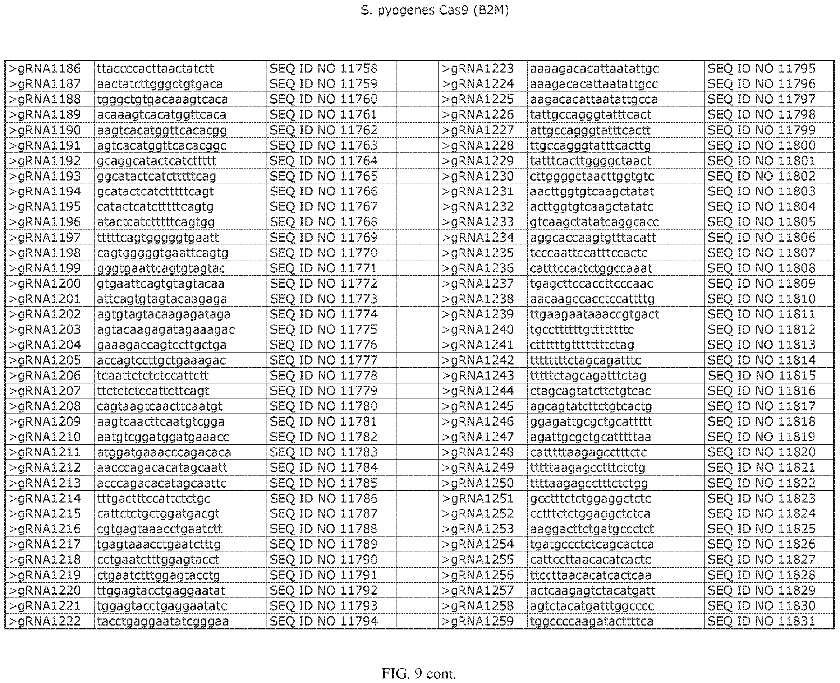

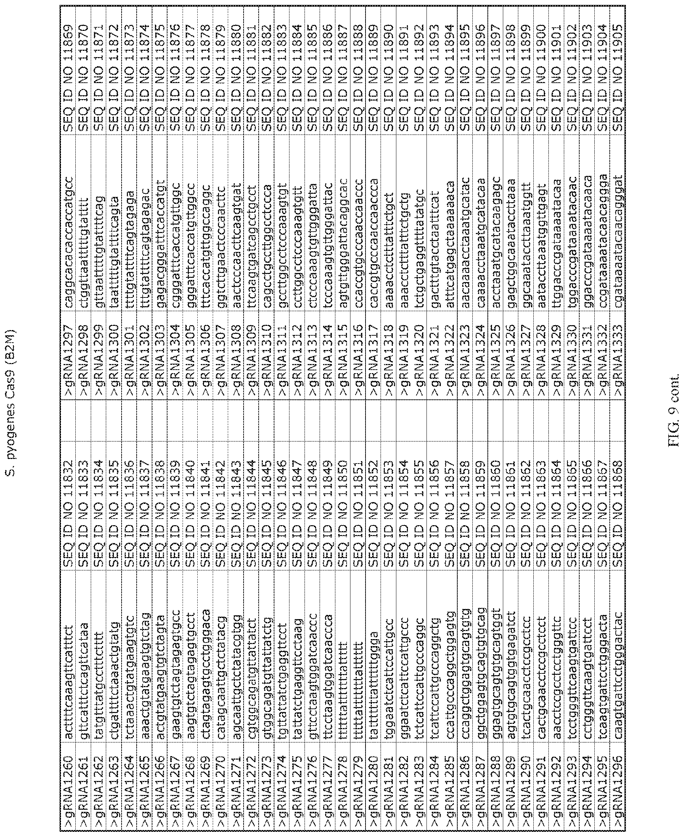

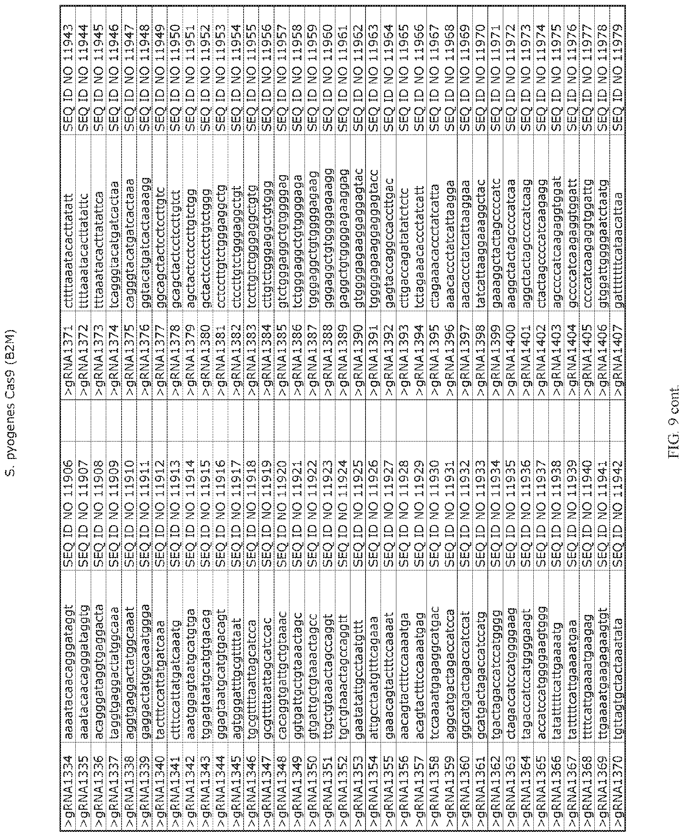

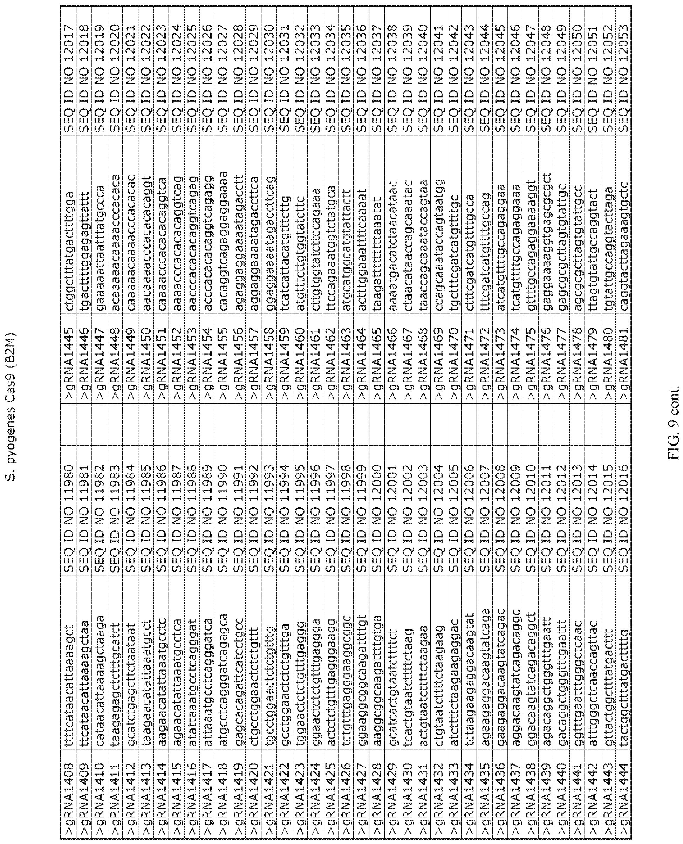

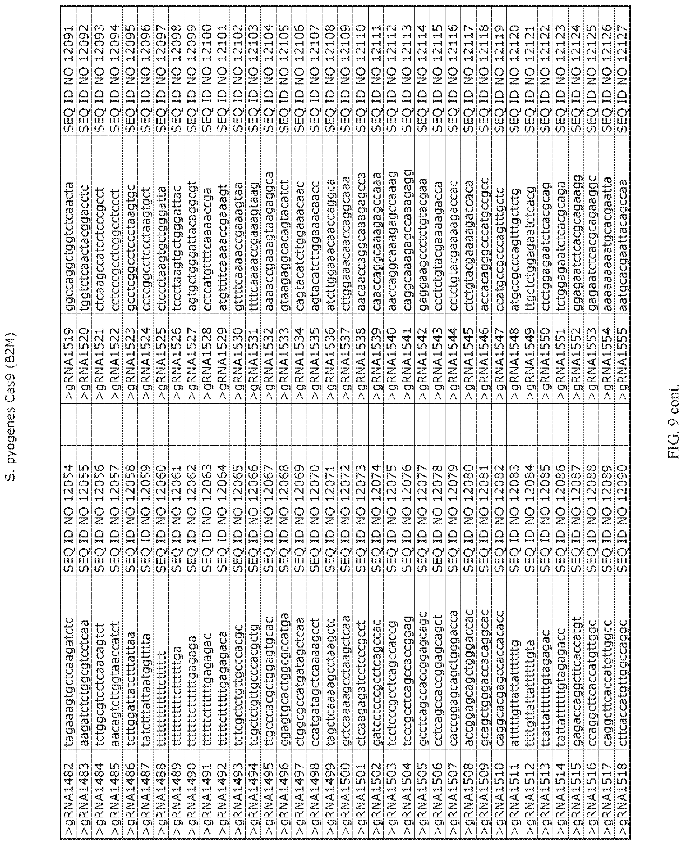

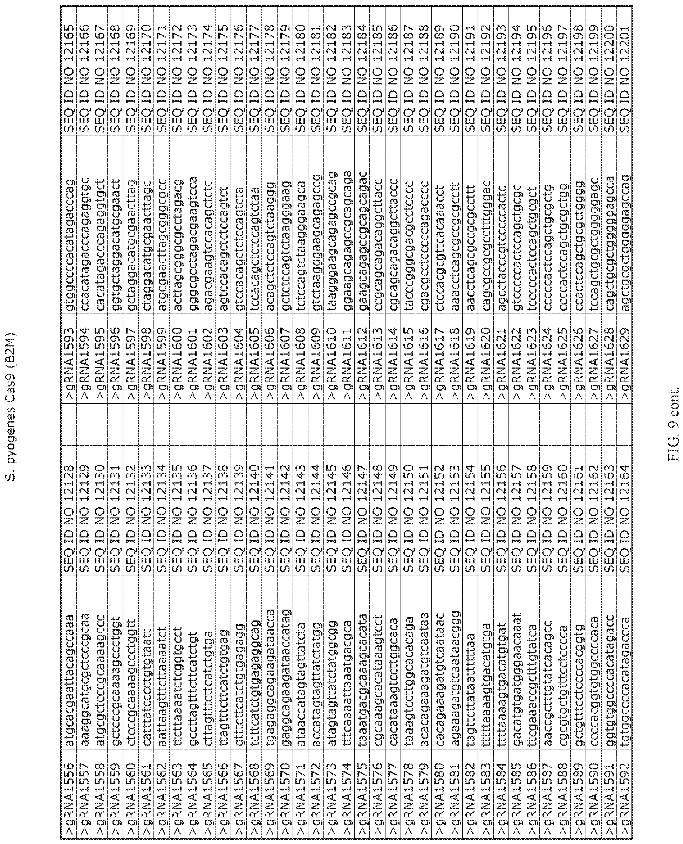

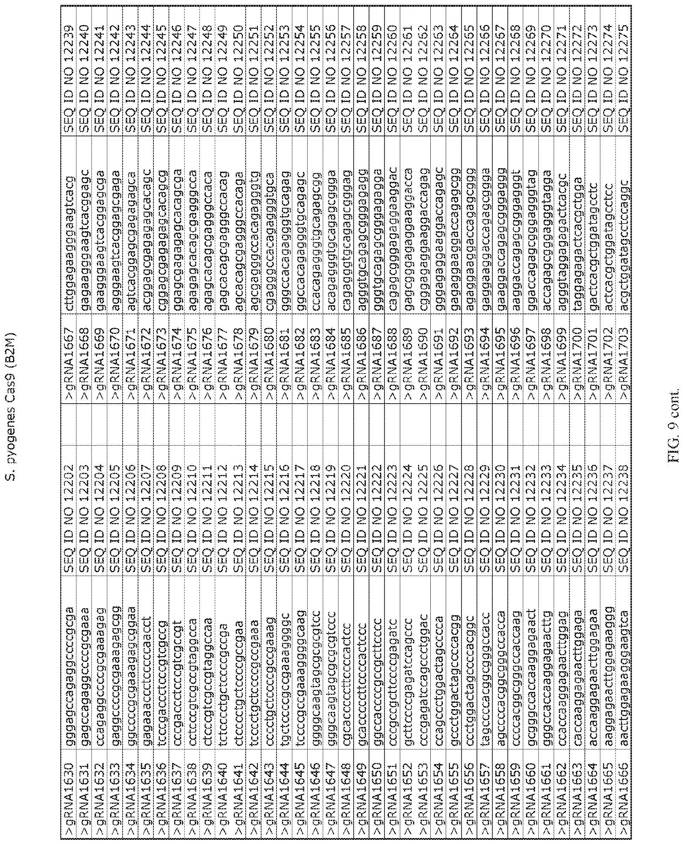

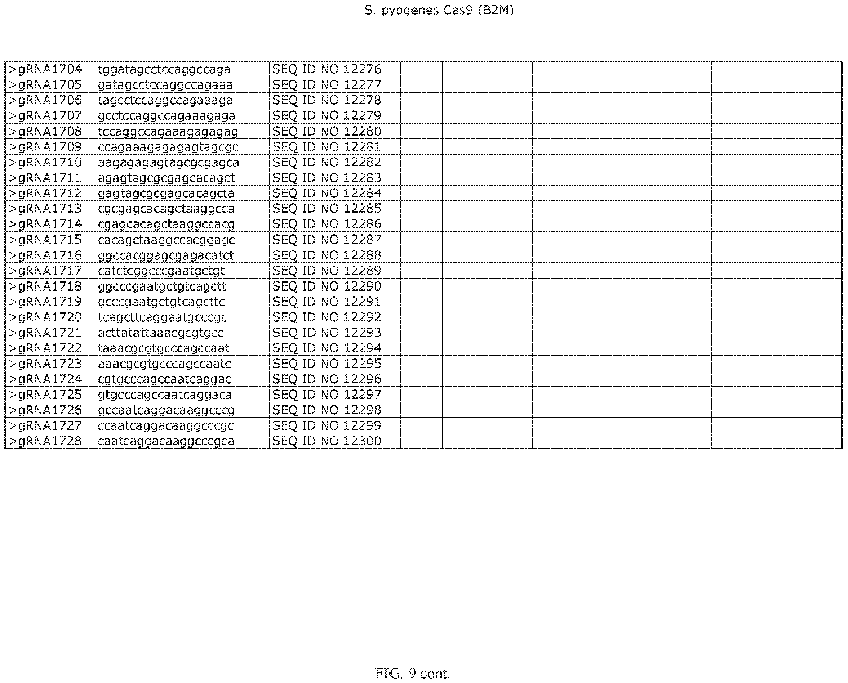

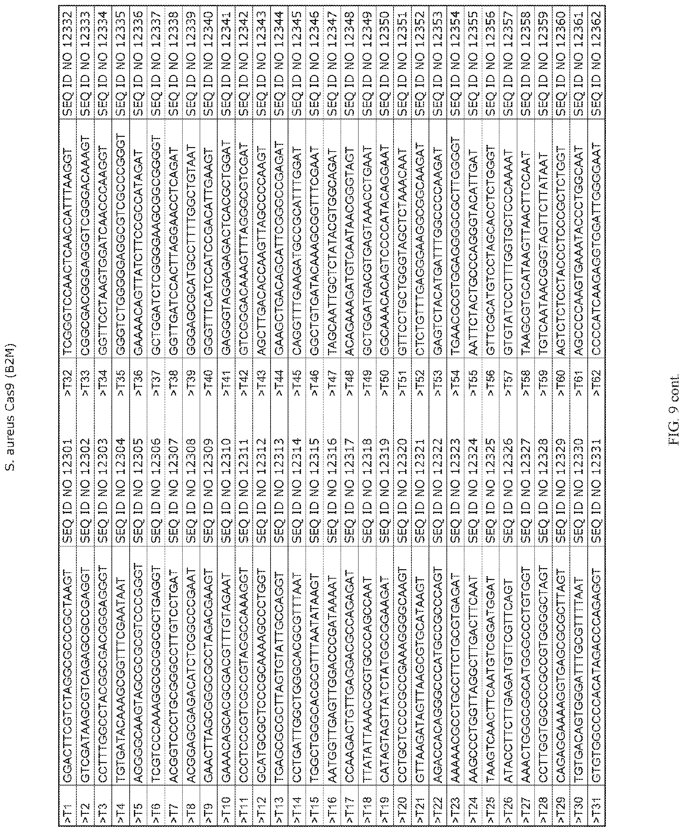

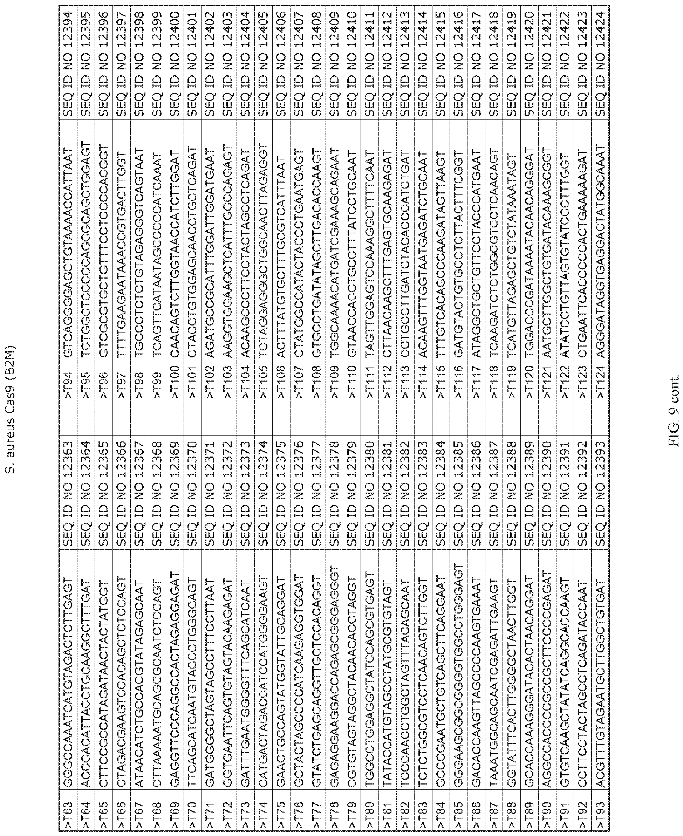

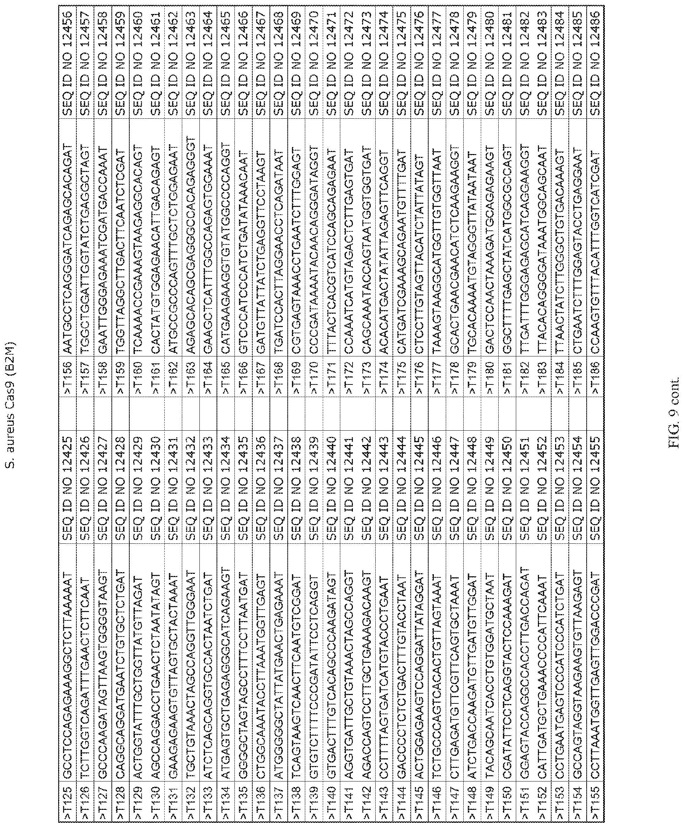

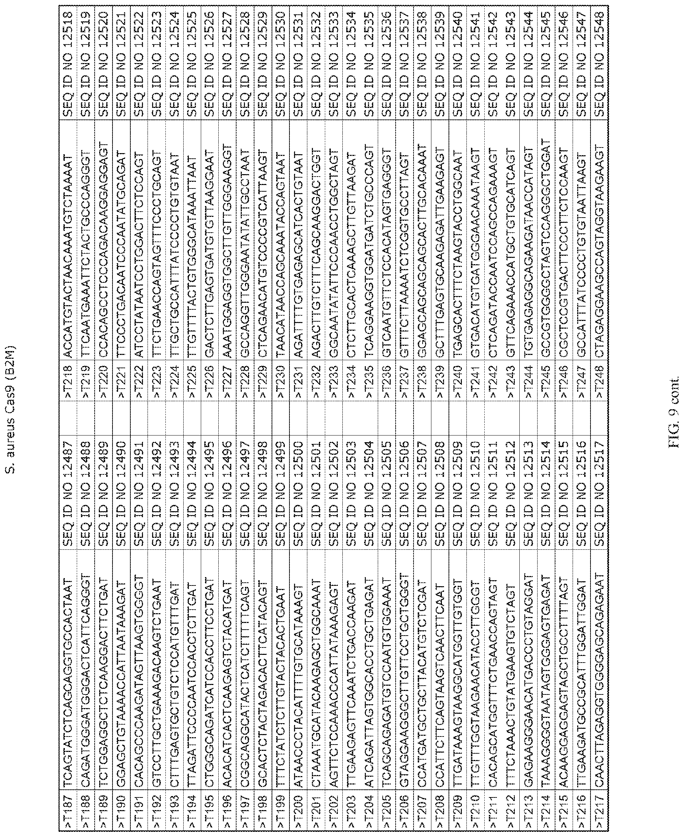

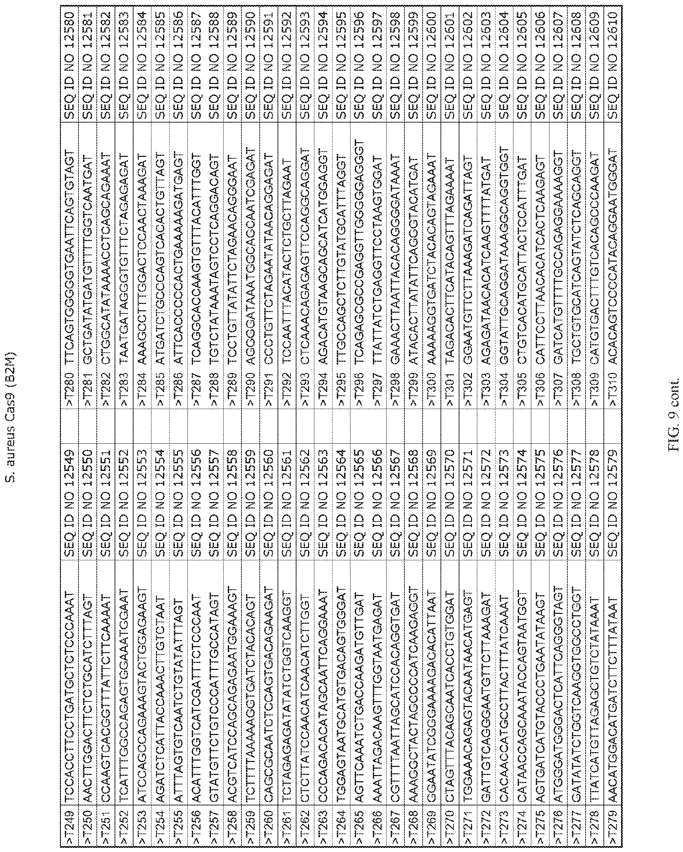

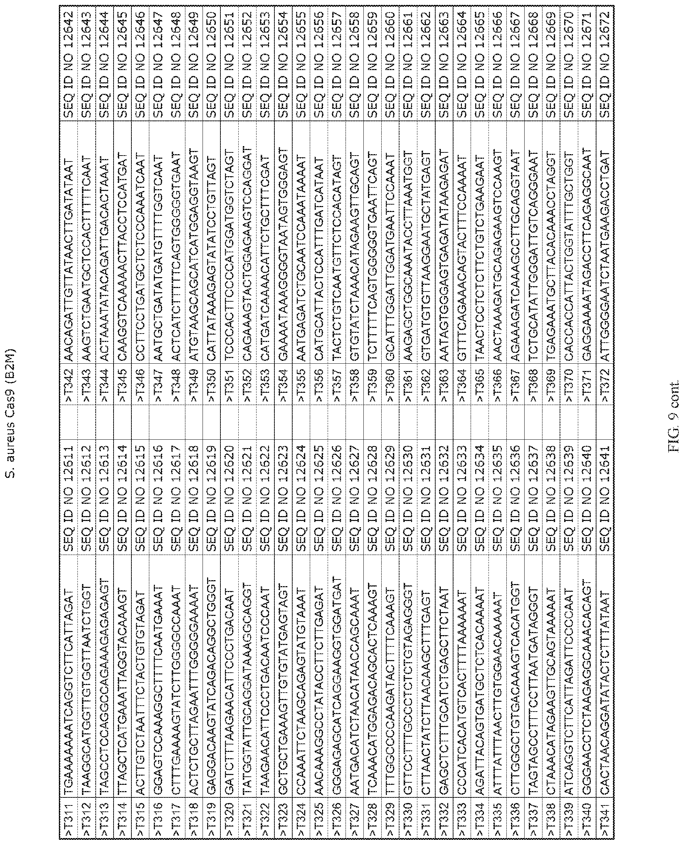

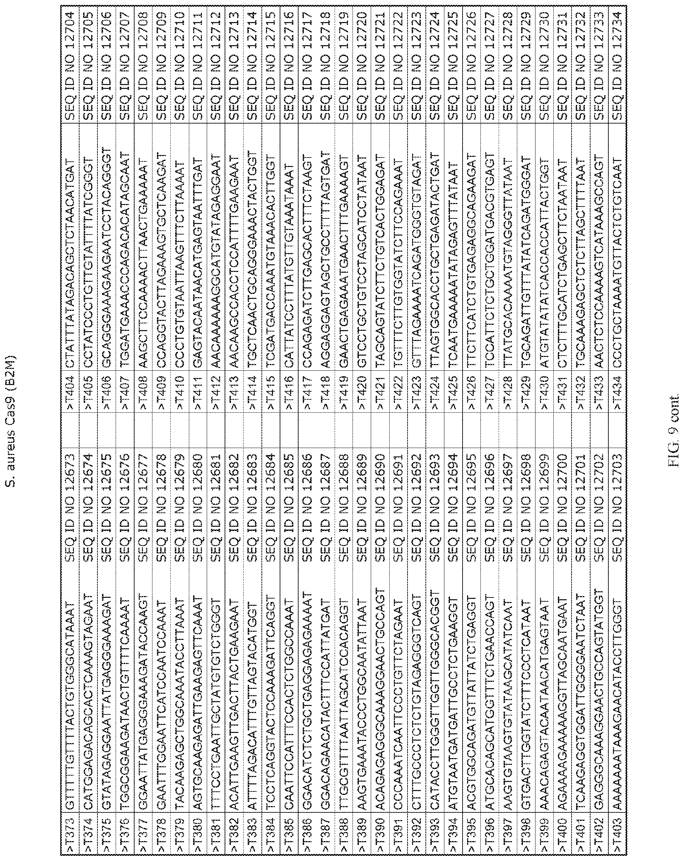

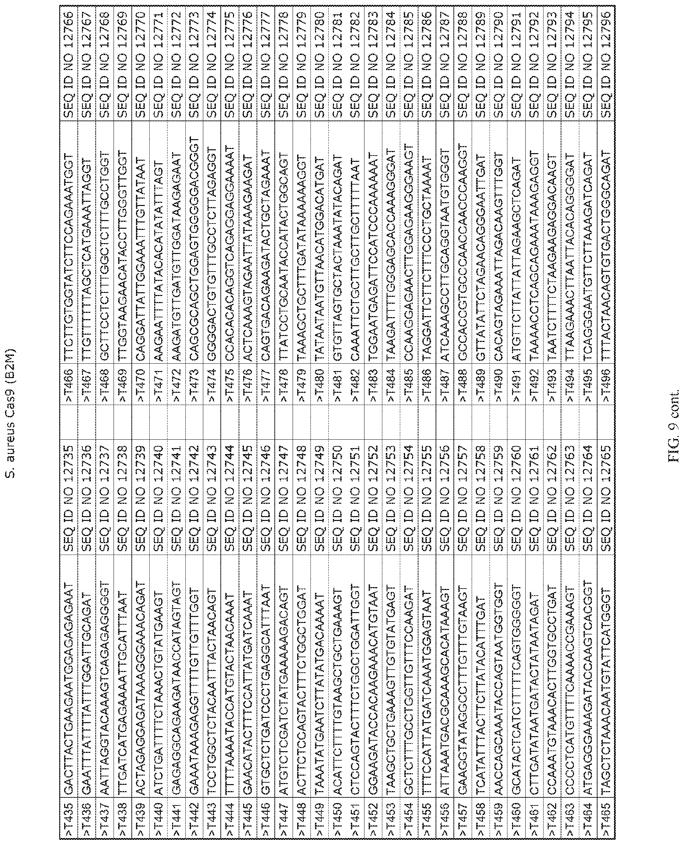

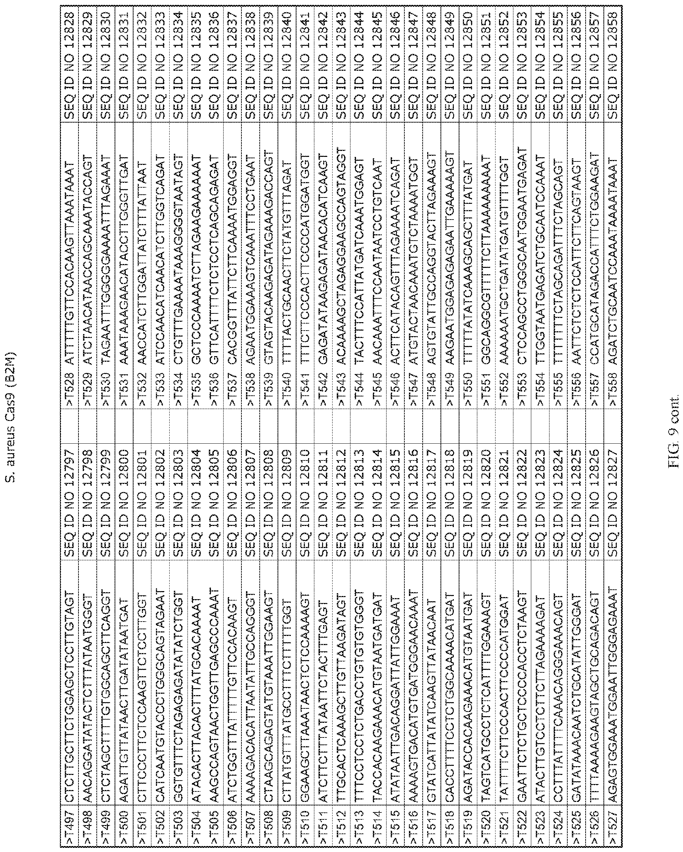

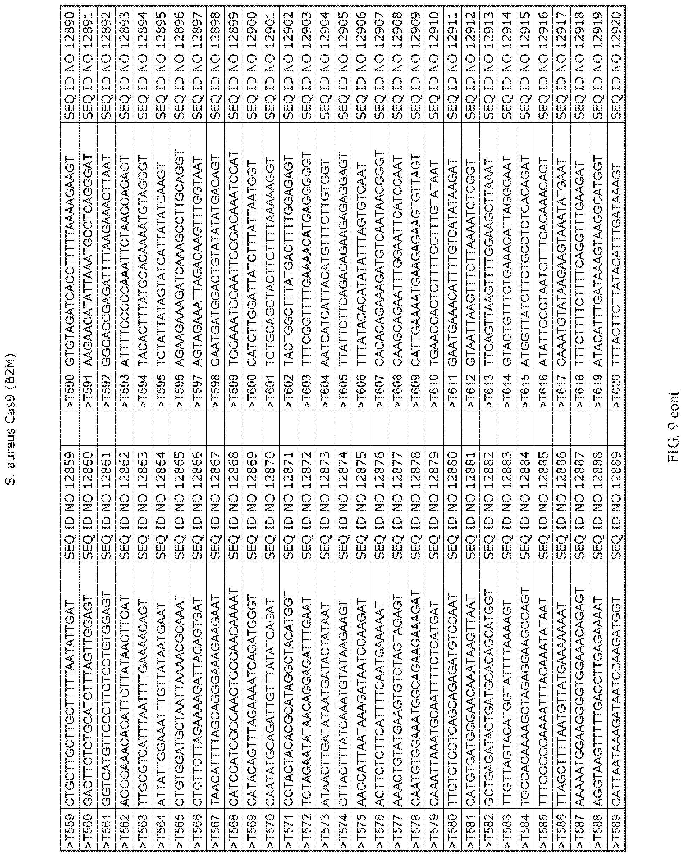

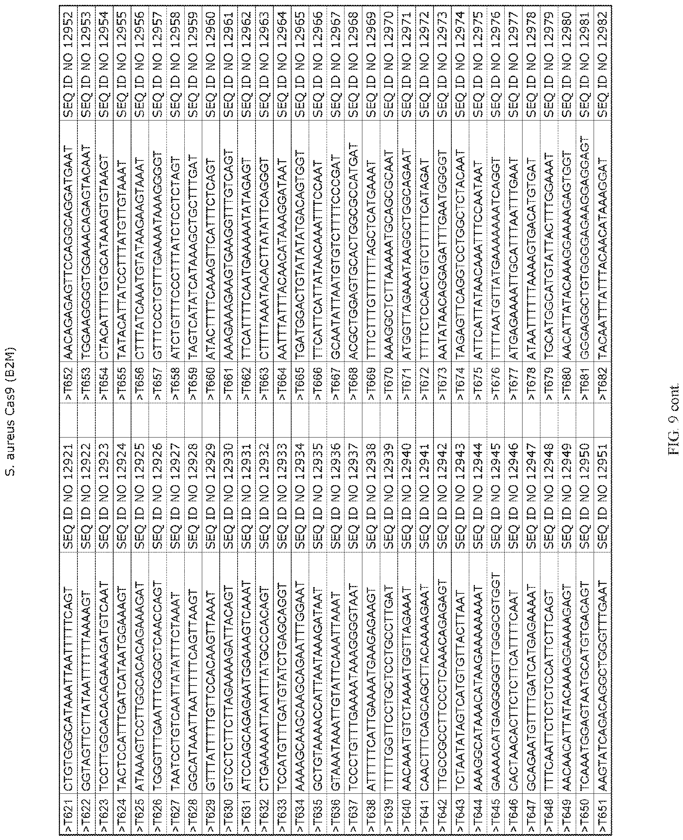

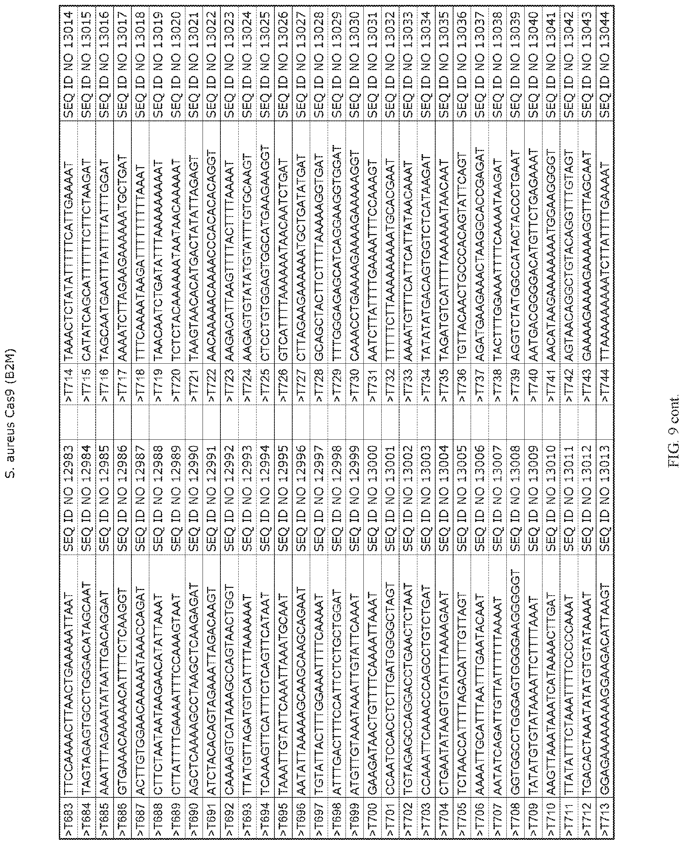

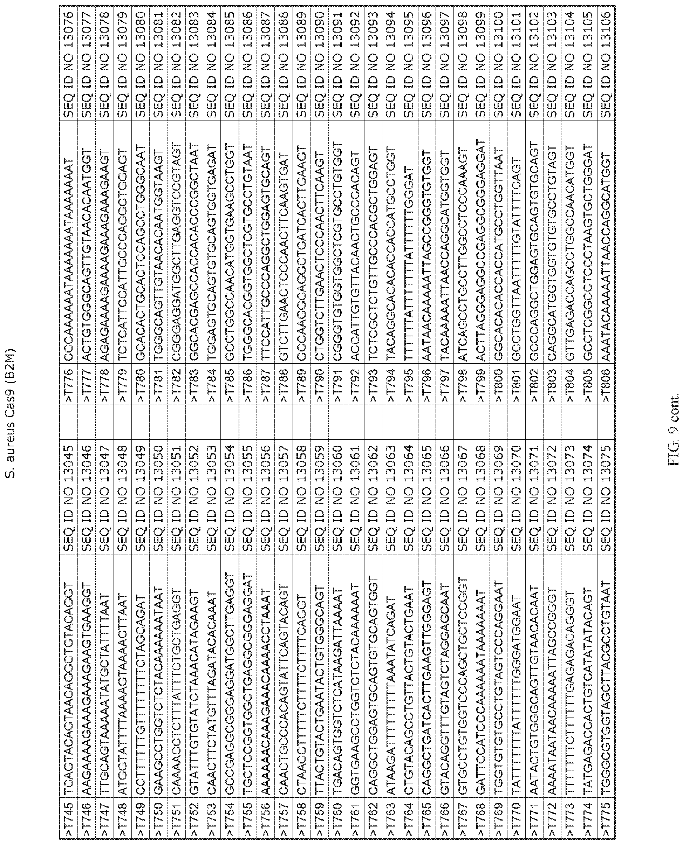

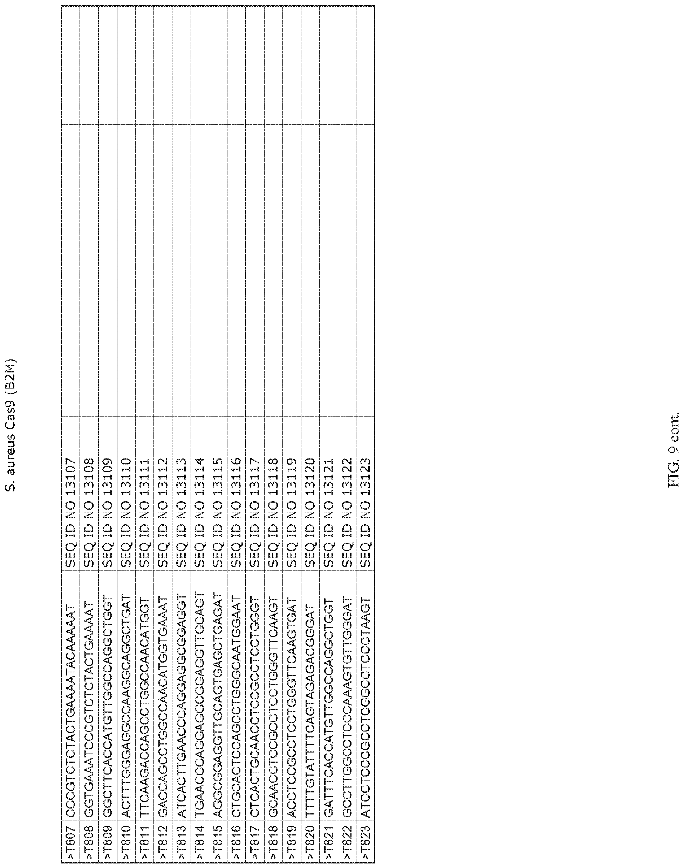

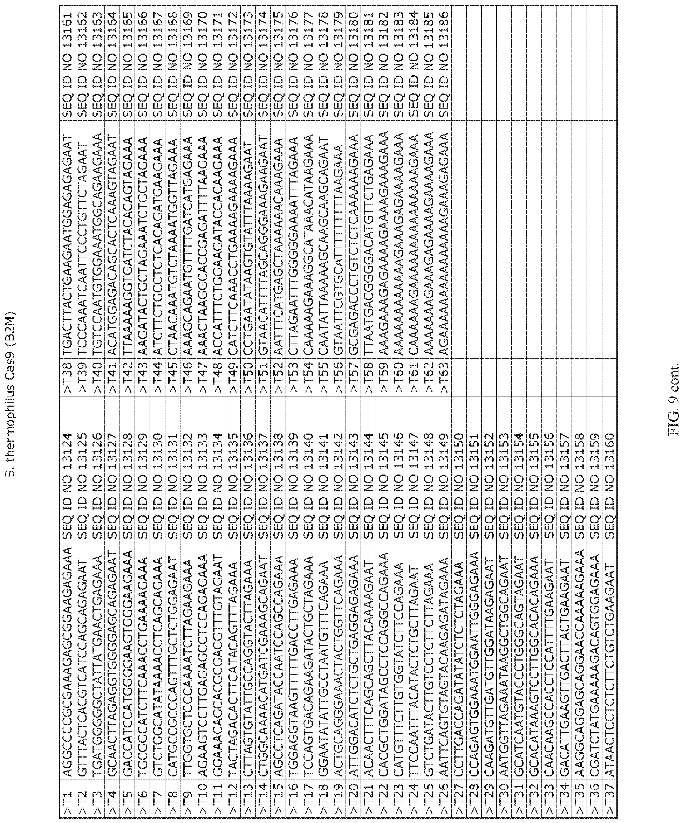

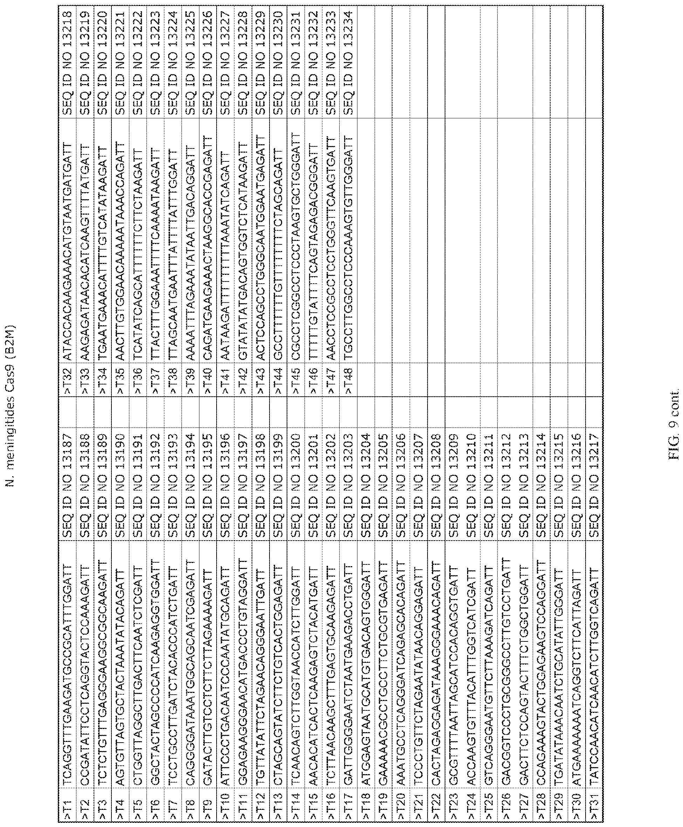

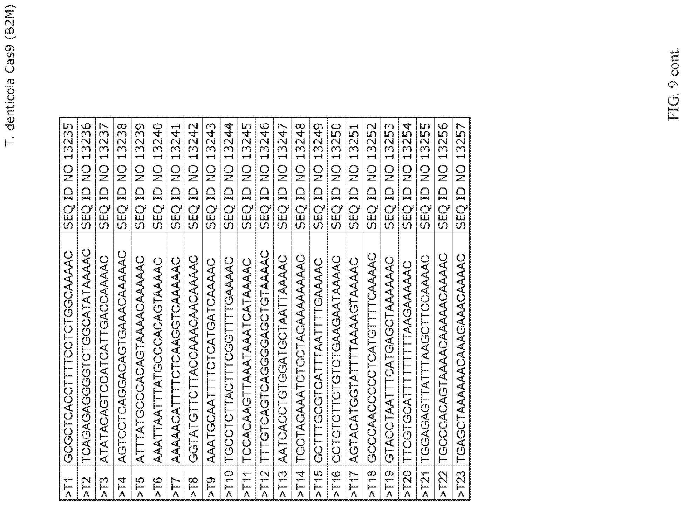

FIG. 9 shows exemplary gRNA sequences useful for targeting and editing the human B2M gene using Cas9.
FIG. 10 demonstrates an exemplary TCR targeting strategy of the present invention. FIG. 10 is a schematic illustration depicting the location of the CRISPR gRNAs targeting the first coding exons of the TCRalpha and TCRbeta chains, respectively.
FIG. 11A and FIG. 11B demonstrate deletion of T cell receptor in Jurkat T cells. FIG. 11A depicts the results of a FACS analysis employing an anti-CD3 antibody, which reveals successful TCR deletion in Jurkat T cells that stably express the Cas9 nuclease. FIG. 11B shows the results of a SURVEYOR^{™} assay confirming cutting at the TCRa and TCRb loci.
FIG. 12A and FIG. 12B demonstrate TCR Deletion in Primary Human CD3+ T Cells. FIG. 12A shows the results of a SURVEYOR^{™} assay demonstrating CRISPR cutting at the TCRa and TCRb loci in CD3+ T cells obtained from two independent donors. FIG. 12B shows the loss of TCR surface expression demonstrated by FACS analysis.
FIG. 13A, FIG. 13B, and FIG. 13C demonstrate an exemplary PD-1 locus targeting strategy of the present invention. FIG. 13A is a schematic representation of the *PD-1* targeting strategy. FIG. 13B demonstrates that the double CRISPR strategy results in cutting by both CRISPRs targeting the PD-1 locus in HEK293T cells. FIG. 13C is a schematic representation of sequencing, which confirmed the predicted deletion in the PD-1 locus after transfection of two CRISPRs targeting the PD-1 gene (*PDCD1*) as shown with reference to SEQ ID NOS: 793 and 794.
FIG. 14A and FIG. 14B demonstrate the loss of PD-1 expression in Jurkat T cells. FIG. 14A shows the results of FACS analysis, demonstrating the loss of PD-1 expression in activated Jurkat T cells. FIG. 14B shows the results of a SURVEYOR^{™} assay confirming cutting at the PD-1 locus.
FIG. 15A, FIG. 15B and FIG. 15C demonstrate an exemplary CTLA4 locus targeting strategy of the present invention. FIG. 15A is a schematic representation of the *CTLA4* targeting strategy. FIG. 15B demonstrates that the double CRISPR strategy results in cutting by both CRISPRs targeting the CTLA4 locus in HEK293T cells. FIG. 15C is a schematic representation of sequencing, which confirmed the predicted deletion in the CTLA4 locus after transfection of two CRISPRs targeting the CTLA4 gene (*CTLA4*) as shown with reference to SEQ ID NOS: 795 and 796.
FIG. 16A and FIG. 16B demonstrate cutting at the CTLA-4 locus in Jurkat T cells. FIG. 16A demonstrates that the double CRISPR strategy results in cutting by both CRISPRs targeting the CTLA4 locus in Jurkat T cells. FIG. 16B shows the results of a SURVEYOR^{™} assay, demonstrating successful cutting by both CTLA4 CRISPRs in Jurkat T cells.

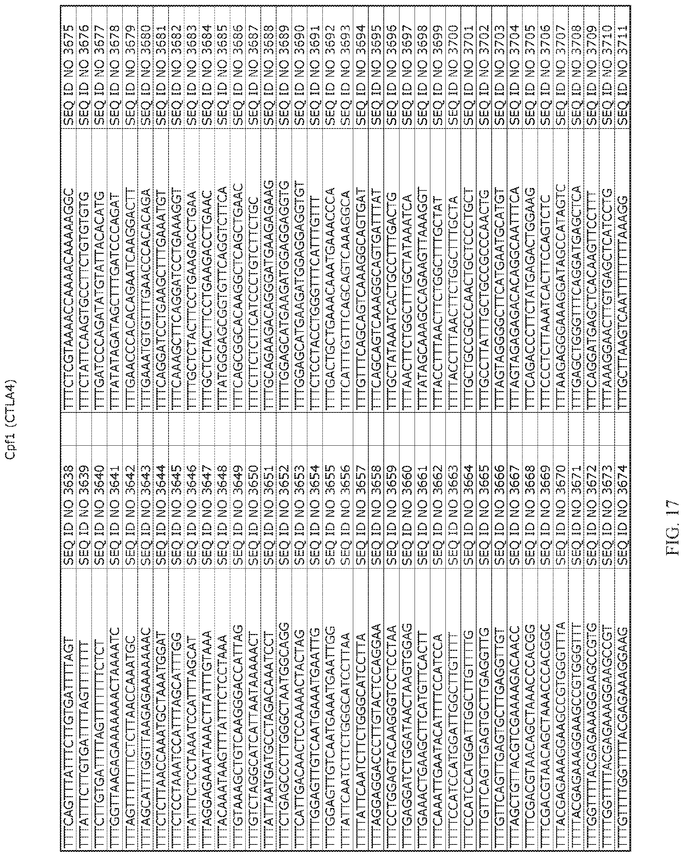

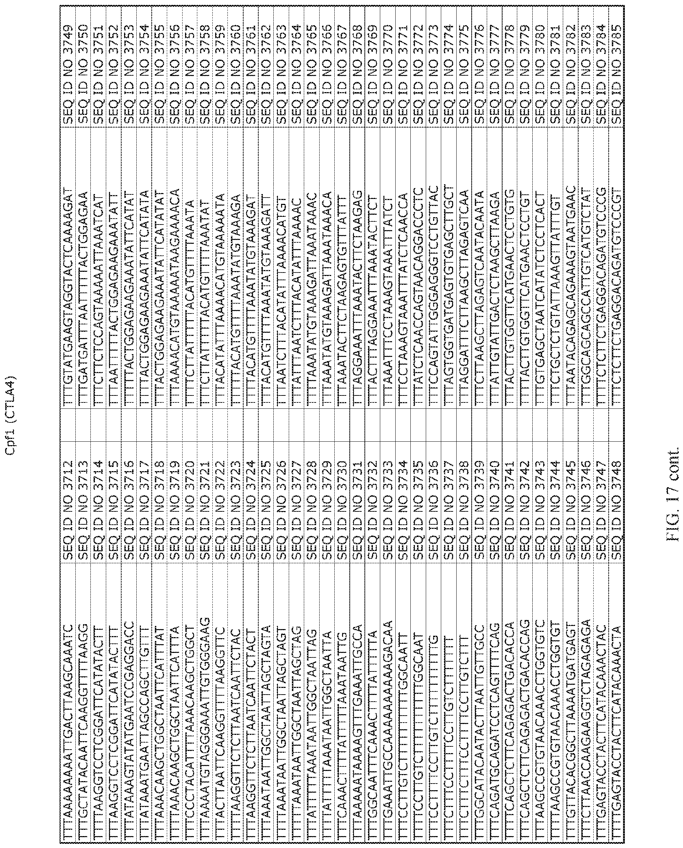

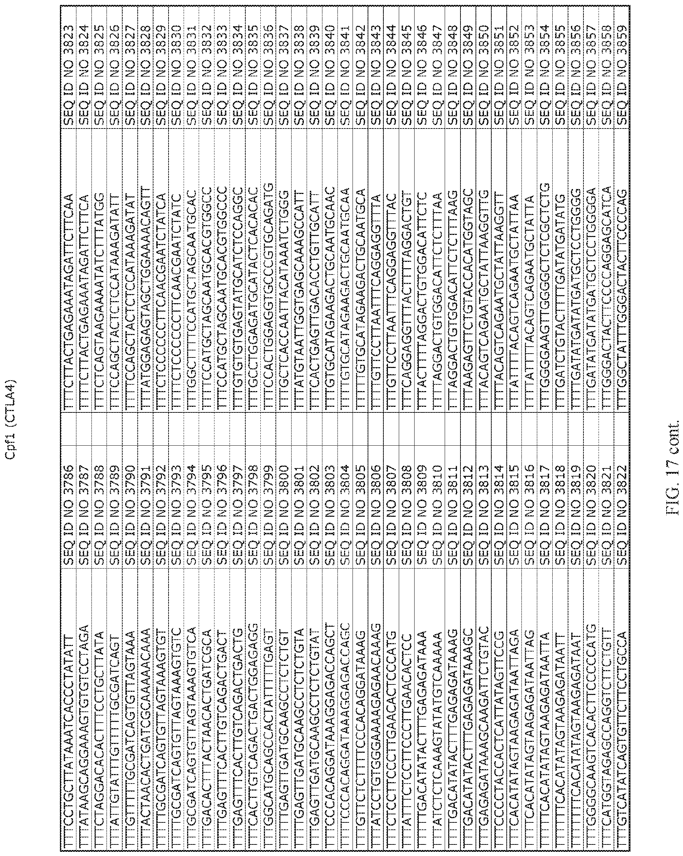

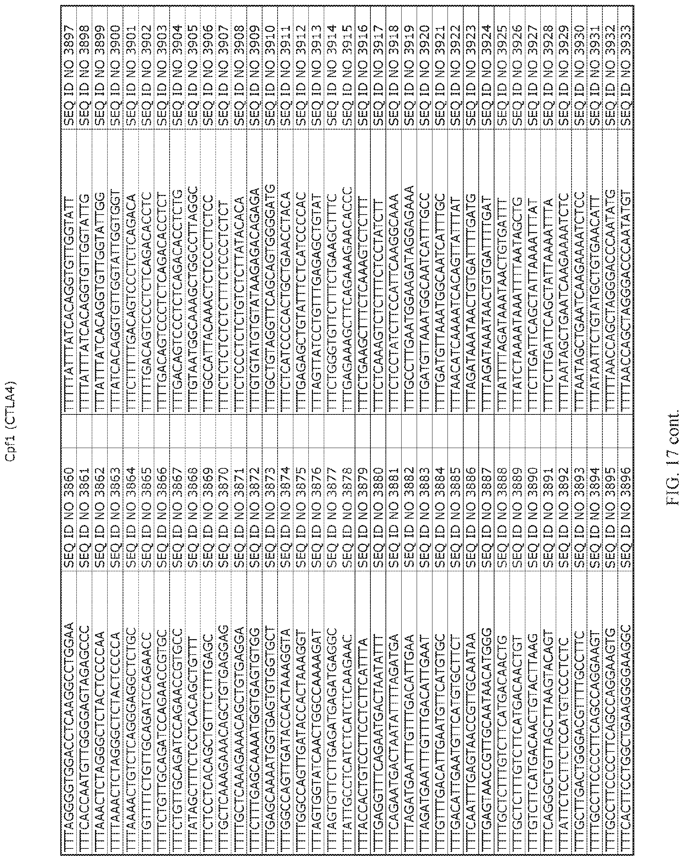

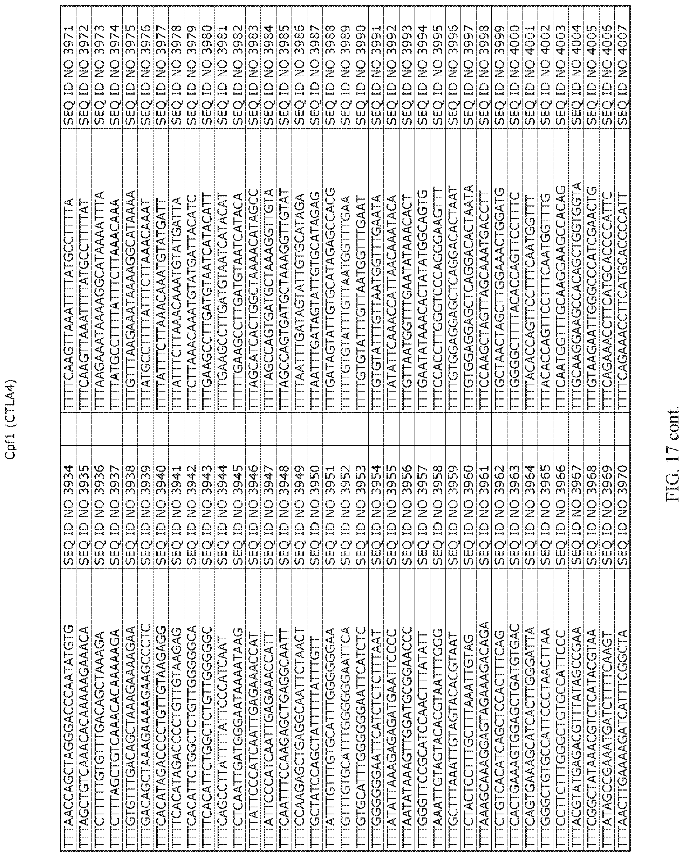

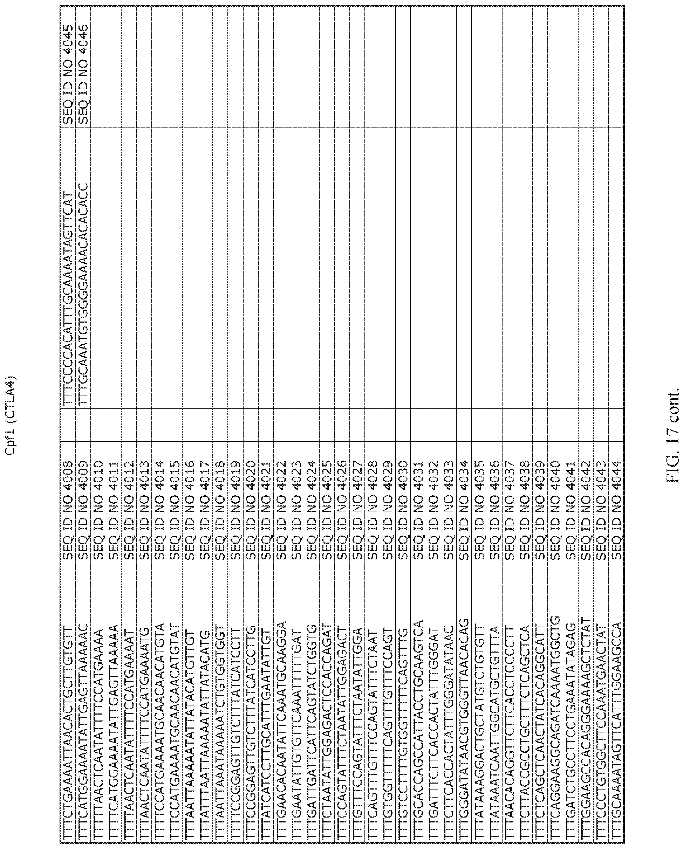

FIG. 17 shows exemplary gRNA sequences uself for targeting the CTLA4 gene using Cpfl.

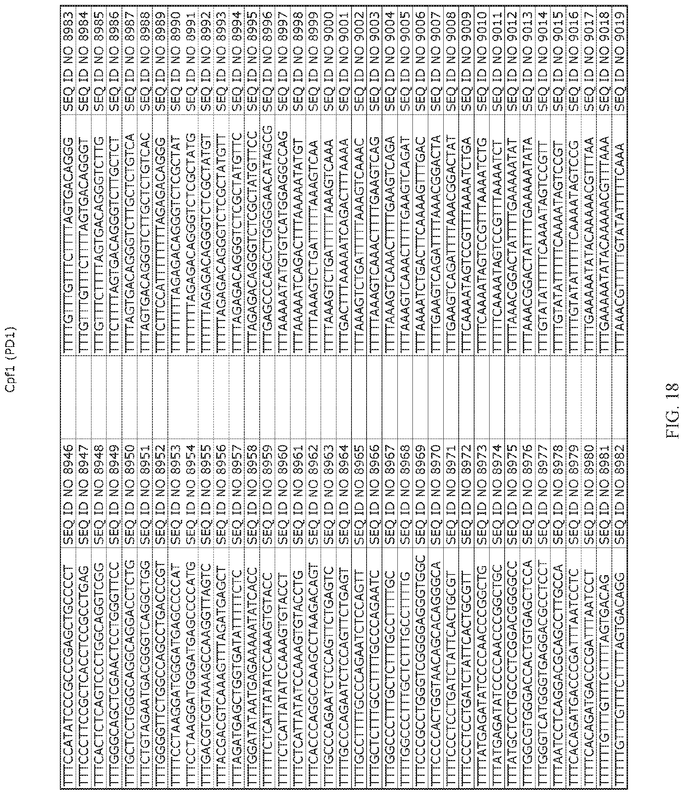

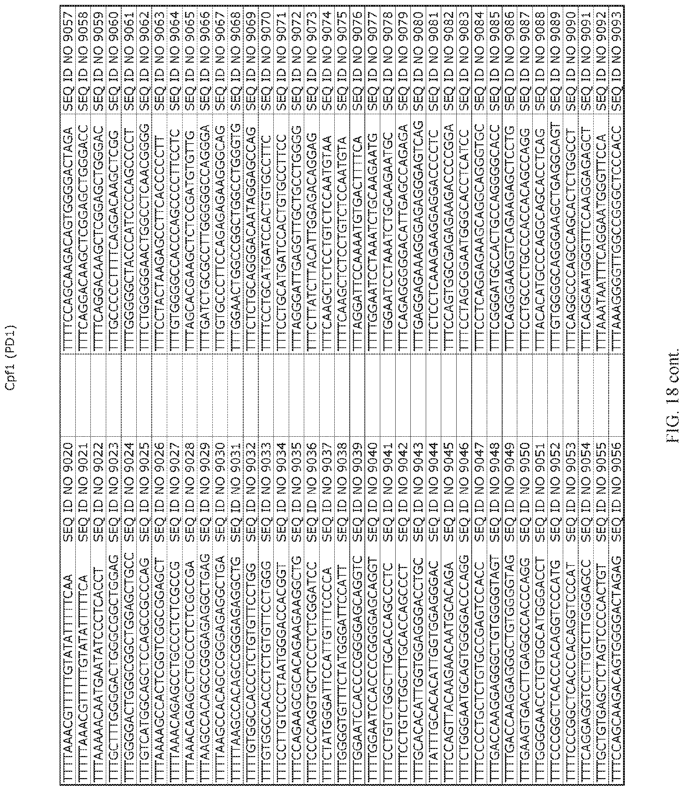

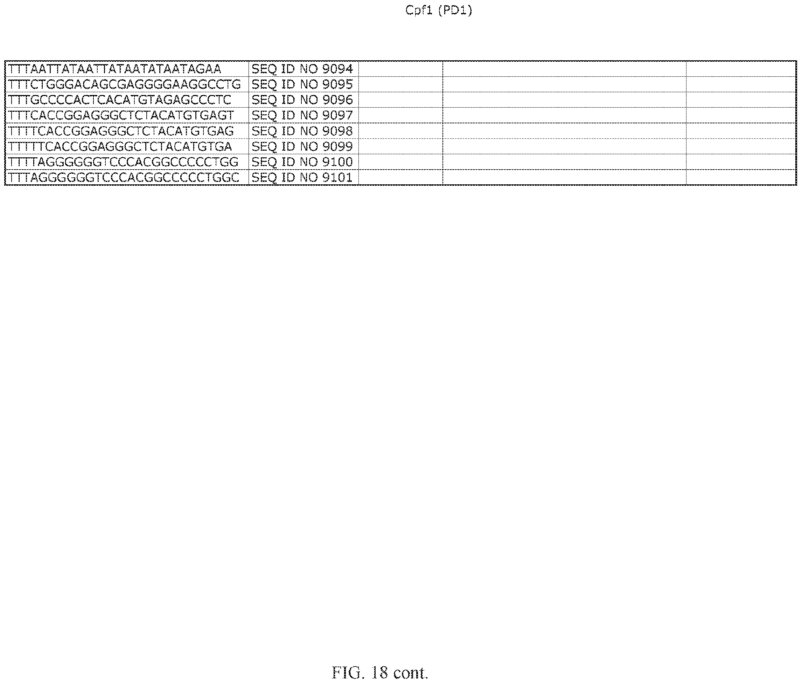

FIG. 18 shows exemplary gRNA sequences useful for targeting the PD1 gene using Cpfl.

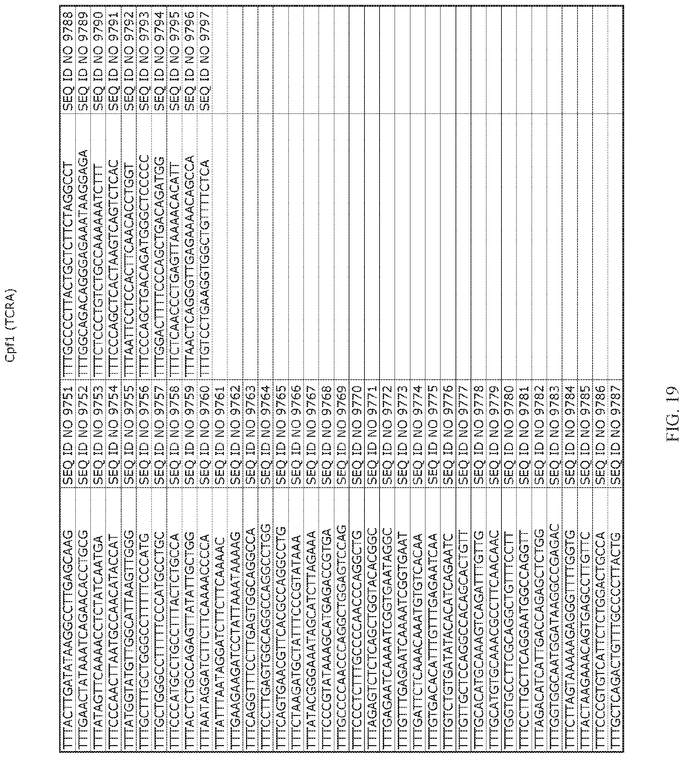

FIG. 19 shows exemplary gRNA sequences useful for targeting the TCRalpha locus using Cpfl.

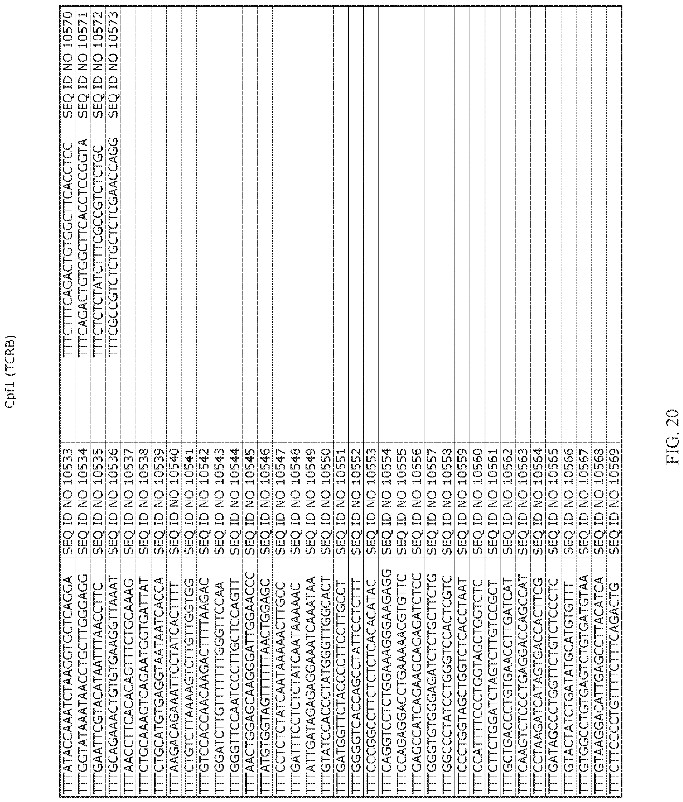

FIG. 20 shows exemplary gRNA sequences useful for targeting the human TCRbeta locus using Cpfl.

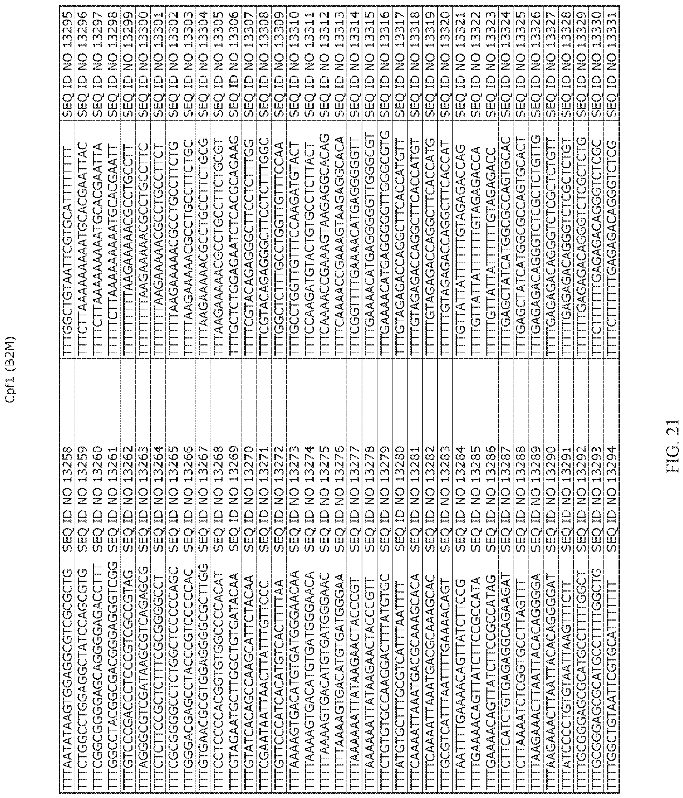

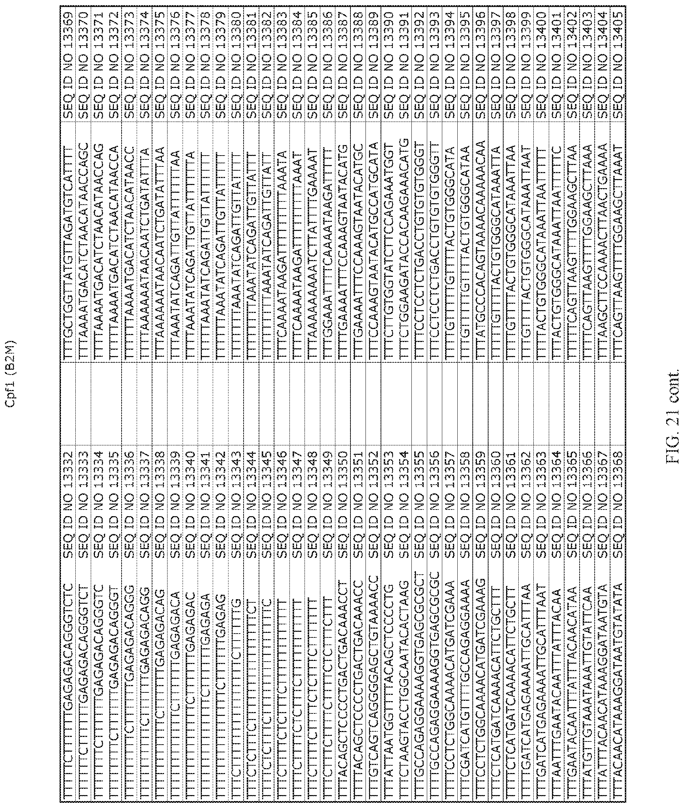

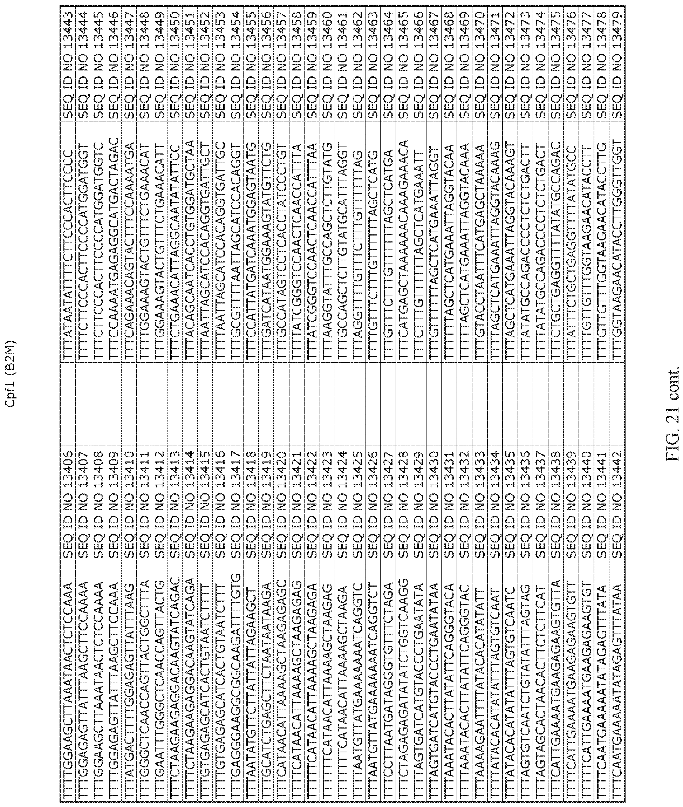

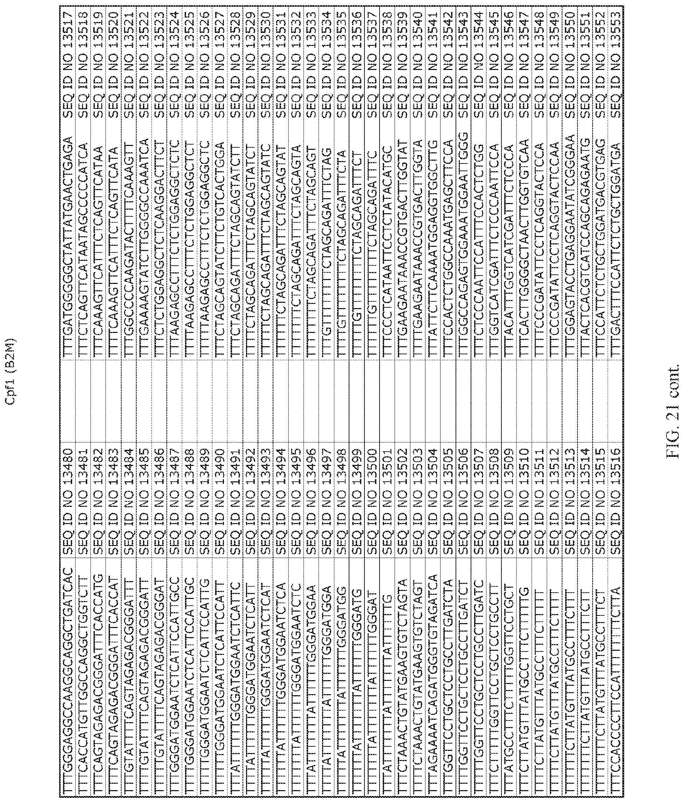

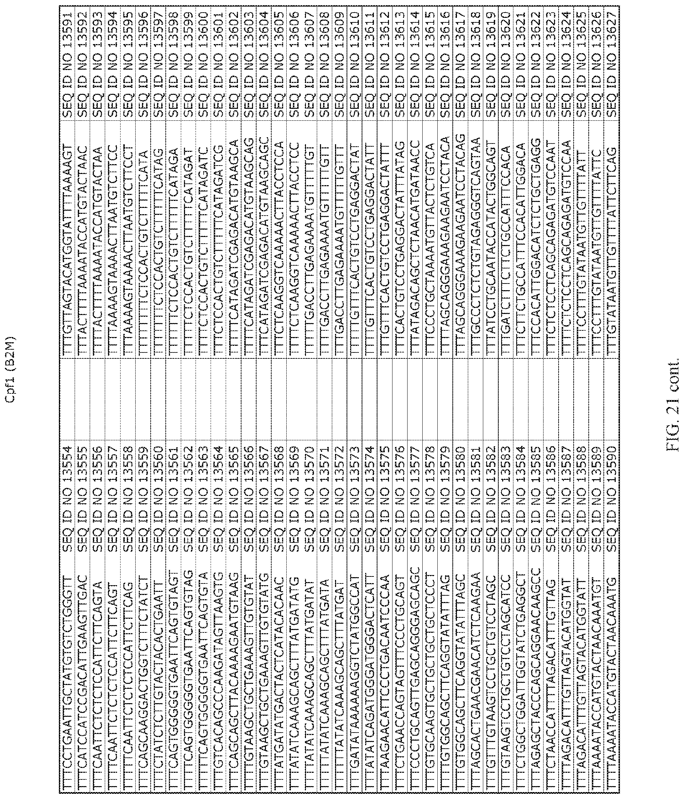

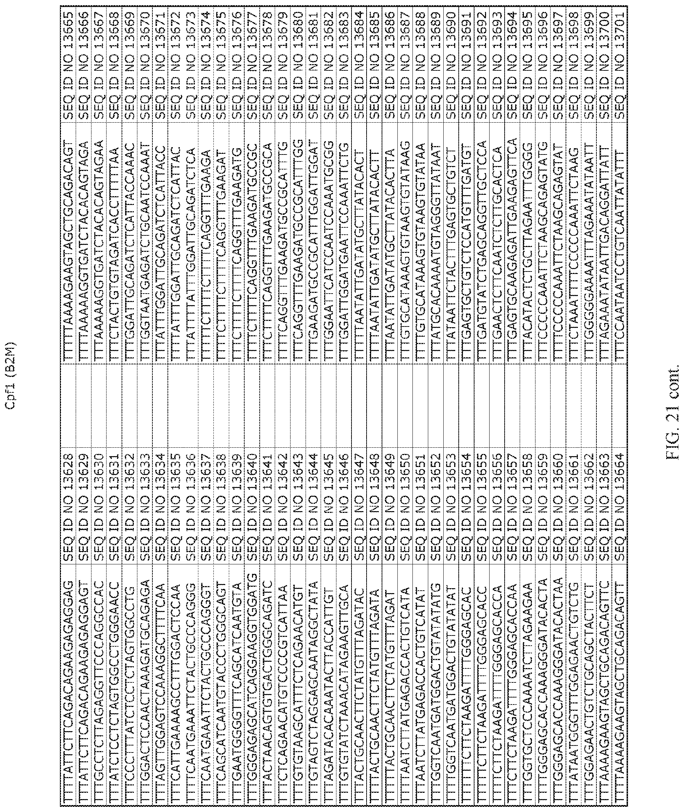

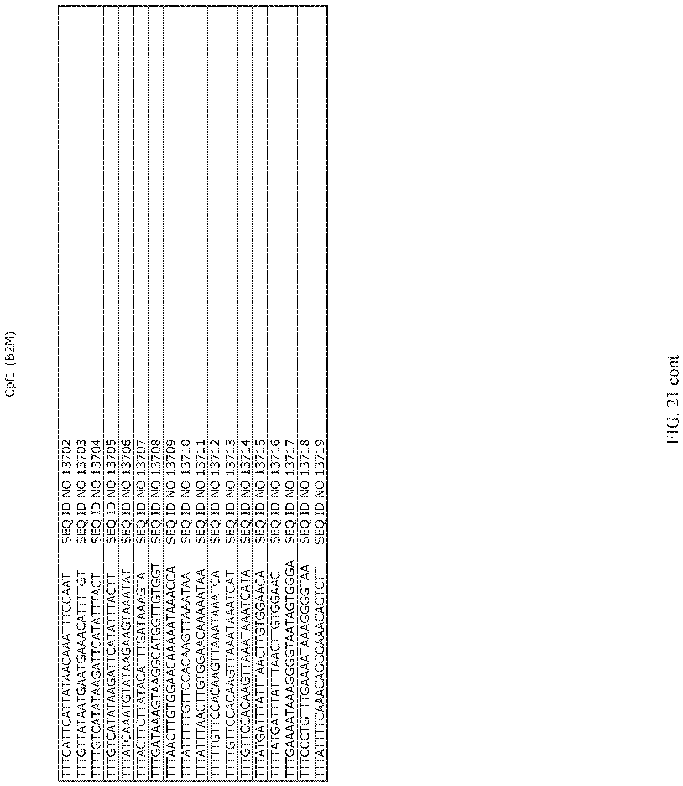

FIG. 21 shows exemplary gRNA sequences useful for targeting and editing the human B2M gene using Cpfl.

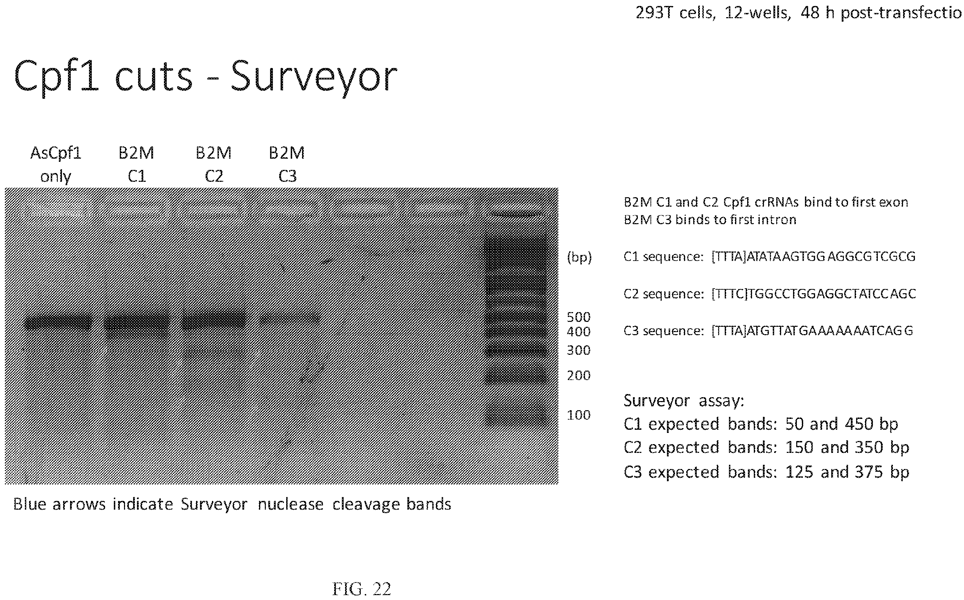

FIG. 22 demonstrates B2M deletion efficiencies of selected guides in 293T cells. Arrows on the Surveyor assays show nuclease cleavage bonds.

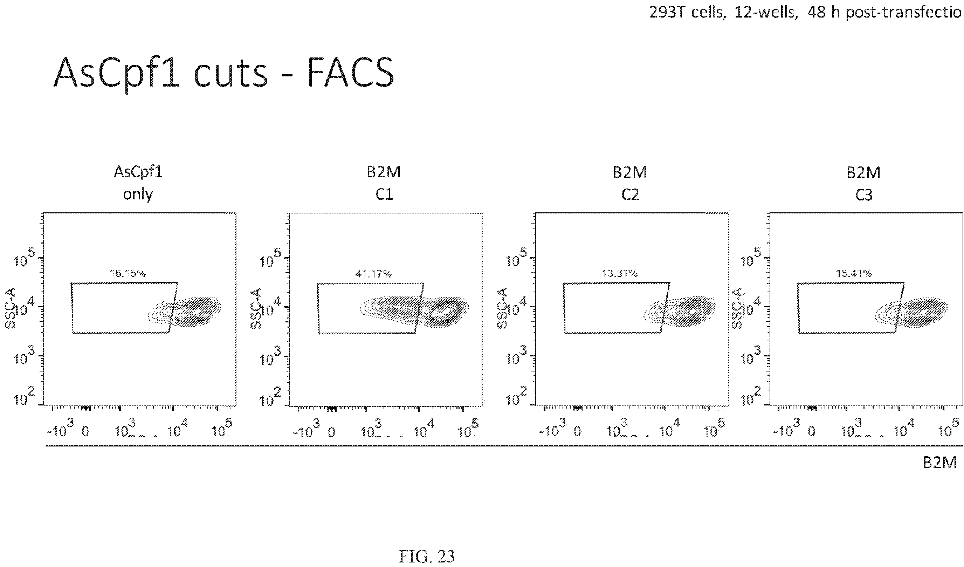

FIG. 23 demonstrates a comparison of B2M surface expression in 293T cells when transfected with AsCpf1 and guide crB2M.

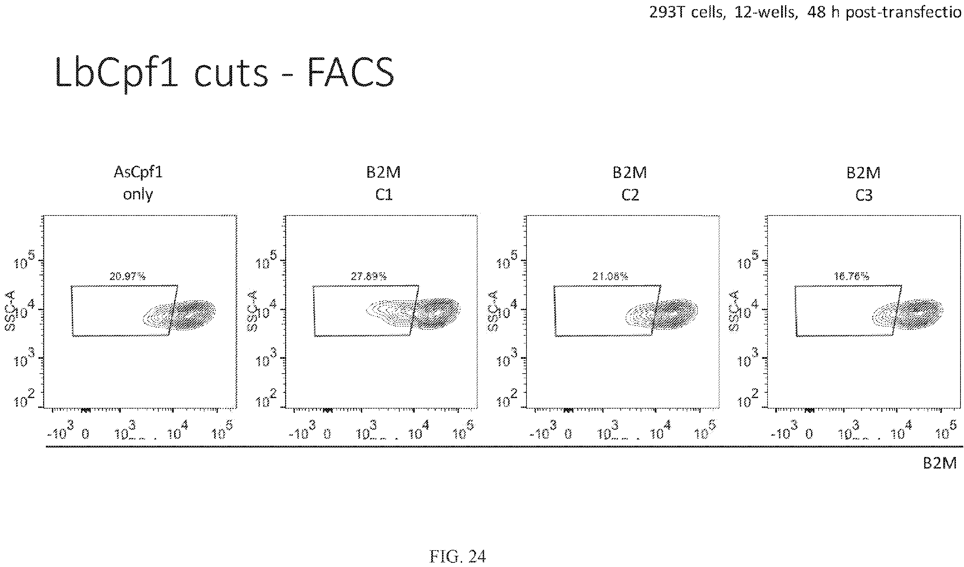

FIG. 24 demonstrates a comparison of B2M surface expression in 293T cells when transfected with LbCpf1 and guide crB2M.

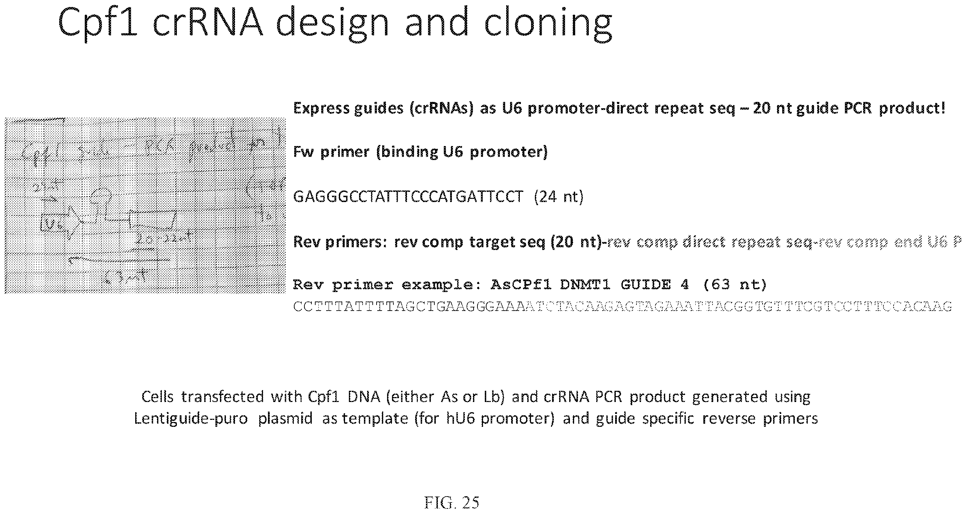

FIG. 25 depicts Cpfl crRNA design and cloning information.

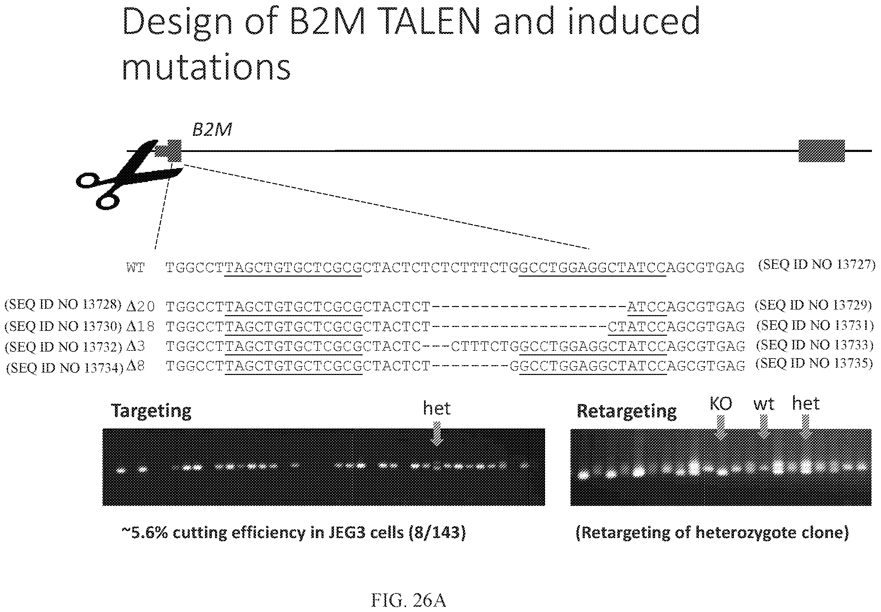

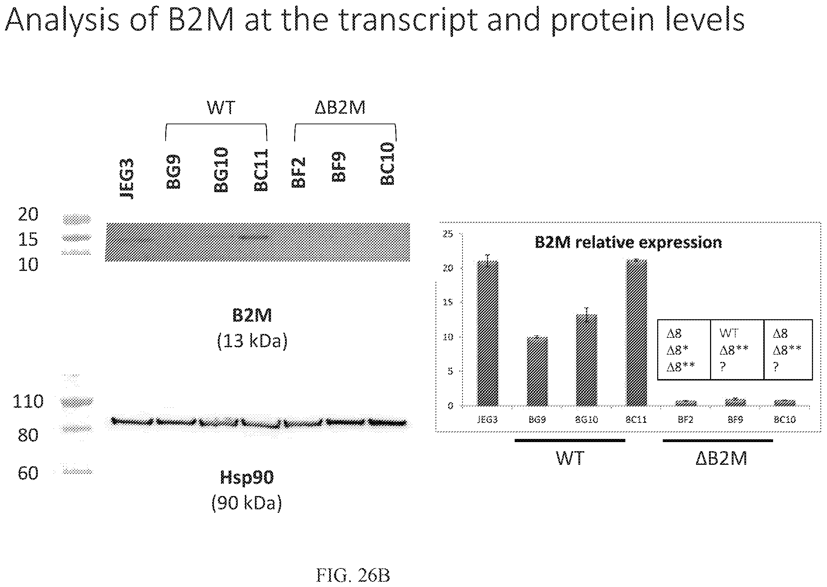

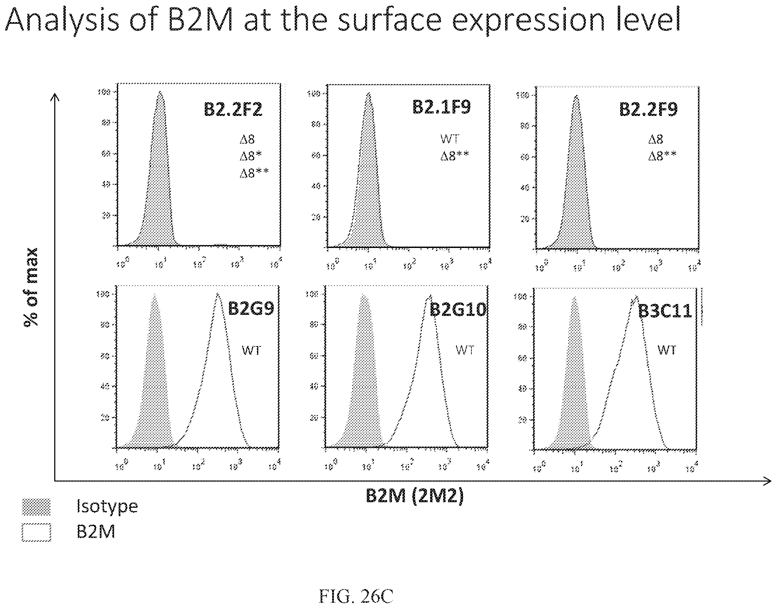

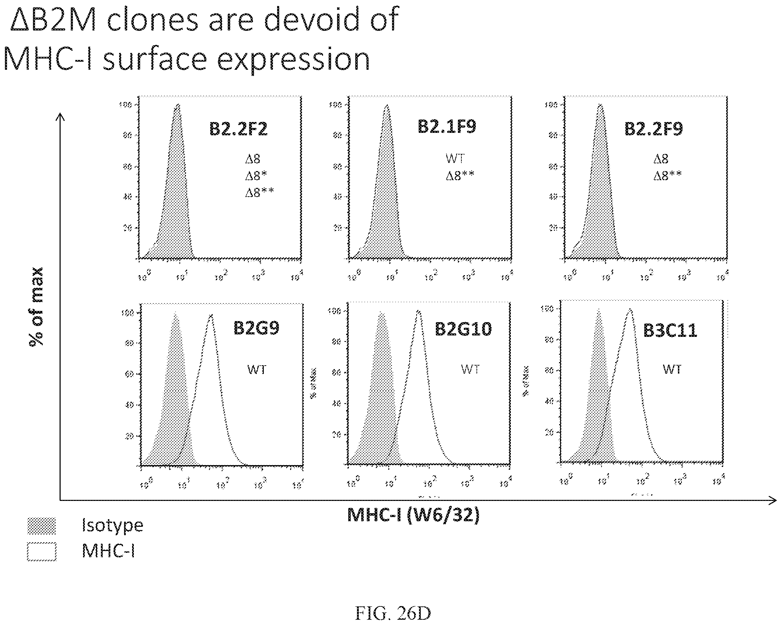

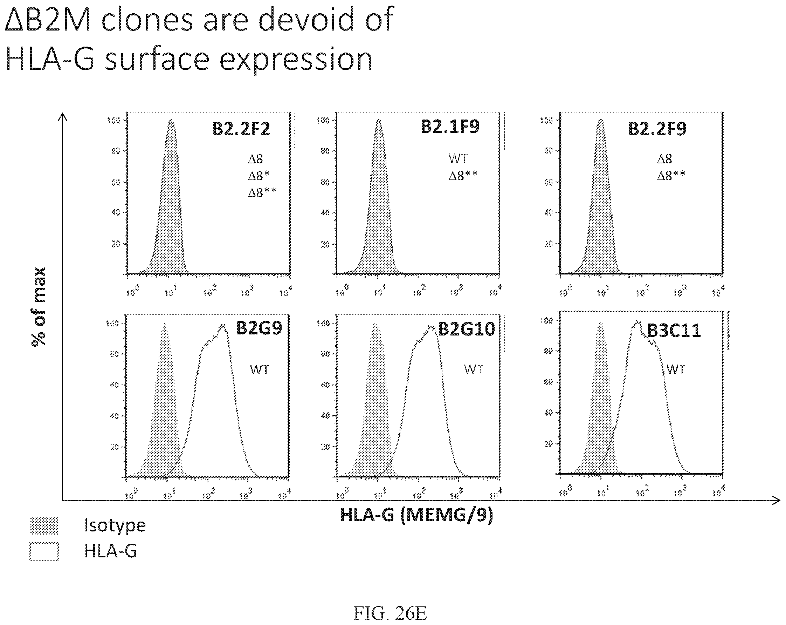

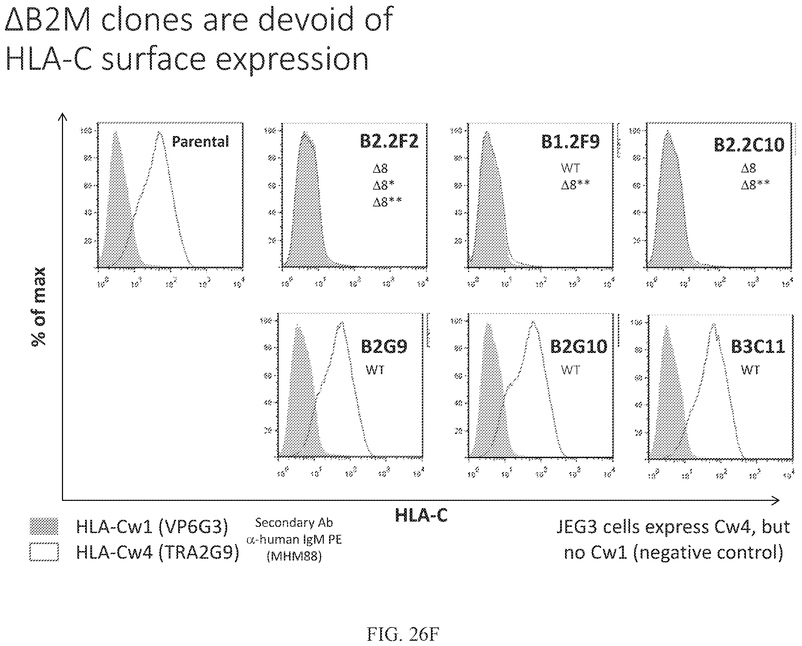

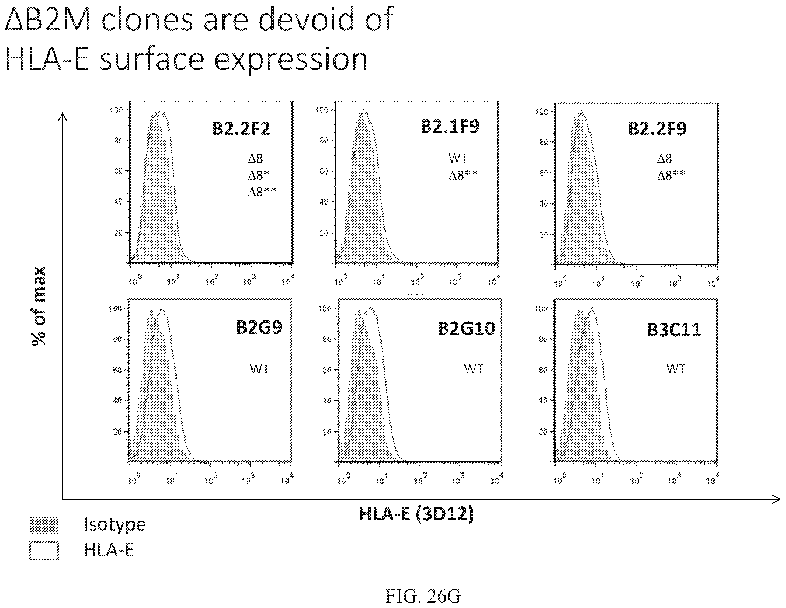

FIG. 26A, FIG. 26B, FIG. 26C, FIG. 26D, FIG. 26E, FIG. 26F, and FIG. 26G demonstrate generation and characterization of B2M KO JEG3 cells using TALENs. FIG. 26A depicts a design of B2M TALEN and induced mutations. FIG. 26B depicts an analysis of B2M at the transcript and protein levels. FIG. 26C demonstrates an analysis of B2M at the surface expression level. FIG. 26D demonstrates that ΔB2M clones are devoid of MHC-I surface expression. FIG. 26E demonstrates that ΔB2M clones are devoid of HLA-G surface expression. FIG. 26F demonstrates that ΔB2M clones are devoid of HLA-C surface expression. FIG. 26G demonstrates that ΔB2M clones are devoid of HLA-E surface expression.

### DETAILED DESCRIPTION OF THE INVENTION

T cell therapy is emerging as a major breakthrough in cancer immunotherapy and currently there are several clinical trials using modified T cells primarily in the treatment of B cell malignancies. T cells can be genetically modified to express tumor-specific chimeric antigen receptors (CAR) with specificity derived from the variable domains of a monoclonal antibody, thus focusing immunoreactivity toward the tumor in a major histocompatibility complex (MHC) non-restricted manner.

Despite these early successes, there are several obstacles to existing CAR T therapies. The predominant roadblock is the continuous presence of the endogenous T cell receptor (TCR), which prevents allotransplantation of CAR T cells, and even if the cells are given back to the same patient may result in autoimmune attack if the cells are cross-reactive with self-antigen, when administered in high dose to a patient. The second obstacle in T cell therapy is that tumors and viruses have evolved mechanisms to suppress T cells by exploiting critical checkpoint regulators of T cell activity. The present invention harnesses the potential of genetic editing systems, such as the CRISPR/Cas or TALEN systems, to overcome the aforementioned roadblocks that prevent the safe translation of this new therapeutic option into the clinic. In particular, work described herein demonstrates the feasibility of generating off-the-shelf universal CAR T cells from allogeneic healthy donors that can be administered to any patient without the risk of immune rejection or graft versus host disease (GvHD) and which are not prone to T cell inhibition. Moreover, the work described herein demonstrates that it is feasible to develop CRISPR guide sequences (gRNAs) that efficiently target the endogenous TCR, as well as critical checkpoint regulators of T cell activity. Work described herein provides gRNAs designed and tested to: (1) prevent autoreactivity by targeting the genes encoding the TCR alpha and beta chains; (2) break down the allobarrier by targeting the TCR and B2M genes; and/or (3) overcome autoreactivity by targeting the checkpoint inhibitors PD-1 and CTLA4.

The present invention contemplates altering target polynucleotide sequences in any manner which is available to the skilled artisan, for example, utilizing a TALEN or a CRISPR/Cas system. Such CRISPR/Cas systems can employ a variety of Cas proteins (Haft et al. PLoS Comput Biol. 2005;1(6)e60). In some embodiments, the CRISPR/Cas system is a CRISPR type I system. In some embodiments, the CRISPR/Cas system is a CRISPR type II system. In some embodiments, the CRISPR/Cas system is a CRISPR type V system. It should be understood that although examples of methods utilizing CRISPR/Cas (e.g., Cas9 and Cpfl) and TALEN are described in detail herein, the invention is not limited to the use of these methods/systems. Other methods of targeting polynucleotide sequences to reduce or ablate expression in target cells known to the skilled artisan can be utilized herein.

According to methods of the present invention, one or more target polynucleotide sequence in a cell are altered, e.g., modified or cleaved. The present invention contemplates altering target polynucleotide sequences in a cell for any purpose but particularly such that the expression or activity of the encoded product is reduced or eliminated. In some embodiments, the target polynucleotide sequence in a cell is altered to produce a mutant cell. As used herein, a "mutant cell" refers to a cell with a resulting genotype that differs from its original genotype. In some instances, a "mutant cell" exhibits a mutant phenotype, for example when a normally functioning gene is altered using the CRISPR/Cas systems of the present invention. In other instances, a "mutant cell" exhibits a wild-type phenotype, for example when a CRISPR/Cas system is used to correct a mutant genotype. In some embodiments, the target polynucleotide sequence in a cell is altered to correct or repair a genetic mutation (e.g., to restore a normal phenotype to the cell). In some embodiments, the target polynucleotide sequence in a cell is altered to induce a genetic mutation (e.g., to disrupt the function of a gene or genomic element).

In some embodiments, the alteration is an indel. As used herein, "indel" refers to a mutation resulting from an insertion, deletion, or a combination thereof. As will be appreciated by those skilled in the art, an indel in a coding region of a genomic sequence will result in a frameshift mutation, unless the length of the indel is a multiple of three. In some embodiments, the alteration is a point mutation. As used herein, "point mutation" refers to a substitution that replaces one of the nucleotides. A CRISPR/Cas system can be used to induce an indel of any length or a point mutation in a target polynucleotide sequence.

In some embodiments, the alteration results in a knock out of the target polynucleotide sequence or a portion thereof. For example, knocking out a target polynucleotide sequence in a cell can be performed *in vitro, in vivo or ex vivo* for both therapeutic and research purposes. Knocking out a target polynucleotide sequence in a cell can be useful for treating or preventing a disorder associated with expression of the target polynucleotide sequence (e.g., by knocking out a mutant allele in a cell *ex vivo* and introducing those cells comprising the knocked out mutant allele into a subject). As used herein, "knock out" includes deleting all or a portion of the target polynucleotide sequence in a way that interferes with the function of the target polynucleotide sequence or its expression product.

In some embodiments, the alteration results in reduced expression of the target polynucleotide sequence. The terms "decrease," "reduced," "reduction," and "decrease" are all used herein generally to mean a decrease by a statistically ssignificant amount. However, for avoidance of doubt, "decreased," "reduced," "reduction," "decrease" includes a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (i.e. absent level as compared to a reference sample), or any decrease between 10-100% as compared to a reference level.

The terms "increased," "increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

The term "statistically significant" or "significantly" refers to statistical significance and generally means a two standard deviation (2SD) below normal, or lower, concentration of the marker. The term refers to statistical evidence that there is a difference. It is defined as the probability of making a decision to reject the null hypothesis when the null hypothesis is actually true. The decision is often made using the p-value.

In some embodiments, the alteration is a homozygous alteration. In some embodiments, the alteration is a heterozygous alteration.

In some embodiments, the alteration results in correction of the target polynucleotide sequence from an undesired sequence to a desired sequence. CRISPR/Cas systems can be used to correct any type of mutation or error in a target polynucleotide sequence. For example, CRISPR/Cas systems can be used to insert a nucleotide sequence that is missing from a target polynucleotide sequence due to a deletion. CRISPR/Cas systems can also be used to delete or excise a nucleotide sequence from a target polynucleotide sequence due to an insertion mutation. In some instances, CRISPR/Cas systems can be used to replace an incorrect nucleotide sequence with a correct nucleotide sequence (e.g., to restore function to a target polynucleotide sequence that is impaired due to a loss of function mutation, i.e., a SNP).

CRISPR/Cas systems can alter target polynucleotides with surprisingly high efficiency. In certain embodiments, the efficiency of alteration is at least about 5%. In certain embodiments, the efficiency of alteration is at least about 10%. In certain embodiments, the efficiency of alteration is from about 10% to about 80%. In certain embodiments, the efficiency of alteration is from about 30% to about 80%. In certain embodiments, the efficiency of alteration is from about 50% to about 80%. In some embodiments, the efficiency of alteration is greater than or equal to about 80%. In some embodiments, the efficiency of alteration is greater than or equal to about 85%. In some embodiments, the efficiency of alteration is greater than or equal to about 90%, about 91%, about 92%, about 93%, about 94%, about 95%, about 96%, about 97%, about 98%, or about 99%. In some embodiments, the efficiency of alteration is equal to about 100%.

CRISPR/Cas systems can be used to alter any target polynucleotide sequence in a cell. Those skilled in the art will readily appreciate that desirable target polynucleotide sequences to be altered in any particular cell may correspond to any genomic sequence for which expression of the genomic sequence is associated with a disorder or otherwise facilitates entry of a pathogen into the cell. For example, a desirable target polynucleotide sequence to alter in a cell may be a polynucleotide sequence corresponding to a genomic sequence which contains a disease-associated single polynucleotide polymorphism (SNP). In such example, CRISPR/Cas systems can be used to correct the disease associated SNP in a cell by replacing it with a wild-type allele. As another example, a polynucleotide sequence of a target gene which is responsible for entry or proliferation of a pathogen into a cell may be a suitable target for deletion or insertion to disrupt the function of the target gene to prevent the pathogen from entering the cell or proliferating inside the cell.

In some embodiments, the target polynucleotide sequence is a genomic sequence. In some embodiments, the target polynucleotide sequence is a human genomic sequence. In some embodiments, the target polynucleotide sequence is a mammalian genomic sequence. In some embodiments, the target polynucleotide sequence is a vertebrate genomic sequence.

### Cytotoxic T-lymphocyte-associated protein 4 (CTLA4)

In some embodiments, the target polynucleotide sequence is CTLA4, or a homolog, ortholog, or variant thereof (Gene ID: 1493, also known as CD; GSE; GRD4; ALPS5; CD152; CTLA-4; IDDM12; CELIAC3). An exemplary CTLA4 human target polynucleotide sequence is shown in Table 1 below (NG_011502.1 RefSeqGene; SEQ ID NO: 782).

| Table 1 - Exemplary human CTLA4 target polynucleotide sequence |
|---|
| 1 cttctgtgtg tgcacatgtg taatacatat ctgggatcaa agctatctat ataaagtcct 61 tgattctgtg tgggttcaaa cacatttcaa agcttcagga tcctgaaagg ttttgctcta 121 cttcctgaag acctgaacac cgctcccata aagccatggc ttgccttgga tttcagcggc 181 acaaggctca gctgaacctg gctaccagga cctggccctg cactctcctg tttttcttc 241 tcttcatccc tgtcttctgc aaaggtgagt gagacttttg gagcatgaag atggaggagg 301 tgtttctcct acctgggttt catttgtttc agcagtcaaa ggcagtgatt tatagcaaag 361 ccagaagtta aaggtaaaac tccaatctgg cttggctggc tctgtattcc agggccagca 421 gggagcagtt gggcggcagc aaataaggca aagagatagc tcagaacaga gcgccaggta 481 tttagtaggg gcttcatgaa tgcatgtgag ttggtttagt agagagacac aggcaatttc 541 agacccttct atgagactgg aagtgattta agagggaaag gatagccata gtcctgaata 601 catttgagct gggtttcagg atgagctcac aagttccttt aaaaaaaatt gacttaagca 661 aatcctggga agagtttttt tgctatacaa ttcaaggttt taaggtcctc ggattcatat 721 actttataaa tgaattagcc agcttgttta aaatgtaggg aaattgtggg aagaatgcct 781 tctttactta attcaaggtt ttaaggttct cttaatcaat tctactagct aattagccaa 841 ttatttaaaa ataaaagttt gaaattgcca aaaaaaaaag acaaggaaaa ggaaagaaag 901 aaagccacca gtctgtttgg catacaatac ttaattgttg cctgacctac gtgtgggttt 961 cagatgcaga tcctcagttt tcagctcttc agagactgac accaggtttg ttacacggct 1021 taaaatgatg agtatatcca ttgaatctca accttatctc tctctagacc ttcttggtta 1081 agaaaccatg tagtttgtat gaagtaggta ctcaaaagat atttgatgat ttaattttta 1141 ctggagaaga aatattcata tatgttttct tatttttaca tgttttaaat atgtaaagat 1201 taaataaaca ctcttagaag tatttaaatt tcctaaagta aatttatctc aaccagtaac |
| 1261 aggaccctcc caatactgga aagttgagtg tgaccgcatt tagtggtgat gagtgtgagc 1321 ttgcttgggg agagggcagg acatttagga tttcttaagc ttagagtcaa tacaataaag 1381 attattgagt gctcacttgg gtgggctata atcactgctc acaggagttc atgaaccaca 1441 agtaaaagag tgaggagata tgattagctc acaaataact ttaatacaga gcagaaagta 1501 atgaactact gcaatggagt tatcacagtg ctaaggatgc tcagagggca tctctgatag 1561 gcagaggtga gggttaggga aggaagctgt agtctagcta gctagagctg ctggaataga 1621 catgacaatg gctgctgcca aactgttttc tcttctgagg acagatgtcc cgtgcaagtg 1681 gcttggtgga agggactagt gtctctaata tagggtgatt tataagcagg aaagtgtgtc 1741 ctagaaattc agaccagagt gatagattgg aattggatca tgggggactc attgaatgtt 1801 atttattgta tttgtttttg cgatcagtgt tagtaaagtg tcaaagggat tgagcagatg 1861 agtgacatca tgcaacacaa gttttgagtt tcacttgtca gactgactgg agagggcct 1921 ggttagttac aggaaggtaa tttggcatgc agccactatt tttgagttga tgcaagcctc 1981 tctgtatgga gagctggtct cctttatcct gtgggaaaag agaacaaagg agcatgggag 2041 tgttcaaggg aaggagaaat aaagggcaga gaggcagcgg tggtgtcagg ggaagcccac 2101 aggagttaac agcagggttg cctcaaccta gagaggaagc gacctggtgc cctcggctct 2161 gtggcttcct tcatctaaca acatcttcca ctctacaaca atgccaggga aggcggaggc 2221 tggtacagtg catcaagaca cagctactcc tgggtgacag aggttcaggg ccagctcact 2281 aagtaggcag aagtttttga catatacttt gagagataaa gcaagattct gtacctcaac 2341 cttcagaatt tcccctacca ctcattatag ttccggagct atatagctcc tatcattcta 2401 tcataacctt agaataccag agaacatatc atctcatcta attatctctt actatatgtg 2461 aaaaaaatga aggacatggg ggaagtgtga cttgccccaa atcacatatt tcatggtaga 2521 gccaggtctt ctgtttgtca tatcagtgtt cttcctgcca caaccatctt gaagaatcta 2581 tttctcagta agaaaatatc tttatggaga gtagctggaa aacagttgag agatggaggg 2641 gaggctgggg gtgtggagag gggaaggggt aagtgataga ttcgttgaag gggggagaaa 2701 aggccgtggg gatgaagcta gaaggcagaa gggcttgcct gggcttggcc atgaaggagc 2761 atgagttcac tgagttccct ttggcttttc catgctagca atgcacgtgg cccagcctgc 2821 tgtggtactg gccagcagcc gaggcatcgc cagctttgtg tgtgagtatg catctccagg 2881 caaagccact gaggtccggg tgacagtgct tcggcaggct gacagccagg tgactgaagt 2941 ctgtgcggca acctacatga tggggaatga gttgaccttc ctagatgatt ccatctgcac 3001 gggcacctcc agtggaaatc aagtgaacct cactatccaa ggactgaggg ccatggacac 3061 gggactctac atctgcaagg tggagctcat gtacccaccg ccatactacc tgggcatagg 3121 caacggaacc cagatttatg taattggtga gcaaagccat ttcactgagt tgacacctgt 3181 tgcattgcag tcttctatgc acaaaaacag ttttgttcct taatttcagg aggtttactt 3241 ttaggactgt ggacattctc tttaagagtt ctgtaccaca tggtagcctt gcttattgtg 3301 ggtggcaacc ttaatagcat tctgactgta aaataaaatg atttggggaa gttggggctc 3361 tcgctctgga gtgctaacca tcatgacgtt tgatctgtac ttttgatatg atatgatgct 3421 cctggggaag tagtcccaaa tagccaaacc tattggtggg ctacccatgc aatttagggg 3481 tggacctcaa ggcctggaag ctctaatgtc cttttttcac caatgttggg gagtagagcc 3541 ctagagttta aaactgtctc agggaggctc tgctttgttt tctgttgcag atccagaacc 3601 gtgcccagat tctgacttcc tcctctggat ccttgcagca gttagttcgg ggttgttttt 3661 ttatagcttt ctcctcacag ctgtttcttt gagcaaaatg gtgagtgtgg tgctgatggt 3721 gcaccatgtc tgatggggat acctttagtg gtatcaactg gccaaaagat gatgttgagt 3781 ttagtgttct tgagatgaga tgaggcaata aatgaagagg aaggacagtg gtaaagaacg 3841 cactagaacc gtaggcattg gcatttgagg tttcagaatg actaatattt tagatgaatt 3901 tgtttgacat tgaatgttca tgtgcttctg agcagggttt caatttgagt aaccgttgca 3961 ataacatggg gcagctgttt tgctctttgt cttcatgaca actgtactta agctaacagc 4021 cctgaaacat gagattaggc tgggcagaat gctgctagag aggaccactt ggatggtctt 4081 tattctcctt ctccatgtcc ctctccatca cctggaagtc acctctgggt gccactctgg 4141 tgccttcctt gtcgaagctg tagctgctca catgacacct atccctgtta tccagtttgc 4201 ttgactggga cgttttgcct tccccttcag ccaggaagtg aaagtcccag tttttattta 4261 tcacaggtgt tggtattggt ggtagaagag gtagaattat ggaatcaggc ctcctgtcag |
| 4321 gatttctttt tgacagtccc tctcagacac ctctgcctaa ggccagcttt gccattacaa 4381 actctccctt ctccctctct cccttcttct cttcctcttc cttcttctcg ctctttctct 4441 ctctctcttt ctccctctct gtctcttata cacatacaca aagatatact ctattccaac 4501 atcctctacc caacctgaca gagatgtcct ttgctgtagg ttcagcagtg ggatgagaa 4561 atacagctct caaacaggat aactaaagct tattatctta tcaagcttgt tcccttgcag 4621 acaagattga tcaattatca taggctttct gggtgttctt tctgaagctt tctcaaagtc 4681 tctttctcct atcttccatt caaggcaaat gattgccatt taacatcaaa atcacagtta 4741 tttatctaaa ataaatttta atagctgaat caagaaaatc tcctgaggtt tataattctg 4801 tatgctgtga acattcattt ttaaccagct agggacccaa tatgtgttga gttctattat 4861 ggttagaagt ggcttccgta ttcctcagta gtaattactg tttctttttg tgtttgacag 4921 ctaaagaaaa gaagccctct tacaacaggg gtctatgtga aaatgccccc aacagagcca 4981 gaatgtgaaa agcaatttca gccttatttt attcccatca attgagaaac cattatgaag 5041 aagagagtcc atatttcaat ttccaagagc tgaggcaatt ctaacttttt tgctatccag 5101 ctatttttat ttgtttgtgc atttgggggg aattcatctc tctttaatat aaagttggat 5161 gcggaaccca aattacgtgt actacaattt aaagcaaagg agtagaaaga cagagctggg 5221 atgtttctgt cacatcagct ccactttcag tgaaagcatc acttgggatt aatatgggga 5281 tgcagcatta tgatgtgggt caaggaatta agttagggaa tggcacagcc caaagaagga 5341 aaaggcaggg agcgagggag aagactatat tgtacacacc ttatatttac gtatgagacg 5401 tttatagccg aaatgatctt ttcaagttaa attttatgcc ttttatttct taaacaaatg 5461 tatgattaca tcaaggcttc aaaaatactc acatggctat gttttagcca gtgatgctaa 5521 aggttgtatt gcatatatac atatatatat atatatatat atatatatat atatatatat 5581 atatatatat atatatttta atttgatagt attgtgcata gagccacgta tgtttttgtg 5641 tatttgttaa tggtttgaat ataaacacta tatggcagtg tctttccacc ttgggtccca 5701 gggaagtttt gtggaggagc tcaggacact aatacaccag gtagaacaca aggtcatttg 5761 ctaactagct tggaaactgg atgaggtcat agcagtgctt gattgcgtgg aattgtgctg 5821 agttggtgtt gacatgtgct ttggggcttt tacaccagtt cctttcaatg gtttgcaagg 5881 aagccacagc tggtggtatc tgagttgact tgacagaaca ctgtcttgaa gacaatggct 5941 tactccagga gacccacagg tatgaccttc taggaagctc cagttcgatg ggcccaattc 6001 ttacaaacat gtggttaatg ccatggacag aagaaggcag caggtggcag aatggggtgc 6061 atgaaggttt ctgaaaatta acactgcttg tgtttttaac tcaatatttt ccatgaaaat 6121 gcaacaacat gtataatatt tttaattaaa taaaaatctg tggtggtcgt ttt (SEQ ID NO: 782) |

The CTLA4 gene is an immunoglobulin superfamily member and its polynucleotide sequence encodes a protein that transmits inhibitory signals to T cells. The protein has a V domain, a transmembrane domain, and a cytoplasmic tail. Various isoforms encoded by alternate splice variants have been characterized. The membrane-bound isoform acts as a homodimer unified by a disulfide bond, whereas the soluble isoform acts as a monomer. CTLA4 genetic mutations have been reportedly associated with insulin-dependent diabetes mellitus, Graves disease, Hashimoto thyroiditis, celiac disease, systemic lupus erythematosus, thyroid-associated orbitopathy, and other autoimmune diseases.

In some aspects, the present disclosure provides a modified primary human cell (e.g., immune cell, e.g., T cell, natural killer cell, etc.) or population thereof comprising a genome in which the CTLA4 gene on chromosome 2 has been edited to reduce or eliminate CTLA4 expression (e.g., cell surface expression) and/or activity (e.g., protein activity) in the cell or population thereof using a genetic editing system (e.g., TALENs, CRISPR/Cas, etc.). In some aspects, the present disclosure provides a modified primary human cell (e.g., immune cell, e.g., T cell, natural killer cell, etc.) or population thereof comprising a genome in which the CTLA4 gene on chromosome 2 has been edited to delete a contiguous stretch of genomic DNA, e.g., from SEQ ID NO: 782, thereby reducing or eliminating CTLA4 expression (e.g., cell surface expression) and/or activity (e.g., protein activity) in the cell or population thereof. The contiguous stretch of genomic DNA can be deleted by contacting a primary human cell (e.g., immune cell, e.g., T cell, natural killer cell, etc.) or population thereof with a Cas protein or a nucleic acid sequence encoding the Cas protein and at least one pair of ribonucleic acids (i.e., CRISPR CTLA4 gRNA pairs, e.g., at least one gRNA pair, at least two gRNA pairs, at least three gRNA pairs, at least four gRNA pairs, at least five gRNA pairs, etc.) selected from the group consisting of SEQ ID NOs: 1-195 and 797-3637.

As used herein, the term "contacting" (e.g., contacting a polynucleotide sequence with a clustered regularly interspaced short palindromic repeats-associated (Cas) protein and/or ribonucleic acids) is intended to include incubating the Cas protein and/or the ribonucleic acids in the cell together *in vitro* (e.g., adding the Cas protein or nucleic acid encoding the Cas protein to cells in culture) or contacting a cell *ex vivo.* The step of contacting a target polynucleotide sequence with a Cas protein and/or ribonucleic acids as disclosed herein can be conducted in any suitable manner. For example, the cells may be treated in adherent culture, or in suspension culture. It is understood that the cells contacted with a Cas protein and/or ribonucleic acids as disclosed herein can also be simultaneously or subsequently contacted with another agent, such as a growth factor or other differentiation agent or environments to stabilize the cells, or to differentiate the cells further.

The present disclosure contemplates reducing or eliminating CTLA4 expression and/or activity in any cell-line or primary human cell (e.g., immune cell, e.g., T cell, natural killer cell, etc.) population thereof to produce cells which reduce or eliminate T cell inhibition. Primary human cells used for genomic editing can be obtained from a subject suffering from, being treated for, diagnosed with, at risk of developing, or suspected of having, a disorder selected from the group consisting of an autoimmune disorder, cancer, a chronic infectious disease, and graft versus host disease (GVHD). Cells can also be obtained from a normal healthy subject not suffering from, being treated for, diagnosed, suspected of having, or at increased risk of developing, the disorder.

The present invention contemplates genomically editing primary human cells to cleave CTLA4 gene sequences, as well as editing the genome of such cells to alter one or more additional target polynucleotide sequences (e.g., PD1, TCRA, TCRB, B2M, etc.). It should be appreciated that cleaving a CTLA4 gene sequence using one or more gRNAs or gRNA pairs described herein can result in partial or complete deletion of the CTLA4 genomic DNA sequence (e.g., SEQ ID NO: 782).

In some aspects, the invention provides a modified primary human T cell or population thereof, each cell comprising a modified genome comprising: (a) a genomic modification (e.g., a first genomic modification) in which the cytotoxic T-lymphocyte-associated protein 4 (CTLA4) gene on chromosome 2 has been edited to delete a first contiguous stretch of genomic DNA comprising an intron flanked by at least a portion of an adjacent upstream exon and at least a portion of an adjacent downstream exon, and the 3' end of the genomic DNA upstream with respect to the 5' end of the deleted first contiguous stretch of genomic DNA is covalently joined to the 5' end of the genomic DNA downstream with respect to the 3' end of the deleted first contiguous stretch of genomic DNA to result in a modified CTLA4 gene on chromosome 2 that lacks the first contiguous stretch of genomic DNA, thereby reducing or eliminating CTLA4 receptor surface expression and/or activity in the cell.

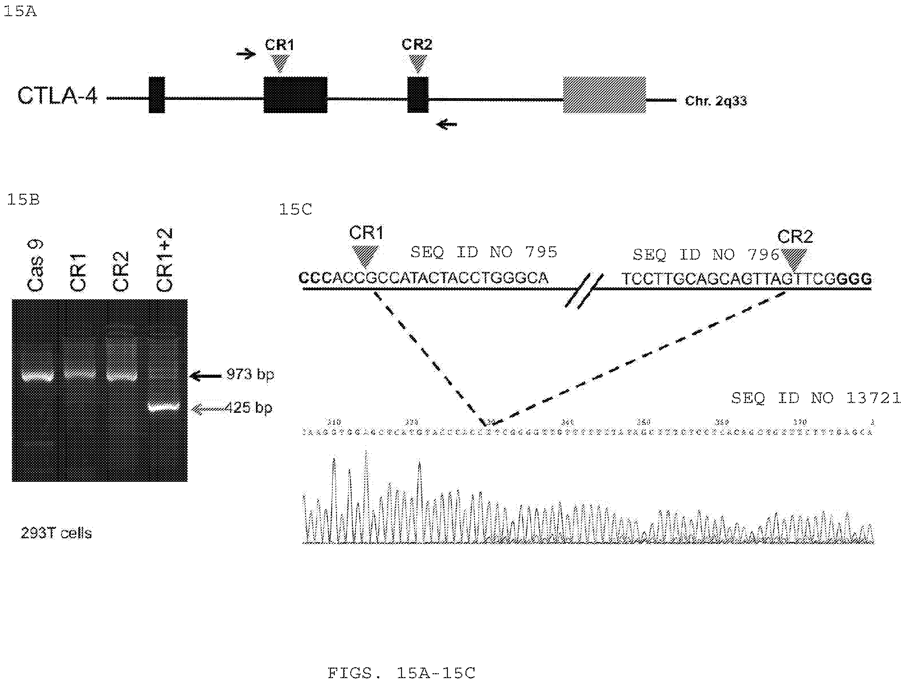

Those skilled in the art will appreciate that deletion of the first contiguous stretch of CTLA4 genomic DNA as such can be achieved using any pair of CRISPR gRNAs targeting exons in the CTLA4 gene where a first CTLA4 gRNA targets a first exon that resides upstream with respect to a second exon downstream with respect to the first exon. Similarly, any exon pair can be targeted using this strategy to result in the cleavage of the first contiguous stretch of genomic DNA with the result that the cell thus modified's DNA repair mechanisms covalently joins the 3' end of the genomic DNA upstream to the CTLA4 gRNA cleavage site in the first exon to the 5' end of the genomic DNA downstream to the CTLA4 gRNA cleavage site. An example of this strategy is shown in FIG. 15A and FIG. 15B using a first pair of ribonucleic acids comprising SEQ ID NO: 128 (CR1) and SEQ ID NO: 72 (CR2). In such example, the first pair of CTLA4 gRNAs are shown targeting exon 2 and exon 3 of the CTLA4 gene. Of course, any two adjacent exons in the CTLA4 gene could be targeted using this strategy (e.g., exon 1 and exon 2, exons 3 and 4). It should further be appreciated that any portion of such CTLA4 exons which contain a CTLA4 target motif can be targeted using this strategy as long as each CTLA4 gRNA of the first pair of CTLA4 gRNAs is directed to one CTLA4 target motif in each of the adjacent CTLA4 exons. In other words, to achieve the strategy, the skilled person need only select a first CTLA4 gRNA from among SEQ ID NOs: 1-195 and 797-3637 that targets a motif in a first exon, and then select a second CTLA4 gRNA from among SEQ ID NOs: 1-195 and 797-3637 that targets a motif in a second (e.g., adjacent) exon that is either upstream or downstream relative to the first exon. In this way, the first pair of gRNAs will guide Cas protein in the cell to the first and second exons, respectively, and cleave those exons as well as the intron (or any other sequence therebetween), thereby permitting the cell's DNA repair mechanisms to covalently join the genomic DNA at the two cleavage sites to create the modified cell or population thereof with a CTLA4 gene lacking the first contiguous stretch of genomic DNA. In addition to, or as an alternate to, targeting adjacent exons, a first pair of gRNAs can be selected to target any two exons in the CTLA4 gene (e.g., exons 1 and 3, exons 1 and 4, exons 2 and 4, etc.) such that the genomic DNA sequence between the cleavage sites in those exons is deleted, and the genomic DNA sequences flanking those cleavage sites are covalently joined to result in a modified cell or population thereof with a CTLA4 gene lacking the first contiguous stretch of genomic DNA.

Table 2 below shows the genomic sequences of each of the four exons in the exemplary human CTLA4 gene. The skilled artisan can readily select pairs of CTLA4 gRNAs from among SEQ ID NOs: 1-195 and SEQ ID NOs 797-3637 to target the exons comprising SEQ ID NOs: 783-786 shown in Table 2 below using the CTLA4 targeting strategy outlined herein to carry out such strategy in a variety of ways. Alternatively, the skilled artisan can readily select at least one CTLA4 gRNA from among SEQ ID NOs: 3638-4046 to target the exons comprising SEQ ID NOs: 783-786 shown in Table 2 below using the CTLA4 targeting strategy outlined herein to carry out such strategy in a variety of ways. The size of the at least the portion of the upstream exon and the at least the portion of the downstream exon deleted using this strategy depends on the location in which the CTLA4 gRNAs direct cleavage in each respective exons. For example, the entire portion of the exon downstream relative to the cleavage site in the upstream exon and the entire portion of the exon upstream relative to the cleavage site in the downstream exon will be deleted using this strategy. Thus, one can delete larger portions of targeted exons using this strategy by selecting CTLA4 gRNAs targeting motifs closest to the 5' end of the upstream exon and closest to the 3' end of the adjacent downstream exon. Conversely, one can delete smaller portions of targeted exons by selecting CTLA4 gRNAs targeting motifs farthest away from the 5' end of the upstream exon and farthest away from the 3' end of the adjacent downstream exon.

**Table 2**

| CTLA4 Exon 1 - location: 5,003...5,266; length 264 bp |
|---|
| 1 cttctgtgtg tgcacatgtg taatacatat ctgggatcaa agctatctat ataaagtcct 61 tgattctgtg tgggttcaaa cacatttcaa agcttcagga tcctgaaagg ttttgctcta 121 cttcctgaag acctgaacac cgctcccata aagccatggc ttgccttgga tttcagcggc 181 acaaggctca gctgaacctg gctaccagga cctggccctg cactctcctg ttttttcttc 241 tcttcatccc tgtcttctgc aaag (SEQ ID NO: 783) |

| CTLA4 Exon 2 - location: 7,801...8,148; length 348 |
|---|
| 1 caatgcacgt ggcccagcct gctgtggtac tggccagcag ccgaggcatc gccagctttg 61 tgtgtgagta tgcatctcca ggcaaagcca ctgaggtccg ggtgacagtg cttcggcagg 121 ctgacagcca ggtgactgaa gtctgtgcgg caacctacat gatggggaat gagttgacct 181 tcctagatga ttccatctgc acgggcacct ccagtggaaa tcaagtgaac ctcactatcc 241 aaggactgag ggccatggac acgggactct acatctgcaa ggtggagctc atgtacccac 301 cgccatacta cctgggcata ggcaacggaa cccagattta tgtaattg (SEQ ID NO: 784) |

| CTLA4 Exon 3 - location: 8,593 ... 8,702; length 110 |
|---|
| 1 atccagaacc gtgcccagat tctgacttcc tcctctggat ccttgcagca gttagttcgg 61 ggttgttttt ttatagcttt ctcctcacag ctgtttcttt gagcaaaatg (SEQ ID NO: 785) |

| CTLA4 Exon 4 - location: 9,923...11,175; length 1,253 |
|---|
| 1 ctaaagaaaa gaagccctct tacaacaggg gtctatgtga aaatgccccc aacagagcca 61 gaatgtgaaa agcaatttca gccttatttt attcccatca attgagaaac cattatgaag 121 aagagagtcc atatttcaat ttccaagagc tgaggcaatt ctaacttttt tgctatccag 181 ctatttttat ttgtttgtgc atttgggggg aattcatctc tctttaatat aaagttggat 241 gcggaaccca aattacgtgt actacaattt aaagcaaagg agtagaaaga cagagctggg 301 atgtttctgt cacatcagct ccactttcag tgaaagcatc acttgggatt aatatgggga 361 tgcagcatta tgatgtgggt caaggaatta agttagggaa tggcacagcc caaagaagga 421 aaaggcaggg agcgagggag aagactatat tgtacacacc ttatatttac gtatgagacg 481 tttatagccg aaatgatctt ttcaagttaa attttatgcc ttttatttct taaacaaatg 541 tatgattaca tcaaggcttc aaaaatactc acatggctat gttttagcca gtgatgctaa 601 aggttgtatt gcatatatac atatatatat atatatatat atatatatat atatatatat 661 atatatatat atatatttta atttgatagt attgtgcata gagccacgta tgtttttgtg 721 tatttgttaa tggtttgaat ataaacacta tatggcagtg tctttccacc ttgggtccca 781 gggaagtttt gtggaggagc tcaggacact aatacaccag gtagaacaca aggtcatttg 841 ctaactagct tggaaactgg atgaggtcat agcagtgctt gattgcgtgg aattgtgctg 901 agttggtgtt gacatgtgct ttggggcttt tacaccagtt cctttcaatg gtttgcaagg 961 aagccacagc tggtggtatc tgagttgact tgacagaaca ctgtcttgaa gacaatggct 1021 tactccagga gacccacagg tatgaccttc taggaagctc cagttcgatg ggcccaattc 1081 ttacaaacat gtggttaatg ccatggacag aagaaggcag caggtggcag aatggggtgc 1141 atgaaggttt ctgaaaatta acactgcttg tgtttttaac tcaatatttt ccatgaaaat 1201 gcaacaacat gtataatatt tttaattaaa taaaaatctg tggtggtcgt ttt (SEQ ID NO: 786) |

In some aspects, the invention provides a modified primary human T cell or population thereof, each cell comprising a modified genome comprising: (a) a genomic modification (e.g., a first genomic modification) in which the cytotoxic T-lymphocyte-associated protein 4 (CTLA4) gene on chromosome 2 has been edited to delete a contiguous stretch (e.g., a first contiguous stretch) of genomic DNA, thereby reducing or eliminating CTLA4 receptor surface expression and/or activity in the cell, wherein the contiguous stretch (e.g., first contiguous stretch) of genomic DNA has been deleted by contacting the cell with a Cas protein or a nucleic acid encoding the Cas protein and a pair (e.g., first pair) of ribonucleic acids having sequences selected from the group consisting of SEQ ID NOs: 1-195 and 797-3637. In some embodiments, the first pair of ribonucleic acids comprises SEQ ID NO: 128 and SEQ ID NO: 72.

In some aspects, the invention provides a method for altering a target CTLA4 polynucleotide sequence in a cell comprising contacting the CTLA4 polynucleotide sequence with a clustered regularly interspaced short palindromic repeats-associated (Cas) protein and from one to two ribonucleic acids, wherein the ribonucleic acids hybridize to the (e.g.) CTLA4 polynucleotide sequence and direct Cas protein to a target motif of the target CTLA4 polynucleotide sequence, wherein the target CTLA4 polynucleotide sequence is cleaved, and wherein at least one of the one to two ribonucleic acids are selected from the group consisting of SEQ ID NOs. 1-195 and 797-3637. In some embodiments, each of the one to two ribonucleic acids is selected from the group consisting of SEQ ID NOs: 1-195 and 797-3637. In some embodiments, the two ribonucleic acids comprise SEQ ID NO: 128 and SEQ ID NO: 72.

In some aspects, the invention provides a modified primary human T cell or population thereof, each cell comprising a modified genome comprising: (a) a genomic modification (e.g., a first genomic modification) in which the cytotoxic T-lymphocyte-associated protein 4 (CTLA4) gene on chromosome 2 has been edited to reduce or eliminate CTLA4 receptor surface expression and/or activity in the cell by contacting the cell with a Cas protein or a nucleic acid encoding the Cas protein and at least one ribonucleic acid having a sequence selected from the group consisting of SEQ ID NOs: 3638-4046.

In some aspects, the invention provides a method for altering a target CTLA4 polynucleotide sequence in a cell comprising contacting the CTLA4 polynucleotide sequence with a clustered regularly interspaced short palindromic repeats-associated (Cas) protein and at least one ribonucleic acid, wherein the ribonucleic acid directs Cas protein to and hybridizes to a target motif of the target CTLA4 polynucleotide sequence, wherein the target CTLA4 polynucleotide sequence is cleaved, and wherein the at least one ribonucleic acid is selected from the group consisting of SEQ ID NOs. 3638-4046. In certain aspects, a subsequent alteration to the target CTLA4 polynucleotide sequence in the cell results in a second cleavage of the target CTLA4 polynucleotide sequence, thereby editing the target CTLA4 polynucleotide sequence to delete a first contiguous stretch of genomic DNA.

### Programmed cell death 1 (PD1)

In some embodiments, the target polynucleotide sequence is PD1, or a homolog, ortholog, or variant thereof (Gene ID: 5133, also known as PD-1, CD279, SLEB2, hPD-1, hPD-I, and hSLE1). An exemplary PD1 human target polynucleotide sequence is shown in Table 3 below (NG_012110.1 RefSeqGene; SEQ ID NO: 787).

| Table 3 - Exemplary human PD1 target polynucleotide sequence |
|---|
| 1 agtttccctt ccgctcacct ccgcctgagc agtggagaag gcggcactct ggtggggctg 61 ctccaggcat gcagatccca caggcgccct ggccagtcgt ctgggcggtg ctacaactgg 121 gctggcggcc aggatggttc ttaggtaggt ggggtcggcg gtcaggtgtc ccagagccag 181 gggtctggag ggaccttcca ccctcagtcc ctggcaggtc ggggggtgct gaggcgggcc 241 tggccctggc agcccagggg tcccggagcg aggggtctgg agggaccttt cactctcagt 301 ccctggcagg tcggggggtg ctgtggcagg cccagccttg gcccccagct ctgcccctta 361 ccctgagctg tgtggctttg ggcagctcga actcctgggt tcctctctgg gccccaactc 421 ctcccctggc ccaagtcccc tctttgctcc tgggcaggca ggacctctgt cccctctcag 481 ccggtccttg gggctgcgtg tttctgtaga atgacgggtc aggctggcca gaaccccaaa 541 ccttggccgt ggggagtctg cgtggcggct ctgccttgcc caggcatcct tggtcctcac 601 tcgagttttc ctaaggatgg gatgagcccc atgtgggact aaccttggct ttacgacgtc 661 aaagtttaga tgagctggtg atatttttct cattatatcc aaagtgtacc tgttcgagtg 721 aggacagttc ttctgtctcc aggatccctc ctgggtgggg attgtgcccg cctgggtctc 781 tgcccagatt ccagggctct ccccgagccc tgttcagacc atccgtgggg gaggccttgg 841 cctcactctc ccggatcgag gagagaggga gcctcttcct gggctgcccg tgaccctggg 901 ccctctgtgt acactgtgac cacagcccgc tcctggaccc tctgtgcccg gctggccctc 961 tgtgcccagc cagcctgcac ctggggatgc caaggcctgg ggagggtggt ttcacccagg 1021 ccaagcctaa gacagtccct ctgggccctg ctgggtaccg gggtgtgaca ccactgggag 1081 gacaagatga ggggcacccc tggggccgcc ctgacacccc ctcgaggctc ctgccccggg 1141 ggtcctggtg ccccttcact gtggcaggcg actgggggtt ccccacctcg gcccctctcc 1201 cggggcctgc tccccggcac ctgaggcagc atccttgtca gggccgtgcc ttcctgcctc 1261 agcgccacct cttaaggttg gcccgtgggt cactcaggac tcagaactgg agattctggg 1321 caaaaggcaa agagcaaagg gccaaaaggc atcccaggga gacgactgcg gggaaccag 1381 agggcagagg ggcgctcgtc acaggggagg gggagctgag cgaggcagga ggggagccga 1441 gcctctcccc ccgtgtcccg gctcttcagg cacgccctcg ggacgccacc ctccccgacc 1501 caggcgggaa agataagagc aaggtgtccg cagcctgaca ctcgtgcctc aggtgcccgc 1561 gcttgtgccg gacaagactc tcacaggtgg catgcctcgg tttccccact ggtaacagca 1621 cagggcactc agcaaggcgc agtgggcatg actggggtcc tgtgggtcct gacccagatg 1681 tggccacccc ggccgcagtg gtcttcattc caggatgcct cttttccctc ctgatctatt 1741 cactgcgttc gccattcggt cattcccggg gccaccactc ccacctcagg tgtgtgcttc 1801 ccttgtgttt tatgagatat ccccaacccg gctgcttatt ggccccgtcc gagggcagga 1861 gcataaataa gagcctctgc tttggcgtgg gaccactgtg agctccagtc agcgctgcca 1921 ctgctgagct ctgggccttc gacaggactt ggccccttac tgacttctcc gtgtgctttg 1981 ggtcatgggt gaggacgcct cctggcaagg ctgcgtcctg aggattaaat cgggtcatct 2041 gtgaaaacta cccagcccag cccctgacac ttttttgttt gtttctttta gtgacagggt 2101 cttgctctgt cacccaggct ggagtgcagt ggtgtgatct cggctcactc gacctccggg 2161 gctcaagcaa ttctcccacc tctgcctcca gagtagctgg gactataggc acgtgccacc 2221 ctgccaggct aatttcttcc atttttttta gagacagggt ctcgctatgt tccccaggct 2281 gggctcaaat ggtcctccca cctcagcctc cccaagtact gggattacag gcataagcca 2341 ctgcatctgg cctccatgac acatattttt aaagtctgat ttttaaagtc aaacttttga 2401 agtcagattt taaacggact attttgaaaa atatacaaaa acgtttaaaa acaatgaata 2461 tccctcacct agaatcaata actaagaata ttgacacatt tgctttgggg actgggcggc 2521 tggagctgcc atgacaaagc tccgccgacc gagtggcttt taaacagagc ctgccctctc 2581 gccgactgag ggctggacgt gcaggatgga gctccgcagg gtcggctccc ctgtgctctg 2641 aggggctctg ctcagcctct cccggctgtg gcttaaaaac agagcctgtc ctcccgccgt |
| 2701 ggggggctgg acatgcagga ccgaggggcc acagggtcgg ctccctgtgc tccgagaggg 2761 ctctgctcag cttctcctgg ctggggggtt ttgtggccac cctctgtgtt cctgggttca 2821 gaagcatccc ccaggctctg ccttcatctc tgcacgggtg actctgtaca ggaagccagg 2881 cctgctggtc aatggccacc cagccctgtg ccctcatctt acctagtccc agctgccgtc 2941 accctattcc taataaggcc gccttctgag gtcatggggt taggacttcc acataggaat 3001 ctgtggggac acggttcggc ccacagccct tcccacctcc acacacacac acgactgtga 3061 ggagttggaa gacctcactc ctcacccctg ccaggtcctc tagggacaag ctcgctgtcc 3121 tcatcccagc acagcccgtg ggacggtttc cttgtcccta atgggaccac ggtcagagat 3181 gccgggtctg gtctgggcca gcaggttcct ccgcccgggg caggcagcct tcttctgtgc 3241 gcttctggaa agcaatgtcc tgtaatgcgg tctctctgcg ggagcacccc caccgccacc 3301 tcacaggcct gttccacagc cccgggatgg gctctgtctc cctcctgacc ctgcataggg 3361 cacagccctc tctcatcaac ccacgatcct acgtggatcc gagagggagc acctggggaa 3421 acaatggaat cccatagaaa caccccaaat ctaacttgat ccaggaccag ccagtggtca 3481 cttctgaata ttcaccttcc tagtagacac taccagccaa gggaggccag gaagccttcc 3541 tggaggaggt ggcctgagga ctggggtgag gcaggccctg cgtgggggtc gccacccagc 3601 acccccacac tgggtgggag ccagtctctg agactggctg ggggaggtgg gagaggggc 3661 tgcttgaact gcagacaccg aggtctagcc cccaccccac ccagccagtt ggtggaggca 3721 ggggaggccg aggggcccag ctggacctgc tccccggggt ggattccaaa ataggggggt 3781 tggggggggc ggaacaggag cccagggtcc tggcttgagg cccagtggct gagggctggt 3841 gcaagccaga caggaaaagg gttgagcctg tcagcgccag cacagatcaa gtcaggagca 3901 ggtccctcca ccaatgtgtg caaataaata gcagctaagt ttccagttac aagaacaatg 3961 cacagatggt cccagggaca ttgcggtgtg gacacacagc ggccattgtc ctgtcgccag 4021 cacctcgccc tacagctggg gggtccctta gcacttccta gccatgcagg gtccctgctc 4081 acagtacccg tgatgacttc tgttcctcac ctgcctgtct gtcccgacag ctgcatggca 4141 gccctggcct gggagatgga gaccccgagg ggctgcctgc ggtggtgggg cccctgggtc 4201 cccactgcat tcccagaaac ccagagggca gggcatttcc cctgctctgt gccgagtcca 4261 cccagcccca gcctaggccc agtaagggct gcagcccacc ctgtcccagg ctgcctccca 4321 ggagccctct tggccctgat gccagaagcc catcttcctc cattcaggca ggtctctgag 4381 tgccctggcc tggctgcctg ctggccctga gagtcacact accccacagc cctccttggt 4441 caaaatccac tctggagtgg ctggaagatt ccccgggccc acgccgcaca cgcctatgca 4501 gggagcttcc cctggccggc cggcagacaa gggcggtctc agagaggggg ctcacctcag 4561 cagccccttg tgtagctggc cctcgcccct gccacctctg ggaacaccac caggaagctg 4621 ggggacaggc acgcaggtga aggaggcgag cgcttgtcag ccgggaggcc atgggcacag 4681 agggaacagg gacaccctgg gtggcctcaa ggtcacttca aacccctcac tcgtcccctg 4741 ggagggtgcc cagtgaggtt ggcactagga gttggtcctg gtcacatgac agacccaccc 4801 acctctggtg tccagccagc acgccgtggg ccagcctggc tgcagggaca cgagggcagc 4861 agccccctcc tcctctgagc tggttgctcc ttgagtcatc accaccgcct gccacggagg 4921 ccgcctgtcc caggaagcag agggaccgca gctgtggcaa ccagggcctg gtctctgtgt 4981 cacctcgctg gggggccgtg cccaggcctg agacggaact gagtgacagt gcactgggtc 5041 tgacagtgtg gggctggcgc catgtttggg gaaccctgtg gcatggaccc tgtgggtgag 5101 ccgggaaaat caccccgttg catggcatct cgggcctgga tcttaagcgc ctgtgttggt 5161 gcctccgcct ggcggaagag ccgcgacccc cacgttgcca tgcgggtatc ccaagccctg 5221 accctggcag gcatatgttt caggaggtcc ttgtcttggg agcccagggt cgggggcccc 5281 gtgtctgtcc acatccgagt caatggccca tctcgtctct gaagcatctt tgctgtgagc 5341 tctagtcccc actgtcttgc tggaaaatgt ggaggcccca ctgcccactg cccagggcag 5401 caatgcccat accacgtggt cccagctccg agcttgtcct gaaaaggggg caaagactgg 5461 accctgagcc tgccaagggg ccacactcct cccagggctg gggtctccat gggcagcccc 5521 ccacccaccc agaccagtta cactcccctg tgccagagca gtgcagacag gaccaggcca 5581 ggatgcccaa gggtcagggg ctggggatgg gtagccccca aacagccctt tctgggggaa 5641 ctggcctcaa cggggaaggg ggtgaaggct cttagtagga aatcagggag acccaagtca 5701 gagccaggtg ctgtgcagaa gctgcagcct cacgtagaag gaagaggctc tgcagtggag |
| 5761 gccagtgccc atccccgggt ggcagaggcc ccagcagaga cttctcaatg acattccagc 5821 tggggtggcc cttccagagc ccttgctgcc cgagggatgt gagcaggtgg ccggggaggc 5881 tttgtggggc cacccagccc cttcctcacc tctctccatc tctcagactc cccagacagg 5941 ccctggaacc cccccacctt ctccccagcc ctgctcgtgg tgaccgaagg ggacaacgcc 6001 accttcacct gcagcttctc caacacatcg gagagcttcg tgctaaactg gtaccgcatg 6061 agccccagca accagacgga caagctggcc gccttccccg aggaccgcag ccagcccggc 6121 caggactgcc gcttccgtgt cacacaactg cccaacgggc gtgacttcca catgagcgtg 6181 gtcagggccc ggcgcaatga cagcggcacc tacctctgtg gggccatctc cctggccccc 6241 aaggcgcaga tcaaagagag cctgcgggca gagctcaggg tgacaggtgc ggcctcggag 6301 gccccggggc aggggtgagc tgagccggtc ctggggtggg tgtcccctcc tgcacaggat 6361 caggagctcc agggtcgtag ggcagggacc ccccagctcc agtccagggc tctgtcctgc 6421 acctggggaa tggtgaccgg catctctgtc ctctagctct ggaagcaccc cagcccctct 6481 agtctgccct cacccctgac cctgaccctc caccctgacc ccgtcctaac ccctgacctt 6541 tgtgcccttc cagagagaag ggcagaagtg cccacagccc accccagccc ctcacccagg 6601 ccagccggcc agttccaaac cctggtggtt ggtgtcgtgg gcggcctgct gggcagcctg 6661 gtgctgctag tctgggtcct ggccgtcatc tgctcccggg ccgcacgagg taacgtcatc 6721 ccagcccctc ggcctgccct gccctaaccc tgctggcggc cctcactccc gcctcccctt 6781 cctccaccct tccctcaccc caccccacct ccccccatct ccccgccagg ctaagtccct 6841 gatgaaggcc cctggactaa gaccccccac ctaggagcac ggctcagggt cggcctggtg 6901 accccaagtg tgtttctctg cagggacaat aggagccagg cgcaccggcc agcccctggt 6961 gagtctcact cttttcctgc atgatccact gtgccttcct tcctgggtgg gcagaggtgg 7021 aaggacaggc tgggaccaca cggcctgcag gactcacatt ctattatagc caggacccca 7081 cctccccagc ccccaggcag caacctcaat ccctaaagcc atgatctggg gccccagccc 7141 acctgcggtc tccgggggtg cccggcccat gtgtgtgcct gcctgcggtc tccaggggtg 7201 cctggcccac gcgtgtgccc gcctgcggtc tctgggggtg cccggcccac atatgtgcct 7261 gcctgcggtc tccaggtgtg cccggcccat gcgtgtgccc acctgcgagg gcgtggggtg 7321 ggcttggtca tttcttatct tacattggag acaggagagc ttgaaaagtc acattttgga 7381 atcctaaatc tgcaagaatg ccagggacat ttcagagggg gacattgagc cagagaggag 7441 gggtggtgtc cccagatcac acagagggca gtggtgggac agctcagggt aagcagctca 7501 tagtgggggg cccaggttcg gtgccggtac tgcagccagg ctgtggagcc gcgggcctcc 7561 ttcctgcggt gggccgtggg gctgactccc tctccctttc tcctcaaaga aggaggaccc 7621 ctcagccgtg cctgtgttct ctgtggacta tggggagctg gatttccagt ggcgagagaa 7681 gaccccggag ccccccgtgc cctgtgtccc tgagcagacg gagtatgcca ccattgtctt 7741 tcctagcgga atgggcacct catcccccgc ccgcaggggc tcagctgacg gccctcggag 7801 tgcccagcca ctgaggcctg aggatggaca ctgctcttgg cccctctgac cggcttcctt 7861 ggccaccagt gttctgcaga ccctccacca tgagcccggg tcagcgcatt tcctcaggag 7921 aagcaggcag ggtgcaggcc attgcaggcc gtccaggggc tgagctgcct gggggcgacc 7981 ggggctccag cctgcacctg caccaggcac agccccacca caggactcat gtctcaatgc 8041 ccacagtgag cccaggcagc aggtgtcacc gtcccctaca gggagggcca gatgcagtca 8101 ctgcttcagg tcctgccagc acagagctgc ctgcgtccag ctccctgaat ctctgctgct 8161 gctgctgctg ctgctgctgc tgcctgcggc ccggggctga aggcgccgtg gccctgcctg 8221 acgccccgga gcctcctgcc tgaacttggg ggctggttgg agatggcctt ggagcagcca 8281 aggtgcccct ggcagtggca tcccgaaacg ccctggacgc agggcccaag actgggcaca 8341 ggagtgggag gtacatgggg ctggggactc cccaggagtt atctgctccc tgcaggccta 8401 gagaagtttc agggaaggtc agaagagctc ctggctgtgg tgggcagggc aggaaacccc 8461 tccaccttta cacatgccca ggcagcacct caggcccttt gtggggcagg gaagctgagg 8521 cagtaagcgg gcaggcagag ctggaggcct ttcaggccca gccagcactc tggcctcctg 8581 ccgccgcatt ccaccccagc ccctcacacc actcgggaga gggacatcct acggtcccaa 8641 ggtcaggagg gcagggctgg ggttgactca ggcccctccc agctgtggcc acctgggtgt 8701 tgggagggca gaagtgcagg cacctagggc cccccatgtg cccaccctgg gagctctcct 8761 tggaacccat tcctgaaatt atttaaaggg gttggccggg ctcccaccag ggcctgggtg |
| 8821 ggaaggtaca ggcgttcccc cggggcctag tacccccgcc gtggcctatc cactcctcac 8881 atccacacac tgcaccccca ctcctggggc agggccacca gcatccaggc ggccagcagg 8941 cacctgagtg gctgggacaa gggatccccc ttccctgtgg ttctattata ttataattat 9001 aattaaatat gagagcatgc taagga (SEQ ID NO: 787) |

The PD1 gene codes for an immunoglobulin superfamily cell surface membrane protein, which is expressed in pro-B-cells and believed to be involved in their differentiation. When mice are injected with anti-CD3 antibodies the expression of this gene is induced in thymus in mice resulting in apoptosis of a large quantity of thymocytes. The product of this gene is also believed to be important in T cell function and play a role in the prevention of autoimmune diseases.

In some aspects, the present disclosure provides a modified primary human cell (e.g., immune cell, e.g., T cell, natural killer cell, etc.) or population thereof comprising a genome in which the PD1 gene on chromosome 2 has been edited to reduce or eliminate PD1 expression (e.g., cell surface expression) and/or activity (e.g., protein activity) in the cell or population thereof using a genetic editing system (e.g., TALENs, CRISPR/Cas, etc.). In some aspects, the present disclosure provides a modified primary human cell (e.g., immune cell, e.g., T cell, natural killer cell, etc.) or population thereof comprising a genome in which the PD1 gene on chromosome 2 has been edited to delete a contiguous stretch of genomic DNA, e.g., from SEQ ID NO: 787, thereby reducing or eliminating PD1 expression (e.g., cell surface expression) and/or activity (e.g., protein activity) in the cell or population thereof. The contiguous stretch of genomic DNA can be deleted by contacting a primary human cell (e.g., immune cell, e.g., T cell, natural killer cell, etc.) or population thereof with a Cas protein or a nucleic acid sequence encoding the Cas protein and at least one pair of ribonucleic acids (i.e., CRISPR PD1 gRNA pairs, e.g., at least one gRNA pair, at least two gRNA pairs, at least three gRNA pairs, at least four gRNA pairs, at least five gRNA pairs, etc.) at least one of which is selected from the group consisting of SEQ ID NOs: 196-531 and 4047-8945.

The present disclosure contemplates reducing or eliminating PD1 expression and/or activity in any cell line or primary human cell (e.g., immune cell, e.g., T cell, natural killer cell, etc.) population thereof to produce cells which reduce or eliminate T cell inhibition. Primary human cells used for genomic editing can be obtained from a subject suffering from, being treated for, diagnosed with, at risk of developing, or suspected of having, a disorder selected from the group consisting of an autoimmune disorder, cancer, a chronic infectious disease, and graft versus host disease (GVHD). Cells can also be obtained from a normal healthy subject not suffering from, being treated for, diagnosed, suspected of having, or at increased risk of developing, the disorder.

The present invention contemplates genomically editing primary human cells to cleave PD1 gene sequences, as well as editing the genome of such cells to alter one or more additional target polynucleotide sequences (e.g., CTLA4, TCRA, TCRB, B2M, etc.). It should be appreciated that cleaving a PD1 gene sequence using one or more gRNAs or gRNA pairs described herein can result in partial or complete deletion of the PD1 genomic DNA sequence (e.g., SEQ ID NO: 787).

In some aspects, the invention provides a modified primary human T cell or population thereof, each cell comprising a modified genome comprising: (b) a genomic modification (e.g., a second genomic modification) in which the PD1 gene on chromosome 2 has been edited to delete a second contiguous stretch of genomic DNA comprising an intron flanked by at least a portion of an adjacent upstream exon and at least a portion of an adjacent downstream exon, and the 3' end of the genomic DNA upstream with respect to the 5' end of the deleted second contiguous stretch of genomic DNA is covalently joined to the 5' end of the genomic DNA downstream with respect to the 3' end of the deleted second contiguous stretch of genomic DNA to result in a modified PD1 gene on chromosome 2 that lacks the second contiguous stretch of genomic DNA, thereby reducing or eliminating PD1 receptor surface expression and/or activity in the cell.

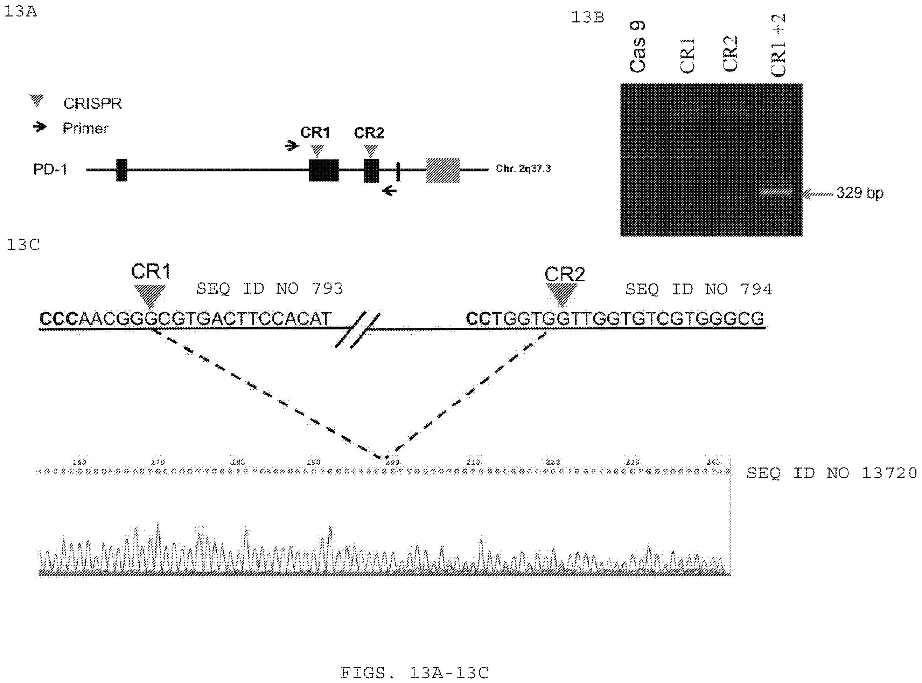

Those skilled in the art will appreciate that deletion of the second contiguous stretch of PD1 genomic DNA as such can be achieved using any pair of CRISPR gRNAs targeting exons in the PD1 gene where a first PD1 gRNA targets a first exon that resides upstream with respect to a second exon downstream with respect to the first exon. Similarly, any exon pair can be targeted using this strategy to result in the cleavage of the second contiguous stretch of genomic DNA with the result that the cell thus modified's DNA repair mechanisms covalently joins the 3' end of the genomic DNA upstream to the PD1 gRNA cleavage site in the first exon to the 5' end of the genomic DNA downstream to the PD1 gRNA cleavage site. An example of this strategy is shown in FIG. 13A and FIG. 13B using a first pair of ribonucleic acids comprising SEQ ID NO: 462 (CR1) and SEQ ID NO: 421 (CR2). In such example, the first pair of PD1 gRNAs are shown targeting exon 2 and exon 3 of the PD1 gene. Of course, any two adjacent exons in the PD1 gene could be targeted using this strategy (e.g., exon 1 and exon 2, exons 3 and 4, exons 4 and 5). It should further be appreciated that any portion of such PD1 exons which contains a CRISPR gRNA PD1 target motif can be targeted using this strategy as long as each PD1 gRNA of the first pair of PD1 gRNAs is directed to one CRISPR gRNA PD1 target motif in each of the adjacent PD1 exons. In other words, to achieve the strategy, the skilled person need only select a first PD1 gRNA from among SEQ ID NOs: 196-531 and 4047-8945 that targets a motif in a first exon, and then select a second PD1 gRNA from among SEQ ID NOs: 196-531 and 4047-8945 that targets a motif in a second (e.g., adjacent) exon that is either upstream or downstream relative to the first exon. In this way, the first pair of gRNAs will guide Cas protein in the cell to the first and second exons, respectively, and cleave those exons as well as the intron (or any other sequence therebetween), thereby permitting the cell's DNA repair mechanisms to covalently join the genomic DNA at the two cleavage sites to create the modified cell or population thereof with a PD1 gene lacking the second contiguous stretch of genomic DNA. In addition to, or as an alternate to, targeting adjacent exons, a first pair of gRNAs can be selected to target any two exons in the PD1 gene (e.g., exons 1 and 3, exons 1 and 4,exons 1 and 5, exons 2 and 4, exons 2 and 5, etc.) such that the genomic DNA sequence between the cleavage sites in those exons is deleted, and the genomic DNA sequences flanking those cleavage sites are covalently joined to result in a modified cell or population thereof with a PD1 gene lacking the second contiguous stretch of genomic DNA.

Table 4 below shows the genomic sequences of each of the five exons in the exemplary human PD1 gene. The skilled artisan can readily select pairs of PD1 gRNAs from among SEQ ID NOs: 196-531 and 4047-8945 to target the exons comprising SEQ ID NOs: 788-792 shown in Table 4 below using the PD1 targeting strategy outlined herein to carry out such strategy in a variety of ways. Alternatively, the skilled artisan can readily select at least one PDlgRNA from among SEQ ID NOs: 8946-9101 to target the exons comprising SEQ ID NOs: 788-792 shown in Table 4 below using the PD1 targeting strategy outlined herein to carry out such strategy in a variety of ways. The size of the at least the portion of the upstream exon and the at least the portion of the downstream exon deleted using this strategy depends on the location in which the PD1 gRNAs direct cleavage in each respective exons. For example, the entire portion of the exon downstream relative to the cleavage site in the upstream exon and the entire portion of the exon upstream relative to the cleavage site in the downstream exon will be deleted using this strategy. Thus, one can delete larger portions of targeted exons using this strategy by selecting PD1 gRNAs targeting motifs closest to the 5' end of the upstream exon and closest to the 3' end of the adjacent downstream exon. Conversely, one can delete smaller portions of targeted exons by selecting PD1 gRNAs targeting motifs farthest away from the 5' end of the upstream exon and farthest away from the 3' end of the adjacent downstram exon.

**Table 4**

| PD1 Exon 1 - location: 5,001...5,144; length 144 bp |
|---|
| 1 agtttccctt ccgctcacct ccgcctgagc agtggagaag gcggcactct ggtggggctg 61 ctccaggcat gcagatccca caggcgccct ggccagtcgt ctgggcggtg ctacaactgg 121 gctggcggcc aggatggttc ttag (SEQ ID NO: 788) |

| PD1 Exon 2 - location: 10,927... 11,286; length 360 |
|---|
| 1 actccccaga caggccctgg aaccccccca ccttctcccc agccctgctc gtggtgaccg 61 aaggggacaa cgccaccttc acctgcagct tctccaacac atcggagagc ttcgtgctaa 121 actggtaccg catgagcccc agcaaccaga cggacaagct ggccgccttc cccgaggacc 181 gcagccagcc cggccaggac tgccgcttcc gtgtcacaca actgcccaac gggcgtgact 241 tccacatgag cgtggtcagg gcccggcgca atgacagcgg cacctacctc tgtggggcca 301 tctccctggc ccccaaggcg cagatcaaag agagcctgcg ggcagagctc agggtgacag (SEQ ID NO: 789) |

| PD1 Exon 3 - location: 11,554...11,709; length 156 |
|---|
| 1 agagaagggc agaagtgccc acagcccacc ccagcccctc acccaggcca gccggccagt 61 tccaaaccct ggtggttggt gtcgtgggcg gcctgctggg cagcctggtg ctgctagtct 121 gggtcctggc cgtcatctgc tcccgggccg cacgag (SEQ ID NO: 790) |

| PD1 Exon 4 - location: 11,924...11,958; length 35 |
|---|
| 1 ggacaatagg agccaggcgc accggccagc ccctg(SEQ ID NO: 791) |

| PD1 Exon 5 - location: 12,610...14,026; length 1,417 |
|---|
| 1 aaggaggacc cctcagccgt gcctgtgttc tctgtggact atggggagct ggatttccag 61 tggcgagaga agaccccgga gccccccgtg ccctgtgtcc ctgagcagac ggagtatgcc 121 accattgtct ttcctagcgg aatgggcacc tcatcccccg cccgcagggg ctcagctgac 181 ggccctcgga gtgcccagcc actgaggcct gaggatggac actgctcttg gcccctctga 241 ccggcttcct tggccaccag tgttctgcag accctccacc atgagcccgg gtcagcgcat 301 ttcctcagga gaagcaggca gggtgcaggc cattgcaggc cgtccagggg ctgagctgcc 361 tgggggcgac cggggctcca gcctgcacct gcaccaggca cagccccacc acaggactca 421 tgtctcaatg cccacagtga gcccaggcag caggtgtcac cgtcccctac agggagggcc 481 agatgcagtc actgcttcag gtcctgccag cacagagctg cctgcgtcca gctccctgaa 541 tctctgctgc tgctgctgct gctgctgctg ctgcctgcgg cccggggctg aaggcgccgt 601 ggccctgcct gacgccccgg agcctcctgc ctgaacttgg gggctggttg gagatggcct 661 tggagcagcc aaggtgcccc tggcagtggc atcccgaaac gccctggacg cagggcccaa 721 gactgggcac aggagtggga ggtacatggg gctggggact ccccaggagt tatctgctcc 781 ctgcaggcct agagaagttt cagggaaggt cagaagagct cctggctgtg gtgggcaggg 841 caggaaaccc ctccaccttt acacatgccc aggcagcacc tcaggccctt tgtggggcag 901 ggaagctgag gcagtaagcg ggcaggcaga gctggaggcc tttcaggccc agccagcact 961 ctggcctcct gccgccgcat tccaccccag cccctcacac cactcgggag agggacatcc 1021 tacggtccca aggtcaggag ggcagggctg gggttgactc aggcccctcc cagctgtggc 1081 cacctgggtg ttgggagggc agaagtgcag gcacctaggg ccccccatgt gcccaccctg 1141 ggagctctcc ttggaaccca ttcctgaaat tatttaaagg ggttggccgg gctcccacca 1201 gggcctgggt gggaaggtac aggcgttccc ccggggccta gtacccccgc cgtggcctat 1261 ccactcctca catccacaca ctgcaccccc actcctgggg cagggccacc agcatccagg 1321 cggccagcag gcacctgagt ggctgggaca agggatcccc cttccctgtg gttctattat 1381 attataatta taattaaata tgagagcatg ctaagga (SEQ ID NO: 792) |

In some aspects, the invention provides a modified primary human T cell or population thereof, each cell comprising a modified genome comprising: (b) a genomic modification (e.g., a second genomic modification) in which the PD1 gene on chromosome 2 has been edited to delete a contiguous stretch (e.g., a second contiguous stretch) of genomic DNA, thereby reducing or eliminating PD1 receptor surface expression and/or activity in the cell, wherein the contiguous stretch (e.g., second contiguous stretch) of genomic DNA has been deleted by contacting the cell with a Cas protein or a nucleic acid encoding the Cas protein and a pair of ribonucleic acids (e.g., second pair) having sequences selected from the group consisting of SEQ ID NOs: 196-531 and 4047-8945. In some embodiments, the second pair of ribonucleic acids comprises SEQ ID NO: 462 and SEQ ID NO: 421.

In some aspects, the invention provides a method for altering a target PD1 polynucleotide sequence in a cell comprising contacting the PD1 polynucleotide sequence with a clustered regularly interspaced short palindromic repeats-associated (Cas) protein and from one to two ribonucleic acids, wherein the ribonucleic acids direct Cas protein to and hybridize to a target motif of the target PD1 polynucleotide sequence, wherein the target PD1 polynucleotide sequence is cleaved, and wherein at least one of the one to two ribonucleic acids are selected from the group consisting of SEQ ID NOs. 196-531 and 4047-8945. In some embodiments, each of the one to two ribonucleic acids is selected from the group consisting of SEQ ID NOs: 196-531 and 4047-8945. In some embodiments, the two ribonucleic acids comprise SEQ ID NO: 462 and SEQ ID NO: 421.

In some aspects, the invention provides a modified primary human T cell or population thereof, each cell comprising a modified genome comprising: (b) a genomic modification (e.g., a second genomic modification) in which the PD1 gene on chromosome 2 has been edited to reduce or eliminate PD1 receptor surface expression and/or activity in the cell by contacting the cell with a Cas protein or a nucleic acid encoding the Cas protein and at least one ribonucleic acid having a sequence selected from the group consisting of SEQ ID NOs: 8946-9101.

In some aspects, the invention provides a method for altering a target PD1 polynucleotide sequence in a cell comprising contacting the PD1 polynucleotide sequence with a clustered regularly interspaced short palindromic repeats-associated (Cas) protein and at least one ribonucleic acid, wherein the ribonucleic acid directs Cas protein to and hybridizes to a target motif of the target PD1 polynucleotide sequence, wherein the target PD1 polynucleotide sequence is cleaved, and wherein the at least one ribonucleic acid is selected from the group consisting of SEQ ID NOs. 8946-9101. In certain aspects, a subsequent alteration to the target PD1 polynucleotide sequence in the cell results in a second cleavage of the target PD1 polynucleotide sequence, thereby editing the target PD1 polynucleotide sequence to delete a second contiguous stretch of genomic DNA.

### T Cell Receptor Alpha Chain (TCRA) and T Cell Receptor Beta Chain (TCRB) Loci

In some embodiments, the target polynucleotide sequence is T cell receptor alpha locus (TCRA), or a homolog, ortholog, or variant thereof (Gene ID: 5133, also known as IMD7, TCRD, TRA@, TRAC, and referred to herein as TCRa, TCRA, TCRalpha, and the like). An exemplary TCRA human target polynucleotide sequence is NCBI Reference Sequence: NC_000014.9. In some embodiments, the target polynucleotide is T cell receptor alpha locus (TCRB), or a homolog, ortholog, or variant thereof (Gene ID: 6957, also known as TCRB; TRB@, and referred to herein as TCRb, TCRB, TCRbeta, and the like). Antigen recognition by T-lymphocytes occurs via a mechanism that is similar to the one used immunoglobulins made by B cells. Two main mature T-cell subtypes exist, namely those expressing alpha and beta chains, and those expressing gamma and delta chains. In contrast to secreted Ig molecules, T-cell receptor chains are membrane bound and function through cell-cell contact. T-cell receptor alpha chain encoding genes are grouped on chromosome 14.

The T-cell receptor alpha chain is formed when one of at least 70 variable (V) genes encoding the N-terminal antigen recognition domain rearranges to one of 61 joining (J) gene segments to form a functional V region exon that is transcribed and spliced to a constant region gene (TRAC) segment that encodes the C-terminal portion. The T-cell receptor beta chain is formed when one of 52 variable (V) genes encoding the N-terminal antigen recognition domain rearranges to a diversity (D) gene and a joining (J) gene to form a functional V region exon that is transcribed and spliced to a constant (C) region gene segment encoding the C-terminal portion. In contrast to the alpha chain locus, the beta chain locus has two separate gene clusters after the V genes, each containing a D gene, several J genes, and a C gene. Following their synthesis the alpha and beta chains combine to produce the alpha-beta T-cell receptor heterodimer (Janeway et. al., 2005).

In some aspects, the present disclosure provides a modified primary human cell (e.g., immune cell, e.g., T cell, natural killer cell, etc.) or population thereof comprising a genome in which the TCR alpha chain locus on chromosome 14 has been edited to reduce or eliminate TCR expression (e.g., cell surface expression) and/or activity (e.g., protein activity) in the cell or population thereof using a genetic editing system (e.g., TALENs, CRISPR/Cas, etc.). In some aspects, the present disclosure provides a modified primary human cell (e.g., immune cell, e.g., T cell, natural killer cell, etc.) or population thereof comprising a genome in which the TCR alpha chain locus on chromosome 14 has been edited to delete a contiguous stretch of genomic DNA, e.g., comprising a coding exon, thereby reducing or eliminating TCR expression (e.g., cell surface expression) and/or activity (e.g., protein activity) in the cell or population thereof. The contiguous stretch of genomic DNA can be deleted by contacting a primary human cell (e.g., immune cell, e.g., T cell, natural killer cell, etc.) or population thereof with a Cas protein or a nucleic acid sequence encoding the Cas protein and at least one pair of ribonucleic acids (i.e., CRISPR TCRA gRNA pairs, e.g., at least one gRNA pair, at least two gRNA pairs, at least three gRNA pairs, at least four gRNA pairs, at least five gRNA pairs, etc.) selected from the group consisting of SEQ ID NOs: 532-609 and 9102-9750.

In some aspects, the present disclosure provides a modified primary human cell (e.g., immune cell, e.g., T cell, natural killer cell, etc.) or population thereof comprising a genome in which the TCR beta chain locus on chromosome 7 has been edited to reduce or eliminate TCR expression (e.g., cell surface expression) and/or activity (e.g., protein activity) in the cell or population thereof using a genetic editing system (e.g., TALENs, CRISPR/Cas, etc.). In some aspects, the present disclosure provides a modified primary human cell (e.g., immune cell, e.g., T cell, natural killer cell, etc.) or population thereof comprising a genome in which the TCR beta chain locus on chromosome 7 has been edited to delete a contiguous stretch of genomic DNA, e.g., comprising a coding exon, thereby reducing or eliminating TCR expression (e.g., cell surface expression) and/or activity (e.g., protein activity) in the cell or population thereof. The contiguous stretch of genomic DNA can be deleted by contacting a primary human cell (e.g., immune cell, e.g., T cell, natural killer cell, etc.) or population thereof with a Cas protein or a nucleic acid sequence encoding the Cas protein and at least one pair of ribonucleic acids (i.e., CRISPR TCRA gRNA pairs, e.g., at least one gRNA pair, at least two gRNA pairs, at least three gRNA pairs, at least four gRNA pairs, at least five gRNA pairs, etc.) selected from the group consisting of SEQ ID NOs: 610-765 and 9798-10532.

The present disclosure contemplates reducing or eliminating TCR expression and/or activity in any cell line or primary human cell (e.g., immune cell, e.g., T cell, natural killer cell, etc.) population thereof to produce cells which reduce or eliminate autoreactivity. The present disclosure further contemplates genomically editing primary human cells to cleave TCR alpha chain locus and/or TCR beta chain locus sequences, as well as editing the genome of such cells to alter one or more additional target polynucleotide sequences (e.g., CTLA4, PD1, and/or B2M). It should be appreciated that cleaving a TCR alpha chain locus sequence and/or TCR beta chain locus sequence using one or more gRNAs or gRNA pairs described herein and a Cas protein can result in partial or complete deletion of the target TCR alpha chain locus and/or TCR beta chain locus DNA sequence (e.g., coding exon, e.g., first coding exon).

In some aspects, the invention provides a modified primary human T cell or population thereof, each cell comprising a modified genome comprising: (c)(i) a genomic modification (e.g., a third genomic modification) in which the TCR alpha chain locus on chromosome 14 has been edited to delete a contiguous stretch (e.g., third contiguous stretch) of genomic DNA comprising at least a portion of a coding exon, and/or (c)(ii) a genomic modification (e.g., a fourth genomic modification) in which the TCR beta chain locus on chromosome 7 has been edited to delete a contiguous stretch (e.g., fourth contiguous stretch) of genomic DNA comprising at least a portion of a coding exon, wherein deletion of the contiguous stretch of genomic DNA from the TCR alpha chain locus on chromosome 14 and/or deletion of the contiguous stretch of genomic DNA from the TCR beta chain locus on chromosome 7 reduces or eliminates TCR surface expression and/or TCR activity in the cell or population thereof.

In some aspects, the invention provides a modified primary human T cell or population thereof, each cell comprising a modified genome comprising: (c)(i) a genomic modification (e.g., a third genomic modification) in which the TCR alpha chain locus on chromosome 14 has been edited to delete a contiguous stretch (e.g., a third contiguous stretch) of genomic DNA, thereby reducing or eliminating TCR surface expression and/or activity in the cell. In some embodiments, the contiguous stretch (e.g., third contiguous stretch) of genomic DNA has been deleted by contacting the cell with a Cas protein or a nucleic acid sequence encoding the Cas protein and a pair of ribonucleic acids (e.g., third pair) having sequences selected from the group consisting of SEQ ID NOs: 532-609 and 9102-9750. In some embodiments, the third pair of ribonucleic acids comprises SEQ ID NO: 550 and SEQ ID NO: 573.

In some aspects, the invention provides a modified primary human T cell or population thereof, each cell comprising a modified genome comprising: (c)(ii) a genomic modification (e.g., a fourth genomic modification) in which the TCR beta chain locus on chromosome 7 has been edited to delete a contiguous stretch (e.g., a fourth contiguous stretch) of genomic DNA, thereby reducing or eliminating TCR surface expression and/or activity in the cell. In some embodiments, the contiguous stretch (e.g., fourth contiguous stretch) of genomic DNA has been deleted by contacting the cell with a Cas protein or a nucleic acid sequence encoding the Cas protein and a pair of ribonucleic acids (e.g., fourth pair) having sequences selected from the group consisting of SEQ ID NOs: 610-765 and 9798-10532. In some embodiments, the fourth pair of ribonucleic acids comprises SEQ ID NO: 773 and SEQ ID NO: 778.

In some aspects, the invention provides a method for altering a target TRCA polynucleotide sequence in a cell comprising contacting the TCRA polynucleotide sequence with a clustered regularly interspaced short palindromic repeats-associated (Cas) protein and from one to two ribonucleic acids, wherein the ribonucleic acids direct Cas protein to and hybridize to a target motif of the target TCRA polynucleotide sequence, wherein the target TCRA polynucleotide sequence is cleaved, and wherein at least one of the one to two ribonucleic acids are selected from the group consisting of SEQ ID NOs. 532-609 and 9102-9750. In some embodiments, each of the one to two ribonucleic acids is selected from the group consisting of SEQ ID NOs: 532-609 and 9102-9750. In some embodiments, the two ribonucleic acids comprise SEQ ID NO: 550 and SEQ ID NO: 573.

In some aspects, the invention provides a method for altering a target TRCB polynucleotide sequence in a cell comprising contacting the TCRB polynucleotide sequence with a clustered regularly interspaced short palindromic repeats-associated (Cas) protein and from one to two ribonucleic acids, wherein the ribonucleic acids direct Cas protein to and hybridize to a target motif of the target TCRB polynucleotide sequence, wherein the target TCRB polynucleotide sequence is cleaved, and wherein at least one of the one to two ribonucleic acids are selected from the group consisting of SEQ ID NOs. 610-765 and 9798-10321. In some embodiments, each of the one to two ribonucleic acids is selected from the group consisting of SEQ ID NOs: 610-765 and 9798-10321. In some embodiments, the two ribonucleic acids comprise SEQ ID NO: 657 and SEQ ID NO: 662.

In some aspects, the invention provides a modified primary human T cell or population thereof, each cell comprising a modified genome comprising: (c)(i) a genomic modification (e.g., a third genomic modification) in which the TCR alpha chain locus on chromosome 14 has been edited to reduce or eliminate TCR surface expression and/or activity in the cell. In some embodiments, the third genomic modification occurs by contacting the cell with a Cas protein or a nucleic acid sequence encoding the Cas protein and at least one ribonucleic acid having a sequence selected from the group consisting of SEQ ID NOs: 9751-9797.

In some aspects, the invention provides a modified primary human T cell or population thereof, each cell comprising a modified genome comprising: (c)(ii) a genomic modification (e.g., a fourth genomic modification) in which the TCR beta chain locus on chromosome 7 has been edited to reduce or eliminate TCR surface expression and/or activity in the cell. In some embodiments, the fourth genomic modification occurs by contacting the cell with a Cas protein or a nucleic acid sequence encoding the Cas protein and at least one ribonucleic acid having a sequence selected from the group consisting of SEQ ID NOs: 10533-10573.

In some aspects, the invention provides a method for altering a target TRCA polynucleotide sequence in a cell comprising contacting the TCRA polynucleotide sequence with a clustered regularly interspaced short palindromic repeats-associated (Cas) protein and at least one ribonucleic acid, wherein the ribonucleic acid directs Cas protein to and hybridizes to a target motif of the target TCRA polynucleotide sequence, wherein the target TCRA polynucleotide sequence is cleaved, and wherein the at least one ribonucleic acid is selected from the group consisting of SEQ ID NOs. 9751-9797. In certain aspects, a subsequent alteration to the target TRCA polynucleotide sequence in the cell results in a second cleavage of the target TRCA polynucleotide sequence, thereby editing the target TRCA polynucleotide sequence to delete a third contiguous stretch of genomic DNA.

In some aspects, the invention provides a method for altering a target TRCB polynucleotide sequence in a cell comprising contacting the TCRB polynucleotide sequence with a clustered regularly interspaced short palindromic repeats-associated (Cas) protein and at least one ribonucleic acid, wherein the ribonucleic acid directs Cas protein to and hybridizes to a target motif of the target TCRB polynucleotide sequence, wherein the target TCRB polynucleotide sequence is cleaved, and wherein the at least one ribonucleic acid is selected from the group consisting of SEQ ID NOs. 10533-10573. In certain aspects, a subsequent alteration to the target TRCB polynucleotide sequence in the cell results in a second cleavage of the target TRCB polynucleotide sequence, thereby editing the target TRCB polynucleotide sequence to delete a fourth contiguous stretch of genomic DNA.

### Beta-2-microglobulin (B2M)

In some embodiments, the target polynucleotide sequence is beta-2-microglobulin (B2M; Gene ID: 567). The B2M polynucleotide sequence encodes a serum protein associated with the heavy chain of the major histocompatibility complex (MHC) class I molecules which are expressed on the surface of virtually all nucleated cells. B2M protein comprises a beta-pleated sheet structure that has been found to form amyloid fibrils in certain pathological conditions. The B2M gene has 4 exons which span approximately 8 kb. B2M has been observed in the serum of normal individuals and in elevated amounts in urine from patients having Wilson disease, cadmium poisoning, and various conditions leading to renal tubular dysfunction. Other pathological conditions known to be associated with B2M include, without limitation, a homozygous mutation (e.g., ala11pro) in the B2M gene has been reported in individuals having familial hypercatabolic hypoproteinemia, a heterozygous mutation (e.g., asp76asn) in the B2M gene has been reported in individuals having familial visceral amyloidosis.

In some embodiments, the target polynucleotide sequence is a variant of B2M. In some embodiments, the target polynucleotide sequence is a homolog of B2M. In some embodiments, the target polynucleotide sequence is an ortholog of B2M.

In some aspects, the present disclosure provides a modified primary human cell (e.g., immune cell, e.g., T cell, natural killer cell, etc.) or population thereof comprising a genome in which the β2-microglobulin (B2M) gene on chromosome 15 has been edited to reduce or eliminate surface expression of MHC class I molecules in the cell or population thereof using a genetic editing system (e.g., TALENs, CRISPR/Cas, etc.). In some aspects, the present disclosure provides a modified primary human T cell or population thereof comprising a genome in which the B2M gene on chromosome 15 has been edited to delete a contiguous stretch of genomic DNA of NCBI Reference Sequence: NG_ 012920.1, thereby reducing or eliminating surface expression of MHC class I molecules in the cell or population thereof. The contiguous stretch of genomic DNA can be deleted by contacting the cell or population thereof with a Cas protein or a nucleic acid sequence encoding the Cas protein and at least one ribonucleic acid or at least one pair of ribonucleic acids selected from the group consisting of SEQ ID NOs: 766-780 and 10574-13719.

The present disclosure contemplates ablating MHC class I molecule surface expression in any cell line or primary human cell population (e.g., immune cells, e.g., T cells, natural killer cells, etc.) to produce cells which reduce or eliminate the likelihood of triggering unwanted host immune responses when transplanted (e.g., allogeneic transplantation). B2M is an accessory chain of the MHC class I proteins which is necessary for the expression of MHC class I proteins on the surface of cells. It is believed that engineering cells (e.g., mutant cells) devoid of surface MHC class I may reduce the likelihood that the engineered cells will be detected by cytotoxic T cells when the engineered cells are administered to a host. Accordingly, in some embodiments, cleavage of the target polynucleotide sequence encoding B2M in the cell or population of cells reduces the likelihood that the resulting cell or cells will trigger a host immune response when the cells are administered to the subject.

The present invention contemplates genomically editing primary human cells to cleave B2M gene sequences, as well as editing the genome of such cells to alter one or more additional target polynucleotide sequences (e.g., CTLA4, PD1, TCRA, and/or TCRB). It should be appreciated that cleaving a B2M genomic sequence using one or more gRNAs or gRNA pairs described herein and a Cas protein can result in partial or complete deletion of the target B2M genomic sequence.

In some aspects, the invention provides a modified primary human T cell or population thereof, each cell comprising a modified genome comprising: (d) a genomic modification (e.g., a fifth genomic modification) in which the B2M gene on chromosome 15 has been edited to delete a contiguous stretch (e.g., fifth contiguous stretch) of genomic DNA, thereby reducing or eliminating MHC Class I molecule surface expression and/or activity in the cell. In some embodiments, the contiguous stretch (e.g., fifth contiguous stretch) of genomic DNA has been deleted by contacting the cell with a Cas protein or a nucleic acid sequence encoding the Cas protein and a pair of ribonucleic acids (e.g., fifth pair) having sequences selected from the group consisting of SEQ ID NOs: 766-780 and 10574-13257. In some embodiments, the fifth pair of ribonucleic acids comprises SEQ ID NO: 773 and SEQ ID NO: 778.

In some aspects, the invention provides a method for altering a target B2M polynucleotide sequence in a cell comprising contacting the B2M polynucleotide sequence with a clustered regularly interspaced short palindromic repeats-associated (Cas) protein and from one to two ribonucleic acids, wherein the ribonucleic acids direct Cas protein to and hybridize to a target motif of the target B2M polynucleotide sequence, wherein the target B2M polynucleotide sequence is cleaved, and wherein at least one of the one to two ribonucleic acids are selected from the group consisting of SEQ ID NOs. 766-780 and 10574-13257. In some embodiments, each of the one to two ribonucleic acids is selected from the group consisting of SEQ ID NOs: 766-780 and 10574-13257. In some embodiments, the fifth pair of ribonucleic acids comprises SEQ ID NO: 773 and SEQ ID NO: 778.

In some aspects, the invention provides a modified primary human T cell or population thereof, each cell comprising a modified genome comprising: (d) a genomic modification (e.g., a fifth genomic modification) in which the B2M gene on chromosome 15 has been edited to reduce or eliminate MHC Class I molecule surface expression and/or activity in the cell. In some embodiments, the fifth genomic modification occurs by contacting the cell with a Cas protein or a nucleic acid sequence encoding the Cas protein and at least one ribonucleic acid having a sequence selected from the group consisting of SEQ ID NOs: 13258-13719.

In some aspects, the invention provides a method for altering a target B2M polynucleotide sequence in a cell comprising contacting the B2M polynucleotide sequence with a clustered regularly interspaced short palindromic repeats-associated (Cas) protein and at least one ribonucleic acid, wherein the ribonucleic acid directs Cas protein to and hybridizes to a target motif of the target B2M polynucleotide sequence, wherein the target B2M polynucleotide sequence is cleaved, and wherein the at least one ribonucleic acid is selected from the group consisting of SEQ ID NOs. 13258-13719. In certain aspects, a subsequent alteration to the target B2M polynucleotide sequence in the cell results in a second cleavage of the target B2M polynucleotide sequence, thereby editing the target B2M polynucleotide sequence to delete a fifth contiguous stretch of genomic DNA.

### Chimeric Antigen Receptors

Aspects of the present invention relate to primary human cells modified to include a chimeric antigen receptor. Chimeric Antigen Receptors (CARs) are molecules designed to target immune cells to specific molecular targets expressed on cell surfaces. In their most basic form, they are receptors introduced to a cell that couple a specificity domain expressed on the outside of the cell to signaling pathways on the inside of the cell such that when the specificity domain interacts with its target, the cell becomes activated. Often CARs are made from variants of T-cell receptors (TCRs) where a specificity domain such as a scFv or some type of receptor is fused to the signaling domain of a TCR. These constructs are then introduced into a T-cell allowing the T-cell to become activated in the presence of a cell expressing the target antigen, resulting in the attack on the targeted cell by the activated T-cell in a non-MHC dependent manner (see Chicaybam et al (2011) Int Rev Immunol 30:294-311). Currently, tumor specific CARs targeting a variety of tumor antigens are being tested in the clinic for treatment of a variety of different cancers. Examples of these cancers and their antigens that are being targeted includes follicular lymphoma (CD20 or GD2), neuroblastoma (CD171), non-Hodgkin lymphoma (CD20), lymphoma (CD19), glioblastoma (IL13Rα2), chronic lymphocytic leukemia or CLL and acute lymphocytic leukemia or ALL (both CD 19). Virus specific CARs have also been developed to attack cells harboring virus such as HIV. For example, a clinical trial was initiated using a CAR specific for Gp100 for treatment of HIV (Chicaybam, ibid).

As used herein, a "chimeric antigen receptor" (CAR) is an artificially constructed hybrid protein or polypeptide comprising a specificity or recognition (i.e. binding) domain linked to an immune receptor responsible for signal transduction in lymphocytes. The binding domain is typically derived from a Fab antibody fragment that has been fashioned into a single chain scFv via the introduction of a flexible linker between the antibody chains within the specificity domain. Other possible specificity domains can include the signaling portions of hormone or cytokine molecules, the extracellular domains of receptors, and peptide ligands or peptides isolated by library (e.g. phage) screening (see Ramos and Dotti, (2011) Expert Opin Bio Ther 11 (7): 855). Flexibility between the signaling and the binding portions of the CAR may be a desirable characteristic to allow for more optimum interaction between the target and the binding domain, so often a hinge region is included. One example of a structure that can be used is the CH2-CH3 region from an immunoglobulin such as an IgG molecule. The signaling domain of the typical CAR comprises intracellular domains of the TCR-CD3 complex such as the zeta chain. Alternatively, the γ chain of an Fc receptor may be used. The transmembrane portion of the typical CAR can comprise transmembrane portions of proteins such as CD4, CD8 or CD28 (Ramos and Dotti, ibid). Characteristics of some CARs include their ability to redirect T-cell specificity and reactivity toward a selected target in a non-MHC-restricted manner. The non-MHC-restricted target recognition gives T-cells expressing CARs the ability to recognize a target independent of antigen processing, thus bypassing a major mechanism of tumor escape.

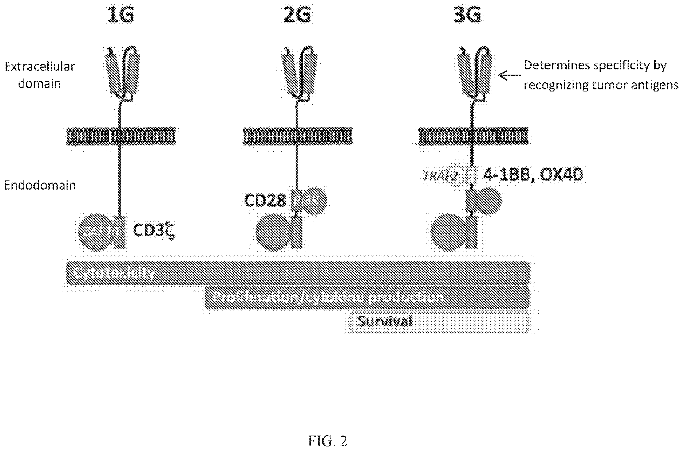

At least three different generations of chimeric antigen receptors contemplated for use in the modified cells, compositions, methods, and kits of the present invention are shown in FIG. 2. The so called "first generation" CARs often comprise a single internal signaling domain such as the CD3 zeta chain, and are thought to be somewhat ineffectual in the clinic, perhaps due to incomplete activation. To increase performance of T-cells bearing these CARs, second generation CARs have been generated with the ability of proving the T-cell additional activation signals by including another stimulatory domain, often derived from the intercellular domains of other receptors such as CD28, CD134/OX40, CD137/4-1BB, Lck, ICOS and DAP10. Additionally, third generation CARs have also been developed wherein the CAR contains three or more stimulatory domains (Ramos and Dotti, ibid). In some instances, CAR can comprise an extracellular hinge domain, transmembrane domain, and optionally, an intracellular hinge domain comprising CD8 and an intracellular T-cell receptor signaling domain comprising CD28; 4-1BB, and CD3ζ CD28 is a T-cell marker important in T-cell co-stimulation. CD8 is also a T-cell marker. 4-1BB transmits a potent costimulatory signal to T-cells, promoting differentiation and enhancing long-term survival of T lymphocytes. CD3ζ associates with TCRs to produce a signal and contains immunoreceptor tyrosine-based activation motifs (ITAMs). In other instances, CARs can comprise an extracellular hinge domain, transmembrane domain, and intracellular T-cell signaling domain comprising CD28 and CD3ζ In further instances, CARs can comprise an extracellular hinge domain and transmembrane domain comprising CD8 and an intracellular T-cell receptor signaling domain comprising CD28 and CD3ζ.

In some embodiments, the modified primary human cells (e.g., immune cells, e.g., T cells, natural killer cells, etc.) further comprise a chimeric antigen receptor or an exogenous nucleic acid encoding the chimeric antigen receptor. The chimeric antigen receptor specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof. Numerous cancer antigens are known in the art and may be targeted by specific CARs. By way of non-limiting examples, see Table 5 for tumor associated antigens that may be targeted by CARs (see Ramos and Dotti, ibid, and Orentas et al (2012), Front in Oncol 2:1).

| Table 5 - Tumor associated antigens suitable for CAR targeting | | |
|---|---|---|
| Tumor type | Antigen | Description |
| Gastrointenstinal | EGP2/EpCam | Epithelial glycoprotein 2/Epithelial cell adhesion molecule |
| Gastrointenstinal | EGP40 | Epithelial glycoprotein 40 |
| Gastrointenstinal | TAG72/CA72-4 | Tumor associated glycoprotein 72/cancer antigen 72-4 |
| Glioblastoma | IL13Rα2 | Interleukin 13 receptor alpha-2 subunit |
| Kidney | G250/MN/CA IX | Carbonic anhydrase IX |
| Lymphoid malignancies | CD19 | |
| Lymphoid malignancies | CD52 | |
| Lymphoid malignancies | CD33 | |
| Lymphoid malignancies | CD20 | Membrane-spanning 4-domains subfamily A member 1 |
| Lymphoid malignancies | TSLPR (CRLF2) | |
| Lymphoid malignancies | CD22 | Sialic acid-binding Ig-like lectin 2 |
| Lymphoid malignancies | CD30 | TNF receptor superfamily member 8 |
| Lymphoid malignancies | κ | Kappa light chain |
| Melanoma | GD3 | GD3-Ganglioside |
| Melanoma | HLA-A1 + MAGE-1 | Human leukocyte antigen A1 + Melanoma antigen 1 |
| Neuroblastoma/Neural | CD171 | L1 cell adhesion molecule |
| tumors | | |
| Neuroblastoma/Neural tumors | ALK | Anaplastic lymphoma kinase |
| Neuroblastoma/Neural tumors | GD2 | GD2-Ganglioside |
| Neuroblastoma/Neural tumors | CD47 | |
| Neuroblastoma/Neural tumors | EGFRvIII | |
| Neuroblastoma/Neural tumors | NCAM | Neural cell adhesion molecule |
| Ovary | FBP/αFR | Folate binding protein/alpha folate receptor |
| Ovary | Le(Y) | Lewis-Y antigen |
| Ovary | MUC1 | Mucin 1 |
| Prostate | PSCA | Prostate stem cell antigen |
| Prostate | PSMA | Prostate-specific membrane antigen |
| Rhadbomyosarcoma | FGFR4 | Fibroblast growth factor receptor 4 |
| Rhadbomyosarcoma | FAR | Fetal acetylcholine receptor |
| Several solid tumors | CEA | Carcinoembryonic antigen |
| Several solid tumors | ERBB2/HER2 | Avian ertyroblastic leukemia viral oncogene homolog 2/Human epidermal growth factor receptor 2 |
| Several solid tumors | ERBB3 + ERBB4 | Avian erthroblastic leukemia viral oncogene homology 3 + 4 |
| Several solid tumors | Mesothelin | |
| Various tumors | CD44v6 | Flyaluronate receptor variant 6 |
| Various tumors | B7-H3 | Adhesion receptor |
| Various tumors | Glypican-3,5 | Cell surface peptidoglycan |
| Various tumors | ROR 1 | |
| Various tumors | Survivin | Anti-apoptotic molecule |
| Various tumors | FOLR1 | a folate receptor |
| Various tumors | WT1 | Wilm's tumor antigen |
| Various tumors | CD70 | |
| Various tumors | VEGFR2/FLK/KD R | Vascular endothelial growth factor 2/Fetal liver kinase 1/Kinase domain insert |

In some embodiments, the CARs may have specificity for a tumor antigen where the CAR specificity domain is a ScFv. In other embodiments, CARs may be specific for a tumor antigen where the CAR specificity domain comprises a ligand or polypeptide. Non-limiting exemplary CARs include those targeted to CD33 (see Dutour et al, (2012)Adv Hematol 2012; 2012:683065), GD2 (Louis et al (2011) Blood 118(23):650-6), CD19 (Savoldo et al, (2011) J Clin Invest 121(5): 1822 and Torikai et al (2012) Blood 119(24): 5697), IL-11Rα (Huang et al, (2012) Cancer Res 72(1):271-81), CD20 (Till et al (2012) Blood 119(17):3940-50), NY-ESO-1 (Schuberth et al, (2012) Gene Ther doi:10.1038/gt2012.48), ErbB2 (Zhao et al, (2009) J. Immunol 183(9): 5563-74), CD70 (Shaffer et al (2011)Blood 116(16):4304-4314), CD38 (Bhattacharayya et al (2012) Blood Cane J2(6) p. e75), CD22 (Haso et al. (2012) Cane Res 72(8) S1, doi: 1158/1158-7445 AM2012-3504), CD74 (Stein et al (2004) Blood 104:3705-3711), CAIX (Lamers et al, (2011) Blood 117(1): 72-82) STEAP1 (see Kiessling et al. (2012) Cancers 4:193-217 for review of target) VEGF-R2 (U.S. Patent Publication No. US20120213783A1), the folate receptor (PCT patent publication WO2012099973) and IL-13 Rα (U.S. Pat. No. 7,514,537).

### ExogenousMolecules Delivered via the Modified Cells

Aspects of the invention relate to using a modified primary human cell or population thereof of the present invention (e.g., immune cell, e.g., T cell, natural killer cell, etc.) to deliver an exogenous molecule to a cell, tissue, or organ to modulate a biological activity/effect of interest in the cell, tissue, or organ. For example, the modified primary human cell or population thereof can be modified to deliver a therapeutic product (e.g., an immunomodulatory cytokine or antagonist thereof to mediate autoimmune activity, for example toward an anti-inflammatory Th2 type response, e.g., transduction of a T cell hybridoma specific for the peptide MBP 87-99 with a viral vector that constitutively expresses IL-4 to target cells to a myelin protein and inhibit Th1 induction and macrophage activation in active CNS lesions, as described further in Johnson and Tuohy, "Targeting Antigen-Specific T Cells for Gene Therapy of Autoimmune Disease," Madame Curie Bioscience Database) or regenerative product (e.g., to repair damaged tissue, generate new or artificial tissue, or both, e.g., using modified T cells to deliver nerve growth factor (NGF) to the central nervous system (CNS), platelet-derived growth factor-A (PDGF-A) to treat experimental autoimmune encephalomyelitis (EAE), etc.) to sites of inflammation and tissue destruction, modulate cellular interactions (e.g., modulation of intercellular functions, such as modulation of signaling pathways, apoptosis induction, stopping epitope spreading, tolerance induction, tolerance reversal, and specificity programming, etc.), or to correct its own genetic defects to ameliorate disease (e.g., autoimmune diseases).

An "exogenous" molecule is a molecule that is not normally present in a cell, but can be introduced into a cell by one or more genetic, biochemical or other methods. "Normal presence in the cell" is determined with respect to the particular developmental stage and environmental conditions of the cell. Thus, for example, a molecule that is present only during embryonic development of neurons is an exogenous molecule with respect to an adult neuron cell. An exogenous molecule can comprise, for example, a functioning version of a malfunctioning endogenous molecule or a malfunctioning version of a normally-functioning endogenous molecule.

An exogenous molecule can be, among other things, a small molecule, such as is generated by a combinatorial chemistry process, or a macromolecule such as a protein, nucleic acid, carbohydrate, lipid, glycoprotein, lipoprotein, polysaccharide, any modified derivative of the above molecules, or any complex comprising one or more of the above molecules. Nucleic acids include DNA and RNA, can be single- or double-stranded; can be linear, branched or circular; and can be of any length. Nucleic acids include those capable of forming duplexes, as well as triplex-forming nucleic acids. See, for example, U.S. Pat. Nos. 5,176,996 and 5,422,251. Proteins include, but are not limited to, DNA-binding proteins, transcription factors, chromatin remodeling factors, methylated DNA binding proteins, polymerases, methylases, demethylases, acetylases, deacetylases, kinases, phosphatases, integrases, recombinases, ligases, topoisomerases, gyrases and helicases.

An exogenous molecule can be the same type of molecule as an endogenous molecule, e.g., an exogenous protein or nucleic acid. In such instances, the exogenous molecule is introduced into the cell at greater concentrations than that of the endogenous molecule in the cell. In some instances, an exogenous nucleic acid can comprise an infecting viral genome, a plasmid or episome introduced into a cell, or a chromosome that is not normally present in the cell. Methods for the introduction of exogenous molecules into cells are known to those of skill in the art and include, but are not limited to, lipid-mediated transfer (i.e., liposomes, including neutral and cationic lipids), electroporation, direct injection, cell fusion, particle bombardment, calcium phosphate co-precipitation, DEAE-dextran-mediated transfer and viral vector-mediated transfer.

In some embodiments, the exogenous molecule comprises a fusion molecule (e.g., fusion protein or nucleic acid). A "fusion" molecule is a molecule in which two or more subunit molecules are linked, preferably covalently. The subunit molecules can be the same chemical type of molecule, or can be different chemical types of molecules. Examples of the first type of fusion molecule include, but are not limited to, fusion proteins (for example, a fusion between a CRISPR DNA-binding domain and a cleavage domain); fusion nucleic acids (for example, a nucleic acid encoding the fusion protein described supra) and fusions between nucleic acids and proteins (e.g., CRISPR/Cas nuclease system). Examples of the second type of fusion molecule include, but are not limited to, a fusion between a triplex-forming nucleic acid and a polypeptide, and a fusion between a minor groove binder and a nucleic acid.

Expression of a fusion molecule in a cell can result from delivery of the fusion molecule to the cell, for instance for fusion proteins by delivery of the fusion protein to the cell or by delivery of a polynucleotide encoding the fusion protein to a cell, wherein the polynucleotide is transcribed, and the transcript is translated, to generate the fusion protein. Trans-splicing, polypeptide cleavage and polypeptide ligation can also be involved in expression of a protein in a cell. Methods for polynucleotide and/or polypeptide delivery to cells are presented elsewhere in this disclosure.

A "gene," for the purposes of the present disclosure, includes a DNA region encoding a gene product (see infra), as well as al1 DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

"Gene expression" refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of an mRNA. Gene products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

"Modulation" of gene expression refers to a change in the expression level of a gene. Modulation of expression can include, but is not limited to, gene activation and gene repression. Modulation may also be complete, i.e. wherein gene expression is totally inactivated or is activated to wildtype levels or beyond; or it may be partial, wherein gene expression is partially reduced, or partially activated to some fraction of wildtype levels. "Eukaryotic" cells include, but are not limited to, fungal cells (such as yeast), plant cells, animal cells, mammalian cells and human cells (e.g., T-cells).

The terms "operative linkage" and "operatively linked" (or "operably linked") are used interchangeably with reference to a juxtaposition of two or more components (such as sequence elements), in which the components are arranged such that both components function normally and allow the possibility that at least one of the components can mediate a function that is exerted upon at least one of the other components. By way of illustration, a transcriptional regulatory sequence, such as a promoter, is operatively linked to a coding sequence if the transcriptional regulatory sequence controls the level of transcription of the coding sequence in response to the presence or absence of one or more transcriptional regulatory factors. A transcriptional regulatory sequence is generally operatively linked in cis with a coding sequence, but need not be directly adjacent to it. For example, an enhancer is a transcriptional regulatory sequence that is operatively linked to a coding sequence, even though they are not contiguous.

With respect to fusion polypeptides, the term "operatively linked" can refer to the fact that each of the components performs the same function in linkage to the other component as it would if it were not so linked. For example, with respect to a fusion polypeptide in which a CasDNA-binding domain is fused to a cleavage domain, the DNA-binding domain and the cleavage domain are in operative linkage if, in the fusion polypeptide, the DNA-binding domain portion is able to bind its target site and/or its binding site, while the cleavage domain is able to cleave DNA in the vicinity of the target site. Similarly, with respect to a fusion polypeptide in which a CasDNA-binding domain is fused to an activation or repression domain, the DNA-binding domain and the activation or repression domain are in operative linkage if, in the fusion polypeptide, the DNA-binding domain portion is able to bind its target site and/or its binding site, while the activation domain is able to upregulate gene expression or the repression domain is able to downregulate gene expression.

A "functional fragment" of a protein, polypeptide or nucleic acid is a protein, polypeptide or nucleic acid whose sequence is not identical to the full-length protein, polypeptide or nucleic acid, yet retains the same function as the full-length protein, polypeptide or nucleic acid. A functional fragment can possess more, fewer, or the same number of residues as the corresponding native molecule, and/or can contain one or more amino acid or nucleotide substitutions. Methods for determining the function of a nucleic acid (e.g., coding function, ability to hybridize to another nucleic acid) are well-known in the art. Similarly, methods for determining protein function are well-known. For example, the DNA-binding function of a polypeptide can be determined, for example, by filter-binding, electrophoretic mobility-shift, or immunoprecipitation assays. DNA cleavage can be assayed by gel electrophoresis. See Ausubel *et al.,* supra. The ability of a protein to interact with another protein can be determined, for example, by co-immunoprecipitation, two-hybrid assays or complementation, both genetic and biochemical. See, for example, Fields et al. (1989) Nature 340:245-246; U.S. Pat. No. 5,585,245 and PCT WO 98/44350.

A "vector" is capable of transferring gene sequences to target cells. Typically, "vector construct," "expression vector," and "gene transfer vector," mean any nucleic acid construct capable of directing the expression of a gene of interest and which can transfer gene sequences to target cells. Thus, the term includes cloning, and expression vehicles, as well as integrating vectors.

A "reporter gene" or "reporter sequence" refers to any sequence that produces a protein product that is easily measured, preferably although not necessarily in a routine assay. Suitable reporter genes include, but are not limited to, sequences encoding proteins that mediate antibiotic resistance (e.g., ampicillin resistance, neomycin resistance, G418 resistance, puromycin resistance), sequences encoding colored or fluorescent or luminescent proteins (e.g., green fluorescent protein, enhanced green fluorescent protein, red fluorescent protein, luciferase), and proteins which mediate enhanced cell growth and/or gene amplification (e.g., dihydrofolate reductase). Epitope tags include, for example, one or more copies of FLAG, His, myc, Tap, HA or any detectable amino acid sequence. "Expression tags" include sequences that encode reporters that may be operably linked to a desired gene sequence in order to monitor expression of the gene of interest.

In some embodiments, the modified primary human cell or population thereof further comprises at least one exogenous protein that modulates a biological effect of interest in an adjacent cell, tissue, or organ, or an exogenous nucleic acid encoding the protein.

### Methods for Producing Modified Cells

Aspects of the invention relate to methods for producing modified primary human cells (e.g., immune cells, e.g., T cells, natural killer cells, etc.). In some embodiments, a method for producing a modified primary human T cell or population thereof includes the step of: (a) editing the cytotoxic T-lymphocyte-associated protein 4 (CTLA4) gene on chromosome 2 in a primary human T cell or population thereof to delete a contiguous stretch (e.g., a first contiguous stretch) of genomic DNA, thereby reducing or eliminating CTLA4 receptor surface expression and/or activity in the cell or population thereof.

In some embodiments, a method for producing a modified primary human T cell or population thereof includes the step of: (b) editing the programmed cell death 1 (PD1) gene on chromosome 2 in the cell or population thereof to delete a contiguous stretch (e.g., second contiguous stretch) of genomic DNA, thereby reducing or eliminating PD1 receptor surface expression and/or activity in the cell or population thereof.

In some embodiments, a method for producing a modified primary human T cell or population thereof includes the step of: (c)(i) editing the gene encoding the T cell receptor (TCR) alpha chain locus on chromosome 14 in the cell or population thereof to delete a contiguous stretch (e.g., third contiguous stretch) of genomic DNA, and/or (c)(ii) editing the gene encoding the TCR beta chain locus on chromosome 7 in the cell or population thereof to delete a contiguous stretch (e.g., fourth contiguous stretch) of genomic DNA, thereby reducing or eliminating TCR surface expression and/or activity in the cell or population thereof.

In some embodiments, a method for producing a modified primary human T cell or population thereof includes the step of: (d) editing the β2-microglobulin (B2M) gene on chromosome 15 in the cell or population thereof to delete a contiguous stretch (e.g., fifth contiguous stretch) of genomic DNA, thereby reducing or eliminating MHC Class I molecule surface expression and/or activity in the cell or population thereof.

In some embodiments, the steps of editing in (a)-(d) comprises contacting the cell or population thereof with a Cas protein or a nucleic acid sequence encoding the Cas protein, and at least one pair (e.g., a first pair) of guide RNA sequences to delete the contiguous stretch (e.g., first contiguous stretch) of genomic DNA from the gene in (a), at least one pair (e.g., a second pair) of guide RNA sequences to delete the contiguous stretch (e.g., second contiguous stretch) of genomic DNA from the gene in (b), at least one pair (e.g., a third pair) of guide RNA sequences to delete the contiguous stretch (e.g., third contiguous stretch) of genomic DNA from the gene in (c)(i), and/or at least one pair (e.g., a fourth pair) of guide RNA sequences to delete the contiguous stretch (e.g., fourth contiguous stretch) of genomic DNA from the gene in (c)(ii), and at least one pair (e.f., a fifth pair) of guide RNA sequences to delete the contiguous stretch (e.g., fifth contiguous stretch) of genomic DNA from the gene in (d).

In some embodiments, a method for producing a modified primary human T cell or population thereof optionally includes the step of: (e) (i) causing the cell or population thereof to express at least one chimeric antigen receptor that specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof, and/or (e)(ii) causing the cell or population thereof to express at least one protein that modulates a biological effect of interest in an adjacent cell, tissue, or organ.

In some aspects, the present invention provides a method for producing a modified primary human T cell or population thereof, the method comprising: (a) editing the cytotoxic T-lymphocyte-associated protein 4 (CTLA4) gene on chromosome 2 in a primary human T cell or population thereof to delete a first contiguous stretch of genomic DNA comprising an intron flanked by at least a portion of an adjacent upstream exon and at least a portion of an adjacent downstream exon, and the 3' end of the genomic DNA upstream with respect to the 5' end of the deleted first contiguous stretch of genomic DNA is covalently joined to the 5' end of the genomic DNA downstream with respect to the 3' end of the deleted first contiguous stretch of genomic DNA to result in a modified CTLA4 gene on chromosome 2 that lacks the first contiguous stretch of genomic DNA, thereby reducing or eliminating CTLA4 receptor surface expression and/or activity in the cell or population thereof; and/or (b) editing the programmed cell death 1 (PD1) gene on chromosome 2 in a primary human T cell or population thereof to delete a second contiguous stretch of genomic DNA comprising an intron flanked by at least a portion of an adjacent upstream exon and at least a portion of an adjacent downstream exon, and the 3' end of the genomic DNA upstream with respect to the deleted second contiguous stretch of genomic DNA is covalently joined to the 5' end of the genomic DNA downstream with respect to the 3' end of the deleted second contiguous stretch of genomic DNA to result in a modified PD1 gene on chromosome 2 that lacks the second contiguous stretch of genomic DNA, thereby reducing or eliminating PD1 receptor surface expression and/or activity in the cell or population thereof.

In some aspects, the invention provides a method for producing a modified primary human T cell or population thereof, the method comprising: (a) contacting a primary human T cell or population thereof with a Cas protein or a nucleic acid sequence encoding the Cas protein and a first pair of ribonucleic acids having sequences selected from the group consisting of SEQ ID NOs: 1-195 and 797-3637, thereby editing the cytotoxic T-lymphocyte-associated protein 4 (CTLA4) gene on chromosome 2 to delete a first contiguous stretch of genomic DNA, and reduce or eliminate CTLA4 receptor surface expression and/or activity in the cell or population thereof; and/or (b) contacting a primary human T cell or population thereof with the Cas protein or the nucleic acid sequence encoding the Cas protein and a second pair of ribonucleic acids having sequences selected from the group consisting of SEQ ID NOs: 196-531 and 4047-8945, thereby editing the programmed cell death 1 (PD1) gene on chromosome 2 to delete a second contiguous stretch of genomic DNA, and reduce or eliminate PD1 receptor surface expression and/or activity in the cell or population thereof.

In some embodiments, the first pair of ribonucleic acids comprises SEQ ID NO: 128 and SEQ ID NO: 72, and the second pair of ribonucleic acids comprises SEQ ID NO: 462 and SEQ ID NO: 421.

In some embodiments, the method for producing a modified primary human T cell or population thereof further comprises: (c)(i) editing the gene encoding the T cell receptor (TCR) alpha chain locus on chromosome 14 in the cell or population thereof to delete a third contiguous stretch of genomic DNA comprising at least a portion of a coding exon, and/or (c)(ii) editing the gene encoding the TCR beta chain locus on chromosome 7 in the cell or population thereof to delete a fourth contiguous stretch of genomic DNA comprising at least a portion of a coding exon, thereby reducing or eliminating TCR surface expression and/or activity in the cell or population thereof. In some embodiments, the editing step in (c)(i) comprises contacting the cell or population thereof with the Cas protein or the nucleic acid sequence encoding the Cas protein and a third pair of ribonucleic acids having sequences selected from the group consisting of SEQ ID NOs: 532-609 and 9102-9750, and/or the editing step in (c)(ii) comprises contacting the cell or population thereof with the Cas protein or the nucleic acid sequence encoding the Cas protein and a fourth pair of ribonucleic acids having sequences selected from the group consisting of SEQ ID NOs: 610-765 and 9798-10532. In some embodiments, the third pair of ribonucleic acids comprises SEQ ID NO: 550 and SEQ ID NO: 573, and/or the fourth pair of ribonucleic acids comprises SEQ ID NO: 657 and SEQ ID NO: 662.

In some embodiments, the method for producing a modified primary human T cell or population thereof further comprises: (d) editing the β2-microglobulin (B2M) gene on chromosome 15 in the cell or population thereof to delete a fifth contiguous stretch of genomic DNA, thereby reducing or eliminating MHC Class I molecule surface expression and/or activity in the cell or population thereof. In some embodiments, the step of editing in (d) comprises contacting the cell with the Cas protein or the nucleic acid sequence encoding the Cas protein and a fifth pair of ribonucleic acids having sequences selected from the group consisting of SEQ ID NOs: 766-780 and 10574-13257. In some embodiments, the fifth pair of ribonucleic acids comprises SEQ ID NO: 773 and SEQ ID NO: 778.

In some aspects, the invention provides a method for producing a modified primary human T cell or population thereof, the method comprising: (a) contacting a primary human T cell or population thereof with a Cas protein or a nucleic acid sequence encoding the Cas protein and a first ribonucleic acid having a sequence selected from the group consisting of SEQ ID NOs: 3638-4046, thereby editing the cytotoxic T-lymphocyte-associated protein 4 (CTLA4) gene on chromosome 2 to reduce or eliminate CTLA4 receptor surface expression and/or activity in the cell or population thereof; and/or (b) contacting a primary human T cell or population thereof with the Cas protein or the nucleic acid sequence encoding the Cas protein and a second ribonucleic acid having a sequence selected from the group consisting of SEQ ID NOs: 8946-9101, thereby editing the programmed cell death 1 (PD1) gene on chromosome 2 to reduce or eliminate PD1 receptor surface expression and/or activity in the cell or population thereof.

In certain aspects, a subsequent alteration to the target TRCB polynucleotide sequence in the cell results in a second cleavage of the target TRCB polynucleotide sequence, thereby editing the target TRCB polynucleotide sequence to delete a fourth contiguous stretch of genomic DNA.

In some embodiments, the method for producing a modified primary human T cell or population thereof further comprises: (c)(i) editing the gene encoding the T cell receptor (TCR) alpha chain locus on chromosome 14 in the cell or population thereof, and/or (c)(ii) editing the gene encoding the TCR beta chain locus on chromosome 7 in the cell or population thereof, thereby reducing or eliminating TCR surface expression and/or activity in the cell or population thereof. In some embodiments, the editing step in (c)(i) comprises contacting the cell or population thereof with the Cas protein or the nucleic acid sequence encoding the Cas protein and a third ribonucleic acid having a sequence selected from the group consisting of SEQ ID NOs: 9751-9797, and/or the editing step in (c)(ii) comprises contacting the cell or population thereof with the Cas protein or the nucleic acid sequence encoding the Cas protein and a fourth ribonucleic acid having a sequence selected from the group consisting of SEQ ID NOs: 10533-10573.

In some embodiments, the method for producing a modified primary human T cell or population thereof further comprises: (d) editing the β2-microglobulin (B2M) gene on chromosome 15 in the cell or population thereof, thereby reducing or eliminating MHC Class I molecule surface expression and/or activity in the cell or population thereof. In some embodiments, the step of editing in (d) comprises contacting the cell with the Cas protein or the nucleic acid sequence encoding the Cas protein and a fifth ribonucleic acid having a sequence selected from the group consisting of SEQ ID NOs: 13258-13719.

In some embodiments, the editing of a gene (e.g., CTLA4, PD1, TCRA, TCRB, and/or B2M), as described herein, results in a cleavage of the polynucleotide sequence of the gene. In certain aspects, a subsequent application of the method for producing a modified primary human T cell causes a second cleavage of the polynucleotide sequence of that gene, thereby deleting a continguous stretch of genomic DNA.

In some embodiments, the methods for producing a modified primary human T cell or population thereof further comprise: causing the cell or population thereof to express at least one chimeric antigen receptor that specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof.

In some embodiments, the methods for producing a modified primary human T cell or population thereof further comprise: causing the cell or population thereof to express at least one protein that modulates a biological effect of interest in an adjacent cell, tissue, or organ when the cell or population thereof is in proximity to the adjacent cell, tissue, or organ.

It should be appreciated that the CRISPR/Cas systems of the present invention can cleave target polynucleotide sequences in a variety of ways. In some embodiments, the target polynucleotide sequence is cleaved such that a double-strand break results. In some embodiments, the target polynucleotide sequence is cleaved such that a single-strand break results.

The methods of the present invention can be used to alter any target polynucleotide sequence in a cell, as long as the target polynucleotide sequence in the cell contains a suitable target motif that allows at least one ribonucleic acid of the CRISPR/Cas system to direct the Cas protein to and hybridize to the target motif. Those skilled in the art will appreciate that the target motif for targeting a particular polynucleotide depends on the CRISPR/Cas system being used, and the sequence of the polynucleotide to be targeted.

In some embodiments, the target motif is 17 to 23 bp in length. In some embodiments, the target motif is at least 20 bp in length. In some embodiments, the target motif is a 20-nucleotide DNA sequence. In some embodiments, the target motif is a 17 to 23-nucleotide DNA sequence and immediately precedes an NRG motif. In some aspects, the NRG motif is NGG or NAG. In some embodiments, the target motif is a 20-nucleotide DNA sequence and immediately precedes an NGG motif recognized by the Cas protein. In some embodiments, the target motif is a 20-nucleotide DNA sequence and immediately precedes an NAG motif recognized by the Cas protein. In some embodiments, the target motif is a 20-nucleotide DNA sequence beginning with G and immediately precedes an NGG motif recognized by the Cas protein. In some embodiments, the target motif is G(N)₁₉NGG. In some embodiments, the target motif is (N)₂₀NGG.

In some embodiments, the target motif is a 17 to 23-nucleotide DNA sequence and immediately precedes an NNGRRT motif. In some embodiments, the target motif is a 20 nucleotide DNA sequence and immediately precedes an NNGRRT motif. In some embodiments, the target motif is a 17 to 23-nucleotide DNA sequence and immediately precedes an NNNRRT motif. In some embodiments, the target motif is a 20 nucleotide DNA sequence and immediately precedes an NNNRRT motif. In some embodiments, the target motif is a 17 to 23-nucleotide DNA sequence and immediately precedes an NNAGAAW motif. In some embodiments, the target motif is a 20 nucleotide DNA sequence and immediately precedes an NNAGAAW motif. In some embodiments, the target motif is a 17 to 23-nucleotide DNA sequence and immediately precedes an NNNNGATT motif. In some embodiments, the target motif is a 20 nucleotide DNA sequence and immediately precedes an NNNNGATT motif. In some embodiments, the target motif is a 17 to 23-nucleotide DNA sequence and immediately precedes an NAAAAC motif. In some embodiments, the target motif is a 20 nucleotide DNA sequence and immediately precedes an NAAAAC motif. In some embodiments, the target motif is a 17 to 23-nucleotide DNA sequence having a 5' T-rich region (e.g., TTTN motif). In some embodiments, the target motif is a 20 nucleotide DNA sequence having a 5' T-rich region (e.g., TTTN motif).

In some embodiments, the target motif is a 17 to 23-nucleotide DNA sequence and immediately precedes an NRG motif (e.g., NGG or NAG) recognized by a *S. pyogenes* Cas9 protein. In some embodiments, the target motif is a 17 to 23-nucleotide DNA sequence and immediately precedes an NNGRRT motif recognized by a *S*. *aureus* Cas9 protein. In some embodiments, the target motif is a 17 to 23-nucleotide DNA sequence and immediately precedes an NNNRRT motif recognized by a *S*. *aureus* Cas9 protein. In some embodiments, the target motif is a 17 to 23-nucleotide DNA sequence and immediately precedes an NNAGAAW motif recognized by a *S*. *thermophilus* Cas9 protein. In some embodiments, the target motif is a 17 to 23-nucleotide DNA sequence and immediately precedes an NNNNGATT motif recognized by *N. meningitides* Cas9 protein. In some embodiments, the target motif is a 17 to 23-nucleotide DNA sequence and immediately precedes an NAAAAC motif recognized by *T. denticola* Cas9 protein. In some embodiments, the target motif is a 17 to 23-nucleotide DNA sequence having a 5' T-rich region (e.g., TTTN motif) recognized by *Acidaminococcus* or *Lachnospiraceae* Cpfl protein.

The target motifs of the present invention can be selected to minimize off-target effects of the CRISPR/Cas systems of the present invention. In some embodiments, the target motif is selected such that it contains at least two mismatches when compared with all other genomic nucleotide sequences in the cell. In some embodiments, the target motif is selected such that it contains at least one mismatch when compared with all other genomic nucleotide sequences in the cell. Those skilled in the art will appreciate that a variety of techniques can be used to select suitable target motifs for minimizing off-target effects (e.g., bioinformatics analyses).

In some embodiments, the target motif comprises a G(N)₁₉NGG or (N)₂₀NGG DNA sequence in the CTLA gene. In some embodiments, the target motif comprises a G(N)₁₉NGG or (N)₂₀NGG DNA sequence in the PD1 gene. In some embodiments, the target motif comprises a G(N)₁₉NGG or (N)₂₀NGG DNA sequence in the TCRA gene. In some embodiments, the target motif comprises a G(N)₁₉NGG or (N)₂₀NGG DNA sequence in the TCRB gene. In some embodiments, the target motif comprises a G(N)₁₉NGG or (N)₂₀NGG DNA sequence in the B2M gene.

In some embodiments, the target motif comprises a G(N)₁₉NGG or (N)₂₀NGG DNA sequence in SEQ ID NO: 782. In some embodiments, the target motif comprises a G(N)₁₉NGG or (N)₂₀NGG DNA sequence in SEQ ID NO: 783. In some embodiments, the target motif comprises a G(N)₁₉NGG or (N)₂₀NGG DNA sequence in SEQ ID NO: 784. In some embodiments, the target motif comprises a G(N)₁₉NGG or (N)₂₀NGG DNA sequence in SEQ ID NO: 785. In some embodiments, the target motif comprises a G(N)₁₉NGG or (N)₂₀NGG DNA sequence in SEQ ID NO: 786. In some embodiments, the target motif comprises a G(N)₁₉NGG or (N)₂₀NGG DNA sequence in SEQ ID NO: 787. In some embodiments, the target motif comprises a G(N)₁₉NGG or (N)₂₀NGG DNA sequence in SEQ ID NO: 788. In some embodiments, the target motif comprises a G(N)₁₉NGG or (N)₂₀NGG DNA sequence in SEQ ID NO: 789. In some embodiments, the target motif comprises a G(N)₁₉NGG or (N)₂₀NGG DNA sequence in SEQ ID NO: 790. In some embodiments, the target motif comprises a G(N)₁₉NGG or (N)₂₀NGG DNA sequence in SEQ ID NO: 791. In some embodiments, the target motif comprises a G(N)₁₉NGG or (N)₂₀NGG DNA sequence in SEQ ID NO: 792.

In some embodiments, the target motif or at least a portion of the target motif comprises a DNA sequence selected from the group consisting of SEQ ID NOs: 1-195 and 797-4046. In some embodiments, the target motif or at least a portion of the target motif comprises a DNA sequence selected from the group consisting of SEQ ID NOs: 196-531 and 4047-9101. In some embodiments, the target motif or at least a portion of the target motif comprises a DNA sequence selected from the group consisting of SEQ ID NOs: 532-609 and 9102-9797. In some embodiments, the target motif or at least a portion of the target motif comprises a DNA sequence selected from the group consisting of SEQ ID NOs: 610-765 and 9798-10573. In some embodiments, the target motif or at least a portion of the target motif comprises a DNA sequence selected from the group consisting of SEQ ID NOs: 766-780 and 10574-13719.

In some embodiments, the target motif comprises a DNA sequence comprising at least two nucleotide mismatches compared to a G(N)₁₉NGG or (N)₂₀NGG DNA sequence in the CTLA gene. In some embodiments, the target motif comprises a DNA sequence comprising at least two nucleotide mismatches compared to a G(N)₁₉NGG or (N)₂₀NGG DNA sequence in the PD1 gene. In some embodiments, the target motif comprises a DNA sequence comprising at least two nucleotide mismatches compared to a G(N)₁₉NGG or (N)₂₀NGG DNA sequence in the TCRA gene. In some embodiments, the target motif comprises a DNA sequence comprising at least two nucleotide mismatches compared to a G(N)₁₉NGG or (N)₂₀NGG DNA sequence in the TCRB gene. In some embodiments, the target motif comprises a DNA sequence comprising at least two nucleotide mismatches compared to a G(N)₁₉NGG or (N)₂₀NGG DNA sequence in the B2M gene.

In some embodiments, the target motif or at least a portion of the target motif comprises a DNA sequence comprising at least two nucleotide mismatches compared to a G(N)₁₉NGG or (N)₂₀NGG DNA sequence in a DNA sequence selected from the group consisting of SEQ ID NOs: 1-195 and 797-2602. In some embodiments, the target motif or at least a portion of the target motif comprises a DNA sequence comprising at least two nucleotide mismatches compared to a G(N)₁₉NGG or (N)₂₀NGG DNA sequence in a DNA sequence selected from the group consisting of SEQ ID NOs: 196-531 and 4047-8128. In some embodiments, the target motif or at least a portion of the target motif comprises a DNA sequence comprising at least two nucleotide mismatches compared to a G(N)₁₉NGG or (N)₂₀NGG DNA sequence in a DNA sequence selected from the group consisting of SEQ ID NOs: 532-609 and 9102-9545. In some embodiments, the target motif or at least a portion of the target motif comprises a DNA sequence comprising at least two nucleotide mismatches compared to a G(N)₁₉NGG or (N)₂₀NGG DNA sequence in a DNA sequence selected from the group consisting of SEQ ID NOs: 610-765 and 9798-10321. In some embodiments, the target motif or at least a portion of the target motif comprises a DNA sequence comprising at least two nucleotide mismatches compared to a G(N)₁₉NGG or (N)₂₀NGG DNA sequence in a DNA sequence selected from the group consisting of SEQ ID NOs: 766-780 and 10574-12300.

In some embodiments, the CRISPR/Cas systems of the present invention utilize homology-directed repair to correct target polynucleotide sequences. In some embodiments, subsequent to cleavage of the target polynucleotide sequence, homology-directed repair occurs. In some embodiments, homology-directed repair is performed using an exogenously introduced DNA repair template. The exogenously introduced DNA repair template can be single-stranded or double-stranded. The DNA repair template can be of any length. Those skilled in the art will appreciate that the length of any particular DNA repair template will depend on the target polynucleotide sequence that is to be corrected. The DNA repair template can be designed to repair or replace any target polynucleotide sequence, particularly target polynucleotide sequences comprising disease associated polymorphisms (e.g., SNPs). For example, homology-directed repair of a mutant allele comprising such SNPs can be achieved with a CRISPR/Cas system by selecting two target motifs which flank the mutant allele, and an designing a DNA repair template to match the wild-type allele.

In some embodiments, a CRISPR/Cas system of the present invention includes a Cas protein or a nucleic acid sequence encoding the Cas protein and at least one to two ribonucleic acids (e.g., gRNAs) that are capable of directing the Cas protein to and hybridizing to a target motif of a target polynucleotide sequence. In some embodiments, a CRISPR/Cas system of the present invention includes a Cas protein or a nucleic acid sequence encoding the Cas protein and at least one pair of ribonucleic acids (e.g., gRNAs) that are capable of directing the Cas protein to and hybridizing to a target motif of a target polynucleotide sequence. As used herein, "protein" and "polypeptide" are used interchangeably to refer to a series of amino acid residues joined by peptide bonds (i.e., a polymer of amino acids) and include modified amino acids (e.g., phosphorylated, glycated, glycosolated, etc.) and amino acid analogs. Exemplary polypeptides or proteins include gene products, naturally occurring proteins, homologs, paralogs, fragments and other equivalents, variants, and analogs of the above.

In some embodiments, a Cas protein comprises one or more amino acid substitutions or modifications. In some embodiments, the one or more amino acid substitutions comprises a conservative amino acid substitution. In some instances, substitutions and/or modifications can prevent or reduce proteolytic degradation and/or extend the half-life of the polypeptide in a cell. In some embodiments, the Cas protein can comprise a peptide bond replacement (e.g., urea, thiourea, carbamate, sulfonyl urea, etc.). In some embodiments, the Cas protein can comprise a naturally occurring amino acid. In some embodiments, the Cas protein can comprise an alternative amino acid (e.g., D-amino acids, beta-amino acids, homocysteine, phosphoserine, etc.). In some embodiments, a Cas protein can comprise a modification to include a moiety (e.g., PEGylation, glycosylation, lipidation, acetylation, end-capping, etc.).

In some embodiments, a Cas protein comprises a core Cas protein. Exemplary Cas core proteins include, but are not limited to Cas1, Cas2, Cas3, Cas4, Cas5, Cas6, Cas7, Cas8 and Cas9. In some embodiments, a Cas protein comprises a Cas protein of an E. coli subtype (also known as CASS2). Exemplary Cas proteins of the E. Coli subtype include, but are not limited to Cse1, Cse2, Cse3, Cse4, and Cas5e. In some embodiments, a Cas protein comprises a Cas protein of the Ypest subtype (also known as CASS3). Exemplary Cas proteins of the Ypest subtype include, but are not limited to Csy1, Csy2, Csy3, and Csy4. In some embodiments, a Cas protein comprises a Cas protein of the Nmeni subtype (also known as CASS4). Exemplary Cas proteins of the Nmeni subtype include, but are not limited to Csn1 and Csn2. In some embodiments, a Cas protein comprises a Cas protein of the Dvulg subtype (also known as CASS1). Exemplary Cas proteins of the Dvulg subtype include Csd1, Csd2, and Cas5d. In some embodiments, a Cas protein comprises a Cas protein of the Tneap subtype (also known as CASS7). Exemplary Cas proteins of the Tneap subtype include, but are not limited to, Cst1, Cst2, Cas5t. In some embodiments, a Cas protein comprises a Cas protein of the Hmari subtype. Exemplary Cas proteins of the Hmari subtype include, but are not limited to Csh1, Csh2, and Cas5h. In some embodiments, a Cas protein comprises a Cas protein of the Apern subtype (also known as CASS5). Exemplary Cas proteins of the Apern subtype include, but are not limited to Csa1, Csa2, Csa3, Csa4, Csa5, and Cas5a. In some embodiments, a Cas protein comprises a Cas protein of the Mtube subtype (also known as CASS6). Exemplary Cas proteins of the Mtube subtype include, but are not limited to Csm1, Csm2, Csm3, Csm4, and Csm5. In some embodiments, a Cas protein comprises a RAMP module Cas protein. Exemplary RAMP module Cas proteins include, but are not limited to, Cmr1, Cmr2, Cmr3, Cmr4, Cmr5, and Cmr6.

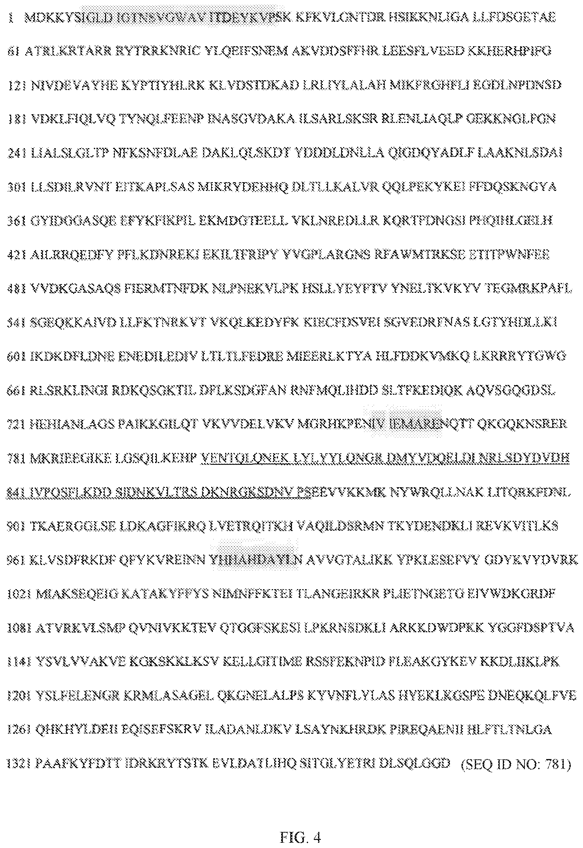

In some embodiments, the Cas protein is a *Streptococcus pyogenes* Cas9 protein or a functional portion thereof. In some embodiments, the Cas protein is a *Staphylococcus aureus* Cas9 protein or a functional portion thereof. In some embodiments, the Cas protein is a *Streptococcus thermophilus* Cas9 protein or a functional portion thereof. In some embodiments, the Cas protein is a *Neisseria meningitides* Cas9 protein or a functional portion thereof. In some embodiments, the Cas protein is a *Treponema denticola* Cas9 protein or a functional portion thereof. In some embodiments, the Cas protein is Cas9 protein from any bacterial species or functional portion thereof. Cas9 protein is a member of the type II CRISPR systems which typically include a trans-coded small RNA (tracrRNA), endogenous ribonuclease 3 (rnc) and a Cas protein. Cas 9 protein (also known as CRISPR-associated endonuclease Cas9/Csn1) is a polypeptide comprising 1368 amino acids. An exemplary amino acid sequence of a Cas9 protein (SEQ ID NO: 781) is shown in FIG. 4. Cas 9 contains 2 enconuclease domains, including an RuvC-like domain (residues 7-22, 759-766 and 982-989) which cleaves target DNA that is noncomplementary to crRNA, and an HNH nuclease domain (residues 810-872) which cleave target DNA complementary to crRNA. In FIG. 4, the RuvC-like domain is highlighted in yellow and the HNH nuclease domain is underlined.

In some embodiments, the Cas protein is Cpfl protein or a functional portion thereof. In some embodiments, the Cas protein is Cpfl from any bacterial species or functional portion thereof. In some aspects, Cpfl is a *Francisella novicida U112* protein or a functional portion thereof. In some aspects, Cpfl is a *Acidaminococcus sp. BY3L6* protein or a functional portion thereof. In some aspects, Cpfl is a *Lachnospiraceae bacterium ND2006* protein or a function portion thereof. Cpfl protein is a member of the type V CRISPR systems. Cpfl protein is a polypeptide comprising about 1300 amino acids. Cpfl contains a RuvC-like endonuclease domain. Cpfl cleaves target DNA in a staggered pattern using a single ribonuclease domain. The staggered DNA double- stranded break results in a 4 or 5-nt 5' overhang.

As used herein, "functional portion" refers to a portion of a peptide which retains its ability to complex with at least one ribonucleic acid (e.g., guide RNA (gRNA)) and cleave a target polynucleotide sequence. In some embodiments, the functional portion comprises a combination of operably linked Cas9 protein functional domains selected from the group consisting of a DNA binding domain, at least one RNA binding domain, a helicase domain, and an endonuclease domain. In some embodiments, the functional portion comprises a combination of operably linked Cpfl protein functional domains selected from the group consisting of a DNA binding domain, at least one RNA binding domain, a helicase domain, and an endonuclease domain. In some embodiments, the functional domains form a complex. In some embodiments, a functional portion of the Cas9 protein comprises a functional portion of a RuvC-like domain. In some embodiments, a functional portion of the Cas9 protein comprises a functional portion of the HNH nuclease domain. In some embodiments, a functional portion of the Cpfl protein comprises a functional portion of a RuvC-like domain.

It should be appreciated that the present invention contemplates various of ways of contacting a target polynucleotide sequence with a Cas protein (e.g., Cas9). In some embodiments, exogenous Cas protein can be introduced into the cell in polypeptide form. In certain embodiments, Cas proteins can be conjugated to or fused to a cell-penetrating polypeptide or cell-penetrating peptide. As used herein, "cell-penetrating polypeptide" and "cell-penetrating peptide" refers to a polypeptide or peptide, respectively, which facilitates the uptake of molecule into a cell. The cell-penetrating polypeptides can contain a detectable label.

In certain embodiments, Cas proteins can be conjugated to or fused to a charged protein (e.g., that carries a positive, negative or overall neutral electric charge). Such linkage may be covalent. In some embodiments, the Cas protein can be fused to a superpositively charged GFP to significantly increase the ability of the Cas protein to penetrate a cell (Cronican et al. ACS Chem Biol.2010;5(8):747-52). In certain embodiments, the Cas protein can be fused to a protein transduction domain (PTD) to facilitate its entry into a cell. Exemplary PTDs include Tat, oligoarginine, and penetratin. In some embodiments, the Cas9 protein comprises a Cas9 polypeptide fused to a cell-penetrating peptide. In some embodiments, the Cas9 protein comprises a Cas9 polypeptide fused to a PTD. In some embodiments, the Cas9 protein comprises a Cas9 polypeptide fused to a tat domain. In some embodiments, the Cas9 protein comprises a Cas9 polypeptide fused to an oligoarginine domain. In some embodiments, the Cas9 protein comprises a Cas9 polypeptide fused to a penetratin domain. In some embodiments, the Cas9 protein comprises a Cas9 polypeptide fused to a superpositively charged GFP. In some embodiments, the Cpfl protein comprises a Cpfl polypeptide fused to a cell-penetrating peptide. In some embodiments, the Cpfl protein comprises a Cpfl polypeptide fused to a PTD. In some embodiments, the Cpfl protein comprises a Cpfl polypeptide fused to a tat domain. In some embodiments, the Cpfl protein comprises a Cpfl polypeptide fused to an oligoarginine domain. In some embodiments, the Cpfl protein comprises a Cpfl polypeptide fused to a penetratin domain. In some embodiments, the Cpfl protein comprises a Cpfl polypeptide fused to a superpositively charged GFP.

In some embodiments, the Cas protein can be introduced into a cell containing the target polynucleotide sequence in the form of a nucleic acid encoding the Cas protein (e.g., Cas9 or Cpfl). The process of introducing the nucleic acids into cells can be achieved by any suitable technique. Suitable techniques include calcium phosphate or lipid-mediated transfection, electroporation, and transduction or infection using a viral vector. In some embodiments, the nucleic acid comprises DNA. In some embodiments, the nucleic acid comprises a modified DNA, as described herein. In some embodiments, the nucleic acid comprises mRNA. In some embodiments, the nucleic acid comprises a modified mRNA, as described herein (e.g., a synthetic, modified mRNA).

In some embodiments, nucleic acids encoding Cas protein and nucleic acids encoding the at least one to two ribonucleic acids are introduced into a cell via viral transduction (e.g., lentiviral transduction).

In some embodiments, the Cas protein is complexed with one to two ribonucleic acids. In some embodiments, the Cas protein is complexed with two ribonucleic acids. In some embodiments, the Cas protein is complexed with one ribonucleic acid. In some embodiments, the Cas protein is encoded by a modified nucleic acid, as described herein (e.g., a synthetic, modified mRNA).

The methods of the present invention contemplate the use of any ribonucleic acid that is capable of directing a Cas protein to and hybridizing to a target motif of a target polynucleotide sequence. In some embodiments, at least one of the ribonucleic acids comprises tracrRNA. In some embodiments, at least one of the ribonucleic acids comprises CRISPR RNA (crRNA). In some embodiments, a single ribonucleic acid comprises a guide RNA that directs the Cas protein to and hybridizes to a target motif of the target polynucleotide sequence in a cell. In some embodiments, at least one of the ribonucleic acids comprises a guide RNA that directs the Cas protein to and hybridizes to a target motif of the target polynucleotide sequence in a cell. In some embodiments, both of the one to two ribonucleic acids comprise a guide RNA that directs the Cas protein to and hybridizes to a target motif of the target polynucleotide sequence in a cell. The ribonucleic acids of the present invention can be selected to hybridize to a variety of different target motifs, depending on the particular CRISPR/Cas system employed, and the sequence of the target polynucleotide, as will be appreciated by those skilled in the art. The one to two ribonucleic acids can also be selected to minimize hybridization with nucleic acid sequences other than the target polynucleotide sequence. In some embodiments, the one to two ribonucleic acids hybridize to a target motif that contains at least two mismatches when compared with all other genomic nucleotide sequences in the cell. In some embodiments, the one to two ribonucleic acids hybridize to a target motif that contains at least one mismatch when compared with all other genomic nucleotide sequences in the cell. In some embodiments, the one to two ribonucleic acids are designed to hybridize to a target motif immediately adjacent to a deoxyribonucleic acid motif recognized by the Cas protein. In some embodiments, each of the one to two ribonucleic acids are designed to hybridize to target motifs immediately adjacent to deoxyribonucleic acid motifs recognized by the Cas protein which flank a mutant allele located between the target motifs.

In some embodiments, at least one of the one to two ribonucleic acids comprises a sequence selected from the group consisting of the ribonucleic acid sequences of SEQ ID NOs: 1-195 and 797-3637. In some embodiments, at least one of the one to two ribonucleic acids comprises a sequence selected from the group consisting of the ribonucleic acid sequences of SEQ ID NOs: 196-531 and 4047-8945. In some embodiments, at least one of the one to two ribonucleic acids comprises a sequence selected from the group consisting of the ribonucleic acid sequences of SEQ ID NOs: 532-609 and 9102-9750. In some embodiments, at least one of the one to two ribonucleic acids comprises a sequence selected from the group consisting of the ribonucleic acid sequences of SEQ ID NOs: 610-765 and 9798-10532. In some embodiments, at least one of the one to two ribonucleic acids comprises a sequence selected from the group consisting of the ribonucleic acid sequences of SEQ ID NOs: 766-780 and 10574-13257.

In some embodiments, at least one ribonucleic acid comprises a sequence selected from the group consisting of the ribonucleic acid sequences of SEQ ID NOs: 3638-4046. In some embodiments, at least one ribonucleic acid comprises a sequence selected from the group consisting of the ribonucleic acid sequences of SEQ ID NOs: 8946-9101. In some embodiments, at least one ribonucleic acid comprises a sequence selected from the group consisting of the ribonucleic acid sequences of SEQ ID NOs: 9751-9797. In some embodiments, at least one ribonucleic acid comprises a sequence selected from the group consisting of the ribonucleic acid sequences of SEQ ID NOs: 10533-10573. In some embodiments, at least one ribonucleic acid comprises a sequence selected from the group consisting of the ribonucleic acid sequences of SEQ ID NOs: 13258-13719.

In some embodiments, at least one of the one to two ribonucleic acids comprises a sequence with a single nucleotide mismatch to a sequence selected from the group consisting of the ribonucleic acid sequences of SEQ ID NOs: 1-195 and 797-3637. In some embodiments, at least one of the one to two ribonucleic acids comprises a sequence with a single nucleotide mismatch to a sequence selected from the group consisting of the ribonucleic acid sequences of SEQ ID NOs: 196-531 and 4047-8945. In some embodiments, at least one of the one to two ribonucleic acids comprises a sequence with a single nucleotide mismatch to a sequence selected from the group consisting of the ribonucleic acid sequences of SEQ ID NOs: 532-609 and 9102-9750. In some embodiments, at least one of the one to two ribonucleic acids comprises a sequence with a single nucleotide mismatch to a sequence selected from the group consisting of the ribonucleic acid sequences of SEQ ID NOs: 610-765 and 9798-10532. In some embodiments, at least one of the one to two ribonucleic acids comprises a sequence with a single nucleotide mismatch to a sequence selected from the group consisting of the ribonucleic acid sequences of SEQ ID NOs: 766-780 and 10574-13257.

In some embodiments, at least one ribonucleic acid comprises a sequence with a single nucleotide mismatch to a sequence selected from the group consisting of the ribonucleic acid sequences of SEQ ID NOs: 3638-4046. In some embodiments, at least one ribonucleic acid comprises a sequence with a single nucleotide mismatch to a sequence selected from the group consisting of the ribonucleic acid sequences of SEQ ID NOs: 8946-9101. In some embodiments, at least one ribonucleic acid comprises a sequence with a single nucleotide mismatch to a sequence selected from the group consisting of the ribonucleic acid sequences of SEQ ID NOs: 9751-9797. In some embodiments, at least one ribonucleic acid comprises a sequence with a single nucleotide mismatch to a sequence selected from the group consisting of the ribonucleic acid sequences of SEQ ID NOs: 10533-10573. In some embodiments, at least one ribonucleic acid comprises a sequence with a single nucleotide mismatch to a sequence selected from the group consisting of the ribonucleic acid sequences of SEQ ID NOs: 13258-13719.

In some embodiments, each of the one to two ribonucleic acids comprises guide RNAs that directs the Cas protein to and hybridizes to a target motif of the target polynucleotide sequence in a cell. In some embodiments, one or two ribonucleic acids (e.g., guide RNAs) are complementary to and/or hybridize to sequences on the same strand of a target polynucleotide sequence. In some embodiments, one or two ribonucleic acids (e.g., guide RNAs) are complementary to and/or hybridize to sequences on the opposite strands of a target polynucleotide sequence. In some embodiments, the one or two ribonucleic acids (e.g., guide RNAs) are not complementary to and/or do not hybridize to sequences on the opposite strands of a target polynucleotide sequence. In some embodiments, the one or two ribonucleic acids (e.g., guide RNAs) are complementary to and/or hybridize to overlapping target motifs of a target polynucleotide sequence. In some embodiments, the one or two ribonucleic acids (e.g., guide RNAs) are complementary to and/or hybridize to offset target motifs of a target polynucleotide sequence.

The present invention also contemplates multiplex genomic editing. Those skilled in the art will appreciate that the description above with respect to genomic editing of a single gene is equally applicable to the multiplex genomic editing embodiments described below.

As used herein, the terms "administering," "introducing" and "transplanting" are used interchangeably in the context of the placement of cells, e.g. cells described herein comprising a target polynucleotide sequence altered according to the methods of the invention into a subject, by a method or route which results in at least partial localization of the introduced cells at a desired site. The cells can be implanted directly to the desired site, or alternatively be administered by any appropriate route which results in delivery to a desired location in the subject where at least a portion of the implanted cells or components of the cells remain viable. The period of viability of the cells after administration to a subject can be as short as a few hours, e. g. twenty-four hours, to a few days, to as long as several years. In some instances, the cells can also be administered a location other than the desired site, such as in the liver or subcutaneously, for example, in a capsule to maintain the implanted cells at the implant location and avoid migration of the implanted cells.

For *ex vivo* methods, cells can include autologous cells, i.e., a cell or cells taken from a subject who is in need of altering a target polynucleotide sequence in the cell or cells (i.e., the donor and recipient are the same individual). Autologous cells have the advantage of avoiding any immunologically-based rejection of the cells. Alternatively, the cells can be heterologous, e.g., taken from a donor. The second subject can be of the same or different species. Typically, when the cells come from a donor, they will be from a donor who is sufficiently immunologically compatible with the recipient, i.e., will not be subject to transplant rejection, to lessen or remove the need for immunosuppression. In some embodiments, the cells are taken from a xenogeneic source, i.e., a non-human mammal that has been genetically engineered to be sufficiently immunologically compatible with the recipient, or the recipient's species. Methods for determining immunological compatibility are known in the art, and include tissue typing to assess donor-recipient compatibility for HLA and ABO determinants. See, e.g., Transplantation Immunology, Bach and Auchincloss, Eds. (Wiley, John & Sons, Incorporated 1994).

Any suitable cell culture media can be used for *ex vivo* methods of the invention.

The terms "subject" and "individual" are used interchangeably herein, and refer to an animal, for example, a human from whom cells can be obtained and/or to whom treatment, including prophylactic treatment, with the cells as described herein, is provided. For treatment of those infections, conditions or disease states which are specific for a specific animal such as a human subject, the term subject refers to that specific animal. The "non-human animals" and "non-human mammals" as used interchangeably herein, includes mammals such as rats, mice, rabbits, sheep, cats, dogs, cows, pigs, and non-human primates. The term "subject" also encompasses any vertebrate including but not limited to mammals, reptiles, amphibians and fish. However, advantageously, the subject is a mammal such as a human, or other mammals such as a domesticated mammal, e.g. dog, cat, horse, and the like, or production mammal, e.g. cow, sheep, pig, and the like.

In some embodiments, the alteration results in reduced expression of the target polynucleotide sequences. In some embodiments, the alteration results in a knock out of the target polynucleotide sequences. In some embodiments, the alteration results in correction of the target polynucleotide sequences from undesired sequences to desired sequences. In some embodiments, each alteration is a homozygous alteration. In some embodiments, the efficiency of alteration at each loci is from about 5% to about 80%. In some embodiments, the efficiency of alteration at each loci is from about 10% to about 80%. In some embodiments, the efficiency of alteration at each loci is from about 30% to about 80%. In some embodiments, the efficiency of alteration at each loci is from about 50% to about 80%. In some embodiments, the efficiency of alteration at each loci is from greater than or equal to about 80%. In some embodiments, the efficiency of alteration at each loci is from greater than or equal to about 85%. In some embodiments, the efficiency of alteration at each loci is greater than or equal to about 90%, 91%, 92%, 93%, 94%, 95%, 96%, 97%, 98%, or 99%. In some embodiments, the efficiency of alteration at each loci is about 100%.

In some embodiments, each target polynucleotide sequence is cleaved such that a double-strand break results. In some embodiments, each target polynucleotide sequence is cleaved such that a single-strand break results.

In some embodiments, the target polynucleotide sequences comprise multiple different portions of CTLA4. In some embodiments, the target polynucleotide sequences comprise multiple different portions of PD1. In some embodiments, the target polynucleotide sequences comprise multiple different portions of TCRA. In some embodiments, the target polynucleotide sequences comprise multiple different portions of TCRB. In some embodiments, the target polynucleotide sequences comprise multiple different portions of B2M.

In some embodiments, each target motif is a 17 to 23 nucleotide DNA sequence. In some embodiments, each target motif is a 20-nucleotide DNA sequence. In some embodiments, each target motif is a 20-nucleotide DNA sequence with a 5' T-rich region. In some embodiments, each target motif is a 20-nucleotide DNA sequence beginning with G and immediately precedes an NGG motif recognized by the Cas protein. In some embodiments, each target motif is a 20-nucleotide DNA sequence and immediately precedes an NGG motif recognized by the Cas protein. In some embodiments, each target motif is G(N)₁₉NGG. In some embodiments, each target motif is (N)₂₀NGG. In some embodiments, each target motif is selected such that it contains at least two mismatches when compared with all other genomic nucleotide sequences in the cell. In some embodiments, each target motif is selected such that it contains at least two mismatches when compared with all other genomic nucleotide sequences in the cell.

In some embodiments, each target motif is a 17 to 23-nucleotide DNA sequence and immediately precedes an NNGRRT motif. In some embodiments, each target motif is a 20 nucleotide DNA sequence and immediately precedes an NNGRRT motif. In some embodiments, each target motif is a 17 to 23-nucleotide DNA sequence and immediately precedes an NNNRRT motif. In some embodiments, each target motif is a 20 nucleotide DNA sequence and immediately precedes an NNNRRT motif. In some embodiments, each target motif is a 17 to 23-nucleotide DNA sequence and immediately precedes an NNAGAAW motif. In some embodiments, each target motif is a 20 nucleotide DNA sequence and immediately precedes an NNAGAAW motif. In some embodiments, each target motif is a 17 to 23-nucleotide DNA sequence and immediately precedes an NNNNGATT motif. In some embodiments, each target motif is a 20 nucleotide DNA sequence and immediately precedes an NNNNGATT motif. In some embodiments, each target motif is a 17 to 23-nucleotide DNA sequence and immediately precedes an NAAAAC motif. In some embodiments, each target motif is a 20 nucleotide DNA sequence and immediately precedes an NAAAAC motif. In some embodiments, each target motif is a 17 to 23-nucleotide DNA sequence having a 5' T-rich region (e.g., TTTN motif). In some embodiments, each target motif is a 20-nucleotide DNA sequence having a 5' T-rich region (e.g. TTTN motif).

In some embodiments, subsequent to cleavage of the target polynucleotide sequences, homology-directed repair occurs. In some embodiments, homology-directed repair is performed using an exogenously introduced DNA repair template. In some embodiments, exogenously introduced DNA repair template is single-stranded. In some embodiments, exogenously introduced DNA repair template is double-stranded.

In some embodiments, the Cas protein (e.g., Cas9 or Cpfl) is complexed with at least one ribonucleic acid. In some embodiments, the Cas protein (e.g., Cas9) is complexed with multiple ribonucleic acids. In some embodiments, the multiple ribonucleic acids are selected to minimize hybridization with nucleic acid sequences other than the target polynucleotide sequence (e.g., multiple alterations of a single target polynucleotide sequence). In some embodiments, the multiple ribonucleic acids are selected to minimize hybridization with nucleic acid sequences other than the target polynucleotide sequences (e.g., one or more alterations of multiple target polynucleotide sequences). In some embodiments, each of the multiple ribonucleic acids hybridize to target motifs that contain at least two mismatches when compared with all other genomic nucleotide sequences in the cell. In some embodiments, each of the multiple ribonucleic acids hybridize to target motifs that contain at least one mismatch when compared with all other genomic nucleotide sequences in the cell. In some embodiments, each of the multiple ribonucleic acids are designed to hybridize to target motifs immediately adjacent to deoxyribonucleic acid motifs recognized by the Cas protein. In some embodiments, each of the multiple ribonucleic acids are designed to hybridize to target motifs immediately adjacent to deoxyribonucleic acid motifs recognized by the Cas protein which flank mutant alleles located between the target motifs.

In some embodiments, the Cas protein (e.g., Cpfl) is complexed with a single ribonucleic acid. In some embodiments, the ribonucleic acid is selected to minimize hybridization with a nucleic acid sequence other than the target polynucleotide sequence (e.g., multiple alterations of a single target polynucleotide sequence). In some embodiments, the ribonucleic acid is selected to minimize hybridization with a nucleic acid sequence other than the target polynucleotide sequences (e.g., one or more alterations of multiple target polynucleotide sequences). In some embodiments, the ribonucleic acid hybridizes to target motifs that contain at least two mismatches when compared with all other genomic nucleotide sequences in the cell. In some embodiments, the ribonucleic acid hybridizes to target motifs that contain at least one mismatch when compared with all other genomic nucleotide sequences in the cell. In some embodiments, the ribonucleic acid is designed to hybridize to target motifs immediately adjacent to deoxyribonucleic acid motifs recognized by the Cas protein. In some embodiments, the ribonucleic acid is designed to hybridize to target motifs immediately adjacent to deoxyribonucleic acid motifs recognized by the Cas protein which flank mutant alleles located between the target motifs.

It should be appreciated that any of the nucleic acid encoding Cas protein or the ribonucleic acids (e.g., SEQ ID NOs: 1-780 and 797-13719) can be expressed from a plasmid. In some embodiments, any of the Cas protein or the ribonucleic acids are expressed using a promoter optimized for increased expression in stem cells (e.g., human stem and/or progenitor cells). In some embodiments, the promoter is selected from the group consisting of a Cytomegalovirus (CMV) early enhancer element and a chicken beta-actin promoter, a chicken beta-actin promoter, an elongation factor-1 alpha promoter, and a ubiquitin promoter.

In some embodiments, the methods of the present invention further comprise selecting cells that express the Cas protein. The present invention contemplates any suitable method for selecting cells. In some embodiments, selecting cells comprises FACS. In some embodiments, FACS is used to select cells which co-express Cas and a fluorescent protein selected from the group consisting of green fluorescent protein and red fluorescent protein.

### Methods of Treatment

The present disclosure contemplates treating and/or preventing a variety of disorders which are associated with expression of a target polynucleotide sequences.

The present disclosure also contemplates various methods of treatment using the modified primary human cells of the present invention, compositions comprising those cells, and compositions of the present invention (e.g., a chimeric nucleic acid). The terms "treat", "treating", "treatment", etc., as applied to an isolated cell, include subjecting the cell to any kind of process or condition or performing any kind of manipulation or procedure on the cell. As applied to a subject, the terms refer to administering a cell or population of cells in which a target polynucleotide sequence (e.g., CTLA4, PD1, TCRA, TCRB, B2M, etc.) has been altered *ex vivo* according to the methods described herein to an individual. The individual is usually ill or injured, or at increased risk of becoming ill relative to an average member of the population and in need of such attention, care, or management.

As used herein, the term "treating" and "treatment" refers to administering to a subject an effective amount of cells with target polynucleotide sequences altered *ex vivo* according to the methods described herein so that the subject has a reduction in at least one symptom of the disease or an improvement in the disease, for example, beneficial or desired clinical results. For purposes of this invention, beneficial or desired clinical results include, but are not limited to, alleviation of one or more symptoms, diminishment of extent of disease, stabilized (i.e., not worsening) state of disease, delay or slowing of disease progression, amelioration or palliation of the disease state, and remission (whether partial or total), whether detectable or undetectable. Treating can refer to prolonging survival as compared to expected survival if not receiving treatment. Thus, one of skill in the art realizes that a treatment may improve the disease condition, but may not be a complete cure for the disease. As used herein, the term "treatment" includes prophylaxis. Alternatively, treatment is "effective" if the progression of a disease is reduced or halted. "Treatment" can also mean prolonging survival as compared to expected survival if not receiving treatment. Those in need of treatment include those already diagnosed with a disorder associated with expression of a polynucleotide sequence, as well as those likely to develop such a disorder due to genetic susceptibility or other factors.

By "treatment," "prevention" or "amelioration" of a disease or disorder is meant delaying or preventing the onset of such a disease or disorder, reversing, alleviating, ameliorating, inhibiting, slowing down or stopping the progression, aggravation or deterioration the progression or severity of a condition associated with such a disease or disorder. In one embodiment, the symptoms of a disease or disorder are alleviated by at least 5%, at least 10%, at least 20%, at least 30%, at least 40%, or at least 50%.

It should be appreciated that the methods and compositions described herein can be used to treat or prevent disorders associated with increased expression of a target polynucleotide sequence, as well as decreased expression of a target polynucleotide sequence in a cell. Increased and decreased expression of a target polynucleotide sequence includes circumstances where the expression levels of the target polynucleotide sequence are increased or decreased, respectively, as well as circumstances in which the function and/or level of activity of an expression product of the target polynucleotide sequence increases or decreases, respectively, compared to normal expression and/or activity levels. Those skilled in the art will appreciate that treating or preventing a disorder associated with increased expression of a target polynucleotide sequence can be assessed by determining whether the levels and/or activity of the target polynucleotide sequence (or an expression product thereof) are decreased in a relevant cell after contacting a cell with a composition described herein. The skilled artisan will also appreciate that treating or preventing a disorder associated with decreased expression of a target polynucleotide sequence can be assessed by determining whether the levels and/or activity of the target polynucleotide sequence (or an expression product thereof) are increased in the relevant cell after contacting a cell with a composition described herein.

In some embodiments, the disorder is cancer. The term "cancer" as used herein is defined as a hyperproliferation of cells whose unique trait-loss of normal controls-results in unregulated growth, lack of differentiation, local tissue invasion, and metastasis. With respect to the inventive methods, the cancer can be any cancer, including any of acute lymphocytic cancer, acute myeloid leukemia, alveolar rhabdomyosarcoma, bladder cancer, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the oral cavity, cancer of the vulva, chronic lymphocytic leukemia, chronic myeloid cancer, colon cancer, esophageal cancer, cervical cancer, fibrosarcoma, gastrointestinal carcinoid tumor, Hodgkin lymphoma, hypopharynx cancer, kidney cancer, larynx cancer, leukemia, liquid tumors, liver cancer, lung cancer, lymphoma, malignant mesothelioma, mastocytoma, melanoma, multiple myeloma, nasopharynx cancer, non-Hodgkin lymphoma, ovarian cancer, pancreatic cancer, peritoneum, omentum, and mesentery cancer, pharynx cancer, prostate cancer, rectal cancer, renal cancer, skin cancer, small intestine cancer, soft tissue cancer, solid tumors, stomach cancer, testicular cancer, thyroid cancer, ureter cancer, and urinary bladder cancer. As used herein, the term "tumor" refers to an abnormal growth of cells or tissues of the malignant type, unless otherwise specifically indicated and does not include a benign type tissue.

In some embodiments, the disorder is a genetic disorder. In some embodiments, the disorder is a monogenic disorder. In some embodiments, the disorder is a multigenic disorder. In some embodiments, the disorder is a disorder associated with one or more SNPs. Exemplary disorders associated with one or more SNPs include a complex disease described in U.S. Patent No. 7,627,436, Alzheimer's disease as described in PCT International Application Publication No. WO/2009/112882, inflammatory diseases as described in U.S. Patent Application Publication No. 2011/0039918, polycystic ovary syndrome as described in U.S. Patent Application Publication No. 2012/0309642, cardiovascular disease as described in U.S. Patent No. 7,732,139, Huntington's disease as described in U.S. Patent Application Publication No. 2012/0136039, thromboembolic disease as described in European Patent Application Publication No. EP2535424, neurovascular diseases as described in PCT International Application Publication No. WO/2012/001613, psychosis as described in U.S. Patent Application Publication No. 2010/0292211, multiple sclerosis as described in U.S. Patent Application Publication No. 2011/0319288, schizophrenia, schizoaffective disorder, and bipolar disorder as described in PCT International Application Publication No. WO/2006/023719A2, bipolar disorder and other ailments as described in U.S. Patent Application Publication No. U.S. 2011/0104674, colorectal cancer as described in PCT International Application Publication No. WO/2006/104370A1, a disorder associated with a SNP adjacent to the AKT1 gene locus as described in U.S. Patent Application Publication No. U.S. 2006/0204969, an eating disorder as described in PCT International Application Publication No. WO/2003/012143A1, autoimmune disease as described in U.S. Patent Application Publication No. U.S. 2007/0269827, fibrostenosing disease in patients with Crohn's disease as described in U.S. Patent No. 7,790,370, and Parkinson's disease as described in U.S. Patent No. 8,187,811. Other disorders associated with one or more SNPs which can be treated or prevented according to the methods of the present invention will be apparent to the skilled artisan.

In some embodiments, the disorder is a chronic infectious disease. A "chronic infectious disease" is a disease caused by an infectious agent wherein the infection has persisted. Such a disease may include hepatitis (A, B, or C), herpes virus (e.g., VZV, HSV-1, HSV-6, HSV-II, CMV, and EBV), and HIV/AIDS. Non-viral examples may include chronic fungal diseases such Aspergillosis, Candidiasis, Coccidioidomycosis, and diseases associated with *Cryptococcus* and Histoplasmosis. None limiting examples of chronic bacterial infectious agents may be *Chlamydia pneumoniae, Listeria monocytogenes,* and *Mycobacterium tuberculosis.* In some embodiments, the disorder is human immunodeficiency virus (HIV) infection. In some embodiments, the disorder is acquired immunodeficiency syndrome (AIDS).

In some embodiments, the disorder is an autoimmune disorder. The term "autoimmune disease" refers to any disease or disorder in which the subject mounts a destructive immune response against its own tissues. Autoimmune disorders can affect almost every organ system in the subject (e.g., human), including, but not limited to, diseases of the nervous, gastrointestinal, and endocrine systems, as well as skin and other connective tissues, eyes, blood and blood vessels. Examples of autoimmune diseases include, but are not limited to Hashimoto's thyroiditis, Systemic lupus erythematosus, Sjogren's syndrome, Graves' disease, *Scleroderma,* Rheumatoid arthritis, Multiple sclerosis, Myasthenia gravis and Diabetes.

In some embodiments, the disorder is graft versus host disease (GVHD).

The methods of the present invention are capable of altering target polynucleotide sequences in a variety of different cells (e.g., altering an immunological checkpoint regulator gene, e.g., CTL4, PD1, etc. to reduce or eliminate T cell inhibition, altering the genes encoding the TCR alpha and beta chains to reduce or eliminate T cell autoreactivity, and/or altering B2M to ablate MHC class I surface expression, and optionally altering one or more additional target polynucleotide sequences associated with a disorder in which altering the target polynucleotide sequences would be beneficial, and/or optionally causing the cell to express a protein that modulates at least one biological process). In some embodiments, the methods of the present invention are used to alter target polynucleotide sequences in cells *ex vivo* for subsequent introduction into a subject.

In some embodiments, the cell or population thereof is a primary cell. In some embodiments, the cell or population thereof is a primary T cell (e.g., human). The T cell can be any T cell, including without limitation, cytotoxic T-cells (e.g., CD8+ cells), helper T-cells (e.g., CD4+ cells), memory T-cells, regulatory T-cells, tissue infiltrating lymphocytes (e.g., tumor infiltrating lymphocytes, e.g., TILs, CD3+ cells), and combinations thereof.

In some embodiments, the cell is a peripheral blood cell. In some embodiments, the cell is a stem cell or a pluripotent cell. In some embodiments, the cell is a hematopoietic stem cell. In some embodiments, the cell is a CD34+ cell. In some embodiments, the cell is a CD34+ mobilized peripheral blood cell. In some embodiments, the cell is a CD34+ cord blood cell. In some embodiments, the cell is a CD34+ bone marrow cell. In some embodiments, the cell is a CD34+CD38-Lineage-CD90+CD45RA- cell. In some embodiments, the cell is a CD4+ cell. In some embodiments, the cell is a CD4+ T cell. In some embodiments, the cell is a hepatocyte. In some embodiments, the cell is a human pluripotent cell. In some embodiments, the cell is a primary human cell. In some embodiments, the cell is a primary CD34+ cell. In some embodiments, the cell is a primary CD34+ hematopoietic progenitor cell (HPC). In some embodiments, the cell is a primary CD4+ cell. In some embodiments, the cell is a primary CD4+ T cell. In some embodiments, the cell is an autologous primary cell. In some embodiments, the cell is an autologous primary somatic cell. In some embodiments, the cell is an allogeneic primary cell. In some embodiments, the cell is an allogeneic primary somatic cell. In some embodiments, the cell is a nucleated cell. In some embodiments, the cell is a non-transformed cell. In some embodiments, the cell is a human choriocarcinoma cell. In some embodiments, the cell is a JEG-3 cell. In some embodiments, the cell is a monocyte cell. In some embodiments, the cell is a Thp-1 cell. In some embodiments, the cell is not a cancer cell. In some embodiments, the cell is not a tumor cell. In some embodiments, the cell is not a transformed cell.

The cell or population thereof can be obtained from any subject, e.g., a subject suffering from, being treated for, diagnosed with, at risk of developing, or suspected of having, a disorder selected from the group consisting of an autoimmune disorder, cancer, a chronic infectious disease, and graft versus host disease (GVHD).

The present invention also provides compositions comprising Cas proteins of the present invention or functional portions thereof, nucleic acids encoding the Cas proteins or functional portions thereof, and ribonucleic acid sequences which direct Cas proteins to and hybridize to target motifs of target polynucleotides in a cell.

In some aspects, disclosed herein are compositions comprising a nucleic acid sequence encoding a Cas 9 protein, and at least one ribonucleic acid sequence selected from the group consisting of SEQ ID NOs: 1-195 and 797-3637. In some aspects, disclosed herein are compositions comprising a nucleic acid sequence encoding a Cas 9 protein, a pair of ribonucleic acid sequences selected from the group consisting of SEQ ID NOs: 1-195 and 797-3637. In some embodiments, the first ribonucleic acid comprises a sequence with a single nucleotide mismatch to a sequence selected from the group consisting of SEQ ID NOs: 1-195 and 797-3637. In some embodiments, the first ribonucleic acid comprises a sequence with a two nucleotide mismatch to a sequence selected from the group consisting of SEQ ID NOs: 1-195 and 797-3637. In some embodiments, the second ribonucleic acid comprises a sequence with a single nucleotide mismatch to a sequence selected from the group consisting of SEQ ID NOs: 1-195 and 797-3637. In some embodiments, the second ribonucleic acid comprises a sequence with a two nucleotide mismatch to a sequence selected from the group consisting of SEQ ID NOs: 1-195 and 797-3637. In some embodiments, the pair of ribonucleic acids comprise sequences with at least one nucleotide mismatch or at least two nucleotide mismatches to a sequence selected from the group consisting of SEQ ID NOs: 1-195 and 797-3637.

In some embodiments, the pair of ribonucleic acid sequences comprises or consists of SEQ ID NO: 128 and SEQ ID NO: 72. In some embodiments, the pair of ribonucleic acids comprise sequences with at least one nucleotide mismatch or at least two nucleotide mismatches to SEQ ID NO: 128 and 72.

In some aspects, disclosed herein are compositions comprising a nucleic acid sequence encoding a Cas 9 protein, and at least one ribonucleic acid sequence selected from the group consisting of SEQ ID NOs: 196-531 and 4047-8945. In some aspects, disclosed herein are compositions comprising a nucleic acid sequence encoding a Cas 9 protein, a pair of ribonucleic acid sequences selected from the group consisting of SEQ ID NOs: 196-531 and 4047-8945. In some embodiments, the first ribonucleic acid comprises a sequence with a single nucleotide mismatch to a sequence selected from the group consisting of SEQ ID NOs: 196-531 and 4047-8945. In some embodiments, the first ribonucleic acid comprises a sequence with a two nucleotide mismatch to a sequence selected from the group consisting of SEQ ID NOs: 196-531 and 4047-8945. In some embodiments, the second ribonucleic acid comprises a sequence with a single nucleotide mismatch to a sequence selected from the group consisting of SEQ ID NOs: 196-531 and 4047-8945. In some embodiments, the second ribonucleic acid comprises a sequence with a two nucleotide mismatch to a sequence selected from the group consisting of SEQ ID NOs: 196-531 and 4047-8945. In some embodiments, the pair of ribonucleic acids comprise sequences with at least one nucleotide mismatch or at least two nucleotide mismatches to a sequence selected from the group consisting of SEQ ID NOs: 196-531 and 4047-8945.

In some embodiments, the pair of ribonucleic acid sequences comprises or consists of SEQ ID NO: 462 and SEQ ID NO: 421. In some embodiments, the pair of ribonucleic acids comprise sequences with at least one nucleotide mismatch or at least two nucleotide mismatches to SEQ ID NO: 462 and SEQ ID NO: 421.

In some aspects, disclosed herein are compositions comprising a nucleic acid sequence encoding a Cas 9 protein, and at least one ribonucleic acid sequence selected from the group consisting of SEQ ID NOs: 532-609 and 9102-9750. In some aspects, disclosed herein are compositions comprising a nucleic acid sequence encoding a Cas 9 protein, a pair of ribonucleic acid sequences selected from the group consisting of SEQ ID NOs: 532-609 and 9102-9750. In some embodiments, the first ribonucleic acid comprises a sequence with a single nucleotide mismatch to a sequence selected from the group consisting of SEQ ID NOs: 532-609 and 9102-9750. In some embodiments, the first ribonucleic acid comprises a sequence with a two nucleotide mismatch to a sequence selected from the group consisting of SEQ ID 532-609 and 9102-9750. In some embodiments, the second ribonucleic acid comprises a sequence with a single nucleotide mismatch to a sequence selected from the group consisting of SEQ ID NOs: 532-609 and 9102-9750. In some embodiments, the second ribonucleic acid comprises a sequence with a two nucleotide mismatch to a sequence selected from the group consisting of SEQ ID NOs: 532-609 and 9102-9750. In some embodiments, the pair of ribonucleic acids comprise sequences with at least one nucleotide mismatch or at least two nucleotide mismatches to a sequence selected from the group consisting of SEQ ID NOs: 532-609 and 9102-9750.

In some embodiments, the pair of ribonucleic acid sequences comprises or consists of SEQ ID NO: 550 and SEQ ID NO: 573. In some embodiments, the pair of ribonucleic acids comprise sequences with at least one nucleotide mismatch or at least two nucleotide mismatches to SEQ ID NO: 550 and SEQ ID NO: 573.

In some aspects, disclosed herein are compositions comprising a nucleic acid sequence encoding a Cas 9 protein, and at least one ribonucleic acid sequence selected from the group consisting of SEQ ID NOs: 610-765 and 9798-10532. In some aspects, disclosed herein are compositions comprising a nucleic acid sequence encoding a Cas 9 protein, a pair of ribonucleic acid sequences selected from the group consisting of SEQ ID NOs: 610-765 and 9798-10532. In some embodiments, the first ribonucleic acid comprises a sequence with a single nucleotide mismatch to a sequence selected from the group consisting of SEQ ID NOs: 610-765 and 9798-10532. In some embodiments, the first ribonucleic acid comprises a sequence with a two nucleotide mismatch to a sequence selected from the group consisting of SEQ ID NOs: 610-765 and 9798-10532. In some embodiments, the second ribonucleic acid comprises a sequence with a single nucleotide mismatch to a sequence selected from the group consisting of SEQ ID NOs: 610-765 and 9798-10532. In some embodiments, the second ribonucleic acid comprises a sequence with a two nucleotide mismatch to a sequence selected from the group consisting of SEQ ID NOs: 610-765 and 9798-10532. In some embodiments, the pair of ribonucleic acids comprise sequences with at least one nucleotide mismatch or at least two nucleotide mismatches to a sequence selected from the group consisting of SEQ ID NOs: 610-765 and 9798-10532.

In some embodiments, the pair of ribonucleic acid sequences comprises or consists of SEQ ID NO: 657 and SEQ ID NO: 662. In some embodiments, the pair of ribonucleic acids comprise sequences with at least one nucleotide mismatch or at least two nucleotide mismatches to SEQ ID NO: 550 and SEQ ID NO: 573.

In some aspects, disclosed herein are compositions comprising a nucleic acid sequence encoding a Cas 9 protein, and at least one ribonucleic acid sequence selected from the group consisting of SEQ ID NOs: 766-780 and 10574-13257. In some aspects, disclosed herein are compositions comprising a nucleic acid sequence encoding a Cas 9 protein, a pair of ribonucleic acid sequences selected from the group consisting of SEQ ID NOs: 766-780 and 10574-13257. In some embodiments, the first ribonucleic acid comprises a sequence with a single nucleotide mismatch to a sequence selected from the group consisting of SEQ ID NOs: 766-780 and 10574-13257. In some embodiments, the first ribonucleic acid comprises a sequence with a two nucleotide mismatch to a sequence selected from the group consisting of SEQ ID NOs: 766-780 and 10574-13257. In some embodiments, the second ribonucleic acid comprises a sequence with a single nucleotide mismatch to a sequence selected from the group consisting of SEQ ID NOs: 766-780 and 10574-13257. In some embodiments, the second ribonucleic acid comprises a sequence with a two nucleotide mismatch to a sequence selected from the group consisting of SEQ ID NOs: 766-780 and 10574-13257. In some embodiments, the pair of ribonucleic acids comprise sequences with at least one nucleotide mismatch or at least two nucleotide mismatches to a sequence selected from the group consisting of SEQ ID NOs: 766-780 and 10574-13257.

In some embodiments, the pair of ribonucleic acid sequences comprises or consists of SEQ ID NO: 773 and SEQ ID NO: 778. In some embodiments, the pair of ribonucleic acids comprise sequences with at least one nucleotide mismatch or at least two nucleotide mismatches to SEQ ID NO: 773 and SEQ ID NO: 778.

In some aspects, disclosed herein are compositions comprising a nucleic acid sequence encoding a Cpfl protein, and a ribonucleic acid having a sequence selected from the group consisting of SEQ ID NOs: 3638-4046. In some embodiments, the ribonucleic acid comprises a sequence with a single nucleotide mismatch to a sequence selected from the group consisting of SEQ ID NOs: 3638-4046. In some embodiments, the ribonucleic acid comprises a sequence with a two nucleotide mismatch to a sequence selected from the group consisting of SEQ ID NOs: 3638-4046.

In some aspects, disclosed herein are compositions comprising a nucleic acid sequence encoding a Cpfl protein, and a ribonucleic acid having a sequence selected from the group consisting of SEQ ID NOs: 8946-9101. In some embodiments, the ribonucleic acid comprises a sequence with a single nucleotide mismatch to a sequence selected from the group consisting of SEQ ID NOs: 8946-9101. In some embodiments, the ribonucleic acid comprises a sequence with a two nucleotide mismatch to a sequence selected from the group consisting of SEQ ID NOs: 8946-9101.

In some aspects, disclosed herein are compositions comprising a nucleic acid sequence encoding a Cpfl protein, and a ribonucleic acid having a sequence selected from the group consisting of SEQ ID NOs: 9751-9797. In some embodiments, the ribonucleic acid comprises a sequence with a single nucleotide mismatch to a sequence selected from the group consisting of SEQ ID NOs: 9751-9797. In some embodiments, the ribonucleic acid comprises a sequence with a two nucleotide mismatch to a sequence selected from the group consisting of SEQ ID 9751-9797.

In some aspects, disclosed herein are compositions comprising a nucleic acid sequence encoding a Cpfl protein, and a ribonucleic acid having a sequence selected from the group consisting of SEQ ID NOs: 10533-10573. In some embodiments, the ribonucleic acid comprises a sequence with a single nucleotide mismatch to a sequence selected from the group consisting of SEQ ID NOs: 10533-10573. In some embodiments, the ribonucleic acid comprises a sequence with a two nucleotide mismatch to a sequence selected from the group consisting of SEQ ID NOs: 10533-10573.

In some aspects, disclosed herein are compositions comprising a nucleic acid sequence encoding a Cpfl protein, and a ribonucleic acid having a sequence selected from the group consisting of SEQ ID NOs: 13258-13719. In some embodiments, the ribonucleic acid comprises a sequence with a single nucleotide mismatch to a sequence selected from the group consisting of SEQ ID NOs: 13258-13719. In some embodiments, the ribonucleic acid comprises a sequence with a two nucleotide mismatch to a sequence selected from the group consisting of SEQ ID NOs: 13258-13719.

In some aspects, the invention provides a composition comprising a chimeric nucleic acid, the chimeric nucleic acid comprising: (a) a nucleic acid sequence encoding a Cas protein; and (b) at least one ribonucleic acid sequence selected from the group consisting of: (i) SEQ ID NOs: 1-195 and 797-3637; (ii) SEQ ID NOs: 196-531 and 4047-8945; (iii) SEQ ID NOs: 532-609 and 9102-9750; (iv) SEQ ID NOs: 610-765 and 9798-10532; and (v) SEQ ID NOs: 766-780 and 10574-13257; and combinations of (i)-(v).

In some embodiments, the at least one ribonucleic acid sequences in (b) is selected from the group consisting of: (i) SEQ ID NO: 128 and SEQ ID NO: 72; (ii) SEQ ID NO: 462 and SEQ ID NO: 421; (iii) SEQ ID NO: 550 and SEQ ID NO: 573; (iv) SEQ ID NO: 657 and SEQ ID NO: 662; and (v) SEQ ID NO: 773 and SEQ ID NO: 778; and combinations of (i)-(v).

In some aspects, the invention provides a composition comprising a chimeric nucleic acid, the chimeric nucleic acid comprising: (a) a nucleic acid sequence encoding a Cas protein; and (b) a ribonucleic acid sequence selected from the group consisting of: (i) SEQ ID NOs: 3638-4046; (ii) SEQ ID NOs: 8946-9101; (iii) SEQ ID NOs: 9751-9797; (iv) SEQ ID NOs: 10533-10573; and (v) SEQ ID NOs: 13258-13719; and combinations of (i)-(v).

In some embodiments, the composition further comprises a nucleic acid sequence encoding a detectable marker.

In some embodiments, the composition includes at least one additional ribonucleic acid sequences for altering a target polynucleotide sequence. In some embodiments, the composition includes at least two additional ribonucleic acid sequences for altering a target polynucleotide sequence. In some embodiments, the composition includes at least three additional ribonucleic acid sequences for altering a target polynucleotide sequence. In some embodiments, the composition includes at least four additional ribonucleic acid sequences for altering a target polynucleotide sequence.

In some embodiments, at least one of the ribonucleic acids in the composition is a modified ribonucleic acid as described herein (e.g., a synthetic, modified ribonucleic acid, e.g., comprising one to two modified nucleotides selected from the group consisting of pseudouridine, 5-methylcytodine, 2-thio-uridine, 5-methyluridine-5'-triphosphate, 4-thiouridine-5' -triphosphate, 5,6-dihydrouridine-5'-triphosphate, and 5-azauridine-5'-triphosphate, or any other modified nucleotides or modifications described herein).

In some embodiments, a composition of the present invention comprises a nucleic acid sequence encoding a Cas protein. In some embodiments, a composition of the present invention comprises nucleic acid sequence encoding Cas9 protein or a functional portion thereof. In some embodiments, a composition of the present invention comprises nucleic acid sequence encoding Cpfl protein or a functional portion thereof.

In some embodiments, at least one of the ribonucleic acids in the composition is a modified ribonucleic acid as described herein (e.g., a synthetic, modified ribonucleic acid, e.g., comprising one to two modified nucleotides selected from the group consisting of pseudouridine, 5-methylcytodine, 2-thio-uridine, 5-methyluridine-5'-triphosphate, 4-thiouridine-5'-triphosphate, 5,6-dihydrouridine-5'-triphosphate, and 5-azauridine-5'-triphosphate, or any other modified nucleotides or modifications described herein). In some embodiments, a pair of ribonucleic acids in the composition is a modified ribonucleic acid as described herein (e.g., a synthetic, modified ribonucleic acid, e.g., comprising one to two modified nucleotides selected from the group consisting of pseudouridine, 5-methylcytodine, 2-thio-uridine, 5-methyluridine-5'-triphosphate, 4-thiouridine-5'-triphosphate, 5,6-dihydrouridine-5'-triphosphate, and 5-azauridine-5'-triphosphate, or any other modified nucleotides or modifications described herein).

In some embodiments, a composition of the present invention comprises a nucleic acid sequence encoding a Cas protein. In some embodiments, a composition of the present invention comprises nucleic acid sequence encoding Cas9 protein or a functional portion thereof. In some embodiments, a composition of the present invention comprises a nucleic acid sequence encoding a Cas protein. In some embodiments, a composition of the present invention comprises nucleic acid sequence encoding Cas9 protein or a functional portion thereof. In some embodiments, a composition of the present invention comprises nucleic acid sequence encoding Cpfl protein or a functional portion thereof.

In some embodiments, the nucleic acid encoding the Cas protein (e.g., Cas9 or Cpfl) comprises a modified ribonucleic acid as described herein (e.g., a synthetic, modified mRNA described herein, e.g., comprising at least one modified nucleotide selected from the group consisting of pseudouridine, 5-methylcytodine, 2-thiouridine, 5-methyluridine-5'-triphosphate, 4-thiouridine-5'-triphosphate, 5,6-dihydrouridine-5'-triphosphate, and 5-azauridine-5'-triphosphate or any other modified nucleotides or modifications described herein).

In some embodiments, a composition of the present invention comprises a nucleic acid sequence encoding a fluorescent protein selected from the group consisting of green fluorescent protein and red fluorescent protein. In some embodiments, a composition of the present invention comprises a promoter operably linked to the chimeric nucleic acid. In some embodiments, the promoter is optimized for increased expression in human cells. In some embodiments, the promoter is optimized for increased expression in human stem cells. In some embodiments, the promoter is optimized for increased expression in primary human cells. In some embodiments, the promoter is selected from the group consisting of a Cytomegalovirus (CMV) early enhancer element and a chicken beta-actin promoter, a chicken beta-actin promoter, an elongation factor-1 alpha promoter, and a ubiquitin promoter. In some embodiments, the Cas protein comprises a Cas9 protein or a functional portion thereof. In some embodiments, the Cas protein comprises a Cpfl protein or a functional portion thereof.

The present invention also provides kits for practicing any of the methods of the present invention, as well as kits comprising the compositions of the present invention, and instructions for using the kits for altering target polynucleotide sequences in a cell or population thereof.

### Administering Cells

In some aspects, the invention provides a method of administering cells to a subject in need of such cells, the method comprising: (a) contacting a cell or population of cells *ex vivo* with a Cas protein or a nucleic acid encoding the Cas protein and at least one ribonucleic acid which directs Cas protein to and hybridize to at least one target polynucleotide sequence selected from the group consisting of target polynucleotide sequences encoding CTLA4, PD1, TCRA, TCRB, B2M, and combinations thereof in the cell or population thereof, wherein the at least one target polynucleotide sequence is cleaved; and (b) administering the resulting cells from (a) to a subject in need of such cells.

In some aspects, the invention provides a method of administering cells to a subject in need of such cells, the method comprising: (a) contacting a cell or population of cells *ex vivo* with (i) a Cas protein or a nucleic acid encoding the Cas protein, and (ii) at least one pair of ribonucleic acids which direct Cas protein to and hybridize to at least one target polynucleotide sequence selected from the group consisting of target polynucleotide sequences encoding CTLA4, PD1, TCRA, TCRB, B2M, and combinations thereof in the cell or population of cells, wherein the target polynucleotide sequences are cleaved; and (b) administering the resulting cell or cells from (a) to a subject in need of such cells.

In some aspects, the invention provides a method of administering cells to a subject in need of such cells, the method comprising: (a) contacting a cell or population of cells *ex vivo* with (i) a Cas protein or a nucleic acid encoding the Cas protein, and (ii) one ribonucleic acid which directs Cas protein to and hybridizes to a target polynucleotide sequence selected from the group consisting of target polynucleotide sequences encoding CTLA4, PD1, TCRA, TCRB, B2M, and combinations thereof in the cell or population of cells, wherein the target polynucleotide sequences are cleaved; and (b) administering the resulting cell or cells from (a) to a subject in need of such cells.

It is contemplated that the methods of administering cells can be adapted for any purpose in which administering such cells is desirable (e.g., for allogeneic administration of cells to a subject in need of such cells). In some embodiments, the subject in need of administration of cells is suffering from a disorder. For example, the subject may be suffering from a disorder in which the particular cells are decreased in function or number, and it may be desirable to administer functional cells obtained from a healthy or normal individual in which the particular cells are functioning properly and to administer an adequate number of those healthy cells to the individual to restore the function provided by those cells (e.g., hormone producing cells which have decreased in cell number or function, immune cells which have decreased in cell number or function, etc.). In such instances, the healthy cells can be engineered to decrease the likelihood of host rejection of the healthy cells. In some embodiments, the disorder comprises a genetic disorder. In some embodiments, the disorder comprises an infection. In some embodiments, the disorder comprises HIV or AIDs. In some embodiments, the disorder comprises cancer. In some embodiments, the disorder comprises an autoimmune disease.

The population of cells can be sorted (e.g., using FACS) prior to administering the cells to select for cells in which their genome has been edited. The sorted cells can then be expanded to an amount of cells needed for transplantation for the particular disorder for which the second subject is in need of such cells. In some embodiments, the method can include, prior to the step of administering, contacting the genomically modified cells with Cas protein and one or more guide RNA sequences targeting one or more additional target polynucleotides that are associated with the disorder for which the second subject (i.e., recipient) is in need of such cells. For example with HIV, the genomically modified cells can be contacted with Cas protein and one or more guide RNA sequences targeting the CCR5 and/or CXCR4 genes, thereby editing the genome of the genomically modified cells to eliminate or reduce surface expression of CCR5 and/or CXCR4. Such cells would be beneficial for administration to a second subject (e.g., suffering from HIV or AIDS) as they would eliminate or reduce the likelihood of an unwanted host immune response due to lack of MHC class I molecule surface expression, and exhibit little or no susceptibility to HIV infection due to the lack of CCR5 and/or CXCR4 surface expression.

As used herein "nucleic acid," in its broadest sense, includes any compound and/or substance that comprise a polymer of nucleotides linked via a phosphodiester bond. Exemplary nucleic acids include ribonucleic acids (RNAs), deoxyribonucleic acids (DNAs), threose nucleic acids (TNAs), glycol nucleic acids (GNAs), peptide nucleic acids (PNAs), locked nucleic acids (LNAs) or hybrids thereof. They may also include RNAi-inducing agents, RNAi agents, siRNAs, shRNAs, miRNAs, antisense RNAs, ribozymes, catalytic DNA, tRNA, RNAs that induce triple helix formation, aptamers, vectors, etc. In some embodiments, the nucleic acid encoding the Cas protein is an mRNA. In some embodiments, the Cas protein is encoded by a modified nucleic acid (e.g., a synthetic, modified mRNA described herein).

The present invention contemplates the use of any nucleic acid modification available to the skilled artisan. The nucleic acids of the present invention can include any number of modifications. In some embodiments, the nucleic acid comprises one or more modifications selected from the group consisting of pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine, and combinations thereof.

Preparation of modified nucleosides and nucleotides used in the manufacture or synthesis of modified RNAs of the present invention can involve the protection and deprotection of various chemical groups. The need for protection and deprotection, and the selection of appropriate protecting groups can be readily determined by one skilled in the art.

The chemistry of protecting groups can be found, for example, in Greene, et al., Protective Groups in Organic Synthesis, 2d. Ed., Wiley & Sons, 199 1.

Modified nucleosides and nucleotides can be prepared according to the synthetic methods described in Ogata et al. Journal of Organic Chemistry 74:2585-2588, 2009; Purmal et al. Nucleic Acids Research 22(1): 72-78, 1994; Fukuhara et al. Biochemistry 1(4): 563-568, 1962; and Xu et al. Tetrahedron 48(9): 1729-1740, 1992.

Modified nucleic acids (e.g., ribonucleic acids) need not be uniformly modified along the entire length of the molecule. Different nucleotide modifications and/or backbone structures may exist at various positions in the nucleic acid. One of ordinary skill in the art will appreciate that the nucleotide analogs or other modification(s) may be located at any position(s) of a nucleic acid such that the function of the nucleic acid is not substantially decreased. A modification may also be a 5' or 3' terminal modification. The nucleic acids may contain at a minimum one and at maximum 100% modified nucleotides, or any intervening percentage, such as at least 50% modified nucleotides, at least 80% modified nucleotides, or at least 90% modified nucleotides.

In some embodiments, at least one ribonucleic acid is a modified ribonucleic acid. In some embodiments, at least one of the one to two ribonucleic acids is a modified ribonucleic acid. In some embodiments, each of the one to two ribonucleic acids is a modified ribonucleic acid. In some embodiments, at least one of the multiple ribonucleic acids is a modified ribonucleic acid. In some embodiments, a plurality of the multiple ribonucleic acids are modified. In some embodiments, each of the multiple ribonucleic acids is modified. Those skilled in the art will appreciate that the modified ribonucleic acids can include one or more of the nucleic acid modification described herein.

In some aspects, provided herein are synthetic, modified RNA molecules encoding polypeptides, where the synthetic, modified RNA molecules comprise one or more modifications, such that introducing the synthetic, modified RNA molecules to a cell results in a reduced innate immune response relative to a cell contacted with synthetic RNA molecules encoding the polypeptides not comprising the one or more modifications. In some embodiments, the Cas protein comprises a synthetic, modified RNA molecule encoding a Cas protein. In some embodiments, the Cas protein comprises a synthetic, modified RNA molecule encoding a Cas9 protein. In some embodiments, the Cas protein comprises a synthetic, modified RNA molecule encoding a Cpfl protein.

The synthetic, modified RNAs described herein include modifications to prevent rapid degradation by endo- and exo-nucleases and to avoid or reduce the cell's innate immune or interferon response to the RNA. Modifications include, but are not limited to, for example, (a) end modifications, e.g., 5' end modifications (phosphorylation dephosphorylation, conjugation, inverted linkages, etc.), 3' end modifications (conjugation, DNA nucleotides, inverted linkages, etc.), (b) base modifications, e.g., replacement with modified bases, stabilizing bases, destabilizing bases, or bases that base pair with an expanded repertoire of partners, or conjugated bases, (c) sugar modifications (e.g., at the 2' position or 4' position) or replacement of the sugar, as well as (d) internucleoside linkage modifications, including modification or replacement of the phosphodiester linkages. To the extent that such modifications interfere with translation (i.e., results in a reduction of 50% or more in translation relative to the lack of the modification-e.g., in a rabbit reticulocyte *in vitro* translation assay), the modification is not suitable for the methods and compositions described herein. Specific examples of synthetic, modified RNA compositions useful with the methods described herein include, but are not limited to, RNA molecules containing modified or non-natural internucleoside linkages. Synthetic, modified RNAs having modified internucleoside linkages include, among others, those that do not have a phosphorus atom in the internucleoside linkage. In other embodiments, the synthetic, modified RNA has a phosphorus atom in its internucleoside linkage(s).

Non-limiting examples of modified internucleoside linkages include phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those) having inverted polarity wherein the adjacent pairs of nucleoside units are linked 3'-5' to 5'-3' or 2'-5' to 5'-2'. Various salts, mixed salts and free acid forms are also included.

Representative U.S. patents that teach the preparation of the above phosphorus-containing linkages include, but are not limited to, U.S. Pat. Nos. 3,687,808; 4,469,863; 4,476,301; 5,023,243; 5,177,195; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,316; 5,550,111; 5,563,253; 5,571,799; 5,587,361; 5,625,050; 6,028,188; 6,124,445; 6,160,109; 6,169,170; 6,172,209; 6,239,265; 6,277,603; 6,326,199; 6,346,614; 6,444,423; 6,531,590; 6,534,639; 6,608,035; 6,683,167; 6,858,715; 6,867,294; 6,878,805; 7,015,315; 7,041,816; 7,273,933; 7,321,029; and U.S. Pat. RE39464.

Modified internucleoside linkages that do not include a phosphorus atom therein have internucleoside linkages that are formed by short chain alkyl or cycloalkyl internucleoside linkages, mixed heteroatoms and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts.

Representative U.S. patents that teach the preparation of modified oligonucleosides include, but are not limited to, U.S. Pat. Nos. 5,034,506; 5,166,315; 5,185,444; 5,214,134; 5,216,141; 5,235,033; 5,64,562; 5,264,564; 5,405,938; 5,434,257; 5,466,677; 5,470,967; 5,489,677; 5,541,307; 5,561,225; 5,596,086; 5,602,240; 5,608,046; 5,610,289; 5,618,704; 5,623,070; 5,663,312; 5,633,360; 5,677,437; and 5,677,439.

Some embodiments of the synthetic, modified RNAs described herein include nucleic acids with phosphorothioate internucleoside linkages and oligonucleosides with heteroatom internucleoside linkage, and in particular -CH2-NH-CH2-, - CH2-N(CH3)-O-CH2-[known as a methylene (methylimino) or MMI], -CH2-ON(CH3)-CH2-, -CH2-N(CH3)-N(CH3)-CH2- and -N(CH3)-CH2-CH2- [wherein the native phosphodiester internucleoside linkage is represented as -O-P-O-CH2-] of the above-referenced U.S. Pat. No. 5,489,677, and the amide backbones of the above-referenced U.S. Pat. No. 5,602,240. In some embodiments, the nucleic acid sequences featured herein have morpholino backbone structures of the above-referenced U.S. Pat. No. 5,034,506.

Synthetic, modified RNAs described herein can also contain one or more substituted sugar moieties. The nucleic acids featured herein can include one of the following at the 2' position: H (deoxyribose); OH (ribose); F; O-, S-, or N-alkyl; O-, S-, or N-alkenyl; O-, S- or N-alkynyl; or O-alkyl-O-alkyl, wherein the alkyl, alkenyl and alkynyl can be substituted or unsubstituted C1 to C10 alkyl or C2 to C10 alkenyl and alkynyl. Exemplary modifications include O[(CH2)nO]mCH3, O(CH2).nOCH3, O(CH2)nNH2, O(CH2)nCH3, O(CH2)nONH2, and O(CH2)nON[(CH2)nCH3)]2, where n and m are from 1 to about 10. In some embodiments, synthetic, modified RNAs include one of the following at the 2' position: C1 to C10 lower alkyl, substituted lower alkyl, alkaryl, aralkyl, O-alkaryl or O-aralkyl, SH, SCH3, OCN, Cl, Br, CN, CF3, OCF3, SOCH3, SO2CH3, ONO2, NO2, N3, NH2, heterocycloalkyl, heterocycloalkaryl, aminoalkylamino, polyalkylamino, substituted silyl, a reporter group, an intercalator, a group for improving the pharmacokinetic properties of an RNA, or a group for improving the pharmacodynamic properties of a synthetic, modified RNA, and other substituents having similar properties. In some embodiments, the modification includes a 2' methoxyethoxy (2'-O-CH2CH2OCH3, also known as 2'-O-(2-methoxyethyl) or 2'-MOE) (Martin et al., Helv. Chim. Acta, 1995, 78:486-504) i.e., an alkoxy-alkoxy group. Another exemplary modification is 2'-dimethylaminooxyethoxy, i.e., a O(CH2)2ON(CH3)2 group, also known as 2'-DMAOE, and 2'-dimethylaminoethoxyethoxy (also known in the art as 2'-O-dimethylaminoethoxyethyl or 2'-DMAEOE), i.e., 2'-O-CH2-O-CH2-N(CH2)2.

Other modifications include 2'-methoxy (2'-OCH3), 2'-aminopropoxy (2'-OCH2CH2CH2NH2) and 2'-fluoro (2'-F). Similar modifications can also be made at other positions on the nucleic acid sequence, particularly the 3' position of the sugar on the 3' terminal nucleotide or in 2'-5' linked nucleotides and the 5' position of 5' terminal nucleotide. A synthetic, modified RNA can also have sugar mimetics such as cyclobutyl moieties in place of the pentofuranosyl sugar. Representative U.S. patents that teach the preparation of such modified sugar structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, certain of which are commonly owned with the instant application.

As non-limiting examples, synthetic, modified RNAs described herein can include at least one modified nucleoside including a 2'-O-methyl modified nucleoside, a nucleoside comprising a 5' phosphorothioate group, a 2'-amino-modified nucleoside, 2'-alkyl-modified nucleoside, morpholino nucleoside, a phosphoramidate or a non-natural base comprising nucleoside, or any combination thereof.

In some embodiments of this aspect and all other such aspects described herein, the at least one modified nucleoside is selected from the group consisting of 5-methylcytidine (5mC), N6-methyladenosine (m6A), 3,2'-O-dimethyluridine (m4tJ), 2-thiouridine (s2U), 2' fluorouridine, pseudouridine, 2'-O-methyluridine (Um), 2' deoxyuridine (2' dU), 4-thiouridine (s4U), 5-methyluridine (m5U), 2'-O-methyladenosine (m6A), N6,2'-O-dimethyladenosine (m6Am), N6,N6,2'-O-trimethyladenosine (m62Am), 2'-O-methylcytidine (Cm), 7-methylguanosine (m7G), 2'-O-methylguanosine (Gm), N2,7-dimethylguanosine (m2,7G), N2,N2,7-trimethylguanosine (m2,2,7G), and inosine (I).

Alternatively, a synthetic, modified RNA can comprise at least two modified nucleosides, at least 3, at least 4, at least 5, at least 6, at least 7, at least 8, at least 9, at least 10, at least 15, at least 20 or more, up to the entire length of the nucleotide. At a minimum, a synthetic, modified RNA molecule comprising at least one modified nucleoside comprises a single nucleoside with a modification as described herein. It is not necessary for all positions in a given synthetic, modified RNA to be uniformly modified, and in fact more than one of the aforementioned modifications can be incorporated in a single synthetic, modified RNA or even at a single nucleoside within a synthetic, modified RNA. However, it is preferred, but not absolutely necessary, that each occurrence of a given nucleoside in a molecule is modified (e.g., each cytosine is a modified cytosine e.g., 5mC). However, it is also contemplated that different occurrences of the same nucleoside can be modified in a different way in a given synthetic, modified RNA molecule (e.g., some cytosines modified as 5mC, others modified as 2'-O-methylcytidine or other cytosine analog). The modifications need not be the same for each of a plurality of modified nucleosides in a synthetic, modified RNA. Furthermore, in some embodiments of the aspects described herein, a synthetic, modified RNA comprises at least two different modified nucleosides. In some such preferred embodiments of the aspects described herein, the at least two different modified nucleosides are 5-methylcytidine and pseudouridine. A synthetic, modified RNA can also contain a mixture of both modified and unmodified nucleosides.

As used herein, "unmodified" or "natural" nucleosides or nucleobases include the purine bases adenine (A) and guanine (G), and the pyrimidine bases thymine (T), cytosine (C) and uracil (U). In some embodiments, a synthetic, modified RNA comprises at least one nucleoside ("base") modification or substitution. Modified nucleosides include other synthetic and natural nucleobases such as inosine, xanthine, hypoxanthine, nubularine, isoguanisine, tubercidine, 2-(halo)adenine, 2-(alkyl)adenine, 2-(propyl)adenine, 2 (amino)adenine, 2-(aminoalkyl)adenine, 2 (aminopropyl)adenine, 2 (methylthio) N6 (isopentenyl)adenine, 6 (alkyl)adenine, 6 (methyl)adenine, 7 (deaza)adenine, 8 (alkenyl)adenine, 8-(alkyl)adenine, 8 (alkynyl)adenine, 8 (amino)adenine, 8-(halo)adenine, 8-(hydroxyl)adenine, 8 (thioalkyl)adenine, 8-(thiol)adenine, N6-(isopentyl)adenine, N6 (methyl)adenine, N6, N6 (dimethyl)adenine, 2-(alkyl)guanine, 2 (propyl)guanine, 6-(alkyl)guanine, 6 (methyl)guanine, 7 (alkyl)guanine, 7 (methyl)guanine, 7 (deaza)guanine, 8 (alkyl)guanine, 8-(alkenyl)guanine, 8 (alkynyl)guanine, 8-(amino)guanine, 8 (halo)guanine, 8-(hydroxyl)guanine, 8 (thioalkyl)guanine, 8-(thiol)guanine, N (methyl)guanine, 2-(thio)cytosine, 3 (deaza) 5 (aza)cytosine, 3-(alkyl)cytosine, 3 (methyl)cytosine, 5-(alkyl)cytosine, 5-(alkynyl)cytosine, 5 (halo)cytosine, 5 (methyl)cytosine, 5 (propynyl)cytosine, 5 (propynyl)cytosine, 5 (trifluoromethyl)cytosine, 6-(azo)cytosine, N4 (acetyl)cytosine, 3 (3 amino-3 carboxypropyl)uracil, 2-(thio)uracil, 5 (methyl) 2 (thio)uracil, 5 (methylaminomethyl)-2 (thio)uracil, 4-(thio)uracil, 5 (methyl) 4 (thio)uracil, 5 (methylaminomethyl)-4 (thio)uracil, 5 (methyl) 2,4 (dithio)uracil, 5 (methylaminomethyl)-2,4 (dithio)uracil, 5 (2-aminopropyl)uracil, 5-(alkyl)uracil, 5-(alkynyl)uracil, 5-(allylamino)uracil, 5 (aminoallyl)uracil, 5 (aminoalkyl)uracil, 5 (guanidiniumalkyl)uracil, 5 (1,3-diazole-1-alkyl)uracil, 5-(cyanoalkyl)uracil, 5-(dialkylaminoalkyl)uracil, 5 (dimethylaminoalkyl)uracil, 5-(halo)uracil, 5-(methoxy)uracil, uracil-5 oxyacetic acid, 5 (methoxycarbonylmethyl)-2-(thio)uracil, 5 (methoxycarbonyl-methyl)uracil, 5 (propynyl)uracil, 5 (propynyl)uracil, 5 (trifluoromethyl)uracil, 6 (azo)uracil, dihydrouracil, N3 (methyl)uracil, 5-uracil (i.e., pseudouracil), 2 (thio)pseudouracil,4 (thio)pseudouracil,2,4-(dithio)psuedouracil,5-(alkyl)pseudouracil, 5-(methyl)pseudouracil, 5-(alkyl)-2-(thio)pseudouracil, 5-(methyl)-2-(thio)pseudouracil, 5-(alkyl)-4 (thio)pseudouracil, 5-(methyl)-4 (thio)pseudouracil, 5-(alkyl)-2,4 (dithio)pseudouracil, 5-(methyl)-2,4 (dithio)pseudouracil, 1 substituted pseudouracil, 1 substituted 2(thio)-pseudouracil, 1 substituted 4 (thio)pseudouracil, 1 substituted 2,4-(dithio)pseudouracil, 1 (aminocarbonylethylenyl)-pseudouracil, 1 (aminocarbonylethylenyl)-2(thio)-pseudouracil, 1 (aminocarbonylethylenyl)-4 (thio)pseudouracil, 1 (aminocarbonylethylenyl)-2,4-(dithio)pseudouracil, 1 (aminoalkylaminocarbonylethylenyl)-pseudouracil, 1 (aminoalkylamino-carbonylethylenyl)-2(thio)-pseudouracil, 1 (aminoalkylaminocarbonylethylenyl)-4 (thio)pseudouracil, 1 (aminoalkylaminocarbonylethylenyl)-2,4-(dithio)pseudouracil, 1,3-(diaza)-2-(oxo)-phenoxazin-1-yl, 1-(aza)-2-(thio)-3-(aza)-phenoxazin-1-yl, 1,3-(diaza)-2-(oxo)-phenthiazin-1-yl, 1-(aza)-2-(thio)-3-(aza)-phenthiazin-1-yl, 7-substituted 1,3-(diaza)-2-(oxo)-phenoxazin-1-yl, 7-substituted 1-(aza)-2-(thio)-3-(aza)-phenoxazin-1-yl, 7-substituted 1,3-(diaza)-2-(oxo)-phenthiazin-1-yl, 7-substituted 1-(aza)-2-(thio)-3-(aza)-phenthiazin-1-yl, 7-(aminoalkylhydroxy)-1,3-(diaza)-2-(oxo)-phenoxazin-1-yl, 7-(aminoalkylhydroxy)-1-(aza)-2-(thio)-3-(aza)-phenoxazin-1-yl, 7-(aminoalkylhydroxy)-1,3-(diaza)-2-(oxo)-phenthiazin-1-yl, 7-(aminoaikylhydroxy)-1-(aza)-2-(thio)-3-(aza)-phenthiazin-1-yl, 7-(guanidiniumalkylhydroxy)-1,3-(diaza)-2-(oxo)-phenoxazin-1-yl, 7-(guanidiniumalkylhydroxy)-1-(aza)-2-(thio)-3-(aza)-phenoxazin-1-yl, 7-(guanidiniumalkylhydroxy)-1,3-(diaza)-2-(oxo)-phenthiazin-1-yl, 7-(guanidiniumalkylhydroxy)-1-(aza)-2-(thio)-3-(aza)-phenthiazin-1-yl, 1,3,5-(triaza)-2,6-(dioxa)-naphthalene, inosine, xanthine, hypoxanthine, nubularine, tubercidine, isoguanisine, inosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, 3-(methyl)isocarbostyrilyl, 5-(methyl)isocarbostyrilyl, 3-(methyl)-7-(propynyl)isocarbostyrilyl, 7-(aza)indolyl, 6-(methyl)-7-(aza)indolyl, imidizopyridinyl, 9-(methyl)-imidizopyridinyl, pyrrolopyrizinyl, isocarbostyrilyl, 7-(propynyl)isocarbostyrilyl, propynyl-7-(aza)indolyl, 2,4,5-(trimethyl)phenyl, 4-(methyl)indolyl, 4,6-(dimethyl)indolyl, phenyl, napthalenyl, anthracenyl, phenanthracenyl, pyrenyl, stilbenzyl, tetracenyl, pentacenyl, difluorotolyl, 4-(fluoro)-6-(methyl)benzimidazole, 4-(methyl)benzimidazole, 6-(azo)thymine, 2-pyridinone, 5 nitroindole, 3 nitropyrrole, 6-(aza)pyrimidine, 2 (amino)purine, 2,6-(diamino)purine, 5 substituted pyrimidines, N2-substituted purines, N6-substituted purines, 06-substituted purines, substituted 1,2,4-triazoles, pyrrolo-pyrimidin-2-on-3-yl, 6-phenyl-pyrrolo-pyrimidin-2-on-3-yl, para-substituted-6-phenyl-pyrrolo-pyrimidin-2-on-3-yl, ortho-substituted-6-phenyl-pyrrolo-pyrimidin-2-on-3-yl, bis-ortho-substituted-6-phenyl-pyrrolo-pyrimidin-2-on-3-yl, para-(aminoalkylhydroxy)-6-phenyl-pyrrolo-pyrimidin-2-on-3-yl, ortho-(aminoalkylhydroxy)-6-phenyl-pyrrolo-pyrimidin-2-on-3-yl, bis-ortho-(aminoalkylhydroxy)-6-phenyl-pyrrolo-pyrimidin-2-on-3-yl, pyridopyrimidin-3-yl, 2-oxo-7-amino-pyridopyrimidin-3-yl, 2-oxo-pyridopyrimidine-3-yl, or any O-alkylated or N-alkylated derivatives thereof. Modified nucleosides also include natural bases that comprise conjugated moieties, e.g. a ligand. As discussed herein above, the RNA containing the modified nucleosides must be translatable in a host cell (i.e., does not prevent translation of the polypeptide encoded by the modified RNA). For example, transcripts containing s2U and m6A are translated poorly in rabbit reticulocyte lysates, while pseudouridine, m5U, and m5C are compatible with efficient translation. In addition, it is known in the art that 2'-fluoro-modified bases useful for increasing nuclease resistance of a transcript, leads to very inefficient translation. Translation can be assayed by one of ordinary skill in the art using e.g., a rabbit reticulocyte lysate translation assay.

Further modified nucleobases include those disclosed in U.S. Pat. No. 3,687,808, those disclosed in Modified Nucleosides in Biochemistry, Biotechnology and Medicine, Herdewijn, P. ed. Wiley-VCH, 2008; those disclosed in Int. Appl. No. PCT/US09/038,425, filed Mar. 26, 2009; those disclosed in The Concise Encyclopedia Of Polymer Science And Engineering, pages 858-859, Kroschwitz, J. L, ed. John Wiley & Sons, 1990, and those disclosed by Englisch et al., Angewandte Chemie, International Edition, 1991, 30, 613.

Representative U.S. patents that teach the preparation of certain of the above noted modified nucleobases as well as other modified nucleobases include, but are not limited to, the above noted U.S. Pat. No. 3,687,808, as well as U.S. Pat. Nos. 4,845,205; 5,130,30; 5,134,066; 5,175,273; 5,367,066; 5,432,272; 5,457,187; 5,457,191; 5,459,255; 5,484,908; 5,502,177; 5,525,711; 5,552,540; 5,587,469; 5,594,121, 5,596,091; 5,614,617; 5,681,941; 6,015,886; 6,147,200; 6,166,197; 6,222,025; 6,235,887; 6,380,368; 6,528,640; 6,639,062; 6,617,438; 7,045,610; 7,427,672; and 7,495,088, and U.S. Pat. No. 5,750,692.

Another modification for use with the synthetic, modified RNAs described herein involves chemically linking to the RNA one or more ligands, moieties or conjugates that enhance the activity, cellular distribution or cellular uptake of the RNA. The synthetic, modified RNAs described herein can further comprise a 5' cap. In some embodiments of the aspects described herein, the synthetic, modified RNAs comprise a 5' cap comprising a modified guanine nucleotide that is linked to the 5' end of an RNA molecule using a 5'-5' triphosphate linkage. As used herein, the term "5' cap" is also intended to encompass other 5' cap analogs including, e.g., 5' diguanosine cap, tetraphosphate cap analogs having a methylene-bis(phosphonate) moiety (see e.g., Rydzik, A M et al., (2009) Org Biomol Chem 7(22):4763-76), dinucleotide cap analogs having a phosphorothioate modification (see e.g., Kowalska, J. et al., (2008) RNA 14(6):1119-1131), cap analogs having a sulfur substitution for a non-bridging oxygen (see e.g., Grudzien-Nogalska, E. et al., (2007) RNA 13(10): 1745-1755), N7-benzylated dinucleoside tetraphosphate analogs (see e.g., Grudzien, E. et al., (2004) RNA 10(9):1479-1487), or anti-reverse cap analogs (see e.g., Jemielity, J. et al., (2003) RNA 9(9): 1108-1122 and Stepinski, J. et al., (2001) RNA 7(10):1486-1495). In one such embodiment, the 5' cap analog is a 5' diguanosine cap. In some embodiments, the synthetic, modified RNA does not comprise a 5' triphosphate.

The 5' cap is important for recognition and attachment of an mRNA to a ribosome to initiate translation. The 5' cap also protects the synthetic, modified RNA from 5' exonuclease mediated degradation. It is not an absolute requirement that a synthetic, modified RNA comprise a 5' cap, and thus in other embodiments the synthetic, modified RNAs lack a 5' cap. However, due to the longer half-life of synthetic, modified RNAs comprising a 5' cap and the increased efficiency of translation, synthetic, modified RNAs comprising a 5' cap are preferred herein.

The synthetic, modified RNAs described herein can further comprise a 5' and/or 3' untranslated region (UTR). Untranslated regions are regions of the RNA before the start codon (5') and after the stop codon (3'), and are therefore not translated by the translation machinery. Modification of an RNA molecule with one or more untranslated regions can improve the stability of an mRNA, since the untranslated regions can interfere with ribonucleases and other proteins involved in RNA degradation. In addition, modification of an RNA with a 5' and/or 3' untranslated region can enhance translational efficiency by binding proteins that alter ribosome binding to an mRNA. Modification of an RNA with a 3' UTR can be used to maintain a cytoplasmic localization of the RNA, permitting translation to occur in the cytoplasm of the cell. In one embodiment, the synthetic, modified RNAs described herein do not comprise a 5' or 3' UTR. In another embodiment, the synthetic, modified RNAs comprise either a 5' or 3' UTR. In another embodiment, the synthetic, modified RNAs described herein comprise both a 5' and a 3' UTR. In one embodiment, the 5' and/or 3' UTR is selected from an mRNA known to have high stability in the cell (e.g., a murine alpha-globin 3' UTR). In some embodiments, the 5' UTR, the 3' UTR, or both comprise one or more modified nucleosides.

In some embodiments, the synthetic, modified RNAs described herein further comprise a Kozak sequence. The "Kozak sequence" refers to a sequence on eukaryotic mRNA having the consensus (gcc)gccRccAUGG , where R is a purine (adenine or guanine) three bases upstream of the start codon (AUG), which is followed by another 'G'. The Kozak consensus sequence is recognized by the ribosome to initiate translation of a polypeptide. Typically, initiation occurs at the first AUG codon encountered by the translation machinery that is proximal to the 5' end of the transcript. However, in some cases, this AUG codon can be bypassed in a process called leaky scanning. The presence of a Kozak sequence near the AUG codon will strengthen that codon as the initiating site of translation, such that translation of the correct polypeptide occurs. Furthermore, addition of a Kozak sequence to a synthetic, modified RNA will promote more efficient translation, even if there is no ambiguity regarding the start codon. Thus, in some embodiments, the synthetic, modified RNAs described herein further comprise a Kozak consensus sequence at the desired site for initiation of translation to produce the correct length polypeptide. In some such embodiments, the Kozak sequence comprises one or more modified nucleosides.

In some embodiments, the synthetic, modified RNAs described herein further comprise a "poly (A) tail", which refers to a 3' homopolymeric tail of adenine nucleotides, which can vary in length (e.g., at least 5 adenine nucleotides) and can be up to several hundred adenine nucleotides). The inclusion of a 3' poly(A) tail can protect the synthetic, modified RNA from degradation in the cell, and also facilitates extra-nuclear localization to enhance translation efficiency. In some embodiments, the poly(A) tail comprises between 1 and 500 adenine nucleotides; in other embodiments the poly(A) tail comprises at least 5, at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, at least 200, at least 225, at least 250, at least 275, at least 300, at least 325, at least 350, at least 375, at least 400, at least 425, at least 450, at least 475, at least 500 adenine nucleotides or more. In one embodiment, the poly(A) tail comprises between 1 and 150 adenine nucleotides. In another embodiment, the poly(A) tail comprises between 90 and 120 adenine nucleotides. In some such embodiments, the poly(A) tail comprises one or more modified nucleosides.

It is contemplated that one or more modifications to the synthetic, modified RNAs described herein permit greater stability of the synthetic, modified RNA in a cell. To the extent that such modifications permit translation and either reduce or do not exacerbate a cell's innate immune or interferon response to the synthetic, modified RNA with the modification, such modifications are specifically contemplated for use herein. Generally, the greater the stability of a synthetic, modified RNA, the more protein can be produced from that synthetic, modified RNA. Typically, the presence of AU-rich regions in mammalian mRNAs tend to destabilize transcripts, as cellular proteins are recruited to AU-rich regions to stimulate removal of the poly(A) tail of the transcript. Loss of a poly(A) tail of a synthetic, modified RNA can result in increased RNA degradation. Thus, in one embodiment, a synthetic, modified RNA as described herein does not comprise an AU-rich region. In particular, it is preferred that the 3' UTR substantially lacks AUUUA sequence elements.

In one embodiment, a ligand alters the cellular uptake, intracellular targeting or half-life of a synthetic, modified RNA into which it is incorporated. In some embodiments a ligand provides an enhanced affinity for a selected target, e.g., molecule, cell or cell type, intracellular compartment, e.g., mitochondria, cytoplasm, peroxisome, lysosome, as, e.g., compared to a composition absent such a ligand. Preferred ligands do not interfere with expression of a polypeptide from the synthetic, modified RNA.

The ligand can be a substance, e.g., a drug, which can increase the uptake of the synthetic, modified RNA or a composition thereof into the cell, for example, by disrupting the cell's cytoskeleton, e.g., by disrupting the cell's microtubules, microfilaments, and/or intermediate filaments. The drug can be, for example, taxol, vincristine, vinblastine, cytochalasin, nocodazole, japlakinolide, latrunculin A, phalloidin, swinholide A, indanocine, or myoservin.

In another aspect, the ligand is a moiety, e.g., a vitamin, which is taken up by a host cell. Exemplary vitamins include vitamin A, E, and K. Other exemplary vitamins include B vitamin, e.g., folic acid, B12, riboflavin, biotin, pyridoxal or other vitamins or nutrients taken up, for example, by cancer cells. Also included are HSA and low density lipoprotein (LDL).

In another aspect, the ligand is a cell-permeation agent, preferably a helical cell-permeation agent. Preferably, the agent is amphipathic. An exemplary agent is a peptide such as tat or antennopedia. If the agent is a peptide, it can be modified, including a peptidylmimetic, invertomers, non-peptide or pseudo-peptide linkages, and use of D-amino acids. The helical agent is preferably an alpha-helical agent, which preferably has a lipophilic and a lipophobic phase.

A "cell permeation peptide" is capable of permeating a cell, e.g., a microbial cell, such as a bacterial or fungal cell, or a mammalian cell, such as a human cell. A microbial cell-permeating peptide can be, for example, an α-helical linear peptide (e.g., LL-37 or Ceropin P1), a disulfide bond-containing peptide (e.g., α-defensin, β-defensin or bactenecin), or a peptide containing only one or two dominating amino acids (e.g., PR-39 or indolicidin). For example, a cell permeation peptide can be a bipartite amphipathic peptide, such as MPG, which is derived from the fusion peptide domain of HIV-1 gp41 and the NLS of SV40 large T antigen (Simeoni et al., Nucl. Acids Res. 31:2717-2724, 2003).

The synthetic, modified RNAs described herein can be synthesized and/or modified by methods well established in the art, such as those described in "Current Protocols in Nucleic Acid Chemistry," Beaucage, S. L. et al. (Edrs.), John Wiley & Sons, Inc., New York, N.Y., USA. Transcription methods are described further herein in the Examples. In one embodiment of the aspects described herein, a template for a synthetic, modified RNA is synthesized using "splint-mediated ligation," which allows for the rapid synthesis of DNA constructs by controlled concatenation of long oligos and/or dsDNA PCR products and without the need to introduce restriction sites at the joining regions. It can be used to add generic untranslated regions (UTRs) to the coding sequences of genes during T7 template generation. Splint mediated ligation can also be used to add nuclear localization sequences to an open reading frame, and to make dominant-negative constructs with point mutations starting from a wild-type open reading frame. Briefly, single-stranded and/or denatured dsDNA components are annealed to splint oligos which bring the desired ends into conjunction, the ends are ligated by a thermostable DNA ligase and the desired constructs amplified by PCR. A synthetic, modified RNA is then synthesized from the template using an RNA polymerase *in vitro.* After synthesis of a synthetic, modified RNA is complete, the DNA template is removed from the transcription reaction prior to use with the methods described herein.

In some embodiments of these aspects, the synthetic, modified RNAs are further treated with an alkaline phosphatase.

The articles "a" and "an" as used herein in the specification and in the claims, unless clearly indicated to the contrary, should be understood to include the plural referents. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process.

Where ranges are given herein, the invention includes embodiments in which the endpoints are included, embodiments in which both endpoints are excluded, and embodiments in which one endpoint is included and the other is excluded. It should be assumed that both endpoints are included unless indicated otherwise. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise. It is also understood that where a series of numerical values is stated herein, the invention includes embodiments that relate analogously to any intervening value or range defined by any two values in the series, and that the lowest value may be taken as a minimum and the greatest value may be taken as a maximum. Numerical values, as used herein, include values expressed as percentages. For any embodiment of the invention in which a numerical value is prefaced by "about" or "approximately", the invention includes an embodiment in which the exact value is recited. For any embodiment of the invention in which a numerical value is not prefaced by "about" or "approximately", the invention includes an embodiment in which the value is prefaced by "about" or "approximately".

As used herein "A and/or B", where A and B are different claim terms, generally means at least one of A, B, or both A and B. For example, one sequence which is complementary to and/or hybridizes to another sequence includes (i) one sequence which is complementary to the other sequence even though the one sequence may not necessarily hybridize to the other sequence under all conditions, (ii) one sequence which hybridizes to the other sequence even if the one sequence is not perfectly complementary to the other sequence, and (iii) sequences which are both complementary to and hybridize to the other sequence.

"Approximately" or "about" generally includes numbers that fall within a range of 1% or in some embodiments within a range of 5% of a number or in some embodiments within a range of 10% of a number in either direction (greater than or less than the number) unless otherwise stated or otherwise evident from the context (except where such number would impermissibly exceed 100% of a possible value). It should be understood that, unless clearly indicated to the contrary, in any methods claimed herein that include more than one act, the order of the acts of the method is not necessarily limited to the order in which the acts of the method are recited, but the invention includes embodiments in which the order is so limited. It should also be understood that unless otherwise indicated or evident from the context, any product or composition described herein may be considered "isolated".

As used herein the term "comprising" or "comprises" is used in reference to compositions, methods, and respective component(s) thereof, that are essential to the invention, yet open to the inclusion of unspecified elements, whether essential or not. As used herein the term "consisting essentially of" refers to those elements required for a given embodiment. The term permits the presence of additional elements that do not materially affect the basic and novel or functional characteristic(s) of that embodiment of the invention.

The term "consisting of" refers to compositions, methods, and respective components thereof as described herein, which are exclusive of any element not recited in that description of the embodiment.

### EXAMPLES

### Example 1: Identification of CRISPR guide ribonucleic acids (gRNAs) that efficiently remove the endogenous TCR from primary human T cells to prevent autoreactivity of CAR T cells.

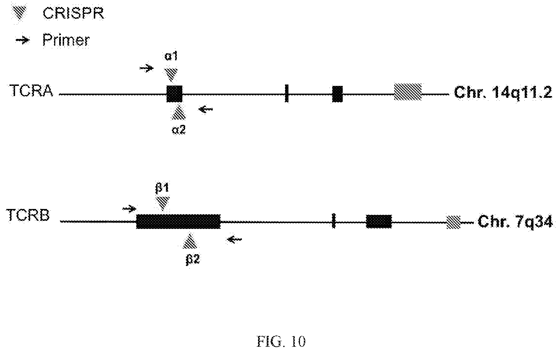

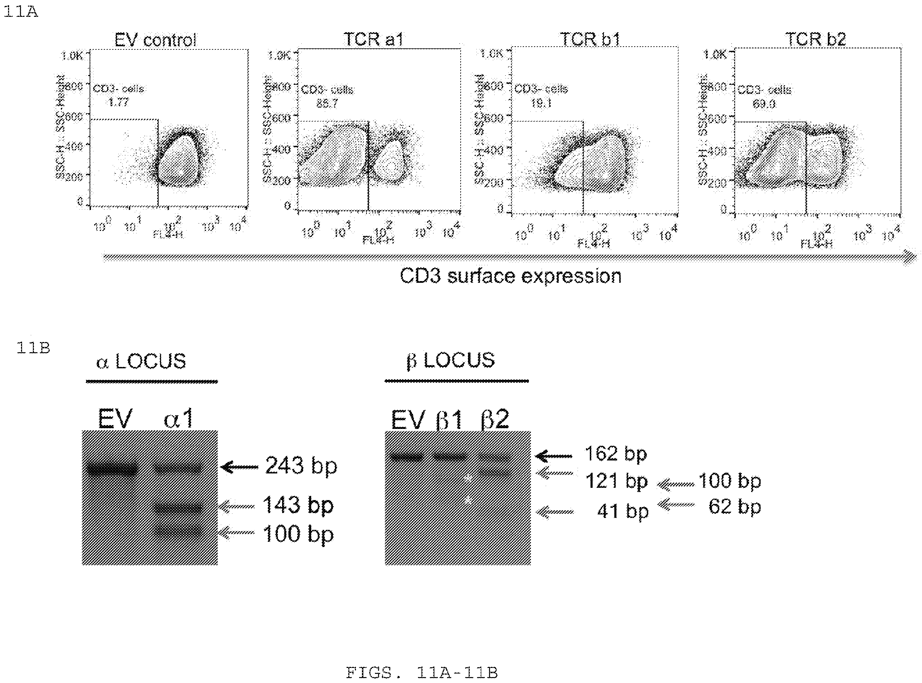

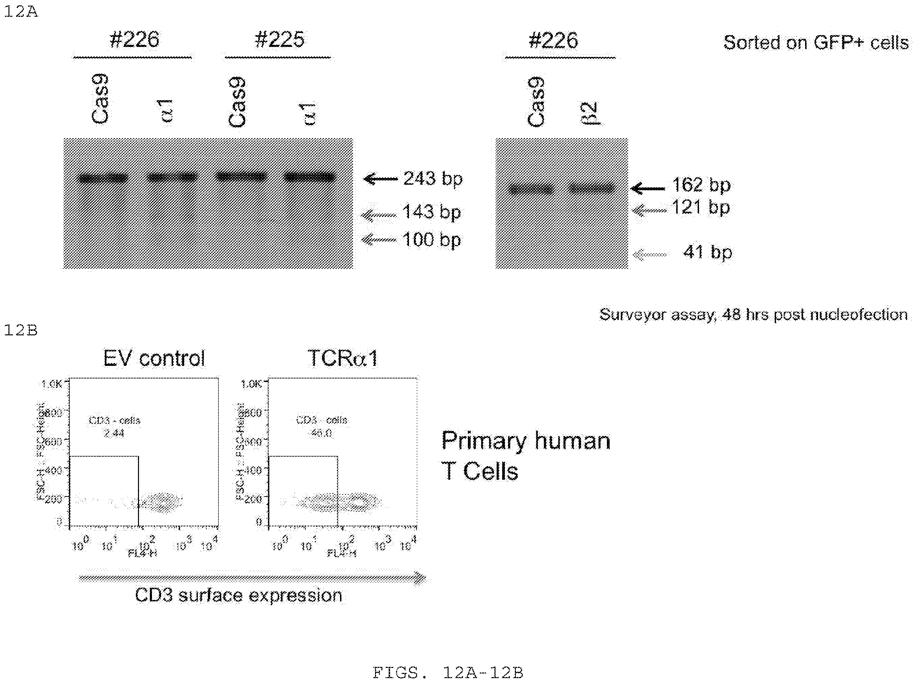

Prevention of autoreactivity is of highest priority to improve on existing T cell-based therapies. The inventors aimed to develop gRNAs with high on-target and no/low off target activity that allow for the efficient and safe deletion of the endogenous T cell receptor (TCR) in primary human T cells. The inventors designed multiple gRNAs against the TCR alpha and beta chain loci *(TRA* and *TRB),* located on chromosome 14 and 7, respectively (FIG. 10). Each gRNA was first tested for the ability to direct site-specific mutations in HEK293T cells, using the SURVEYOR^{™} assay (data not shown). Candidate gRNAs with the highest on-target efficiency were subsequently evaluated for functional TCR ablation in Jurkat T cells by flow cytometry (FACS) and SURVEYOR^{™} assay (FIG. 11A and FIG. 11B). Guides were either delivered by lentiviral transduction or transiently by nucleofection. We observed loss of TCR expression by FACS analysis in primary human T cells (FIG. 12A) and confirmed CRISPR cutting in T cells obtained from two independent donors using the SURVEYOR^{™} assay (FIG. 12B).

### Example 2: Allogeneic T cells for CAR-T therapies.

T cell-based therapies are currently limited to infusion of autologous cells obtained from the same patient. Extending the T cell origin to an allogeneic source by creating a universally applicable cell product would not only greatly reduce the costs but also open the door to a much broader range of patients for this novel and promising class of therapies.

Complete loss of MHC class I surface expression can be accomplished using CRISPR gRNAs targeting the gene encoding the accessory chain beta2microglobulin *(B2M).* The inventors have recently identified g RNAs targeting *B2M* with unrivaled high on-target efficiency that may already be suitable for gene therapy (Mandal et al., "Efficient Ablation of Genes in Human Hematopoietic Stem and Effector Cells using CRISPR/Cas9" Cell Stem Cell, 15:5, 643 - 652 (2014); Meissner et al., "Genome editing for human gene therapy," Methods in enzymology, 546, 273-295 (2014); U.S. Appl. No.: 62/076,424 and PCT application PCT/US2015/059621). As the CRISPR/Cas9 system allows multiplexing, B2M gRNA can be successfully used together with gRNAs for the TCRa and TCRb chains in primary T cells and subsequently in an adoptive transfer model in humanized mice, for example, to enable the use of allogeneic T cells for CAR-T therapies.

In addition, the inventors recently generated and characterized B2M knock out JEG3 cells using TALENs. These results demonstrated that genomic deletion of B2M using the TALENs genomic editing system results in complete abrogation of surface expression of B2M and all MHC class I molecules (FIGS. 26A-26G).

### Example 3: Prevention of T cell inhibition by targeting the checkpoint regulators of T cell activation, PD-1 and CTLA4.

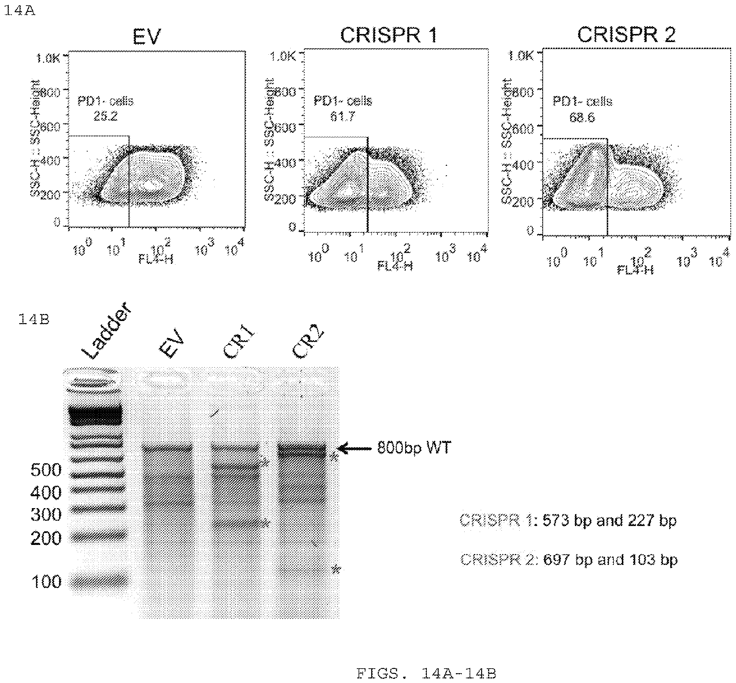

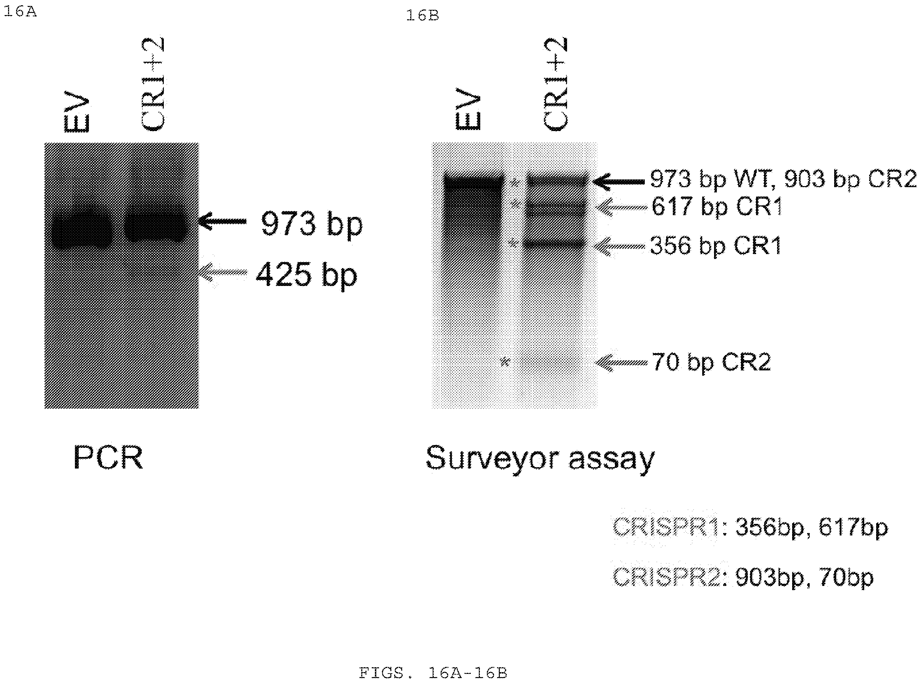

To overcome T cell inhibition, for example by cancerous cells or the tumor environment, the inventors developed gRNAs targeting critical checkpoint regulators of T cell activity. A similar approach as described for the TCR alpha and beta chains was taken: multiple gRNAs directed against the genes encoding PD-1 (*PDCD1*) and CTLA4 (*CTLA4*), both located on human chromosome 2, were first tested for their on-target cutting efficiency in HEK293T cells (FIG. 13 and FIG. 15, respectively). Cutting was confirmed at both loci using a PCR-based strategy followed by sequencing (FIG. 13C and FIG. 15C), as well as by SURVEYOR^{™} assay (FIG. 14B and FIG. 16B). A reduction of PD-1 expression was confirmed by FACS analysis in activated Jurkat T cells (FIG. 14A).

## Claims

1. A modified primary human T cell comprising a modified genome comprising:
(a) a first genomic modification in which the cytotoxic T-lymphocyte-associated protein 4 (CTLA4) gene on chromosome 2 has been edited to reduce or eliminate CTLA4 receptor surface expression and/or activity in the cell;
(b) a second genomic modification in which the programmed cell death 1 (PD1) gene on chromosome 2 has been edited to reduce or eliminate PD1 receptor surface expression and/or activity in the cell;
(c)(i) a third genomic modification in which the gene encoding the T cell receptor (TCR) alpha chain locus on chromosome 14 has been edited to reduce or eliminate TCR surface expression and/or activity in the cell, and/or
(c)(ii) a fourth genomic modification in which the gene encoding the TCR beta chain locus on chromosome 7 has been edited to reduce or eliminate TCR surface expression and/or activity in the cell; and
(d) a fifth genomic modification in which the β2-microglobulin (B2M) gene on chromosome 15 has been edited to reduce or eliminate MHC Class I molecule surface expression and/or activity in the cell;
each cell optionally comprising:
(e)(i) at least one chimeric antigen receptor that specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof, or an exogenous nucleic acid encoding the at least one chimeric antigen receptor, and/or
(e)(ii) at least one exogenous protein that modulates a biological effect of interest in an adjacent cell, tissue, or organ, or an exogenous nucleic acid encoding the protein.

2. The modified primary human T cell according to claim 1, wherein
(a) the first genomic modification in which the cytotoxic T-lymphocyte-associated protein 4 (CTLA4) gene on chromosome 2 has been edited to delete a first contiguous stretch of genomic DNA, thereby reducing or eliminating CTLA4 receptor surface expression and/or activity in the cell;
(b) the second genomic modification in which the programmed cell death 1 (PD1) gene on chromosome 2 has been edited to delete a second contiguous stretch of genomic DNA, thereby reducing or eliminating PD1 receptor surface expression and/or activity in the cell;
(c)(i) the third genomic modification in which the gene encoding the T cell receptor (TCR) alpha chain locus on chromosome 14 has been edited to delete a third contiguous stretch of genomic DNA, and/or
(c)(ii) the fourth genomic modification in which the gene encoding the TCR beta chain locus on chromosome 7 has been edited to delete a fourth contiguous stretch of genomic DNA, thereby reducing or eliminating TCR surface expression and/or activity in the cell; and
(d) the fifth genomic modification in which the β2-microglobulin (B2M) gene on chromosome 15 has been edited to delete a fifth contiguous stretch of genomic DNA, thereby reducing or eliminating MHC Class I molecule surface expression and/or activity in the cell.

3. A modified primary human T cell comprising a modified genome comprising:
(a) a first genomic modification in which the cytotoxic T-lymphocyte-associated protein 4 (CTLA4) gene on chromosome 2 has been edited to delete a first contiguous stretch of genomic DNA comprising an intron flanked by at least a portion of an adjacent upstream exon and at least a portion of an adjacent downstream exon, and the 3' end of the genomic DNA upstream with respect to the 5' end of the deleted first contiguous stretch of genomic DNA is covalently joined to the 5' end of the genomic DNA downstream with respect to the 3' end of the deleted first contiguous stretch of genomic DNA to result in a modified CTLA4 gene on chromosome 2 that lacks the first contiguous stretch of genomic DNA, thereby reducing or eliminating CTLA4 receptor surface expression and/or activity in the cell; and
(b) a second genomic modification in which the programmed cell death 1 (PD1) gene on chromosome 2 has been edited to delete a second contiguous stretch of genomic DNA comprising an intron flanked by at least a portion of an adjacent upstream exon and at least a portion of an adjacent downstream exon, and the 3' end of the genomic DNA upstream with respect to the deleted second contiguous stretch of genomic DNA is covalently joined to the 5' end of the genomic DNA downstream with respect to the 3' end of the deleted second contiguous stretch of genomic DNA to result in a modified PD1 gene on chromosome 2 that lacks the second contiguous stretch of genomic DNA, thereby reducing or eliminating PD1 receptor surface expression and/or activity in the cell.

4. The cell of claim 3, further comprising:
(c)(i) a third genomic modification in which the gene encoding the T cell receptor (TCR) alpha chain locus on chromosome 14 has been edited to delete a third contiguous stretch of genomic DNA comprising at least a portion of a coding exon, and/or
(c)(ii) a fourth genomic modification in which the gene encoding the TCR beta chain locus on chromosome 7 has been edited to delete a fourth contiguous stretch of genomic DNA comprising at least a portion of a coding exon, thereby reducing or eliminating TCR surface expression and/or activity in the cell; optionally further comprising:
(d) a fifth genomic modification in which the β2-microglobulin (B2M) gene on chromosome 15 has been edited to delete a fifth contiguous stretch of genomic DNA, thereby reducing or eliminating MHC Class I molecule surface expression and/or activity in the cell.

5. The cell of any one of claims 3 to 4, further comprising a chimeric antigen receptor or an exogenous nucleic acid encoding the chimeric antigen receptor; optionally wherein the chimeric antigen receptor specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof.

6. The cell of any one of claims 3 to 5, further comprising at least one exogenous protein that modulates a biological effect of interest in an adjacent cell, tissue, or organ, or an exogenous nucleic acid encoding the protein.

7. The cell of any one of claims 1 to 6, wherein the T cell is selected from the group consisting of cytotoxic T-cells, helper T-cells, memory T-cells, regulatory T-cells, tissue infiltrating lymphocytes, and combinations thereof.

8. The cell of any one of claims 1 to 7, wherein the cell is obtained from a subject suffering from, being treated for, diagnosed with, at risk of developing, or suspected of having, a disorder selected from the group consisting of an autoimmune disorder, cancer, a chronic infectious disease, and graft versus host disease (GVHD).

9. A method for producing a modified primary human T cell, the method comprising:
(a) editing the cytotoxic T-lymphocyte-associated protein 4 (CTLA4) gene on chromosome 2 in a primary human T cell to delete a first contiguous stretch of genomic DNA, thereby reducing or eliminating CTLA4 receptor surface expression and/or activity in the cell;
(b) editing the programmed cell death 1 (PD1) gene on chromosome 2 in the cell to delete a second contiguous stretch of genomic DNA, thereby reducing or eliminating PD1 receptor surface expression and/or activity in the cell;
(c)(i) editing the gene encoding the T cell receptor (TCR) alpha chain locus on chromosome 14 in the cell to delete a third contiguous stretch of genomic DNA, and/or
(c)(ii) editing the gene encoding the TCR beta chain locus on chromosome 7 in the cell to delete a fourth contiguous stretch of genomic DNA, thereby reducing or eliminating TCR surface expression and/or activity in the cell; and
(d) editing the β2-microglobulin (B2M) gene on chromosome 15 in the cell to delete a fifth contiguous stretch of genomic DNA, thereby reducing or eliminating MHC Class I molecule surface expression and/or activity in the cell; and
optionally comprising
(e) (i) causing the cell to express at least one chimeric antigen receptor that specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof, and/or
(e)(ii) causing the cell to express at least one protein that modulates a biological effect of interest in an adjacent cell, tissue, or organ,
wherein the editing in (a)-(d) comprises contacting the cell with a Cas protein or a nucleic acid encoding the Cas protein, and
at least one first pair of guide RNA sequences to delete the first contiguous stretch of genomic DNA from the gene in (a),
at least one second pair of guide RNA sequences to delete the second contiguous stretch of genomic DNA from the gene in (b),
at least one third pair of guide RNA sequences to delete the third contiguous stretch of genomic DNA from the gene in (c)(i), and/or
at least one fourth pair of guide RNA sequences to delete the fourth contiguous stretch of genomic DNA from the gene in (c)(ii), and
at least one fifth pair of guide RNA sequences to delete the fifth contiguous stretch of genomic DNA from the gene in (d).

10. A method for producing a modified primary human T cell, the method comprising:
(a) editing the cytotoxic T-lymphocyte-associated protein 4 (CTLA4) gene on chromosome 2 in a primary human T cell to delete a first contiguous stretch of genomic DNA comprising an intron flanked by at least a portion of an adjacent upstream exon and at least a portion of an adjacent downstream exon, and the 3' end of the genomic DNA upstream with respect to the 5' end of the deleted first contiguous stretch of genomic DNA is covalently joined to the 5' end of the genomic DNA downstream with respect to the 3' end of the deleted first contiguous stretch of genomic DNA to result in a modified CTLA4 gene on chromosome 2 that lacks the first contiguous stretch of genomic DNA, thereby reducing or eliminating CTLA4 receptor surface expression and/or activity in the cell; and
(b) editing the programmed cell death 1 (PD1) gene on chromosome 2 in a primary human T cell to delete a second contiguous stretch of genomic DNA comprising an intron flanked by at least a portion of an adjacent upstream exon and at least a portion of an adjacent downstream exon, and the 3' end of the genomic DNA upstream with respect to the deleted second contiguous stretch of genomic DNA is covalently joined to the 5' end of the genomic DNA downstream with respect to the 3' end of the deleted second contiguous stretch of genomic DNA to result in a modified PD1 gene on chromosome 2 that lacks the second contiguous stretch of genomic DNA, thereby reducing or eliminating PD1 receptor surface expression and/or activity in the cell.

11. The method of claim 10, further comprising:
(c)(i) editing the gene encoding the T cell receptor (TCR) alpha chain locus on chromosome 14 in the cell to delete a third contiguous stretch of genomic DNA comprising at least a portion of a coding exon, and/or
(c)(ii) editing the gene encoding the TCR beta chain locus on chromosome 7 in the cell to delete a fourth contiguous stretch of genomic DNA comprising at least a portion of a coding exon, thereby reducing or eliminating TCR surface expression and/or activity in the cell; optionally further comprising:
(d) editing the β2-microglobulin (B2M) gene on chromosome 15 in the cell to delete a fifth contiguous stretch of genomic DNA, thereby reducing or eliminating MHC Class I molecule surface expression and/or activity in the cell.

12. The method of any one of claims 10 to 11, further comprising causing the cell to express at least one chimeric antigen receptor that specifically binds to an antigen or epitope of interest expressed on the surface of at least one of a damaged cell, a dysplastic cell, an infected cell, an immunogenic cell, an inflamed cell, a malignant cell, a metaplastic cell, a mutant cell, and combinations thereof.

13. The method of any one of claims 10 to 12, further comprising causing the cell to express at least one protein that modulates a biological effect of interest in an adjacent cell, tissue, or organ when the cell is in proximity to the adjacent cell, tissue, or organ.

14. The method of any one of claims 9 to 13, wherein the T cell is selected from the group consisting of cytotoxic T-cells, helper T-cells, memory T-cells, regulatory T-cells, tissue infiltrating lymphocytes, and combinations thereof.

15. The method of any one of claims 9 to 14, wherein the cell is obtained from a subject suffering from, being treated for, diagnosed with, at risk of developing, or suspected of having, a disorder selected from the group consisting of an autoimmune disorder, cancer, a chronic infectious disease, and graft versus host disease (GVHD).

16. A composition comprising the cell according to any one of claims 1 to 8, or produced according to the method of any one of claims 9 to 15.

## Patentansprüche

1. Modifizierte primäre menschliche T-Zelle, welche ein modifiziertes Genom umfasst, das umfasst:
(a) eine erste genomische Modifikation, bei der das Gen für das zytotoxische T-Lymphozyten-assoziierte Protein 4 (CTLA4) auf Chromosom 2 editiert wurde, um die Oberflächenexpression und/oder Aktivität des CTLA4-Rezeptors in der Zelle zu reduzieren oder zu eliminieren;
(b) eine zweite genomische Modifikation, bei der das Gen für den programmierten Zelltod 1 (PD1) auf Chromosom 2 editiert wurde, um die PD1-Rezeptor-Oberflächenexpression und/oder -aktivität in der Zelle zu reduzieren oder zu eliminieren;
(c)(i) eine dritte genomische Modifikation, bei der das Gen, das den T-Zell-Rezeptor (TCR)-Alpha-Ketten-Locus auf Chromosom 14 kodiert, editiert wurde, um die TCR-Oberflächenexpression und/oder -aktivität in der Zelle zu reduzieren oder zu eliminieren, und/oder
(c)(ii) eine vierte genomische Modifikation, bei der das Gen, das den TCR-Beta-Ketten-Locus auf Chromosom 7 kodiert, editiert wurde, um die TCR-Oberflächenexpression und/oder -aktivität in der Zelle zu reduzieren oder zu eliminieren; und
(d) eine fünfte genomische Modifikation, bei der das β2-Mikroglobulin (B2M)-Gen auf Chromosom 15 editiert wurde, um die Oberflächenexpression und/oder -aktivität des MHC-Klasse-I-Moleküls in der Zelle zu reduzieren oder zu eliminieren;
wahlweise worin jede Zelle umfasst:
(e)(i) mindestens einen chimären Antigenrezeptor, der spezifisch an ein Antigen oder Epitop von Interesse bindet, das auf der Oberfläche von mindestens einem von einer geschädigten Zelle, einer dysplastischen Zelle, einer infizierten Zelle, einer immunogenen Zelle, einer entzündeten Zelle, einer malignen Zelle, einer metaplastischen Zelle, einer mutierten Zelle und Kombinationen davon exprimiert wird, oder eine exogene Nukleinsäure, die den mindestens einen chimären Antigenrezeptor kodiert, und/oder
(e)(ii) mindestens ein exogenes Protein, das eine biologische Wirkung von Interesse in einer benachbarten Zelle, einem benachbarten Gewebe oder Organ moduliert, oder eine exogene Nukleinsäure, die das Protein kodiert.

2. Modifizierte primäre menschliche T-Zelle nach Anspruch 1, worin
(a) die erste genomische Modifikation, bei der das Gen für das zytotoxische T-Lymphozyten-assoziierte Protein 4 (CTLA4) auf Chromosom 2 editiert wurde, um einen ersten zusammenhängenden Abschnitt genomischer DNA zu löschen, wodurch die CTLA4-Rezeptoroberflächenexpression und/oder -aktivität in der Zelle reduziert oder eliminiert wird;
(b) die zweite genomische Modifikation, bei der das Gen für den programmierten Zelltod 1 (PD1) auf Chromosom 2 editiert wurde, um einen zweiten zusammenhängenden Abschnitt genomischer DNA zu löschen, wodurch die PD1-Rezeptor-oberflächenexpression und/oder - aktivität in der Zelle reduziert oder eliminiert wird;
(c)(i) die dritte genomische Modifikation, bei der das Gen, das den T-Zell-Rezeptor (TCR)-Alpha-Ketten-Locus auf Chromosom 14 kodiert, editiert wurde, um einen dritten zusammenhängenden Abschnitt genomischer DNA zu löschen, und/oder
(c)(ii) die vierte genomische Modifikation, bei der das Gen, das den TCR-Beta-Ketten-Locus auf Chromosom 7 kodiert, editiert wurde, um einen vierten zusammenhängenden Abschnitt genomischer DNA zu löschen, wodurch die TCR-Oberflächenexpression und/oder -aktivität in der Zelle reduziert oder eliminiert wird; und
(d) die fünfte genomische Modifikation, bei der das β2-Mikroglobulin (B2M)-Gen auf Chromosom 15 editiert wurde, um einen fünften zusammenhängenden Abschnitt genomischer DNA zu löschen, wodurch die Oberflächenexpression und/oder -aktivität in der Zelle des MHC-Klasse-I-Moleküls reduziert oder eliminiert wird.

3. Modifizierte primäre menschliche T-Zelle, welche ein modifiziertes Genom umfasst, das umfasst:
(a) eine erste genomische Modifikation, bei der das Gen für das zytotoxische T-Lymphozyten-assoziierte Protein 4 (CTLA4) auf Chromosom 2 editiert wurde, um einen ersten zusammenhängenden Abschnitt der genomischen DNA zu löschen, der ein Intron umfasst, das von mindestens einem Teil eines benachbarten stromaufwärts gelegenen Exons und mindestens einem Teil eines benachbarten stromabwärts gelegenen Exons flankiert wird, und das 3'-Ende der genomischen DNA stromaufwärts in Bezug auf das 5'-Ende des gelöschten ersten zusammenhängenden Abschnitts der genomischen DNA kovalent mit dem 5'-Ende der genomischen DNA stromabwärts in Bezug auf das 3'-Ende des gelöschten ersten zusammenhängenden Abschnitts der genomischen DNA verbunden ist, um ein modifiziertes CTLA4-Gen auf Chromosom 2 zu ergeben, dem der erste zusammenhängende Abschnitt genomischer DNA fehlt, wodurch die CTLA4-Rezeptoroberflächenexpression und/oder - aktivität in der Zelle reduziert oder eliminiert wird; und
(b) eine zweite genomische Modifikation, bei der das Gen für den programmierten Zelltod 1 (PD1) auf Chromosom 2 editiert wurde, um einen zweiten zusammenhängenden Abschnitt genomischer DNA zu löschen, der ein Intron umfasst, das von mindestens einem Teil eines benachbarten stromaufwärts gelegenen Exons und mindestens einem Teil eines benachbarten stromabwärts gelegenen Exons flankiert ist, und das 3'-Ende der genomischen DNA stromaufwärts in Bezug auf den gelöschten zweiten zusammenhängenden Abschnitt genomischer DNA kovalent mit dem 5'-Ende der genomischen DNA stromabwärts in Bezug auf das 3'-Ende des gelöschten zweiten zusammenhängenden Abschnitts der genomischen DNA verbunden ist, um ein modifiziertes PD1-Gen auf Chromosom 2 zu ergeben, dem der zweite zusammenhängende Abschnitt genomischer DNA fehlt, wodurch die PD1-Rezeptoroberflächenexpression und/oder -aktivität in der Zelle reduziert oder eliminiert wird.

4. Zelle nach Anspruch 3, welche ferner umfasst:
(c)(i) eine dritte genomische Modifikation, bei der das Gen, das den T-Zell-Rezeptor (TCR)-Alpha-Ketten-Locus auf Chromosom 14 kodiert, so editiert wurde, dass ein dritter zusammenhängender Abschnitt genomischer DNA, der mindestens einen Teil eines kodierenden Exons umfasst, gelöscht wird, und/oder
(c)(ii) eine vierte genomische Modifikation, bei der das Gen, das den TCR-Beta-Ketten-Locus auf Chromosom 7 kodiert, so editiert wurde, dass ein vierter zusammenhängender Abschnitt genomischer DNA, der mindestens einen Teil eines kodierenden Exons umfasst, gelöscht wird, wodurch die TCR-Oberflächenexpression und/oder -aktivität in der Zelle reduziert oder eliminiert wird;
wahlweise worin die Modifikation zudem umfasst:
(d) eine fünfte genomische Modifikation, bei der das β2-Microglobulin (B2M)-Gen auf Chromosom 15 editiert wurde, um einen fünften zusammenhängenden Abschnitt genomischer DNA zu löschen, wodurch die Oberflächenexpression und/oder -aktivität von MHC-Klasse-I-Molekülen in der Zelle reduziert oder eliminiert wird.

5. Zelle nach einem der Ansprüche 3 bis 4, welche ferner einen chimären Antigenrezeptor oder eine exogene Nukleinsäure umfasst, die den chimären Antigenrezeptor kodiert, wahlweise worin der chimäre Antigenrezeptor spezifisch an ein Antigen oder Epitop von Interesse bindet, das auf der Oberfläche von mindestens einer geschädigten Zelle, einer dysplastischen Zelle, einer infizierten Zelle, einer immunogenen Zelle, einer entzündeten Zelle, einer malignen Zelle, einer metaplastischen Zelle, einer mutierten Zelle und Kombinationen davon exprimiert ist.

6. Zelle nach einem der Ansprüche 3 bis 5, welche ferner mindestens ein exogenes Protein umfasst, das eine biologische Wirkung von Interesse in einer benachbarten Zelle, einem benachbarten Gewebe oder Organ moduliert, oder eine exogene Nukleinsäure, die das Protein kodiert.

7. Zelle nach einem der Ansprüche 1 bis 6, worin die T-Zelle ausgewählt ist aus der Gruppe bestehend aus zytotoxischen T-Zellen, Helfer-T-Zellen, Gedächtnis-T-Zellen, regulatorischen T-Zellen, gewebeinfiltrierenden Lymphozyten und Kombinationen davon.

8. Zelle nach einem der Ansprüche 1 bis 7, worin die Zelle von einer Person erhalten wurde, die an einer Störung leidet, wegen welcher die Person behandelt wird, bei der ein Risiko besteht, eine solche zu entwickeln, oder bei der der Verdacht besteht, dass die Person eine solche hat, ausgewählt aus der Gruppe bestehend aus einer Autoimmunerkrankung, Krebs, einer chronischen Infektionskrankheit und der Transplantat-gegen-Wirt-Krankheit (GVHD).

9. Verfahren zur Herstellung einer modifizierten primären menschlichen T-Zelle, wobei das Verfahren umfasst:
(a) Editieren des Gens für das zytotoxische T-Lymphozyten-assoziierte Protein 4 (CTLA4) auf Chromosom 2 in einer primären menschlichen T-Zelle, um einen ersten zusammenhängenden Abschnitt genomischer DNA zu löschen, wodurch die CTLA4-Rezeptor-Oberflächenexpression und/oder -aktivität in der Zelle reduziert oder eliminiert wird;
(b) Editieren des Gens für den programmierten Zelltod 1 (PD1) auf Chromosom 2 in der Zelle, um einen zweiten zusammenhängenden Abschnitt genomischer DNA zu löschen, wodurch die PD1-Rezeptor-Oberflächenexpression und/oder -aktivität in der Zelle reduziert oder eliminiert wird;
(c)(i) Editieren des Gens, das den T-Zell-Rezeptor (TCR)-Alpha-Ketten-Locus auf Chromosom 14 in der Zelle kodiert, um einen dritten zusammenhängenden Abschnitt genomischer DNA zu löschen, und/oder
(c)(ii) Editieren des Gens, das den TCR-Beta-Ketten-Locus auf Chromosom 7 in der Zelle kodiert, um einen vierten zusammenhängenden Abschnitt genomischer DNA zu löschen, wodurch die TCR-Oberflächenexpression und/oder -aktivität in der Zelle reduziert oder eliminiert wird; und
(d) Editieren des B2-Microg|obu|in (B2M)-Gens auf Chromosom 15 in der Zelle, um einen fünften zusammenhängenden Abschnitt genomischer DNA zu löschen, wodurch die Oberflächenexpression und/oder -aktivität des MHC-Klasse-I-Moleküls in der Zelle verringert oder beseitigt wird; und
wahlweise wobei das Verfahren umfasst:
(e) (i) Bewirken, dass die Zelle mindestens einen chimären Antigenrezeptor exprimiert, der spezifisch an ein Antigen oder Epitop von Interesse bindet, das auf der Oberfläche von mindestens einem von einer geschädigten Zelle, einer dysplastischen Zelle, einer infizierten Zelle, einer immunogenen Zelle, einer entzündeten Zelle, einer bösartigen Zelle, einer metaplastischen Zelle, einer mutierten Zelle und Kombinationen davon exprimiert ist, und/oder
(e)(ii) Bewirken, dass die Zelle mindestens ein Protein exprimiert, das eine biologische Wirkung von Interesse in einer benachbarten Zelle, einem benachbarten Gewebe oder Organ moduliert,
wobei das Editieren in (a)-(d) das Inkontaktbringen der Zelle mit einem Cas-Protein oder einer Nukleinsäure umfasst, die das Cas-Protein kodiert, und
mindestens ein erstes Paar von *guide*-RNA-Sequenzen, um den ersten zusammenhängenden Abschnitt genomischer DNA aus dem Gen in (a) zu löschen,
mindestens ein zweites Paar von *guide*-RNA-Sequenzen, um den zweiten zusammenhängenden Abschnitts genomischer DNA aus dem Gen in (b) zu löschen,
mindestens ein drittes Paar von *guide*-RNA-Sequenzen, um den dritten zusammenhängenden Abschnitt genomischer DNA aus dem Gen in (C)(i) zu löschen, und/oder
mindestens ein viertes Paar von *guide*-RNA-Sequenzen, um den vierten zusammenhängenden Abschnitt genomischer DNA aus dem Gen in (c)(ii) zu löschen, und
mindestens ein fünftes Paar von *guide*-RNA-Sequenzen, um den fünften zusammenhängenden Abschnitt genomischer DNA aus dem Gen in (d) zu löschen.

10. Verfahren zur Herstellung einer modifizierten primären menschlichen T-Zelle, wobei das Verfahren umfasst:
(a) Editieren des zytotoxischen T-Lymphozyten-assoziierten Proteins 4 (CTLA4) Gens auf Chromosom 2 in einer primären menschlichen T-Zelle, um einen ersten zusammenhängenden Abschnitt genomischer DNA zu löschen, der ein Intron umfasst, das von mindestens einem Teil eines benachbarten stromaufwärts gelegenen Exons und mindestens einem Teil eines benachbarten stromabwärts gelegenen Exons flankiert wird, und das 3'-Ende der genomischen DNA stromaufwärts in Bezug auf das 5'-Ende des gelöschten ersten zusammenhängenden Abschnitts der genomischen DNA kovalent mit dem 5'-Ende der genomischen DNA stromabwärts in Bezug auf das 3'-Ende des gelöschten ersten zusammenhängenden Abschnitts der genomischen DNA verbunden wird, um zu einem modifizierten CTLA4-Gen auf Chromosom 2 zu führen, dem der erste zusammenhängende Abschnitt genomischer DNA fehlt, wodurch die CTLA4-Rezeptoroberflächenexpression und/oder -aktivität in der Zelle reduziert oder eliminiert wird; und
(b) Editieren des Gens für den programmierten Zelltod 1 (PD1) auf Chromosom 2 in einer primären menschlichen T-Zelle, um einen zweiten zusammenhängenden Abschnitt genomischer DNA zu löschen, der ein Intron umfasst, das von mindestens einem Teil eines benachbarten stromaufwärts gelegenen Exons und mindestens einem Teil eines benachbarten stromabwärts gelegenen Exons flankiert wird, und das 3'-Ende der genomischen DNA stromaufwärts in Bezug auf den gelöschten zweiten zusammenhängenden Abschnitt genomischer DNA kovalent mit dem 5'-Ende der genomischen DNA stromabwärts in Bezug auf das 3'-Ende des gelöschten zweiten zusammenhängenden Abschnitts genomischer DNA verbunden ist, um ein modifiziertes PD1-Gen auf Chromosom 2 zu ergeben, dem der zweite zusammenhängende Abschnitt genomischer DNA fehlt, wodurch die PD1-Rezeptor-Oberflächenexpression und/oder -aktivität in der Zelle reduziert oder eliminiert wird.

11. Verfahren nach Anspruch 10, welches ferner umfasst:
(c)(i) Editieren des Gens, das den T-Zell-Rezeptor (TCR)-Alpha-Ketten-Locus auf Chromosom 14 in der Zelle kodiert, um einen dritten zusammenhängenden Abschnitt genomischer DNA zu löschen, der mindestens einen Teil eines kodierenden Exons umfasst, und/oder
(c)(ii) Editieren des Gens, das den TCR-Beta-Ketten-Locus auf Chromosom 7 in der Zelle kodiert, um einen vierten zusammenhängenden Abschnitt genomischer DNA zu löschen, der mindestens einen Teil eines kodierenden Exons umfasst, wodurch die TCR-Oberflächenexpression und/oder -aktivität in der Zelle reduziert oder eliminiert wird; wahlweise wobei das Verfahren weiter umfasst:
(d) Editieren des β2-Mikroglobulin (B2M)-Gens auf Chromosom 15 in der Zelle, um einen fünften zusammenhängenden Abschnitt genomischer DNA zu löschen, wodurch die Oberflächenexpression und/oder -aktivität des MHC-Klasse-I-Moleküls in der Zelle reduziert oder eliminiert wird.

12. Verfahren nach einem der Ansprüche 10 bis 11, welches ferner das Veranlassen der Zelle umfasst, mindestens einen chimären Antigenrezeptor zu exprimieren, der spezifisch an ein Antigen oder Epitop von Interesse bindet, das auf der Oberfläche von mindestens einem von einer geschädigten Zelle, einer dysplastischen Zelle, einer infizierten Zelle, einer immunogenen Zelle, einer entzündeten Zelle, einer malignen Zelle, einer metaplastischen Zelle, einer mutierten Zelle und Kombinationen davon exprimiert wird.

13. Verfahren nach einem der Ansprüche 10 bis 12, welches ferner das Veranlassen der Zelle umfasst, mindestens ein Protein zu exprimieren, das eine biologische Wirkung von Interesse in einer benachbarten Zelle, einem benachbarten Gewebe oder Organ moduliert, wenn sich die Zelle in der Nähe der benachbarten Zelle, des benachbarten Gewebes oder Organs befindet.

14. Verfahren nach einem der Ansprüche 9 bis 13, wobei die T-Zelle ausgewählt ist aus der Gruppe bestehend aus zytotoxischen T-Zellen, Helfer-T-Zellen, Gedächtnis-T-Zellen, regulatorischen T-Zellen, gewebeinfiltrierenden Lymphozyten und Kombinationen davon.

15. Verfahren nach einem der Ansprüche 9 bis 14, wobei die Zelle von einer Person erhalten wurde, die an einer Störung leidet, wegen welcher die Person behandelt wird, bei der eine solche Störung diagnostiziert wurde, bei der das Risiko besteht, dass die Person eine solche Störung entwickelt, oder bei der der Verdacht besteht, dass die Person eine solche Störung hat, ausgewählt aus der Gruppe bestehend aus einer Autoimmunerkrankung, Krebs, einer chronischen Infektionskrankheit und der Transplantat-gegen-Wirt-Krankheit (GVHD).

16. Zusammensetzung, welche die Zelle nach einem der Ansprüche 1 bis 8 umfasst oder welche nach dem Verfahren eines der Ansprüche 9 bis 15 hergestellt wurde.

## Revendications

1. Lymphocyte T humain primaire modifié comprenant un génome modifié comprenant :
(a) une première modification génomique dans laquelle le gène de la protéine 4 associée aux lymphocytes T cytotoxiques (CTLA4) sur le chromosome 2 a été édité pour réduire ou éliminer l'expression de surface et/ou l'activité du récepteur CTLA4 dans la cellule ;
(b) une deuxième modification génomique dans laquelle le gène de mort cellulaire programmée 1 (PD1) sur le chromosome 2 a été édité pour réduire ou éliminer l'expression de surface et/ou l'activité du récepteur PD1 dans la cellule ;
(c) (i) une troisième modification génomique dans laquelle le gène codant pour le locus de la chaîne alpha du récepteur des lymphocytes T (TCR) sur le chromosome 14 a été édité pour réduire ou éliminer l'expression de surface et/ou l'activité du TCR dans la cellule, et/ou
(c) (ii) une quatrième modification génomique dans laquelle le gène codant pour le locus de la chaîne bêta du TCR sur le chromosome 7 a été édité pour réduire ou éliminer l'expression de surface et/ou l'activité du TCR dans la cellule ; et
(d) une cinquième modification génomique dans laquelle le gène de la β2-microglobuline (B2M) sur le chromosome 15 a été édité pour réduire ou éliminer l'expression de surface et/ou l'activité des molécules du CMH classe I dans la cellule ;
chaque cellule comprenant facultativement :
(e)(i) au moins un récepteur antigénique chimérique qui se lie spécifiquement à un antigène ou un épitope d'intérêt exprimé sur la surface d'au moins l'une d'une cellule endommagée, d'une cellule dysplasique, d'une cellule infectée, d'une cellule immunogène, d'une cellule inflammatoire, d'une cellule maligne, d'une cellule métaplasique, d'une cellule mutante, et de combinaisons de celles-ci, ou un acide nucléique exogène codant pour l'au moins un récepteur antigénique chimérique, et/ou
(e) (ii) au moins une protéine exogène qui module un effet biologique d'intérêt dans une cellule, un tissu ou un organe adjacents, ou un acide nucléique codant pour la protéine.

2. Lymphocyte T humain primaire modifié selon la revendication 1, dans lequel
(a) la première modification génomique dans laquelle le gène de la protéine 4 associée aux lymphocytes T cytotoxiques (CTLA4) sur le chromosome 2 a été édité pour déléter un premier étirement contigu d'ADN génomique, réduisant ou éliminant ainsi l'expression de surface et/ou l'activité du récepteur CTLA4 dans la cellule ;
(b) la deuxième modification génomique dans laquelle le gène de mort cellulaire programmée 1 (PD1) sur le chromosome 2 a été édité pour déléter un deuxième étirement contigu d'ADN génomique, réduisant ou éliminant ainsi l'expression de surface et/ou l'activité du récepteur PD1 dans la cellule ;
(c)(i) la troisième modification génomique dans laquelle le gène codant pour le locus de la chaîne alpha du récepteur des lymphocytes T (TCR) sur le chromosome 14 a été édité pour déléter un troisième étirement contigu d'ADN génomique, et/ou
(c)(ii) la quatrième modification génomique dans laquelle le gène codant pour le locus de la chaîne bêta du TCR sur le chromosome 7 a été édité pour déléter un quatrième étirement contigu d'ADN génomique, réduisant ou éliminant ainsi l'expression de surface et/ou l'activité du TCR dans la cellule ; et
(d) la cinquième modification génomique dans laquelle le gène de la β2-microglobuline (B2M) sur le chromosome 15 a été édité pour déléter un cinquième étirement contigu d'ADN génomique, réduisant ou éliminant ainsi l'expression de surface et/ou l'activité des molécules du CMH classe I dans la cellule.

3. Lymphocyte T humain primaire modifié comprenant un génome modifié comprenant :
(a) une première modification génomique dans laquelle le gène de la protéine 4 associée aux lymphocytes T cytotoxiques (CTLA4) sur le chromosome 2 a été édité pour déléter un premier étirement contigu d'ADN génomique comprenant un intron flanqué par au moins une partie d'un exon amont adjacent et au moins une partie d'un exon aval adjacent, et l'extrémité 3' de l'ADN génomique en amont par rapport à l'extrémité 5' du premier étirement contigu délété d'ADN génomique est jointe de manière covalente à l'extrémité 5' de l'ADN génomique en aval par rapport à l'extrémité 3' du premier étirement contigu délété d'ADN génomique pour résulter en un gène CTLA4 modifié sur le chromosome 2 qui ne présente pas le premier étirement contigu d'ADN génomique, réduisant ou éliminant ainsi l'expression de surface et/ou l'activité du récepteur CTLA4 dans la cellule ; et
(b) une deuxième modification génomique dans laquelle le gène de mort cellulaire programmée 1 (PD1) sur le chromosome 2 a été édité pour déléter un deuxième étirement contigu d'ADN génomique comprenant un intron flanqué par au moins une partie d'un exon amont adjacent et au moins une partie d'un exon aval adjacent, et l'extrémité 3' de l'ADN génomique en amont par rapport au deuxième étirement contigu délété d'ADN génomique est jointe de manière covalente à l'extrémité 5' de l'ADN génomique en aval par rapport à l'extrémité 3' du deuxième étirement contigu délété d'ADN génomique pour résulter en un gène PD1 modifié sur le chromosome 2 qui ne présente pas le deuxième étirement contigu d'ADN génomique, réduisant ou éliminant ainsi l'expression de surface et/ou l'activité du récepteur PD1 dans la cellule.

4. Cellule selon la revendication 3, comprenant en outre :
(c) (i) une troisième modification génomique dans laquelle le gène codant pour le locus de la chaîne alpha du récepteur des lymphocytes T (TCR) sur le chromosome 14 a été édité pour déléter un troisième étirement contigu d'ADN génomique comprenant au moins une partie d'un exon codant, et/ou
(c) (ii) une quatrième modification génomique dans laquelle le gène codant pour le locus de la chaîne bêta du TCR sur le chromosome 7 a été édité pour déléter un quatrième étirement contigu d'ADN génomique comprenant au moins une partie d'un exon codant, réduisant ou éliminant ainsi l'expression de surface et/ou l'activité du TCR dans la cellule ; comprenant en outre facultativement :
(d) une cinquième modification génomique dans laquelle le gène de la β2-microglobuline (B2M) sur le chromosome 15 a été édité pour déléter un cinquième étirement contigu d'ADN génomique, réduisant ou éliminant ainsi l'expression de surface et/ou l'activité des molécules du CMH classe I dans la cellule.

5. Cellule selon l'une quelconque des revendications 3 à 4, comprenant en outre un récepteur antigénique chimérique ou un acide nucléique exogène codant pour le récepteur antigénique chimérique ; facultativement dans laquelle le récepteur antigénique chimérique se lie spécifiquement à un antigène ou un épitope d'intérêt exprimé sur la surface d'au moins l'une d'une cellule endommagée, d'une cellule dysplasique, d'une cellule infectée, d'une cellule immunogène, d'une cellule inflammatoire, d'une cellule maligne, d'une cellule métaplasique, d'une cellule mutante, et de combinaisons de celles-ci.

6. Cellule selon l'une quelconque des revendications 3 à 5, comprenant en outre au moins une protéine exogène qui module un effet biologique d'intérêt dans une cellule, un tissu ou un organe adjacents, ou un acide nucléique codant pour la protéine.

7. Cellule selon l'une quelconque des revendications 1 à 6, dans laquelle le lymphocyte T est sélectionné dans le groupe constitué des lymphocytes T cytotoxiques, des lymphocytes T auxiliaires, des lymphocytes T mémoires, des lymphocytes T régulateurs, des lymphocytes d'infiltration tissulaire, et de combinaisons de ceux-ci.

8. Cellule selon l'une quelconque des revendications 1 à 7, dans laquelle la cellule est obtenue d'un sujet souffrant de, étant traité pour, diagnostiqué comme ayant, à risque de développer, ou suspecté d'avoir, un trouble sélectionné dans le groupe constitué d'un trouble auto-immun, d'un cancer, d'une maladie infectieuse chronique, et d'une maladie du greffon contre l'hôte (GVHD).

9. Procédé de production d'un lymphocyte T humain primaire modifié, le procédé comprenant :
(a) l'édition du gène de la protéine 4 associée aux lymphocytes T cytotoxiques (CTLA4) sur le chromosome 2 dans un lymphocyte T humain primaire pour déléter un premier étirement contigu d'ADN génomique, réduisant ou éliminant ainsi l'expression de surface et/ou l'activité du récepteur CTLA4 dans la cellule ;
(b) l'édition du gène de mort cellulaire programmée 1 (PD1) sur le chromosome 2 dans la cellule pour déléter un deuxième étirement contigu d'ADN génomique, réduisant ou éliminant ainsi l'expression de surface et/ou l'activité du récepteur PD1 dans la cellule ;
(c) (i) l'édition du gène codant pour le locus de la chaîne alpha du récepteur des lymphocytes T (TCR) sur le chromosome 14 dans la cellule pour déléter un troisième étirement contigu d'ADN génomique, et/ou
(c) (ii) l'édition du gène codant pour le locus de la chaîne bêta du TCR sur le chromosome 7 dans la cellule pour déléter un quatrième étirement contigu d'ADN génomique, réduisant ou éliminant ainsi l'expression de surface et/ou l'activité du TCR dans la cellule ; et
(d) l'édition du gène de la β2-microglobuline (B2M) sur le chromosome 15 dans la cellule pour déléter un cinquième étirement contigu d'ADN génomique, réduisant ou éliminant ainsi l'expression de surface et/ou l'activité des molécules du CMH classe I dans la cellule ; et
comprenant facultativement
(e) (i) le fait d'amener la cellule à exprimer au moins un récepteur antigénique chimérique qui se lie spécifiquement à un antigène ou un épitope d'intérêt exprimé sur la surface d'au moins l'une d'une cellule endommagée, d'une cellule dysplasique, d'une cellule infectée, d'une cellule immunogène, d'une cellule inflammatoire, d'une cellule maligne, d'une cellule métaplasique, d'une cellule mutante, et de combinaisons de celles-ci, et/ou
(e) (ii) le fait d'amener la cellule à exprimer au moins une protéine exogène qui module un effet biologique d'intérêt dans une cellule, un tissu ou un organe adjacents,
dans lequel l'édition en (a) à (d) comprend la mise en contact de la cellule avec une protéine Cas ou un acide nucléique codant pour la protéine Cas, et
au moins une première paire de séquences d'ARN guide pour déléter le premier étirement contigu d'ADN génomique du gène en (a),
au moins une deuxième paire de séquences d'ARN guide pour déléter le deuxième étirement contigu d'ADN génomique du gène en (b),
au moins une troisième paire de séquences d'ARN guide pour déléter le troisième étirement contigu d'ADN génomique du gène en (c)(i), et/ou
au moins une quatrième paire de séquences d'ARN guide pour déléter le quatrième étirement contigu d'ADN génomique du gène en (c)(ii), et
au moins une cinquième paire de séquences d'ARN guide pour déléter le cinquième étirement contigu d'ADN génomique du gène en (d).

10. Procédé de production d'un lymphocyte T humain primaire modifié, le procédé comprenant:
(a) l'édition du gène de la protéine 4 associée aux lymphocytes T cytotoxiques (CTLA4) sur le chromosome 2 dans un lymphocyte T humain primaire pour déléter un premier étirement contigu d'ADN génomique comprenant un intron flanqué par au moins une partie d'un exon amont adjacent et au moins une partie d'un exon aval adjacent, et l'extrémité 3' de l'ADN génomique en amont par rapport à l'extrémité 5' du premier étirement contigu délété d'ADN génomique est jointe de manière covalente à l'extrémité 5' de l'ADN génomique en aval par rapport à l'extrémité 3' du premier étirement contigu délété d'ADN génomique pour résulter en un gène CTLA4 modifié sur le chromosome 2 qui ne présente pas le premier étirement contigu d'ADN génomique, réduisant ou éliminant ainsi l'expression de surface et/ou l'activité du récepteur CTLA4 dans la cellule ; et
(b) l'édition du gène de mort cellulaire programmée 1 (PD1) sur le chromosome 2 dans un lymphocyte T humain primaire pour déléter un deuxième étirement contigu d'ADN génomique comprenant un intron flanqué par au moins une partie d'un exon amont adjacent et au moins une partie d'un exon aval adjacent, et l'extrémité 3' de l'ADN génomique en amont par rapport au deuxième étirement contigu délété d'ADN génomique est jointe de manière covalente à l'extrémité 5' de l'ADN génomique en aval par rapport à l'extrémité 3' du deuxième étirement contigu délété d'ADN génomique pour résulter en un gène PD1 modifié sur le chromosome 2 qui ne présente pas le deuxième étirement contigu d'ADN génomique, réduisant ou éliminant ainsi l'expression de surface et/ou l'activité du récepteur PD1 dans la cellule.

11. Procédé selon la revendication 10, comprenant en outre :
(c)(i) l'édition du gène codant pour le locus de la chaîne alpha du récepteur des lymphocytes T (TCR) sur le chromosome 14 dans la cellule pour déléter un troisième étirement contigu d'ADN génomique comprenant au moins une partie d'un exon codant, et/ou
(c) (ii) l'édition du gène codant pour le locus de la chaîne bêta du TCR sur le chromosome 7 dans la cellule pour déléter un quatrième étirement contigu d'ADN génomique comprenant au moins une partie d'un exon codant, réduisant ou éliminant ainsi l'expression de surface et/ou l'activité du TCR dans la cellule ; comprenant en outre facultativement :
(d) l'édition du gène de la β2-microglobuline (B2M) sur le chromosome 15 dans la cellule pour déléter un cinquième étirement contigu d'ADN génomique, réduisant ou éliminant ainsi l'expression de surface et/ou l'activité des molécules du CMH classe I dans la cellule.

12. Procédé selon l'une quelconque des revendications 10 à 11, comprenant en outre le fait d'amener la cellule à exprimer au moins un récepteur antigénique chimérique qui se lie spécifiquement à un antigène ou un épitope d'intérêt exprimé sur la surface d'au moins l'une d'une cellule endommagée, d'une cellule dysplasique, d'une cellule infectée, d'une cellule immunogène, d'une cellule inflammatoire, d'une cellule maligne, d'une cellule métaplasique, d'une cellule mutante, et de combinaisons de celles-ci.

13. Procédé selon l'une quelconque des revendications 10 à 12, comprenant en outre le fait d'amener la cellule à exprimer au moins une protéine qui module un effet biologique d'intérêt dans une cellule, un tissu ou un organe adjacents, lorsque la cellule est à proximité de la cellule, du tissu ou de l'organe adjacents.

14. Procédé selon l'une quelconque des revendications 9 à 13, dans lequel le lymphocyte T est sélectionné dans le groupe constitué des lymphocytes T cytotoxiques, des lymphocytes T auxiliaires, des lymphocytes T mémoires, des lymphocytes T régulateurs, des lymphocytes d'infiltration tissulaire, et de combinaisons de ceux-ci.

15. Procédé selon l'une quelconque des revendications 9 à 14, dans lequel la cellule est obtenue d'un sujet souffrant de, étant traité pour, diagnostiqué comme ayant, à risque de développer, ou suspecté d'avoir, un trouble sélectionné dans le groupe constitué d'un trouble auto-immun, d'un cancer, d'une maladie infectieuse chronique, et d'une maladie du greffon contre l'hôte (GVHD).

16. Composition comprenant la cellule selon l'une quelconque des revendications 1 à 8, ou produite conformément au procédé selon l'une quelconque des revendications 9 à 15.
